(19) Europäisches Patentamt
European Patent Office
Office européen des brevets

(11) Publication number: **0 235 463 A2**

(12) **EUROPEAN PATENT APPLICATION**

(21) Application number: **86310045.9**

(22) Date of filing: **22.12.86**

(51) Int. Cl.4: **C 07 D 211/14**, C 07 D 211/70, C 07 D 207/08, C 07 D 401/12, C 07 D 405/06, C 07 D 405/12, A 61 K 31/445, A 61 K 31/47

(30) Priority: **17.01.86 US 819701**
**20.12.85 US 811799**

(43) Date of publication of application: **09.09.87**
**Bulletin 87/37**

(84) Designated Contracting States: **AT BE CH DE ES FR GB GR IT LI LU NL SE**

(71) Applicant: **A.H. ROBINS COMPANY, INCORPORATED, 1407 Cummings Drive P.O. Box 26609, Richmond Virginia 23261-6609 (US)**

(72) Inventor: **Shanklin, James Robert, 8318 Brookfield Road, Richmond Virginia 23227 (US)**
Inventor: **Proakis, Anthony George, 1811 Carbon Hill Drive, Midlothian Virginia 23113 (US)**

(74) Representative: **Sheard, Andrew Gregory et al, Kilburn & Strode 30, John Street, London WC1N 2DD (GB)**

# (54) N-substituted-arylalkyl and arylalkylene aminoheterocyclics as cardiovascular antihistaminic and antisecretory agents.

(57) Cardiovascular disturbances and the effects of histamine and excessive gastric secretion can be countered by compounds expressed generally by the formula:

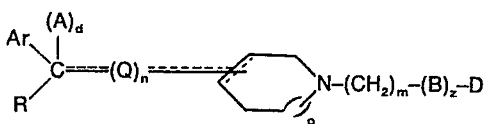

wherein;

p is zero, one or two;

m is one to six inclusive;

A is hydrogen, $-O-R^1$, $-C\equiv N$, $-(O)NR^1R^2$, $-C(O)R^1$, $-C(O)-OR^1$, $-CH_2OR^1$, $-CH_2NR^1R^2$, or $-OC(O)R^1$;

Q is $-CH-$, $-CH_2-$ or $-C(OH)(H)-$;

d and n are zero or one and the dotted lines represent double bonds which may form consistent with the valence of carbon;

B is O, S, $-S(O)-$, $-S(O)_2-$, $-N(R^1)-$, and $-N-C(O)OR^1$;

Ar, D and R are selected from phenyl and substituted phenyl with certain limitations, pyridinyl, thienyl, furanyl or naphthyl and, in addition, R may have the values: benzyl, substituted benzyl, cycloalkyl or loweralkyl, and D may additionally have the values: 2H-1-benzopyran-2-one, 4-oxo-4H-1-benzopyran-2-carboxylic acid loweralkyl ester, 1,4-benzodioxanloweralkyl-2-yl or quinolinyl, and the pharmaceutically acceptable addition salts thereof.

EP 0 235 463 A2

# N-SUBSTITUTED-ARYLALKYL AND ARYLALKYLENE AMINOHETEROCYCLICS AS CARDIOVASCULAR ANTIHISTAMINIC AND ANTISECRETORY AGENTS

The present invention relates to certain N-substituted arylalkyl and arylalkylenepyrrolidines, piperidines and homopiperidines useful in methods of treating cardiovascular disfunctions, countering effects of histamine in allergies and countering gastric secretion excesses. Certain of the compounds are novel and all the methods of treatment novelly use the compounds.

U. S. Patent 3,956,296 and a divisional patent thereof, U. S. 4,032,642, disclose pertinent compounds, among which some would fall within a generic structure as follows:

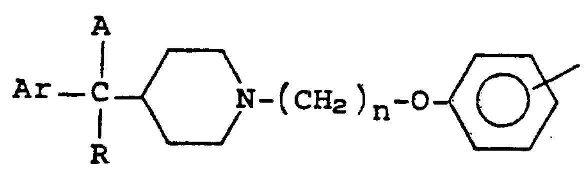

wherein Ar is phenyl, p-fluorophenyl or m-trifluorophenyl; R is phenyl, p-fluorophenyl or cyclohexyl and A is hydrogen or hydroxy, the compounds having utility as anti-inflammatory agents, sedatives and tranquilizers and pharmaceutical compositions therefor. The compounds of this structure are within the scope of novel uses of the present invention but are excluded from formulas representing novel compounds.

U. S. Patent 3,922,276 discloses compounds, among which would fall within a generic structure as follows:

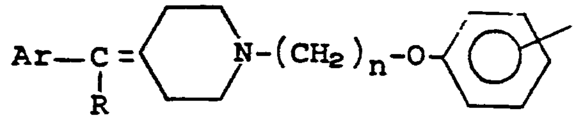

wherein Ar is phenyl or p-fluorophenyl and R is phenyl, p-fluorophenyl, m-trifluorophenyl or cyclohexyl, the compounds having utility as anti-inflammatory agents and tranquilizers and pharmaceutical compositions therefor. The compounds of this structure are within the scope of novel treatment methods of the present invention but are excluded from formulas representing novel compounds.

U. S. Patent 4,163,790 discloses compounds which fall within a generic structure as follows:

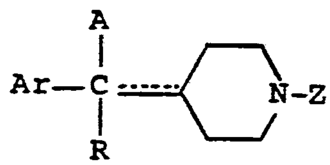

wherein Ar and R are phenyl and p-fluorophenyl and Z is hydrogen, acetyl, p-fluorobenzoylpropyl, carbamoyl, N-methylcarbamoyl, N,N-dimethylcarbamoyl, phenylcarbamoyl, or N-($\omega$-morpholinoethyl)carbamoyl; A is hydrogen, hydroxy or forms a double bond as indicated by the dotted line. The compounds were active in increasing coronary blood flow; however, the compounds while substituted in the 4-piperidine position and also disclosed in the other above-mentioned patents, differ substantially in structure from the compounds of the present invention in the substitution in the 1-position of the piperidine radical and are not within the scope of generic formulas hereof of compounds for novel treatment methods or novel compounds.

1-Benzyl-($\alpha$,$\alpha$-diphenyl)-4-benzylidinepiperidines of the formula 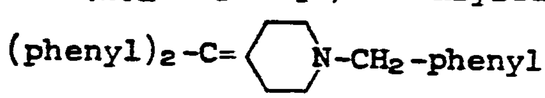 are disclosed in Ger. Offen. 2,800,919 as having anticonvulsant and vasodilating properties. Similar 1-benzyl-(α,α-diphenyl-4-benzylidinepiperidines of the formula
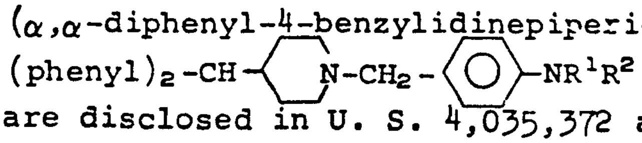
are disclosed in U. S. 4,035,372 as having vasodilating properties. The compounds are excluded from the present invention by proviso.

1-Benzyl-(α α-diphenyl)-4-benzylpiperidines of the general formula 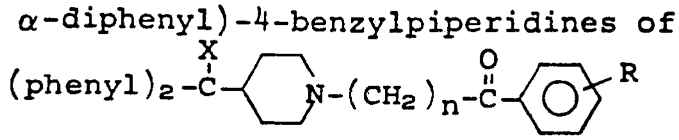

are disclosed in U. S. 3,965,257 as having antihistamine activity. The compounds are ketones and, as contrasted to the ethers, useful in the present methods.

4-(Diphenylmethylene)-1-benzylpiperidines of the general formula

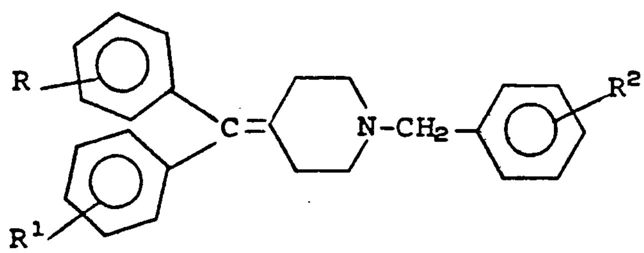

having hemodynamic, antiarrhythmic and antihistaminic activities are disclosed in U. S. Patent 3,759,928 and are excluded from the present invention by proviso.

U. S. Patent 3,984,557 discloses compounds which fall within a generic structure as follows:

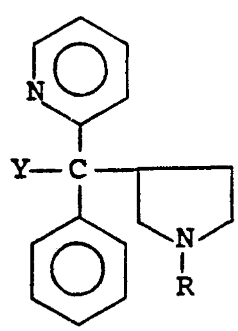

wherein R represents loweralkyl, lowercycloalkyl or phenylloweralkyl and Y is carbamoyl, cyano or hydrogen, the compounds having utility as antiarrhythmic agents. In the compounds of the present invention, the radical on the 1-position of the cycloalkylamino moiety has an aryloxy,

arylamino or an aryl group other than phenyl on the alkyl chain.

A separate application, directed to the use of certain compounds of Formula I as anti-allergy agents wherein $(B)_z$ is confined to oxygen and A is hydrogen, hydroxy, cyano or forms a double bond has also been filed today, the specification of which is hereby incorporated by reference.

The present invention is concerned with correcting cardiovascular disturbances, and as anti-histiminic and antisecretory agents in animals and humans utilizing heterocyclic amines of the general formula I and certain novel compounds thereof as composition of matter. The compounds useful in the invention have the formula:

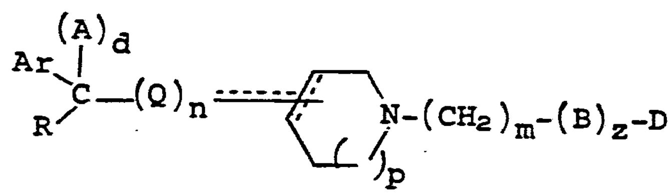

Formula I

wherein;

p is zero, one or two;

A is hydrogen, $-O-R^1$, $-C{\equiv}N$, $-\underset{O}{\overset{}{C}}NR^1R^2$, $-\overset{O}{C}-R^1$, $-\overset{O}{C}-O-R^1$, $-O-\overset{O}{C}-R^1$, $-CH_2OR^1$, $-CH_2NR^1R^2$;

m is zero to six inclusive;

Q is $-CH-$, $-CH_2-$ or $-\overset{OH}{\underset{H}{C}}-$;

d and n are selected from zero or one and the dotted lines represent double bonds which may form consistent with the valence of carbon;

Ar, D and R are selected from the group consisting of

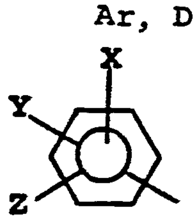 , 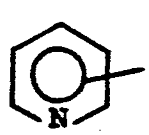 , 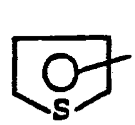 , 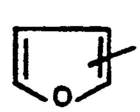 or 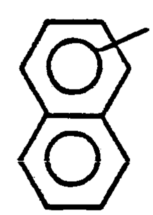

and in addition, R may have the values:

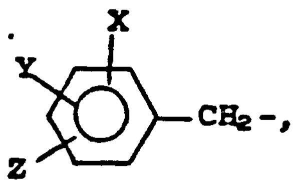 cycloalkyl or loweralkyl, and D may have additionally the values:

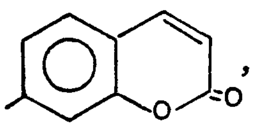, 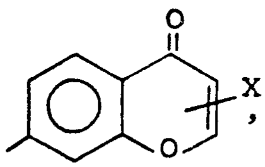, 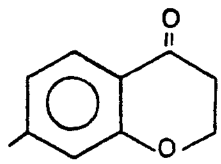, 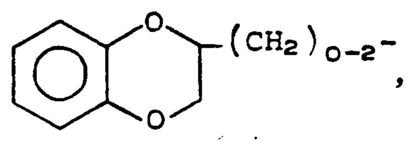,

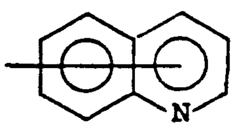, or $Ar(CH_2)_{1-4}$, X, Y and Z are selected from the group consisting of hydrogen, loweralkyl, halogen, $-NO_2$, $-O-R^1$, $-C(O)-R^1$, $-CF_3$, $-C{\equiv}N$, $-C(O)-N(R^1)_2$, $-N(R^1)_2$, $-C(O)OR^1$, $SO_2R^2$, $-SR^2$, $-S(O)R^2$, $-N(R^1)-C(O)-R^1$, $-CH_2COOM$, $-SO_2N(R^1)(R^2)$, $-N(R^1)SO_2CH_3$, $-N(R^1)C(O)-N(R^1)(R^2)$, or $-N(R^1)C(O)-OR^2$; B is selected from O, S, $-S(O)-$, $-S(O_2)-$, $-N(R^1)-$ and $-N-C(O)-O-R^1$; z is one or zero with the proviso that z cannot be zero at the same time n is zero when one of the following occurs at the same time that D is phenyl or substituted phenyl: $(A)_d$ is hydrogen, $(A)_d$ is cyano, $(A)_d$ is aminocarbonyl, or a double bond forms between the α carbon and a carbon of the central heterocyclic amine-ring; $R^1$ is selected from hydrogen, loweralkyl, phenyl and phenylloweralkyl; $R^2$ is selected from loweralkyl, phenyl and phenylloweralkyl; M is a pharmaceutically acceptable metal ion and the pharmaceutically acceptable salts thereof, including acid addition salts, quaternary salts, and hydrates and alcoholates thereof.

Preferred compound include, independently:

those wherein Ar is unsaturated phenyl or 4-saturated phenyl;

those wherein Ar is halo-substituted phenyl, trifluoromethyl-substituted phenyl, loweralkyl-substituted phenyl or loweralkoxy-substituted phenyl;

those wherein R is phenyl, 4-substituted phenyl or loweralkyl;

those wherein R is halo-substituted phenyl, loweralkyl-substituted phenyl or loweralkoxy-substituted phenyl;

those wherein M is two to five inclusive;

those wherein p is one;

and/or those wherein the left hand substituent in the formula, as drawn, is in the 4-position relative to the ring nitrogen atom.

In the further definition of symbols in the formulas hereof and where they appear elsewhere throughout this specification and in the claims, the terms have the following significance.

The term "loweralkyl" as used herein, unless otherwise specified, includes straight and branched chain radicals of up to eight carbons inclusive and is exemplified by such groups as methyl, ethyl, propyl, isopropyl, butyl, sec-butyl, tert-butyl, amyl, isoamyl, hexyl, heptyl and octyl radicals and the like. The term "loweralkoxy" has the formula -O-loweralkyl.

The term "cycloalkyl" as used herein includes primarily cyclic alkyl radicals containing 3-7 carbon atoms inclusive and includes such groups as cyclopropyl, cyclobutyl, cyclopentyl, cyclohexyl, cycloheptyl and methylcyclohexyl and the like.

The term "halo" or "halogen" when referred to herein includes fluorine, chlorine, bromine, and iodine unless otherwise stated.

The term "central heterocyclic amine ring" refers to that portion of Formula I represented by 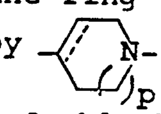 wherein the dotted line may represent a double bond. The term "saturated central heterocyclic amine ring" refers to the foregoing radical having no double bond.

The term "phenylloweralkyl" includes phenyl connected by hydrocarbon chains exemplified by loweralkyl above and wherein phenyl may be substituted by non-reactive or non-interfering radicals such as halo, loweralkyl, loweralkoxy and the like.

"Pharmaceutically acceptable salts" include acid addition salts, hydrates, alcoholates and quaternary salts of the compounds of Formula I which are physiologically compatible in warm-blooded animals. The acid addition salts may be formed by either strong or weak acids. Representative of strong acids are hydrochloric, hydrobromic, sulfuric and phosphoric acids. Representative of weak acids are fumaric, maleic, mandelic, tartaric, citric, oxalic, succinic, hexamic and the like. Suitable quaternary salts include the loweralkyl halides and loweralkyl sulfates.

The compounds of Formula I have been found to be calcium antagonists with potential use as coronary vasodilators, antihypertensives, antiarrhythmic, antiallergy,

antihistaminic and antisecretory agents. As stated above, an application directed to use of certain compounds of Formula I as antiallergy agents wherein $(B)_z$ is confined to oxygen and A is hydrogen, hydroxy, cyano or forms a double bond has also been filed today, is hereby incorporated by reference and serves to demonstrate utility of those compounds as antiallergy agents.

Pharmacological testing methods used for screening in support of methods of treatment of this invention excluding antiallergy method of treatment are described hereinbelow.

Certain compounds encompassed by Formula I are novel as represented by Formula Ia.

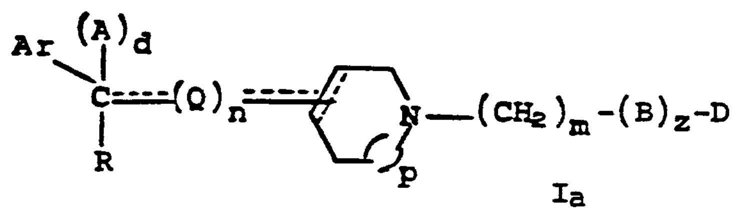

Ia

wherein p, m, d, Q, n, A, Ar, D, R, $R^1$, $R^2$, $Ar^1$, M, B, z, X, Y and Z are as defined under Formula I with the following additional proviso:

> $(B)_z$ cannot represent oxygen at the same time D is phenyl or substituted phenyl when n is zero and $(A)_d$ is hydrogen or hydroxyl; or when d is zero and a double bond forms between the $\alpha$-carbon of a saturated central heterocyclic amine ring.

DETAILED DESCRIPTION OF THE INVENTION

The compounds of Formula I may be prepared by methods described in U. S. Patents 3,922,276 and 4,032,642 and as set forth in Charts I, II, III, IV and V in the description of intermediate preparation, the preparations and examples hereinbelow. One of the general methods used is outlined by equations in Chart I. This reaction can be carried out in alcoholic solvents, preferably refluxing butanol, or in dimethylformamide or dimethoxyethane in the presence of an acid receptor, as for example, an alkali-metal carbonate and preferably using potassium iodide catalyst. The

reaction time may vary from a few hours to 24 hr, depending on reactivity of the halide and temperature. Temperature may vary from 80°C. to 125°C. Products are usually isolated by partitioning in a solvent such as methylene chloride, chloroform or benzene and the like and a weak basic solution and washing, drying, and concentrating the organic layer to give the free base which may then be converted, if desired, to an acid addition salt in a conventional manner.

Alternate Method B is shown by equation in Chart II. This reaction may be carried out in a suitable solvent such as tetrahydrofuran at room temperature for several hours. Preparation and isolation of free base and a salt is typically described in Example 4.

Alternate Method C is shown by equation in Chart III. Mesylation or tosylation with such as mesyl or tosyl chloride is conducted in the presence of an acid receptor such as a tertiary amine; e.g., triethylamine, while cooling. The final reaction of the mesylate or tosylate with the $DO^{\ominus}M^{\oplus}$ is conducted in a suitable organic solvent and the free base is isolated by conventional means such as washing, extracting with an acid solution and an organic solvent and evaporating the solvent. Salts may be prepared as described hereinabove.

Alternate Method D is shown by equation in Chart IV. When the halo compound is reacted with the $DO^{\ominus}M^{\oplus}$ compound, a suitable solvent is dimethylsulfoxide and a suitable temperature is about 25°C. When the halo compound is reacted with the $HN(R)-D$ compound, excess $HN(R)-D$ compound may be used as solvent and a temperature of about 100°C. or above is suitable.

Alternate Method E is shown by equation in Chart V. The method is limited to preparation of certain derivatives such as wherein D is 2-pyridinyl or 2-quinolinyl. Dimethyl sulfoxide is a suitable solvent and 60°C. is a suitable temperature for the reaction.

CHART I

Preparation of Compounds of Formula I:

Method A.

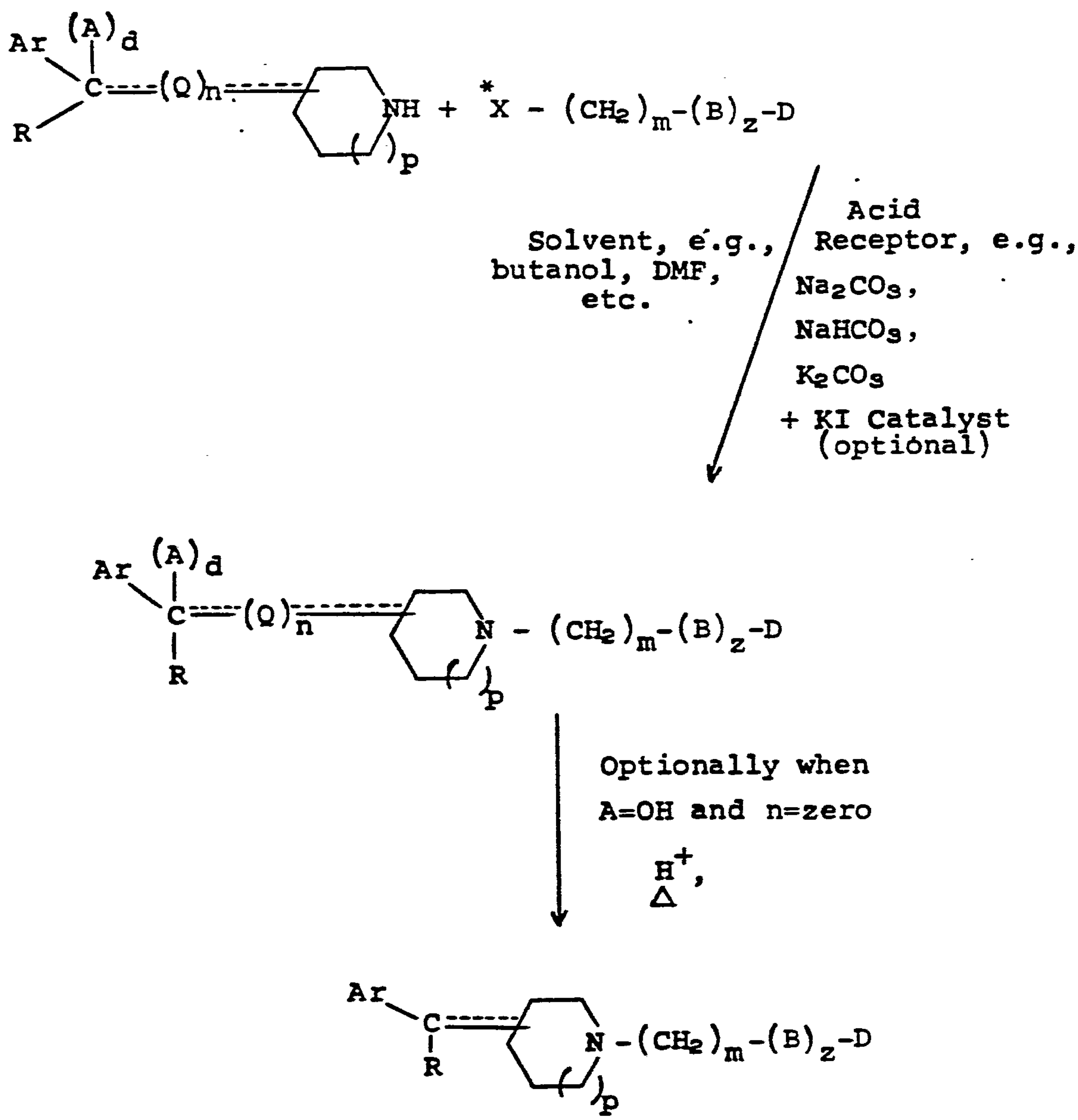


*X = halide

## CHART II

Alternate Preparation of Compounds of Formula I:

Method B.

(R = Ar, n of $(Q)_n$ is zero)

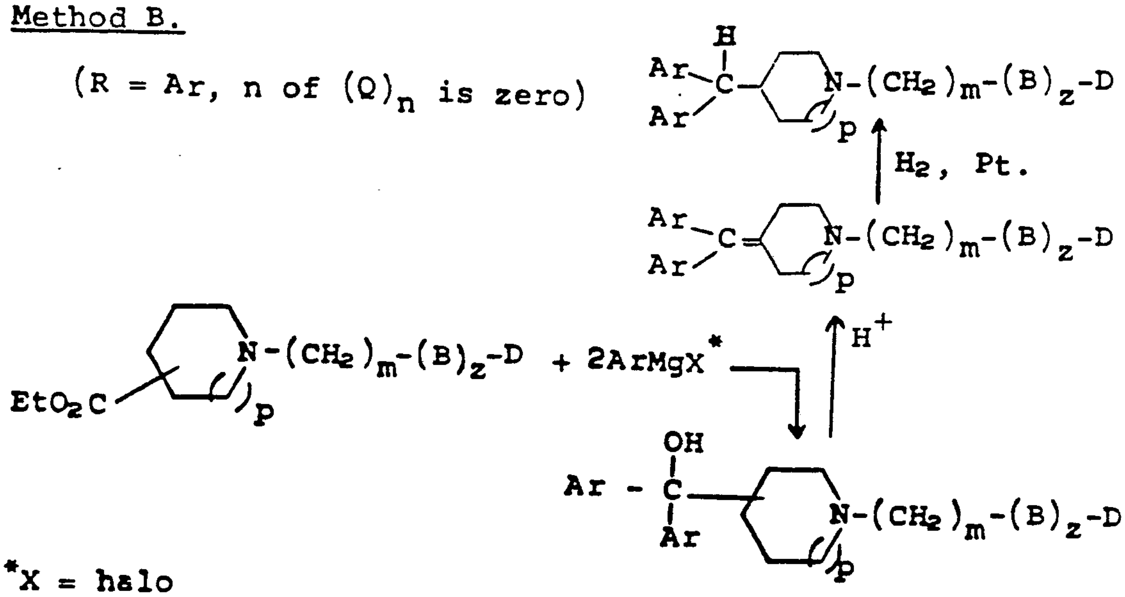


*X = halo

## CHART III

Alternate Preparation of Compounds of Formula I:

Method C.

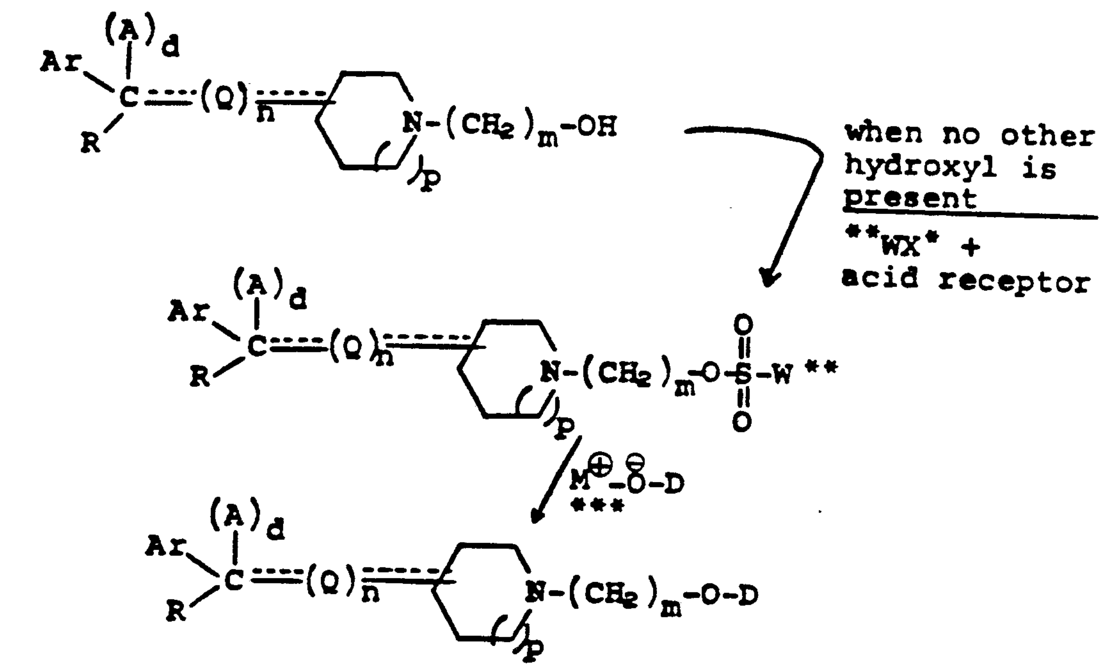


Footnotes:

*X = halo.

**W = mesyl, tosyl, etc.

***M = alkali-metal.

CHART IV

Alternate Preparation of Compounds of Formula I:

Method D.

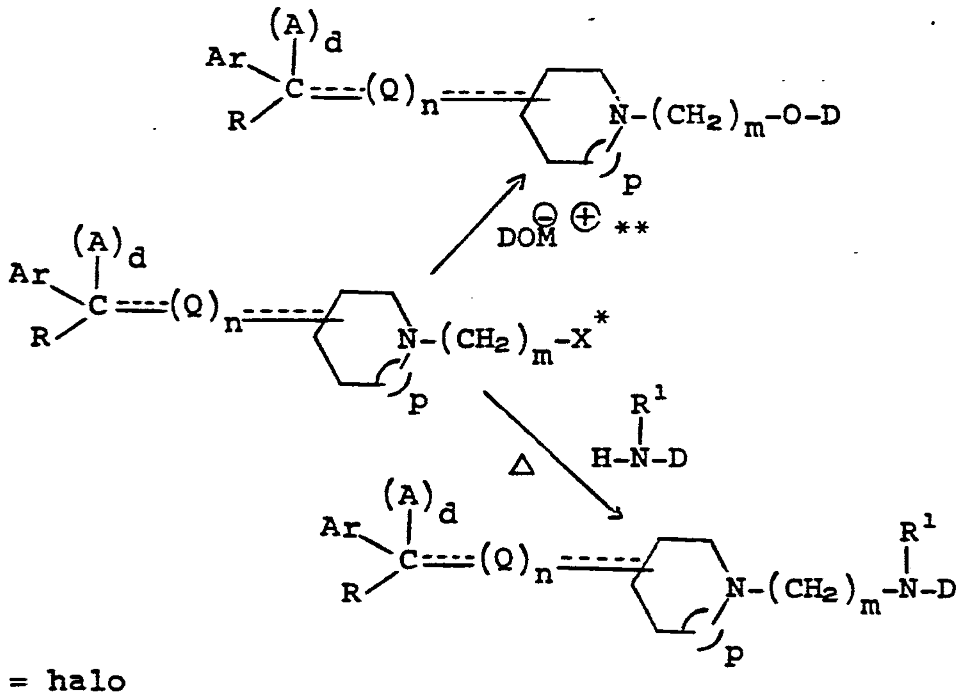


*X = halo

**M = alkali metal

CHART V

Alternate Preparation of Certain Compounds of Formula I:

Method E.

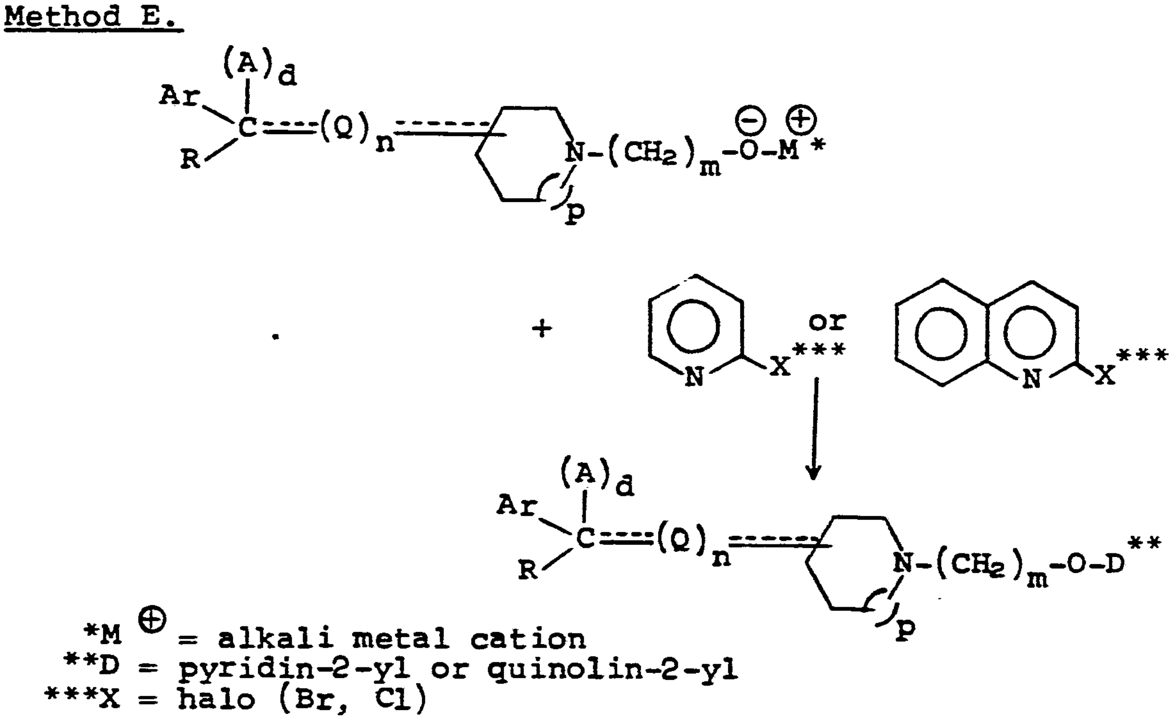


*M ⊕ = alkali metal cation

**D = pyridin-2-yl or quinolin-2-yl

***X = halo (Br, Cl)

To prepare acid addition salts, the free base is reacted with the calculated amount of organic or inorganic acid in aqueous miscible solvent such as ethanol or isopropanol, with isolation by concentration and/or cooling, or the base is reacted with an excess of the acid in aqueous immiscible solvent such as diethyl ether or isopropyl ether, with the desired salt separating directly. Exemplary of such organic salts are those formed with oxalic, maleic, fumaric, benzoic, ascorbic, pamoic, succinic, methanesulfonic, acetic, propionic, tartaric, citric, lactic, malic, citraconic, itaconic, hexamic, p-aminobenzoic, glutamic and stearic acid and the like. Exemplary of such inorganic salts are those formed with hydrochloric, hydrobromic, sulfuric, sulfamic, phosphoric and nitric acids.

If desired, the free base may be regenerated by proportioning the acid addition salt between an organic solvent such as methylene chloride and aqueous weakly basic solution of, for example, sodium bicarbonate and separating the methylene chloride layer and evaporating it.

Precursors (chemical intermediates) used in the synthesis of compounds of Formula I are prepared in a number of ways as illustrated by the following 1 to 10 sets of equations which are also applicable to pyrrolidinyl and homopiperidinyl derivatives: See also U. S. Patents 3,922,276 and 3,956,296.

(1)

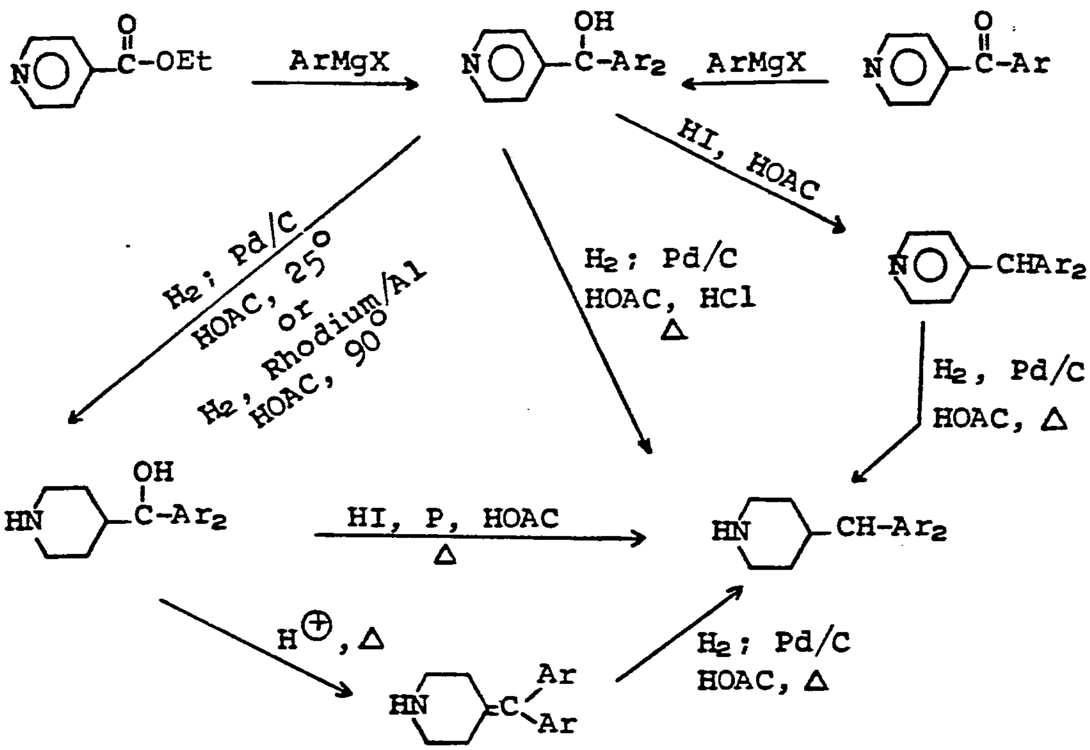
ArMgX
ArMgX
HI, HOAC
H2; Pd/C
HOAC, 25°
or
H2, Rhodium/Al
HOAC, 90°
H2; Pd/C
HOAC, HCl
Δ
H2, Pd/C
HOAC, Δ
HI, P, HOAC
Δ
H⊕, Δ
H2; Pd/C
HOAC, Δ

(2)

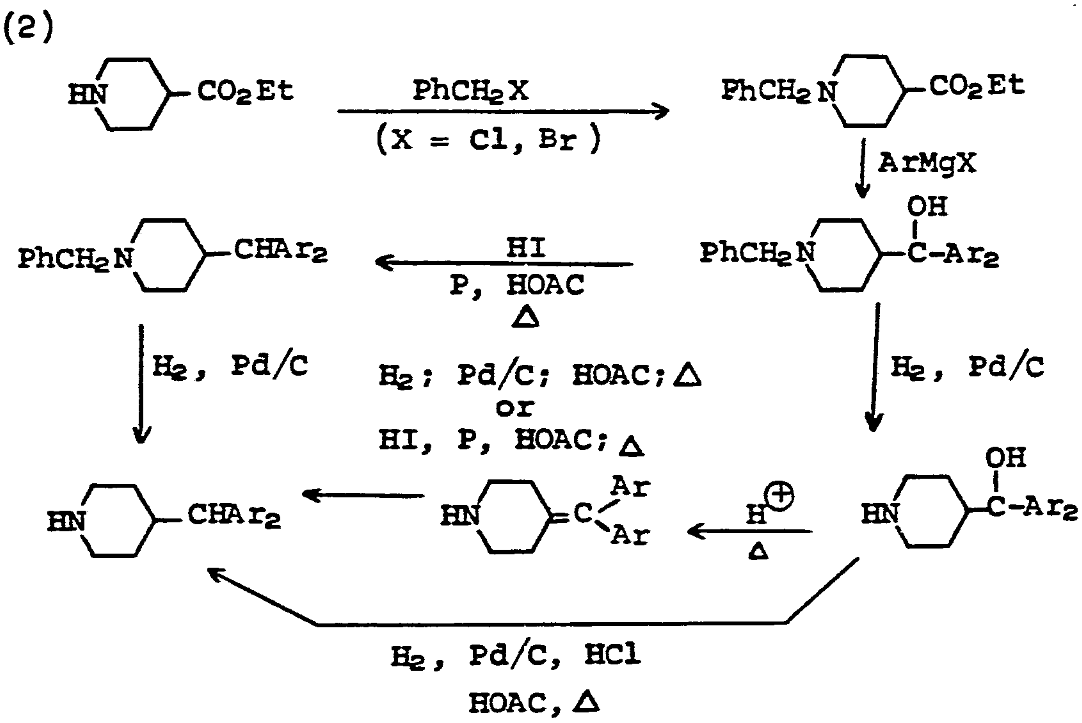
PhCH2X
(X = Cl, Br)
ArMgX
HI
P, HOAC
Δ
H2, Pd/C
H2; Pd/C; HOAC; Δ
or
HI, P, HOAC; Δ
H2, Pd/C
H⊕
Δ
H2, Pd/C, HCl
HOAC, Δ

Ph = phenyl

(3)

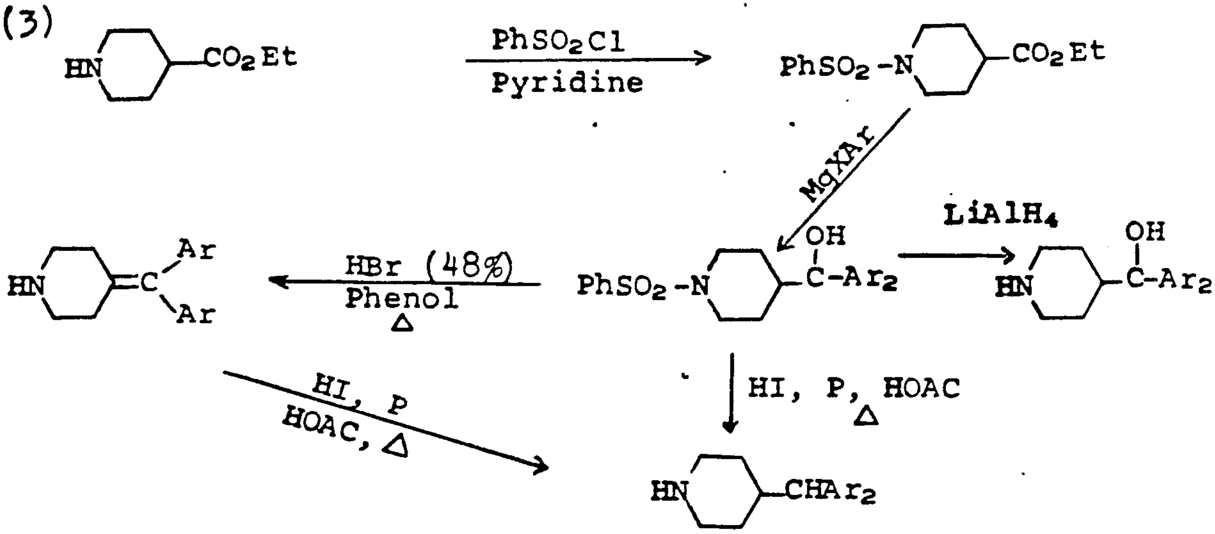


(4)

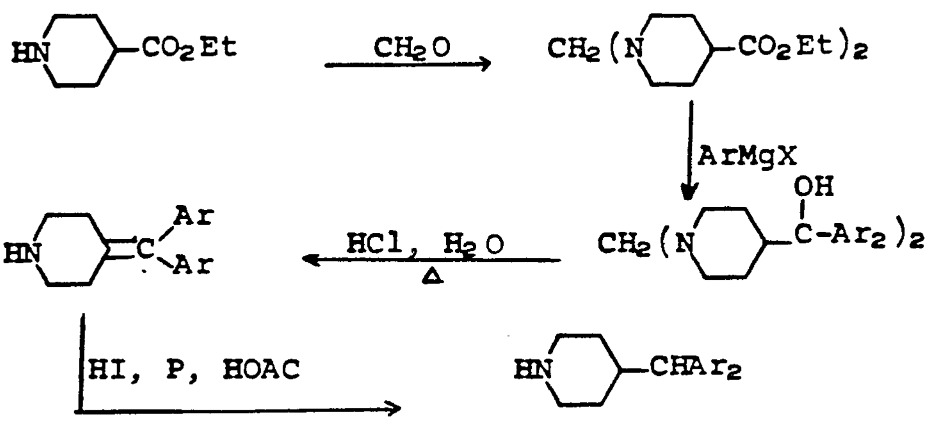


(5)

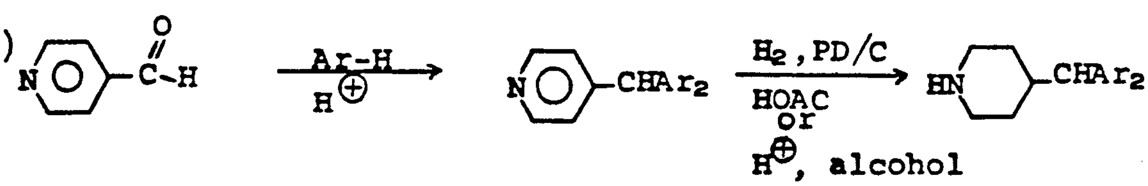


(6)

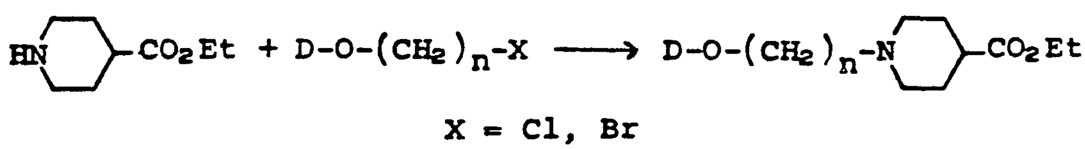


Ph = phenyl.

(7)

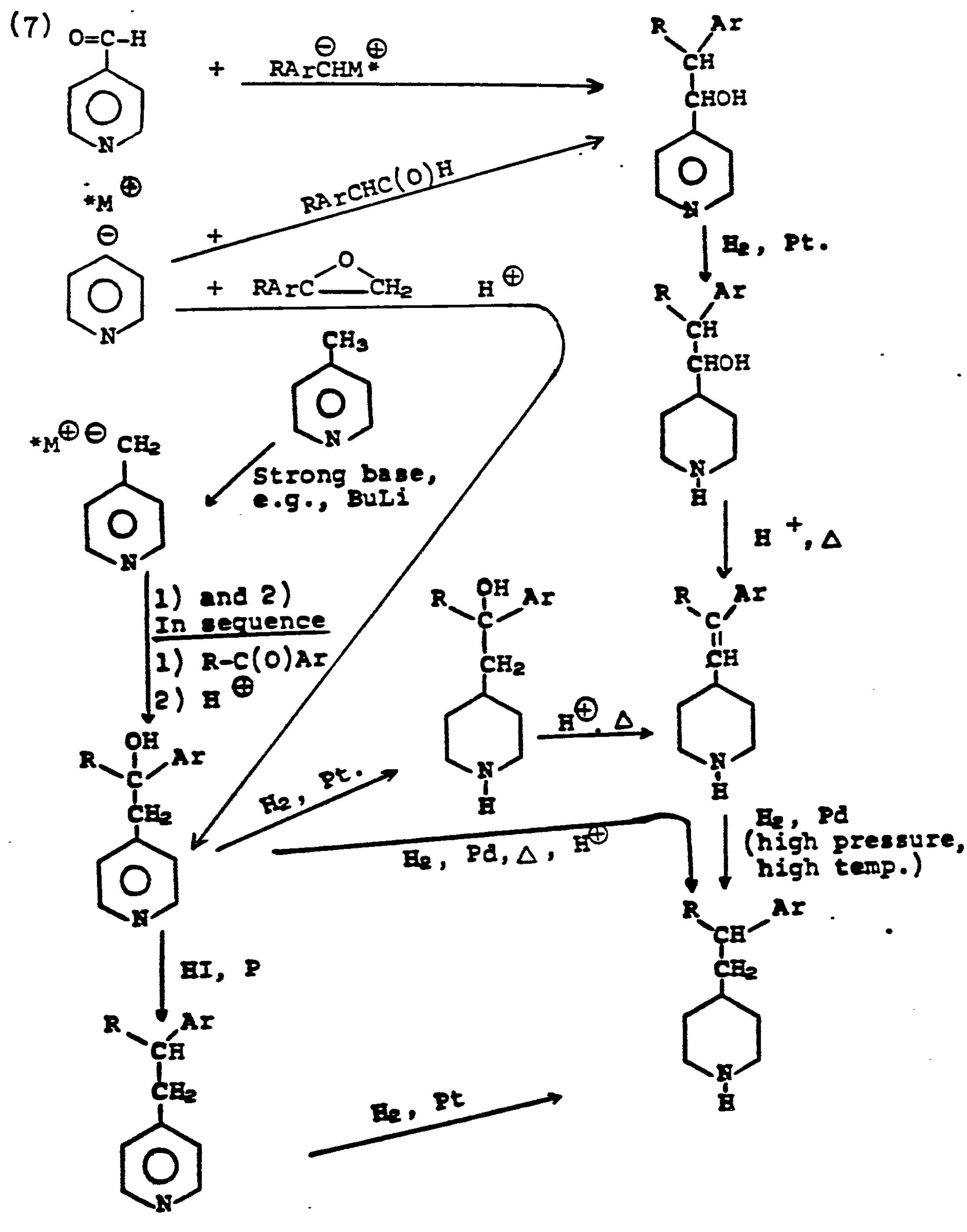

Strong base,
e.g., BuLi
1) and 2)
In sequence
1) R-C(O)Ar
2) H ⊕
H₂, Pt.
H ⁺, Δ
H⊕, Δ
H₂, Pd, Δ, H⊕
H₂, Pd
(high pressure,
high temp.)
HI, P
H₂, Pt


* M⊕ = Li⊕ or MgBr⊕

(8)

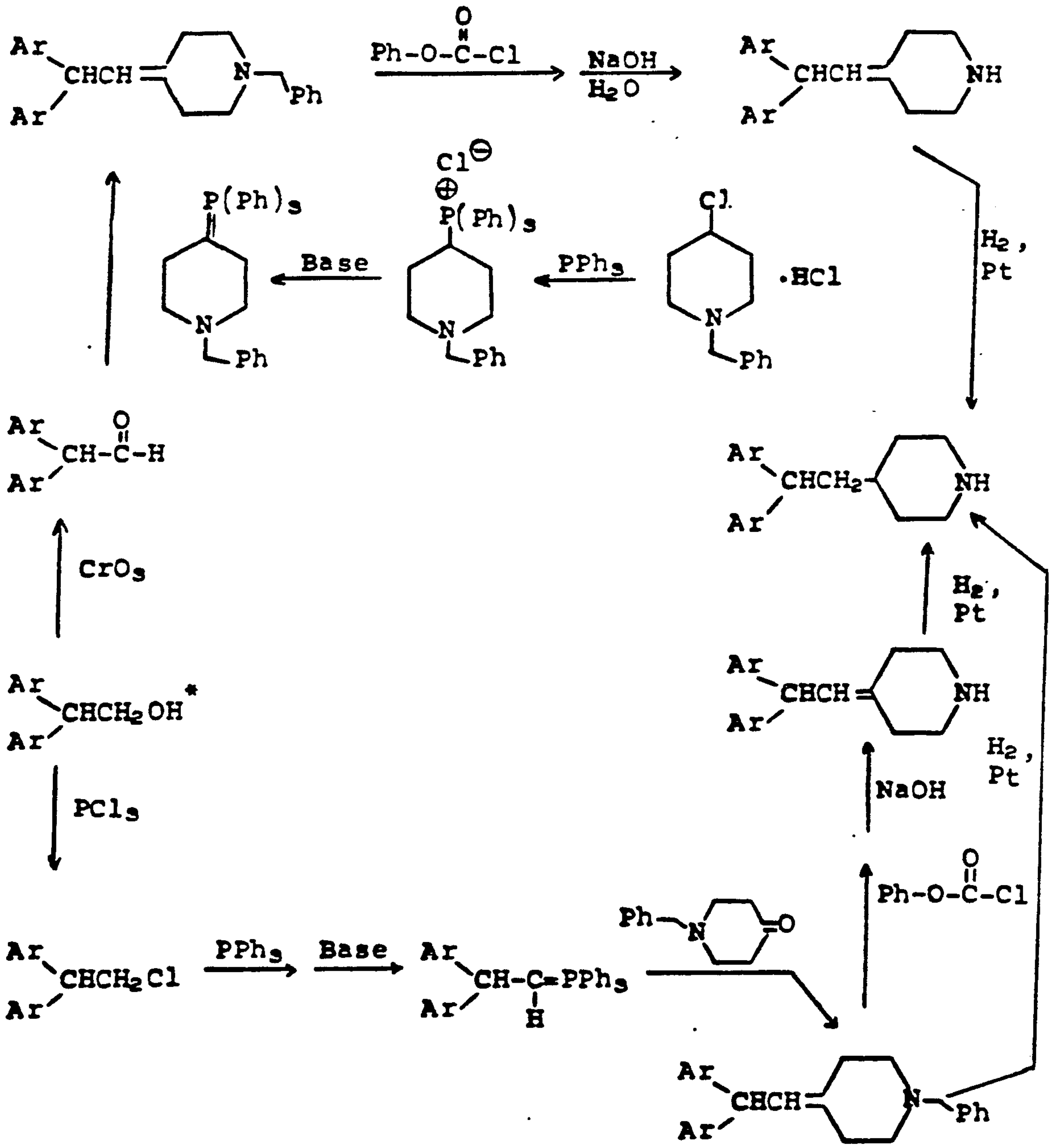


*Commercially available
Ph = phenyl

(9)

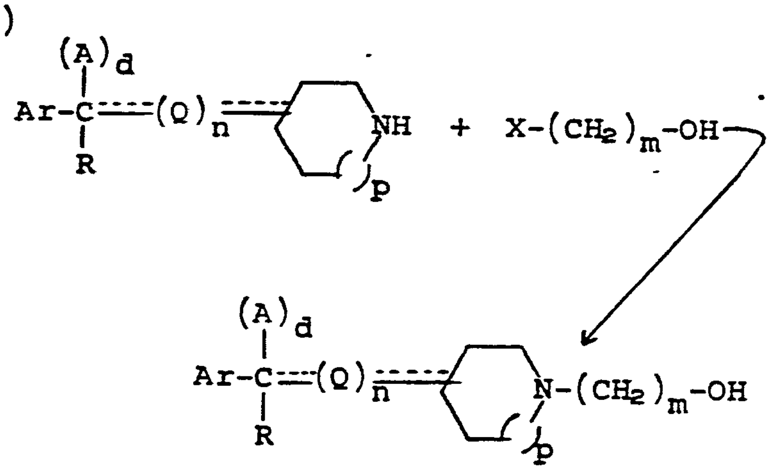

X = halo.

(10)

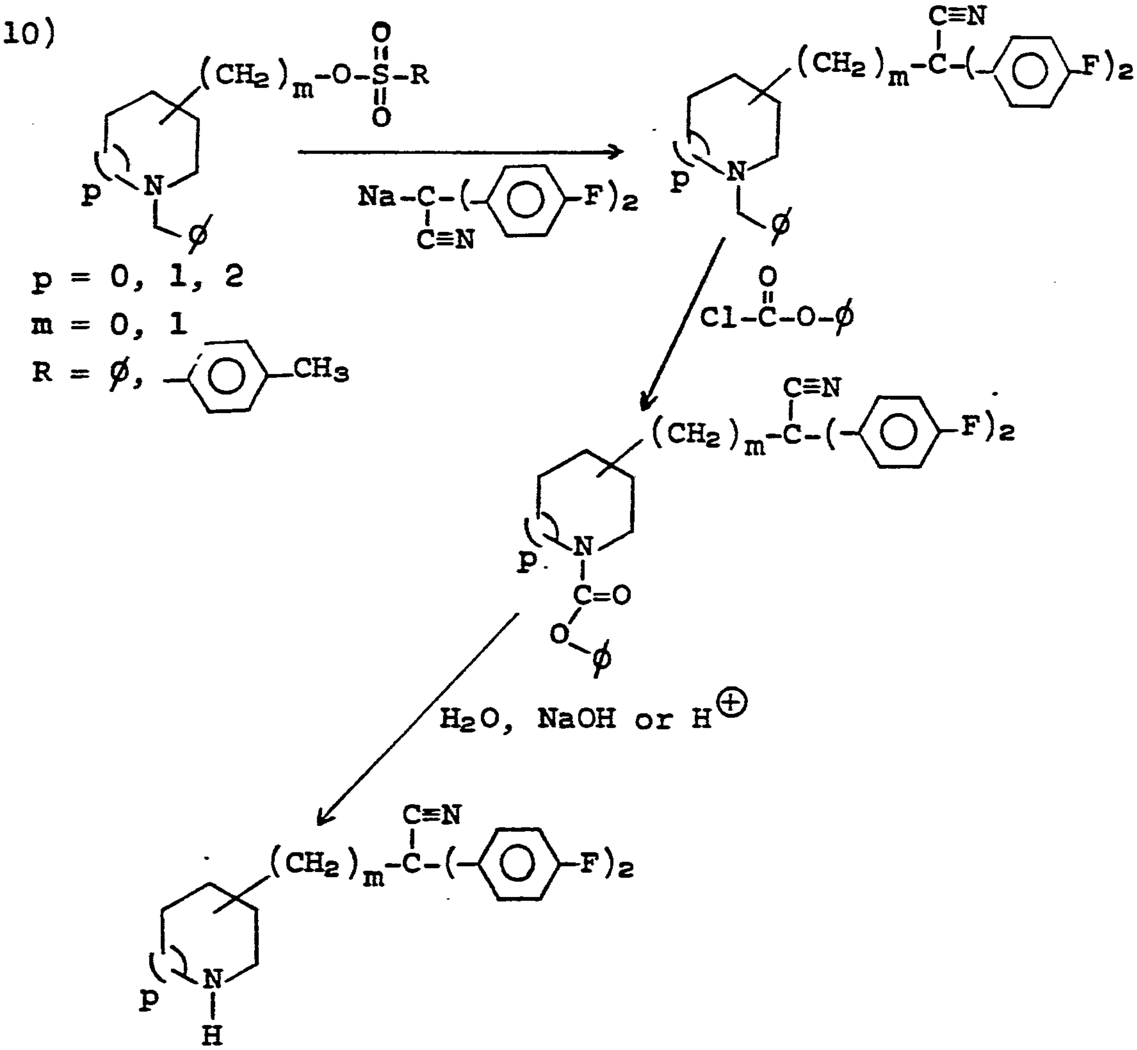

The method of preparation of certain starting materials wherein D is phenyl substituted by hydroxy is illustrated by the following equations:

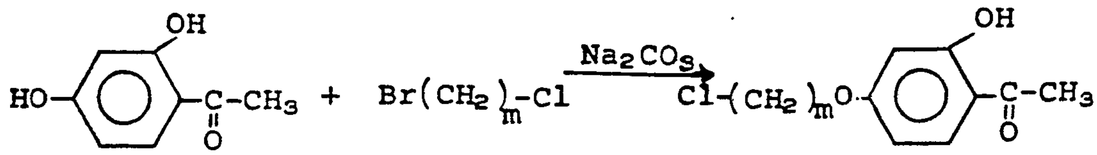


The preparation of other hydroxyphenyl intermediates and compounds is illustrated by the following equation:

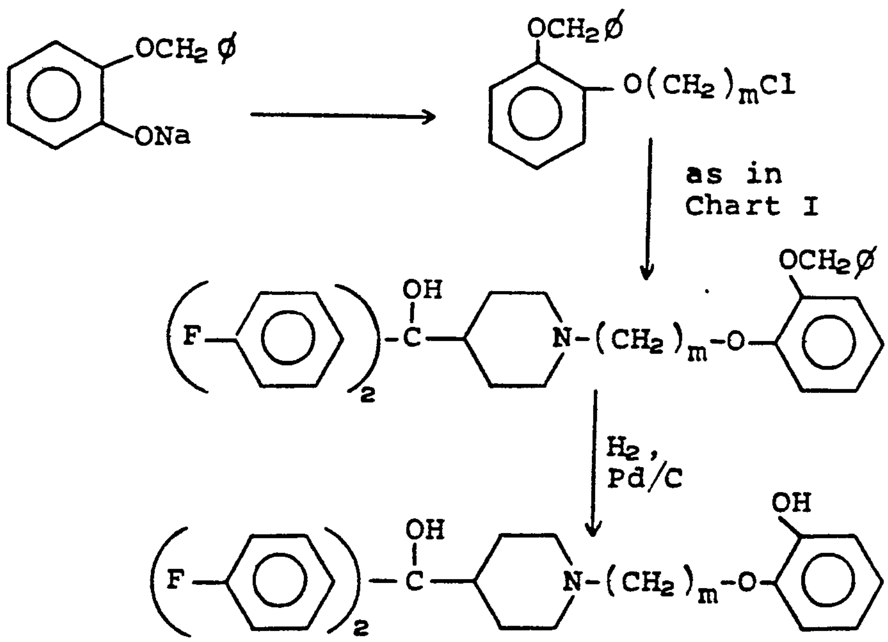


$\phi$ = phenyl.

The preparation of certain substituted phenol starting materials is illustrated by the following equations:

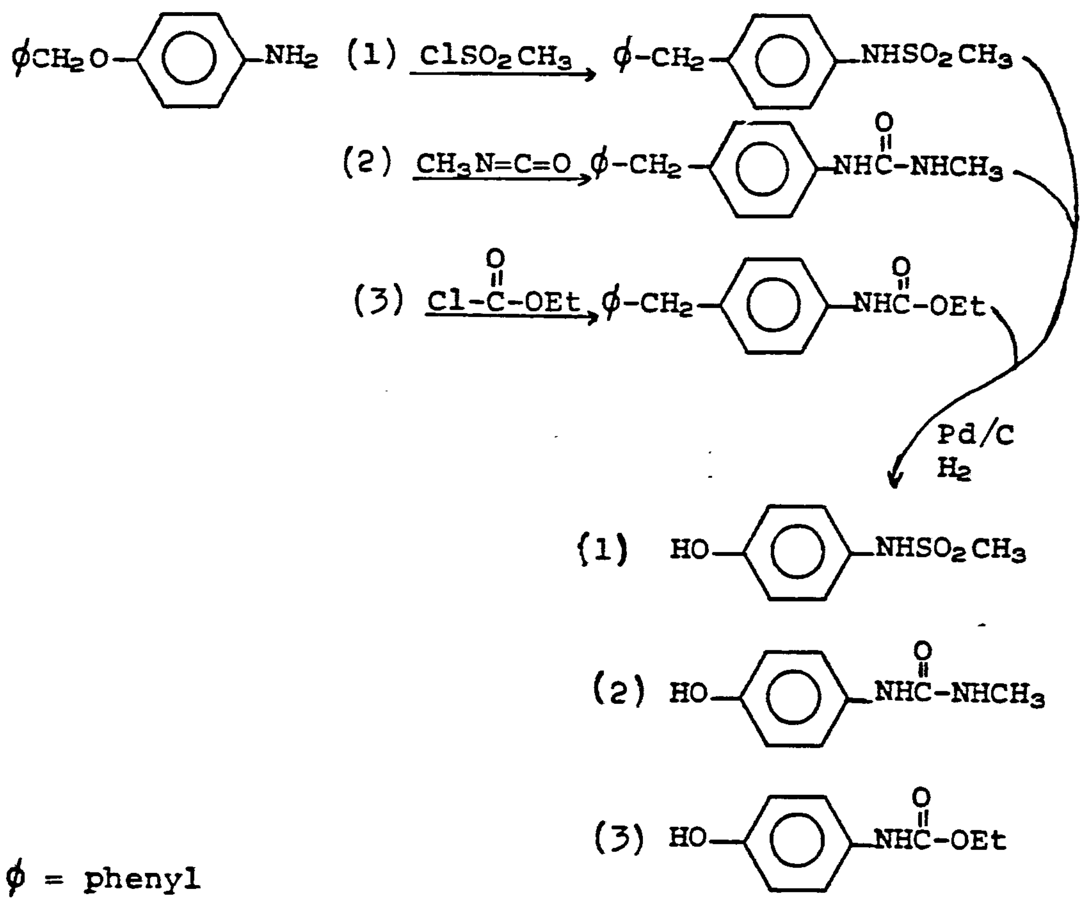


ϕ = phenyl

The preparation of chemical intermediates is further illustrated in the following Preparations 1 to 84. Examples 1 to 147 illustrate the synthesis methods for preparing compounds of Formula I. The scope of the invention is not limited by the descriptive methods and procedures of the preparations and examples, however.

Preparation 1

4-Diphenylmethylenepiperidine.

A solution of 7.0 g of 1-acetyl-4-diphenylhydroxymethylpiperidine in 30 ml of absolute alcohol and 76 ml of concentrated hydrochloric acid was heated at reflux for seven hours, cooled and made basic with 50% sodium hydroxide. The oil which separated was extracted with benzene and the combined extracts washed with water. After drying over magnesium sulfate the solvent was evaporated at reduced pressure. The residual oil which crystallized on cooling was recrystallized twice from petroleum ether to give 4.0 g (73.0%) of white crystals, m.p. 85-86°C.

Analysis: Calculated for $C_{18}H_{19}N$: C,86.70; H,7.68; N,5.62
Found : C,86.70; H,7.83; N,5.73

Preparation 2

[α,α-Bis(p-fluorophenyl)]-4-piperidinemethanol hydrochloride hydrate [1:1:0.5].

This compound was prepared by the method described in Preparation 1 of U. S. Patent 4,032,642, m.p. 243-243.5°C. from the Grignard reagent formed with p-fluorobromobenzene and 1-acetyl-4-(p-fluorobenzoyl)piperidine followed by hydrolysis and conversion to the salt.

Preparation 3

1-(Phenylmethyl)-4-piperidinecarboxylic acid ethyl ester hydrochloride [1:1].

A mixture of 100 g (0.637 mole) of ethyl isonipecotate, 80.64 g (0.64 mole) of benzyl chloride and 67.84 g (0.64 mole) of sodium carbonate in 1 liter of absolute ethanol was refluxed for 8 hours and then was stirred at room temperature for 10 hours. The solvent was removed *in vacuo*, and the residue was partitioned between methylene chloride and dilute sodium hydroxide. The methylene chloride phase was dried over magnesium sulfate and the solvent was removed *in vacuo* to give the free base of the title compound as a liquid. The free base was converted to the hydrochloric acid salt, and the salt was recrystallized from ethanol-ether to give 89.33 g (49.7%) of white crystalline solid, m.p. 154-155°C.

Analysis: Calculated for $C_{15}H_{22}NO_2Cl$: C,63.48; H,7.81; N,4.94
Found : C,63.07; H,7.82; N,4.91

Preparation 4

α,α-Bis-(4-fluorophenyl)-1-(phenylmethyl)-4-piperidinemethanol.

To a 6.08 g (0.25 mole) of magnesium turnings and an iodine crystal in 600 ml of dry tetrahydrofuran and under an atmosphere of nitrogen was added, dropwise, a solution of p-bromofluorobenzene in 125 ml of tetrahydrofuran. The temperature of the reaction was kept below 10°C. by cooling in an ice-methanol bath. The mixture was stirred at room temperature for 1.5 hours. A solution of 24.7 g (0.10 mole) of 1-(phenylmethyl)-4-piperidinecarboxylic acid ethyl ester hydrochloride in tetrahydrofuran was added, and the mixture was stirred at room temperature for 17 hours. The reaction was poured into an icy, aqueous solution of ammonium chloride, and the resulting solution was extracted with methylene chloride. The methylene chloride solution was extracted with dilute sodium hydroxide and was dried over magnesium sulfate. The solvent was removed *in vacuo* to give an oil. This was crystallized from ether-hexane to give 19.87 g (51%) of the title compound, m.p. 113-115°C.
Analysis: Calculated for $C_{25}H_{25}NOF_2$: C,76.31; H,6.40; N,3.56
Found : C,76.24; H,6.38; N,3.50

Preparation 5

[α,α-Bis(p-fluorophenyl)]-4-piperidinemethanol.

A solution of 31.2 g (0.079 mole) of α,α-bis-(4-fluorophenyl)-1-(phenylmethyl)-4-piperidinemethanol in 400 ml of absolute ethanol was hydrogenated at 50 psi and 70°C. over 5% palladium on carbon over the weekend. The mixture was filtered and the filtrate was concentrated under reduced pressure to give a gum as residue. Methylene chloride was added to the residue and the gum crystallized. The mixture was diluted with petroleum ether and the solid was collected by filtration, washed with petroleum ether, and dried to yield 22 g (92%) of white solid which was recrystallized from isopropyl ether-2-propanol, m.p. 159.5-160.5°C.

Analysis: Calculated for $C_{18}H_{19}F_2NO$: C,71.27; H,6.31; N,4.62
Found : C,70.93; H,6:71; N,4.38

Preparation 6

1-(Phenylsulfonyl)-4-piperidinecarboxylic acid, ethyl ester.

To a solution of 10.1 g (0.0642 mole) of ethyl isonipecotate in 300 ml of pyridine and cooled in an ice bath was added 13.2 g (0.075 mole) of benzene sulfonyl chloride. The mixture was stirred for 2 hours at room temperature, and the solvent was removed *in vacuo*. The residue was partitioned between methylene chloride and dilute sodium hydroxide. The methylene chloride solution was dried over magnesium sulfate, and the solvent was removed *in vacuo* to give a solid. This was recrystallized from ethanol-ether to give 4.59 g (24.1%) of crystalline solid, m.p. 85-86.5.

Analysis: Calculated for $C_{14}H_{19}NO_4S$: C,56.55; H,6.44; N,4.71
Found : C,56.53; H,6.55; N,4.67

In another preparation, 100 g (0.634 mole) of ethyl nipecotate and 130.4 g (0.74 mole) of benzene sulfonyl chloride were reacted by the above procedure for 4-1/2 hr. to give the title product in 78.1% yield.

Preparation 7

α,α-Bis(4-fluorophenyl)-1-(phenylsulfonyl)-4-piperidinemethanol.

To a suspension of 33.78 g (1.39 mole) of magnesium trimmings in 1 liter of tetrahydrofuran (dried over molecular sieves 5A) under an atmosphere of $N_2$ and cooled in an ice bath was added dropwise a solution of 243.25 g (1.39 mole) of p-bromofluorobenzene in 150 ml of tetrahydrofuran. The mixture was stirred for 2 hr after the addition was completed. To this mixture was added 103 g (0.346 mole) of 1-(phenylsulfonyl)-4-piperidinecarboxylic acid ethyl ester as a solid, and the solution was stirred at ambient temperature for 5 hr. The reaction was poured into an icy aqueous solution of ammonium chloride. The phases were separated, and the solvent was removed *in vacuo* from the organic phase. The

residue was partitioned between methylene chloride and dilute sodium hydroxide. The methylene chloride solution was dried over magnesium sulfate and was reduced in vacuo to ≈1 liter volume. The title compound was obtained by adding hexane and cooling, recrystallizing the precipitate from ethyl acetate and hexane and drying the solid under high vacuum at 130°C. for 45 min. at which time the product had partially melted, m.p. 142.5-144°C.

Analysis: Calculated for $C_{24}H_{23}NO_3SF_2$: C,65.00; H,5.23; N,3.16
Found : C,65.21; H,5.30; N,3.10

## Preparation 8

4-[Bis(4-fluorophenyl)methylene]-1-(phenylsulfonyl) piperidine.

A solution of 5.23 g (0.0118 mole) of α,α-bis(4-fluoro-phenyl)-1-(phenylsulfonyl)-4-piperidine methanol in 100 ml of acetic acid and 20 ml of 2M sulfuric acid was refluxed for 2-1/2 hr and then was poured over ice. The mixture was made basic with 50% sodium hydroxide and the basic mixture was extracted with methylene chloride. The methylene chloride solution was dried (anhydrous sodium sulfate), and the solvent was removed in vacuo. The residue was recrystallized from ether-hexane to give 3.23 g (64.4%) of white crystalline solid, m.p. 90-92.5°C.

Analysis: Calculated for $C_{24}H_{21}NO_2SF_2$: C,67.75; H,4.98; N,3.29
Found : C,67.73; H,5.00; N,3.21

## Preparation 9

4-[Bis(4-fluorophenyl)methylene]piperidine hydrobromide [1:1].

A mixture of 164 g (0.342 mole) of α,α-[bis(4-fluoro-phenyl)]-1-(phenylsulfonyl)-4-piperidinemethanol and 80 g (0.85 mole) of phenol in 700 ml of 48% hydrobromic acid was refluxed for 7 hr and then was stirred at room temperature for 9 hr. The hydrobromic acid solution was decanted from a gum in the bottom of the reaction flask. The gum was triturated with ~1 liter of ether, and a tan solid formed.

The solid was washed with several portions of ether and was dried under high vacuum to give 9.13 g (73%) of slightly impure title product, m.p. 211-215°C. A small sample of this solid was recrystallized from methanol to give an analytically pure sample as a crystalline solid, m.p. 216-218°C.

Analysis: Calculated for $C_{18}H_{18}NBrF_2$: C,59.03; H,4.95; N,3.82

Found : C,58.96; H,4.98; N,3.76

Preparation 10

4-[Bis(4-fluorophenyl)methyl]piperidine fumarate hydrate [1:1:0.5].

A mixture of 30.6 (0.99 mole) of phosphorous and 15.1 g (0.059 mole) of iodine in 90 ml of glacial acetic acid was strrred for 20 min at room temperature. A mixture of 6 ml of water, 70 ml of methanesulfonic acid, 56.19 g (0.197 mole) of 4-[bis(4-fluorophenyl)methylene]piperidine and 110 ml of glacial acetic acid was added, and the mixture was refluxed for 7 hr. The solvent was removed in vacuo, and the resulting viscous liquid was poured over ice. The icy mixture was made basic with 50% sodium hydroxide, and the basic suspension was extracted with methylene chloride. The methylene chloride solution was extracted with an aqueous solution of sodium thiosulfate and was dried over anhydrous sodium sulfate, and the solution was filtered through celite. The solvent was removed in vacuo to give a gum. The gum was dissolved in 400 ml of hot methanol, and 4.25 g of an unknown tan solid was collected from the warm solution. Fumaric acid (22 g, 0.190 mole) was added to the methanolic solution followed by the addition of ether. A white precipitate was collected to give 22.55 g (32.3%) of crystalline solid, m.p. 208-209°C.

Analysis: Calculated for $C_{20}H_{22}NO_{2.5}F_2$: C, 67.78; H,6.26; N,3.95

Found : C,67.86; H,6.12; N,3.81

Preparation 11

4-[α-(p-Fluorophenyl)-α-phenylmethyl]piperidine hydrochloride [1:1].

This compound was prepared as described in U. S. Patent 4,032,642 by hydrogenation of α-(p-fluorophenyl)benzylidine-piperidine over palladium charcoal catalyst, m.p. 81-82°C.

Analysis: Calculated for $C_{18}H_{21}ClFN$: C,70.69; H,6.92; N,4.58
Found : C,70.69; H,6.93; N,4.52

Preparation 12

1-[4-(3-Chloropropoxy)-3-methoxyphenyl]ethanone.

To a mixture of 15.15 kg (96.26 mole) of 1-bromo-3-chloropropane and 25 liter of water heated to 86°C. was added a solution of 8 kg (48.13 mole) of acetovanillone in 3.93 kg (48.6 mole) of 50% aqueous sodium hydroxide and 89 liter of water over a 2.5 hr period. The mixture was heated at 80-85°C. for 2-5 hr after addition was complete. The mixture was cooled and extracted twice with 49 kg portions of toluene. The combined extracts were washed once with 1.9 kg of 50% sodium hydroxide diluted to 5 gal and once with 5 gal of water. The toluene layer was dried over 3 lb of anhydrous sodium sulfate and concentrated under reduced pressure. The residue was heated to reflux in 15 gal of diisopropylether, filtered, and the filtrate cooled. The crystallized title compound obtained by filtration together with additional compound obtained by concentrating the filtrate to 25% of its original volume amounted to 4.2 kg (36%). Acetovanillone recovered was 3.4 kg. The product was recrystallized twice from cyclohexane and twice from ligroin, m.p. 57.8-58.5°C.

Analysis: Calculated for $C_{12}H_{15}ClO_3$: C,59.39; H,6.23;
Found : C,59.07; H,6.22

Preparation 13

1-(3-Phenoxypropyl)-4-piperidinecarboxylic acid ethyl ester oxalate [1:1].

A mixture of ethyl isonipecotate (35.5 g, 0.226 mole) 3-phenoxy-1-bromopropane (51.6 g, 0.24 mole) and sodium carbonate (25.4 g, 0.24 mole) in 500 ml of absolute ethanol was refluxed for 16 hr. The solvent was removed *in vacuo*, and the residue was partitioned between methylene chloride and dilute sodium hydroxide. The solution was dried over anhydrous sodium sulfate and the solvent was removed *in vacuo* to give a liquid. The liquid was dissolved in absolute ethanol, and a solution of oxalic acid (~0.23 mole) in absolute ethanol was added. The product 73.43 g (87.7%) precipitated as a white, crystalline solid, m.p. 180-181.5°C.

Analysis: Calculated for $C_{19}H_{27}NO_7$: C,59.83; H,7.14; N,3.67
Found : C,59.76; H,7.17; N,3.64

Preparation 14

4-[Bis(4-fluorophenyl)methylene]-1-(phenylmethyl)-piperidine maleate [1:1].

A mixture of α α-bis(4-fluorophenyl)-1-(phenylmethyl)-4-piperidinemethanol (5.09 g, 0.013 mole) in 200 ml of acetic acid and 10 ml of 2M sulfuric acid was refluxed for 2 hr. The solvent was removed *in vacuo*, and the residue was partitioned between methylene chloride and dilute sodium hydroxide. The methylene chloride solution was dried over magnesium sulfate and the solvent was removed *in vacuo* to give the free base of the title compound as a solid. The free base was dissolved in methanol-diethylether and maleic acid (excess) was added. The product 5.24 g (82.1%) precipitated as a white, crystalline solid, m.p. 180-181.5°C.

Analysis: Calculated for $C_{29}H_{27}NF_2O_4$: C,70.86; H,5.54; N,2.85
Found : C,70.80; H,5.45; N,2.79

Preparation 15

α,α-Bis(4-fluorophenyl)-4-pyridinemethanol.

The Grignard reagent was prepared from 4-bromofluorobenzene (66.6 g, 0.381 mole) and magnesium (9.13 g, 0.381 mole) in tetrahydrofuran (ice bath). The Grignard reagent was stirred at room temperature for 1-1/2 hr. and transferred (under $N_2$) to an addition funnel. This solution was added dropwise to a tetrahydrofuran solution of ethyl isonicotinate (25.0 g, 0.165 mole) (ice bath cooling). The reaction mixture was stirred 3 hr at room temperature and poured onto ice containing ammonium chloride (28 g, 0.5 mole). The mixture was allowed to stand overnight. The reaction mixture was diluted to 3 liter with water and extracted with chloroform. The chloroform layer was back extracted with dilute sodium hydroxide. Removal of chloroform gave a gummy brown solid. The brown solid was triturated with methanol-diethyl ether (10-120 v/v) and placed in the refrigerator freezer. Solid was filtered off and dried overnight in vacuo at 80°C. to give 11.86 g (24%) of white crystalline product, m.p. 185-189°C.

Analysis: Calculated for $C_{18}H_{13}NOF_2$: C,72.72; H,4.41; N,4.71
Found : C,72.76; H,4.39; N,4.67

Preparation 16

4-[Bis(4-fluorophenyl)methyl]-1-(phenylmethyl)-piperidine, fumarate [1:1].

A mixture of 4.3 g (0.139 mole) of phosphorous, 44 g (0.196 mole) of a 57% aqueous solution of hydrogen iodide and 4.15 g (0.0106 mole) of 4-[bis(4-fluorophenyl)methylene]-1-(phenylmethyl)piperidine in 60 ml of glacial acetic was refluxed for 1 hr. The mixture was poured over ice and was made basic with 50% sodium hydroxide. The aqueous mixture was extracted with methylene chloride. The methylene chloride solution was extracted with an aqueous solution of sodium sulfite and was dried over magnesium sulfate. The solvent was removed in vacuo to give 3.89 g (89%) of the free base of the title compound. The free base was converted to the fumarate salt, and the salt was recrystallized from methanol-ether to give 3.62 g (69.3%) white

solid, m.p. 201-202°C.

Analysis: Calculated for $C_{29}H_{29}NO_4F_2$: C,70.57; H,5.92; N,2.84
Found : C,70.69; H,5.95; N,2.81

Preparation 17

4-(2-Chloroethoxy)benzoic acid ethyl ester.

A mixture of 71.7 g (0.5 mole) of 1-bromo-2-chloroethane, 83.1 g (0.5 mole) of ethyl p-hydroxybenzoate and 69.1 g (0.5 mole) of potassium carbonate in 200 ml of acetone was heated at reflux for 40 hr. The solids were removed by filtration and the filtrate was evaporated under reduced pressure to leave a semi-solid residue. The residue was triturated with 200 ml of 5% sodium hydroxide solution and filtered.[1] The filter cake was washed with water (100 ml) and dried to give 42.4 g (37.2 %) of a solid. A sample was recrystallized from benzene-petroleum ether (30-60°C) to give white solid, m.p. 74-76°C.

Analysis: Calculated for $C_{11}H_{13}ClO_3$: C,57.78; H,5.73
Found : C,57.87; H,5.82

[1]The filtrate pH was adjusted to 2 with concentrated hydrochloric acid. The resulting solid was collected by filtration, washed with water (100 ml) and dried to give 44.4 g of ethyl p-hydroxybenzoate.

Preparation 18

1-[4-(2-Chloroethoxy)-3-methoxyphenyl]ethanone.

To a solution of 12.7 g (0.55 mole) of sodium metal in 750 ml of absolute ethanol was added 83.1 g (0.5 mole) of acetovanillone to give a slurry. This slurry was then added over a 3 hr period to a solution of 107.6 g (0.75 mole) 1-bromo-2-chloroethane in 500 ml of absolute ethanol at reflux. An additional 250 ml of ethanol was used to wash the slurry into the reaction mixture. The mixture was heated at reflux overnight and then concentrated under reduced pressure to give a solid as residue. The solid was partitioned between 1 liter of benzene and 1 liter of water. The aqueous layer was extracted with 500 ml of benzene and the combined organic layers were washed successively with

three 200 ml portions of a 5% sodium hydroxide solution, once with water and once with brine. The benzene solution was dried over anhydrous sodium sulfate and concentrated under reduced pressure to give an oil which gradually crystallized. The solid was triturated with petroleum ether, collected by filtration and recrystallized from 2-propanol to yield 48.5 g (42%) of off-white solid. An analytical sample was prepared from isopropyl ether, m.p. 69-71°C.

Analysis: Calculated for $C_{11}H_{13}ClO_3$: C,57.78; H,5.73
Found : C,57.55; H,5.74

Preparation 19

1-[4-(4-Bromobutoxy)-3-methoxyphenyl]ethanone.

To a warm solution of 12.7 g (0.55 mole) of sodium metal in 500 ml of absolute ethanol was added a slurry of 83.1 g (0.5 mole) of acetovanillone in 250 ml of absolute ethanol. All solids dissolved and then a solid precipitated. The mixture was stirred at ambient temperature for 1 hr and then added over a 3 hr period to a solution at reflux of 177 g (0.82 mole) of 1,4-dibromobutane in 500 ml of absolute ethanol. After addition was complete, the mixture was heated at reflux overnight. The mixture was concentrated under reduced pressure and the residue was partitioned between 1.5 liter of benzene and 1 liter of water. The mixture was filtered to remove undesirable insoluble material. The filtrate layers were separated and the organic layer was washed with four 300 ml portions of a 5% sodium hydroxide solution once with water and once with brine, dried over anhydrous sodium sulfate and concentrated under reduced pressure to give 138 g of gummy solid as residue. This solid was purified by column chromatography on 1 kg of silica gel, eluting with 2% ethyl acetate in benzene to yield 69.6 g (46%) of title compound as an off-white solid. The solid was recrystallized from isopropyl ether, m.p. 52-54°C.

Analysis: Calculated for $C_{13}H_{17}BrO_3$: C,51.84; H,5.69;
Found : C,52.03; H,5.76

## Preparation 20

4-(Diphenylmethyl)pyridine.

A mixture of 99 g (0.379 mole) of diphenyl-4-pyridyl-methanol, 50 ml of conc. hydrochloric acid, 200 ml of 57% hydroiodic acid and 200 ml of glacial acetic acid was refluxed for 4-1/2 hr and then was stirred at room temperature for 12 hr. The reaction mixture was poured over ice and was made basic with 50% sodium hydroxide. An aqueous solution of sodium thiosulfate was added, and the mixture was extracted with methylene chloride. The methylene chloride solution was dried over magnesium sulfate and the solvent was removed in vacuo. The residue was recrystallized from a mixture of methylene chloride-ether-hexane to give two crops of crystalline solids: Crop I, 40.87 g (44.0%), m.p. 124-126; Crop II, 25.38 g (27.3%), m.p. 123-125. Analysis of the mixture of the Crops I and II was as follows:

Analysis: Calculated for $C_{18}H_{15}N$: C,88.13; H,6.16; N,5.71

Found : C,87.67; H,6.01; N,5.56

## Preparation 21

1-(3-Chloropropoxy)-4-methoxybenzene.

A solution of sodium hydroxide 20.0 g (0.5 mole) in 300 ml of water and p-methoxyphenol, 62.1 g (0.5 mole) in 300 ml of dioxane was stirred for 1 hour at room temperature. 1-Chloro-3-bromopropane (472.35 g, 3.0 mole) in 100 ml of dioxane was added, and the reaction mixture was stirred overnight at 80°C. The lower layer was separated and the aqueous layer extracted with hexane. The lower layer and hexane layer were combined, dried, and solvent was removed in vacuo. The residue was dissolved in chloroform and extracted with 5% sodium hydroxide; removal of chloroform by evaporation gave a yellow oil. A 10 g sample of the oil was subjected to column chromatography on silica gel with an elution series composed of hexane-methylene chloride-methanol. This furnished 9.64 g (79.3% based on the aliquot taken)of pure clear oil.

Analysis: Calculated for $C_{10}H_{13}O_2Cl$: C,59.86; H,6.53

Found : C,59.39; H,6.56

Preparation 22

1-[4-(3-Chloropropoxy)phenyl]ethanone.

The sodium salt of p-hydroxyacetophenone was prepared in 200 ml of dioxane-400 ml of water from p-hydroxyacetophenone 68.08 g (0.5 mole) and sodium hydroxide 20.0 g, (0.5 mole). The reaction mixture was stirred 3/4 hr at room temperature. Next, chlorobromopropane, 472.35 g (3.0 mole), was added along with 200 ml of dioxane and the mixture was heated at 80-90°C. overnight with stirring. The mixture was diluted to 4 liters with water; the aqueous phase was extracted with hexane and chloroform. These were combined and back extracted with 5% sodium hydroxide. The solvent was removed *in vacuo* with heating. A 10 g sample of the oil was subject to column chromatography on silica gel using hexane-methylene chloride-methanol. Fractions with similar TLCs were combined and solvent removed. The oil from the column did not analyze, therefore a short-path bulb-bulb distillation was carried out. This produced 4.38 g (37.9%) of clear oil.

| | | |
|---|---|---|
| Analysis: | Calculated for $C_{11}H_{13}O_2Cl$: | C,62.12; H,6.16; |
| | Found | : C,61.70; H,6.17 |

$^1H$ NMR ($CDCl_3$) Analysis:

| | | | |
|---|---|---|---|
| δ 8.1 | doublet | aromatic portons | 2H |
| δ 6.8-7.0 | doublet | aromatic portons | 2H |
| δ 4.1-4.3 | triplet | $CH_2$ | 2H |
| δ 3.6-3.8 | triplet | $-CH_2-$ | 2H |
| δ 2.5 | singlet | $\underset{O}{C}-CH_3$ or $COCH_3$ | 2H |
| δ 2-2.4 | triplet | $-CH_2-$ | 2H |

Preparation 23

4-(Diphenylmethyl)piperidine hydrochloride [1:1].

A mixture of 62.69 g (0.256 mole) of diphenyl-4-pyridyl-methane and 6.4 g of 10% palladium on carbon (0.0060 mole) in 300 ml of glacial acetic acid and under an atmosphere of hydrogen (44 psi) was shaken on a Parr apparatus at 85° for 4 days. The reaction mixture was filtered, and the solvent was removed *in vacuo* from the filtrate. The residue was partitioned between methylene chloride and dilute sodium hydroxide. The methylene chloride solution was dried over

magnesium sulfate, and the solvent was removed *in vacuo* to give a solid. This was dissolved in a mixture of methanol and acetonitrile, and excess ethereal hydrogen chloride was added. A precipitate was collected to give 59.13 g (80.3%) of slightly impure title compound as a white crystalline solid, m.p. 273-274 C. Part of this was recrystallized from methanol-ether to give an analytically pure sample, m.p. 275.5-277°C.

Analysis: Calculated for $C_{18}H_{22}NCl$: C,75.11; H,7.70; N,4.87
Found : C,75.03; H,7.73; N,4.93

## Preparation 24

### α-(4-Fluorophenyl)-α-phenyl-4-pyridinemethanol.

To a suspension of 18.5 g (0.761 mole) of magnesium turnings and several crystals of iodine in 800 ml of anhydrous diethyl-ether, cooled in an ice bath and under an atmosphere of argon was slowly added a solution of p-bromofluorobenzene in 200 ml of diethyl-ether. The solution was stirred for 2 hr at 25°C. and 97.02 g (0.530 mole) of 4-benzoylpyridine was added as a solid. An additional 1 liter of anhydrous diethyl-ether was added, and the solution was stirred at 25°C. for 3 hr. The reaction mixture was poured into an icy, aqueous solution of ammonium chloride. The mixture stood in the hood overnight and a white solid was collected. The solid was dissolved in a mixture of methanol-methylene chloride. The solution was filtered and the solvent was removed *in vacuo*. The residue was crystallized from chloroform-hexane to give 66.68 g (45%) of title compound as a white, crystalline solid, m.p. 189-192°C. Part of this was recrystallized from methylene chloride-acetonitrile-hexane, m.p. 190-192°C.

Analysis: Calculated for $C_{18}H_{14}NOF$: C,77.40; H,5.05; N,5.02
Found : C,77.24; H,5.03; N,4.90

Preparation 25

α,α-Bis(4-chlorophenyl)-1-(phenylsulfonyl)-4-piperidinemethanol.

Following the procedure of Preparation 7, but substituting p-bromochlorobenzene for p-bromofluorobenzene, the title compound was prepared.

Preparation 26

4-[Bis(4-chlorophenyl)methylene]piperidine hydrobromide hydrate [1:1:1].

A mixture of 69.33 g (0.146 mole) of α,α-bis(4-chlorophenyl)-1-(phenylsulfonyl)-4-piperidinemethanol and 26 g (0.277 mole) of phenol in 400 ml of 48% hydrobromic acid was refluxed for 6 hr and then was stirred at room temperature for 10 hr. The reaction solution was decanted from a gum which had formed in the bottom of the reaction flask. The gum was washed with several portions of water and then was crystallized from ether to give a solid. The solid was recrystallized from a mixture of methanol-diethyl ether to give 26.52 g (43.6%) of white crystalline solid, m.p. 106-109°C.

| | | | |
|---|---|---|---|
| Analysis: Calculated for $C_{18}H_{20}NBrCl_2O$: | C,51.83; | H,4.83; | N,3.36 |
| Found: | C,52.13; | H,4.62; | N,3.38 |

Preparation 27

1-Chloro-4-(3-chloropropoxy)benzene.

A mixture of 77.2 g (0.60 mole) of p-chlorophenol, 189 g (1.2 mole) of 1-bromo-3-chloropropane, 249 g (1.8 mole) of anhydrous potassium carbonate, and 600 ml of acetone was stirred vigorously and heated to reflux for 16 hr under a nitrogen atmosphere. The potassium carbonate was removed by suction filtration, and the acetone and excess bromochloropropane were removed by heating under reduced pressure. The residue was dissolved in petroleum ether, and the resulting solution was cooled in an ice-isopropyl alcohol bath to produce a white solid. The solid was collected by filtration and washed with cold petroleum ether. The filtrate was concentrated and cooled to yield two more crops of white crystals. The combined solids were dried

under vacuum at ambient temperature to yield 107 g (87%) of white, flaky solid, m.p. 35-36°C.

Analysis: Calculated for $C_8H_{10}OCl_2$: C,52.71; H,4.92
Found : C,52.99; H,4.87

Preparation 28

4-(3-Chloropropoxy)benzoic acid methyl ester.

Ethyl 4-hydroxybenzoate 83.1 g (0.50 mole), 107 ml (1.0 mole) of 1-bromo-3-chloropropane, and potassium carbonate (1.5 mole, 207.3 g) were mechanically stirred in 600 ml of refluxing acetone under nitrogen overnight. The potassium carbonate was removed by filtration, and the filtrate was evaporated under reduced pressure to give 122 g of a liquid. This liquid was dissolved in 250 ml of petroleum ether and with stirring and cooling in an ice/ 2-propanol bath. A white precipitate formed and was collected by filtration and washed with cold petroleum ether to yield 108 g of a solid. An additional 6 g of the product was obtained from the mother liquor. A small sample of the solid was dissolved in petroleum ether at room temperature. The solution was stirred and cooled in an ice bath. White crystals were collected by filtration, washed with cold petroleum ether and dried under vacuum at room temperature, m.p. 24-25°C.

Analysis: Calculated for $C_{12}H_{15}O_3Cl$: C,59.39; H,6.23;
Found : C,59.69; H,6.30

Preparation 29

1-(3-Chloropropoxy)-4-nitrobenzene.

A mixture of 7.0 g (0.05 mole) of 4-nitrophenol, 15.7 g (0.1 mole) of 1-bromo-3-chloropropane and 20.7 g (0.15 mole) of anhydrous potassium carbonate in 350 ml of acetone was heated at reflux for 17 hr. The mixture was cooled, filtered, and the filtrate was concentrated to give an oil which crystallized. The solid was collected by filtration, washed with petroleum ether, and dried to yield 10.1 g (94%) of the title compound. An analytical sample was prepared from ethyl ether-petroleum ether, m.p. 37-39°C.

Analysis: Calculated for $C_9H_{10}ClNO_3$: C,50.13; H,4.67; N,6.50
Found : C,49.95; H,4.71; N,6.51

Preparation 30

4-[Bis(4-fluorophenyl)methyl]-1-piperidinepropanol oxalate monohydrate.

A mixture of 10.67 g (0.0372 mole) of 4-[bis(4-fluorophenyl)methyl]piperidine, 5.42 g (0.039 mole) of 3-bromo-1-propanol and 8 g (0.095 mole) of sodium bicarbonate in 400 ml of 1-butanol was refluxed for 21 hr. The solvent was removed *in vacuo*, and the residue was partitioned between methylene chloride and dilute sodium hydroxide. The methylene chloride solution was dried over magnesium sulfate and the solvent was removed *in vacuo* to give 8.88 g (67.3%) of oil, the free base of the title compound. A small sample of this oil was converted to the oxalate salt, and the salt was recrystallized from methanol-ether to give a white solid, m.p. 89-94°C. Overall yield was calculated to be 75.1%.

Analysis: Calculated for $C_{23}H_{29}NO_6F_2$: C,60.92; H,6.45; N,3.09
Found : C,61.49; H,6.15; N,3.03

Preparation 31

4-(3-Chloropropoxy)-3-methoxybenzoic acid methyl ester.

A mixture of 100 g (0.549 mole) of methylvanillate, 172.8 g (1.1 mole) of 1-bromo-3-chloropropane and 228 g (1.65 mole) of anhydrous potassium carbonate in 1 liter of acetone was heated at reflux for 20 hr. The mixture was cooled, filtered, and the filtrate concentrated to give a white solid as residue. The solid was triturated with petroleum ether, collected by filtration, and dried to yield 137.8 g (97%) of white powder which was recrystallized from isopropyl alcohol, m.p. 104-105°C.

Analysis: Calculated for $C_{12}H_{15}ClO_4$: C,55.71; H,5.84
Found : C,55.87; H,5.94

Preparation 32

4-[Bis(4-methoxyphenyl)methyl]pyridine.

Anisole, 108.13 g (1.0 mole) was cooled in an ice bath. Concentrated sulfuric acid, 115.3 ml (2.0 mole) was added while stirring the mixture in an ice bath. The temperature rose to 55°C. The reaction was then cooled in the ice bath. To this solution was added 4-pyridine carboxaldehyde, 53.5 g (0.5 mole). The temperature rose to 95°C. and further cooling and stirring brought the temperature down to 20°C. The reaction mixture was heated at 70°C. for 3-1/2 hr. The red gel was made alkaline with 50% sodium hydroxide-ice mix. The alkaline phase was extracted with toluene and the toluene extracted with a saturated sodium chloride solution. The product crystallized from the toluene solution while standing at room temperature. The white solid can be recrystallized from hot hexane-isopropyl alcohol.

A small 2.2 g sample of the product was recrystallized from methylene chloride-hexanes (1:9 v/v) and dried overnight at 80°C. in vacuo. This furnished 1.08 g (48.6% yield based on the aliquot taken) of white crystalline product in 49% yield, m.p. 111.5-113.5°C.

Analysis: Calculated for $C_{20}H_{19}NO_2$: C,78.66; H,6.27; N,4.59

Found : C,78.14; H,6.24; N,4.54

Preparation 33

4-[Bis(4-methoxyphenyl)methyl]piperidine hydrochloride hydrate [1:1:1].

The precursor pyridine derivative 4-[bis-4-methoxyphenyl) methyl]pyridine was prepared from the reaction of anisole and 4-pyridine carboxaldehyde in the presence of sulfuric acid.

To prepare the title compound, a solution of 4-[bis-4-methoxyphenyl)methyl]pyridine (70.8 g, 0.232 mole) in 350 ml of acetic acid was hydrogenated with 5% palladium on carbon (7.08 g) for five hours with heat. The hydrogenation was continued overnight at room temperature. The reaction mixture was filtered and rinsed with methanol. The filtrate was stripped of solvent via a rotary evaporator and the residue was partitioned between 5% sodium hydroxide and toluene. The aqueous layer was back extracted with toluene. The organic layer was dried over anhydrous sodium sulfate and filtered. Removal of solvent by means of a rotary evaporator gave 64 g (88.6%) of white solid, the free base. The free base was then converted to the hydrochloride salt by dissolving it in methanol and treating with ethereal hydrogen chloride. The white solid was filtered off and dried overnight at 80°C. *in vacuo* in the amount of 2.08 g (69.3%), m.p. 132-135°C.

Analysis: Calculated for $C_{20}H_{28}NO_3Cl$: C,65.65; H,7.71; N,3.83
Found : C,65.63; H,7.53; N,3.90

Preparation 34

4-[Bis(4-methylphenyl)methyl]piperidine hydrochloride [1:1].

The free base of the title compound was prepared by hydrogenation of 4-[(bis-4-methylphenyl)methyl]pyridine in acetic acid using palladium on carbon as catalyst and converted to the hydrochloride salt in methanol-diethyl ether. The salt was recrystallized from methanol-diethyl ether and isopropanol-diethyl ether and dried overnight *in vacuo* at 80°C. White solid amounting to 46% yield, m.p. 232°C. was obtained.

Analysis: Calculated for $C_{20}H_{26}NCl$: C,76.05; H,8.30; N,4.43
Found : C,75.51; H,8.33; N,4.33

## Preparation 35

N-[4-(3-Chloropropoxy)phenyl]acetamide.

A mixture of 4-acetamidophenol, 182.2 g (1.2 mole), bromochloropropane, 157.4 g (1.0 mole), and potassium carbonate, 145.0 g (1.05 mole) was refluxed overnight in 700 ml of acetone. The acetone solution was refrigerated overnight and white crystals formed. This white solid was filtered and washed with acetone. The filtrate was stripped to dryness and the residue was dissolved in chloroform and extracted with 5% sodium hydroxide. Removal of chloroform gave an oil. The white solid was also dissolved in chloroform and extracted with 5% sodium hydroxide. Removal of chloroform gave a white solid. The white solid and oil were combined and placed in acetone in the refrigerator; white crystals were obtained. The white crystals were recrystallized twice from acetone. A 5 g sample of the white crystals was recrystallized from acetone. This furnished 1.76 g (after drying *in vacuo* overnight at 80°C.) (23%) of white crystalline product, m.p. 125-127°C.

Analysis: Calculated for $C_{11}H_{14}NO_{2\,7}Cl$: C,58.03; H,6.20; N,6.15

Found : C,58.21; H,6.28; N,6.15

## Preparation 36

1-(3-Chloropropoxy)-3,5-dimethoxybenzene.

A mixture of 3,5-dimethoxyphenol, 100.0 g (0.6486 mole), chlorobromopropane, 148.0 g (0.96 mole) and potassium carbonate, 89.6 g (0.96 mole) was heated overnight at gentle reflux in 600 ml of acetone. The reaction mixture was cooled to room temperature, filtered, and stripped to dryness via a rotary evaporator. The resulting oil was dissolved in chloroform and the solution extracted with 5% aqueous sodium hydroxide; removal of chloroform gave a dark brown oil. A 5 g sample of the oil was pumped *in vacuo* overnight at 80°C. This produced 3.23 g (53.2% yield based on the aliquot taken) of dark brown oil.

H' ($CDCl_3$): $\delta$ 2-2.4 (quintuplet, center methylene protons, 2H), 3.6-4.2 (m, aliphatic protons, 4H), 3.8 (s, $OCH_3$, 6H),

6.1 (s, aromatic protons, 3H).

Analysis: Calculated for $C_{11}H_{15}O_3Cl$: C,57.27; H,6.56;
Found : C,56.96; H,6.49

Preparation 37

4-(3-Chloropropoxy)benzonitrile.

A mixture of 4-cyanophenol, 125.0 g (1.05 mole), bromochloropropane, 189.0 g (1.2 mole) and potassium carbonate, 145.0 g (1.05 mole) was heated overnight at reflux in 750 ml of acetone. The reaction mixture was filtered and stripped to dryness. The resulting residue was dissolved in chloroform and extracted with 5% sodium hydroxide. Removal of chloroform gave an oil which crystallized to give a white solid. A 5 g sample was recrystallized from isopropyl ether. This furnished 1.22 g (24.4% based on 5 g sample) of white solid, m.p. 40-44°C. which contained a dimer impurity.

Analysis: Calculated for $C_{10}H_{20}NOCl$: C,61.39; H,5.15; N,7.16
Found : C,61.57; H,5.14; N,7.20

Preparation 38

1-[4-(3-Chloropropoxy)-3-methylphenyl]ethanone.

A mixture of 25 g (0.166 mole) of 4-hydroxy-3-methylacetophenone, 45.8 g (0.33 mole) of 1-bromo-3-chloropropane and 69.1 g (0.5 mole) of anhydrous potassium carbonate in 500 ml of acetone was heated at reflux for 20 hr. The mixture was cooled, filtered, and the filtrate concentrated under reduced pressure to give an oil as residue. The oil was crystallized in petroleum ether. The solid was collected by filtration, washed with petroleum ether and dried to yield 35.8 g (95%) of an off-white powder. An analytical sample, m.p. 41.5-42.5°C., was prepared from petroleum ether.

Analysis: Calculated for $C_{12}H_{15}ClO_2$: C,63.58; H,6.67;
Found : C,63.40; H,6.64

Preparation 39

4-(3-Chloropropoxy)benzamide.

A mixture of 50 g (0.365 mole) of 4-hydroxybenzamide, 114.8 g (0.729 mole) of 1-bromo-3-chloropropane and 151.3 g (1.1 mole) of anhydrous potassium carbonate in 1 liter of acetone was heated at reflux for 20 hr. The mixture was concentrated under reduced pressure and the residue was stirred with 1.2 liter of water to remove inorganic solids. The mixture was filtered and the filter cake was washed with water and petroleum ether and dried to yield 75.5 g (97%) of a white solid. The solid was recrystallized from ethyl acetate, m.p. 142-143°C.

Analysis: Calculated for $C_{10}H_{12}ClNO_2$: C,56.22; H,5.66; N,6.56
Found : C,55.92; H,5.61; N,6.56

Preparation 40

1-[4-(5-Chloropentoxy)-3-methoxyphenyl]ethanone.

A mixture of 59.7 g (0.36 mole) of acetovanillone, 100 g (0.539 mole) of 1-bromo-5-chloropentane and 138 g (1 mole) of anhydrous potassium carbonate in 1 liter of acetone was heated at reflux for 20 hr. The mixture was filtered and the filtrate was concentrated under reduced pressure to give an oil which crystallized in petroleum ether (30-60°C.). The solid was collected by filtration, washed with petroleum ether and dried to yield 81.4 g (84%) of fluffy, white solid. The solid was recrystallized from isopropyl ether, m.p. 57-58°C.

Analysis: Calculated for $C_{14}H_{19}ClO_3$: C,62.11; H,7.07;
Found : C,62.14; H,7.10

Preparation 41

4-(3-Chloropropoxy)-3-methoxybenzeneacetic acid ethyl ester.

A mixture of 50 g (0.238 mole) of ethyl homovanillate, 75 g (0.476 mole) of 1-bromo-3-chloropropane and 98.7 g (0.71 mole) of anhydrous potassium carbonate in 1 liter of acetone was heated at reflux for 24 hr. The mixture was filtered and the filtrate was concentrated under reduced

pressure to give an oil which gradually crystallized to a semi-solid. The solid was recrystallized from ethyl ether-petroleum ether (30-60°C.) to yield 44.4 g (65%) of white solid, m.p. 36-38°C.

Analysis: Calculated for $C_{14}H_{19}ClO_4$: C,58.64; H,6.68
Found : C,58.74; H,6.74

Preparation 42

1-(3-Chloropropoxy)-4-(methylsulfonyl)benzene.

To a solution of 21.7 g (0.1 mole) of 1-(3-chloropropoxy)-4-(methylthio)benzene in 100 ml of chloroform was cautiously added a slurry of 51.8 g (0.3 mole) of m-chloroperbenzoic acid in 450 ml of chloroform. The mixture was stirred at ambient temperature for 2 days and then filtered. The filtrate was washed with four portions of a solution comprised of 110 ml of saturated sodium bicarbonate, 110 ml of water, and 30 ml of 20% sodium hydroxide, once with brine, dried (sodium sulfate) and concentrated under reduced pressure to give a solid as residue. The solid was triturated with petroleum ether, collected by filtration and air dried to yield 24.3 g (98%) of white solid. An analytical sample, m.p. 84-86°C. was recrystallized from 2-propanol.

Analysis: Calculated for $C_{10}H_{13}ClO_3S$: C,48.29; H,5.27
Found : C,48.38; H,5.30

Preparation 43

5-Oxo-1-(phenylmethyl)-3-pyrrolidinecarboxylic acid, methyl ester.

A solution of 158.2 g (1.0 mole) of dimethylitaconate and 107.2 g (1.0 mole) of benzylamine in 750 ml of methanol was let stand at ambient temperature over the weekend. The solution was filtered and the filtrate was concentrated under reduced pressure to give an oil as residue. The oil crystallized when it was triturated with petroleum ether (30-60°C.). The solid was collected by filtration and dried to yield 225.5 g (97%) of white powder. An analytical sample, m.p. 63-65°C. was prepared from diisopropyl ether.

Analysis: Calculated for $C_{13}H_{15}NO_3$: C,66.94; H,6.48; N,6.01
Found : C,66.82; H,6.48; N,6.01

## Preparation 44

### 1-Benzyl-3-hydroxymethyl-pyrrolidine oxalate [1:1].

A solution of (60.0 g, 0.2553 mole) 5-oxo-1-(phenylmethyl)-3-pyrrolidinecarboxylic acid methyl ester in dry dimethoxyethane was added to a mixture of dimethoxyethane and 47.0 g (1.23 mole) of lithium aluminum hydride. The reaction mixture was stirred 2 hrs at room temperature and then heated at reflux 2 hrs. The mixture was then stirred overnight at room temperature, then quenched by the slow addition of ethyl acetate. More ethyl acetate was added and the use of celite allowed the solid material to be separated from filtrate by filtration. The filtrate was stripped to dryness and dissolved in chloroform. The chloroform layer was extracted with 10% sodium hydroxide. The chloroform layer was dried, filtered, and solvent removed to give an oil. A portion of the oil was converted to the oxalate salt. The salt was recrystallized from methanol-diethyl ether and dried at 80°C. *in vacuo* overnight to give 2.27 g (39.4% yield based on aliquot taken) of white crystalline solid, m.p. 98-102°C.

Analysis: Calculated for $C_{14}H_{19}NO_5$: C,59.78; H,6.81; N,4.98
Found : C,59.43; H,6.79; N,4.95

## Preparation 45

### 1-[4-(6-Chlorohexyloxy)-3-methoxyphenyl]ethanone.

A mixture of 41.6 g (0.25 mole) of acetylvanillone, 76 g (0.375 mole) of 1-bromo-6-chlorohexane and 103.7 g (0.75 mole) of anhydrous potassium carbonate in 750 ml of acetone was heated at reflux 20 hr. The mixture was cooled, filtered, and the filter cake washed with acetone. The combined filtrates were concentrated under vacuum pump pressure at 90°C. to give an oil which gradually crystallized. The residue was triturated with petroleum ether (30-60°C.), collected by filtration,and dried to yield 59.6 g (84%) of off-white solid. An analytical sample, m.p. 35-38°C., was prepared from isopropyl ether.

Analysis: Calculated for $C_{15}H_{21}ClO_3$: C,63.26; H,7.43;
Found : C,63.50; H,7.60

## Preparation 46

### 4-(3-Chloropropoxy)benzenesulfonamide.

A mixture of 25 g (0.144 mole) of p-hydroxybenzenesulfonamide, 45.5 g (0.289 mole) of 1-bromo-3-chloropropane and 59.7 g (0.432 mole) of anhydrous potassium carbonate in 500 ml of acetone was heated at reflux for 24 hr. The mixture was cooled, filtered and the filtrate concentrated under vacuum pump pressure at 90°C. to give 32.2 g of tan gum as residue. The gum was purified by column chromatography on 600 g of silica gel. Fractions containing the title compound eluted with 8% acetone in benzene were combined and concentrated under reduced pressure to yield 12.2 g (34%) of white solid; m.p. 105-107.5 on recrystallization from 2-propanol.

Analysis: Calculated for $C_9H_{12}NO_3S$: C,43.29; H,4.84; N,5.61
Found : C,43.48; H,4.92; N,5.62

## Preparation 47

### 7-(3-Chloropropoxy)-2H-1-benzopyran-2-one.

A mixture of 16.8 g (0.104 mole) of 7-hydroxycoumarin, 31.6 g (0.2 mole) of 1-bromo-3-chloropropane and 41.5 g (0.3 mole) of anhydrous potassium carbonate in 500 ml of acetone was heated at reflux for 24 hr. The mixture was filtered with difficulty to give a milky filtrate. The filtrate was treated with charcoal and filtered through celite to give a clear filtrate. The filtrate was concentrated under reduced pressure to give a solid residue. The solid was triturated with petroleum ether (30-60°C.), collected by filtration, and dried to yield 19.1 g (77%) of fluffy, white solid. An analytical sample, m.p. 100-102°C., was obtained on recrystallization from 2-propanol.

Analysis: Calculated for $C_{12}H_{11}ClO_3$: C,60.39; H,4.65
Found : C,60.35; H,4.68

Preparation 48

7-(3-Chloropropoxy)-4-oxo-4H-1-benzopyran-2-carboxylic acid ethyl ester.

A mixture of 23.4 g (0.1 mole) of 7-hydroxy-4-oxo-4H-1-benzopyran-2-carboxylic acid ethyl ester, 31.6 g (0.2 mole) of 1-bromo-3-chloropropane and 41.5 g (0.3 mole) of anhydrous potassium carbonate in 500 ml of acetone was heated at reflux for 20 hr. The mixture was cooled and filtered through Celite. The filtrate was concentrated under reduced pressure to give a solid residue. The solid was triturated with petroleum ether (30-60°C.), collected by filtration, and recrystallized from 2-propanol to yield 22.5 g (73%) of white solid, m.p. 107-108°C.

Analysis: Calculated for $C_{15}H_{15}ClO_5$: C,57.98; H,4.87
Found : C,58.21; H,4.88

Preparation 49

1-[4-(3-Chloropropoxy)-2-methoxyphenyl]ethanone.

A mixture of 10.6 g (0.637 mole) of 1-(4-hydroxy-2-methoxyphenyl)ethanone, 20 g (0.127 mole) of 1-bromo-3-chloropropane and 26.4 g (0.19 mole) of anhydrous potassium carbonate in 250 ml of acetone was heated at reflux for 20 hr. The mixture was cooled, filtered and the filtrate concentrated under vacuum pump pressure at 90°C. to give an oil which gradually crystallized. The solid was triturated with petroleum ether (30-60°C.), collected by filtration and dried to yield 14.6 g (94%) of white solid, m.p. 47-49°C. on recrystallizing from isopropyl ether.

Analysis: Calculated for $C_{12}H_{15}ClO_3$: C,59.39; H,6.23
Found : C,59.32; H,6.26

Preparation 50

1-(3-Chloropropoxy)-4-sulfinylbenzene.

The title compound is prepared by treating 1-(3-chloropropoxy)-4-methylthiobenzene with sodium perborate in glacial acetic acid.

Preparation 51

2-(3-Chloropropoxy)benzonitrile.

A mixture of 2-cyanophenol (50.0 g, 0.42 mole), 1-bromo-3-chloropropane (67.7 g, 0.43 mole), and potassium carbonate (58.0 g, 0.42 mole) was heated overnight at gentle reflux in 500 ml of acetone. The reaction mixture was stripped to dryness and the residue was dissolved in chloroform. The chloroform layer was extracted several times with 5% sodium hydroxide. The chloroform layer was dried (anhydrous sodium sulfate), filtered, and the solvent was removed in vacuo to give a brown oil (80.09 g). A ten gram portion of this oil was subjected to flash chromatography on silica gel with 10% ethyl acetate-hexanes and 20% ethyl acetate-hexanes used for elution. Fractions were combined and solvent removed in vacuo. The clear oil obtained was dried 18 hrs in vacuo at room temperature and 8 hrs at 80°C. in vacuo. This furnished 5.24 g (50.0% yield - based on aliquot taken) of clear oil. $^1H$ NMR ($CDCl_3$); $\delta$ 2.1-2.5 (q, 2, $-CH_2$), 3.8 (t, 2, $-ClCH_2$), 4.2 (t, 2, $-OCH_2$), 6.9 (m, 2, aromatic protons ortho and para to ether), 7.5 (m, 2, aromatic protons ortho and para to CN group).

| | | | | |
|---|---|---|---|---|
| Analysis: | Calculated for $C_{10}H_{10}NOCl$: | C,61.39; | H,5.15; | N,7.16 |
| | Found : | C,61.27; | H,5.15; | N,7.14 |

Preparation 52

1-Phenylmethyl-3-pyrrolidinemethanol methanesulfonate (ester) oxalate [1:1].

A solution of 113.80 g (0.596 mole) of 1-benzyl-3-hydroxymethylpyrrolidine and triethylamine, 66.6 g (0.66 mole) in 600 ml of acetonitrile was prepared. This solution was cooled in an ice bath. A solution of tosyl chloride, 125.9 g (0.66 mole) in 300 ml of acetonitrile was added dropwise with stirring. The solution was allowed to stir overnight at room temperature. A solid precipitated and the solution was filtered. The solvent was removed by rotary evaporator and the residue was dissolved in chloroform. The chloroform layer was extracted with 5% sodium hydroxide and water. The chloroform layer was dried (anhydrous sodium sulfate),

filtered, and solvent removed to give 232.9 g of a dark brown oil. This oil was converted to the oxalate salt and recrystallized from methanol-diethyl ether. After drying at 80°C. *in vacuo* overnight, 181.63 g of white crystalline solid was obtained. A five gram sample was recrystallized again from methanol-diethyl ether and dried at 80°C. *in vacuo* overnight. A yield of 1.41 g (19.7% overall adjusted for the aliquot taken) of white crystalline solid, m.p. 147-149°C. was obtained.

Analysis: Calculated for $C_{21}H_{25}NO_7S$: C,57.92; H,5.79; N,3.22
Found : C,57.62; H,5.82; N,3.22

## Preparation 53

### α,α-Diphenyl-3-pyrrolidineacetamide maleate [1:1].

A 1.13 g sample of 1-benzyl-α,α-diphenyl-3-pyrrolidineacetamide dissolved in 50 ml of methanol was hydrogenated with 0.5 g of 10% palladium-on-charcoal catalyst at 75°C. overnight in a Parr hydrogenation apparatus. After removal of the catalyst by filtration the filtrate was concentrated to give 0.783 g (80%) of light tan gum. The mass spectra and infrared spectra were consistent with its structure. A sample of this free base in methanol was treated with one molar equivalent of a solution of maleic acid in methanol. After evaporation of the methanol, the residue crystallized and was recrystallized twice from isopropanol-ether. The material was dried at 110°C./0.1 mm for 3 hr, m.p. 110-145°C. (softens and turns liquid). The material appears to be an amorphous solid.

Analysis: Calculated for $C_{22}H_{24}N_2O_5$: C,66.65; H,6.10; N,7.07
Found : C,66.79; H,6.05; N,7.04

## Preparation 54

### α,α-Diphenyl-3-pyrrolidineacetamide N-cyclohexylsulfamate hydrate [1:1:1.5].

A 1.15 g sample of free base of the above compound obtained by proportioning α,α-diphenyl-3-pyrrolidineacetamide maleate in chloroform and aqueous basic solution and evaporating the chloroform layer and 0.735 g of hexamic acid

were dissolved in 10 ml of ethanol. The solvent was evaporated, the residue crystallized, and then was recrystallized from ethanol, m.p. 103-106°C.

Analysis: Calculated for $C_{24}H_{33}N_3SO_4 \cdot 1.5\ H_2O$: C,59.24; H,7.46; N,8.64
Found :C,58.97; H,6.98; N,8.51

Preparation 55

1-(Phenylmethyl)-4-piperidinol ester with 4-methylbenzenesulfonic acid maleate [1:1].

A solution of 100 g (0.524 mole) of N-benzyl-4-hydroxypiperidine and 13 g (0.684 mole) of tosylchloride in 600 ml of pyridine was stirred at room temperature overnight. One liter of methylene chloride and 500 ml of 0.5 M aqueous sodium hydroxide were added to the reaction mixture. The reaction mixture was stirred for 10 min and the phases were separated. The methylene chloride layer was extracted with several portions of dilute sodium hydroxide, dried over magnesium sulfate and evaporated _in vacuo_ to give an oil, the free base of the title compound. The free base was converted to the maleate salt, which was recrystallized from methylene chloride-diethyl ether to give white crystalline solid, m.p. 159-160°C.

Analysis: Calculated for $C_{23}H_{27}NO_7S$: C,59.86; H,5.90; N,3.04
Found : C,59.79; H,5.86; N,2.95

Preparation 56

1-[2-(Phenylthio)ethyl]-4-piperidinecarboxylic acid ethyl ester hydrochloride [1:1].

A mixture of 69.3 g (0.40 mole) of 2-chloroethylphenylsulfide, 61.65 g (0.393 mole) of ethyl isonipecotate and 53 g (0.50 mole) of sodium carbonate in 1 liter of absolute ethanol was refluxed for 30 hr in the presence of molecular 3A sieves. The reaction mixture was filtered, and the solvent was removed _in vacuo_ from the filtrate. The residue was partitioned between methylene chloride and dilute sodium hydroxide, and the methylene chloride solution was dried over anhydrous sodium sulfate. The solvent was removed _in vacuo_ to give the free base of the title compound as an oil.

The free base was converted to the hydrochloride salt, and the salt was recrystallized from absolute ethanol-ether to give 38.62 g (29.8%) of white crystalline solid, m.p. 125-126°C.

Analysis: Calculated for $C_{16}H_{24}NO_2SCl$: C,58.26; H,7.33; N,4.25
Found : C,58.11; H,7.32; N,4.20

Preparation 57

1-[(4-Methylphenyl)sulfonyl]-4-piperidinol ester with 4-methylbenzenesulfonic acid.

A solution of 1.63 g (0.016 mole) of 4-hydroxypiperidine and 13.9 g (0.0732 mole) of tosyl chloride in 80 ml of pyridine was stirred overnight at 25°C. The mixture was quenched in 200 ml of water and the aqueous mixture was extracted with several portions of methylene chloride. The combined methylene chloride layer was extracted with several portions of 1 M sulfuric acid followed by 1 M sodium hydroxide and dried over magnesium sulfate. The solvent was removed in vacuo to give a solid. The solid was recrystallized from methylene chloride-diethyl ether to give 4.82 g (73.3%) of the product, m.p. 140.5-141°C.

Analysis: Calculated for $C_{19}H_{23}NO_5S_2$: C,55.73; H,5.66; N,3.42
Found : C,55.60; H,5.64; N,3.39

Preparation 58

1-[(4-Methylphenyl)sulfonyl]-α,α-diphenyl-4-piperidineacetonitrile.

The sodium salt of diphenylacetonitrile was formed in toluene from diphenylacetonitrile, 94.5 g (0.488 mole) and sodium hydride, 19.6 g (0.488 mole). The reaction mixture was heated at reflux for approximately two hours. A color change from green to brown was detected during the reaction. 1-[(4-Methylphenyl)sulfonyl]-4-piperidinol ester with methylbenzenesulfonic acid (200.0 g, 0.488 mole) was added in small portions as a solid while stirring the reaction mixture under nitrogen at room temperature. The solution became green in color. The solution/mixture was stirred overnight at 100°C. The mixture was filtered and the toluene was removed by rotary evaporation.

The filter cake and the residue from removal of toluene were combined and dissolved in chloroform. The chloroform was extracted several times with 5% sodium hydroxide followed by extraction with 1N sulfuric acid and sodium hydroxide. The chloroform was removed *in vacuo* to give a reddish-brown oil.

A sample of the oil was crystallized from toluene and washed with isopropyl ether. The solid obtained was then recrystallized from methylene chloride-hexanes and dried at 80°C *in vacuo* overnight to give white solid title compound in 26.7% yield based on aliquot taken, m.p. 183-184°C.

Analysis: Calculated for $C_{26}H_{26}N_2O_2S$: C,72.53; H,6.09; N,6.51

Found : C,72.11; H,6.07; N,6.45

Preparation 59

α,α-Diphenyl-4-piperidineacetonitrile oxalate [2:1].

A solution of 1-[(4-methylphenyl)sulfonyl]-α,α-diphenyl-4-piperidineacetonitrile (183.83 g, 0.428 mole) and phenol (150.0 g, 1.60 mole) in 750 ml of 48% hydrobromic acid was stirred vigorously and heated at reflux for 3-1/2 hrs. The reaction mixture was cooled and made alkaline with ice/50% sodium hydroxide. The reaction mixture was extracted with chloroform, and the chloroform layer was back extracted with 5% sodium hydroxide. The chloroform layer was dried, filtered over anhydrous sodium sulfate and solvent removed to furnish a red oil. This oil, the free base of the title compound, was converted to the oxalate salt using methanol-diethyl ether mixture. A sample of the oxalate salt was recrystallized from methanol-diethyl ether to give white crystalline product in 17.9% yield, m.p. 275-276°C.

Analysis: Calculated for $C_{20}H_{21}N_2O_2$: C,74.74; H,6.59; N,8.72

Found : C,74.51; H,6.52; N,8.60

## Preparation 60

α,α-Diphenyl-4-piperidineacetamide fumarate [2:3].

α-α-Diphenyl-4-piperidineacetonitrile oxalate [2:1] 60.34 g (0.165 mole) was converted to the free base by partitioning in 5% sodium hydroxide and chloroform. Removal of chloroform from the chloroform layer gave an oil which was then dissolved in a mixture of 280 ml concentrated sulfuric acid and 30 ml of water. The solution was stirred overnight at 90°C. The reaction mixture was poured into ice and carefully made alkaline with 50% sodium hydroxide. The aqueous layer was extracted several times with chloroform. The chloroform layer was dried over anhydrous sodium sulfate, filtered, and solvent removed to give an oil which cyrstallized. A two gram sample of the oil was converted to the fumarate salt and the salt was recrystallized from methanol-diethyl ether. A white solid was obtained (47.9% yield based on aliquot taken) which was dried overnight in vacuo at 80°C., m.p. 234-235°C.

Analysis: Calculated for $C_{25}H_{28}N_2O_7$: C,64.09; H,6.02; N,5.98
Found : C,63.82; H,6.14; N,5.82

## Preparation 61

1-[(4-Methylphenyl)sulfonyl]-α,α-diphenyl-3-piperidine-propanenitrile.

The sodium salt of diphenylacetonitrile was formed in 400 ml of dimethylsulfoxide from sodium hydride (60%, 39.0 g, 0.975 mole) and diphenylacetonitrile (188.90 g, 0.975 mole). The resulting solution was stirred under nitrogen for one hr at room temperature. A 90-10 mixture of 3-(chloromethyl)-1-[(4-methylphenyl)sulfonyl]piperidine and 4-methylbenzenesulfonic acid 1-[(4-methylphenyl)sulfonyl]piperidin-3-yl methyl ester, 221.42 g (0.975 mole) dissolved in 400 ml of dimethylsulfoxide was added. The reaction mixture was heated to 85°C. and stirred overnight at 73°C. The dimethylsulfoxide was removed in vacuo and the residue obtained was dissolved in chloroform. The chloroform layer was extracted with 1N sulfuric acid. The chloroform layer was dried, filtered, and the chloroform was removed by rotary evaporator. A brown residue was obtained which was triturated with isopropyl

ether to give a brown solid. A five gram sample was recrystallized from ethyl acetate-isopropyl ether. This provided four grams (56.8% yield based on aliquot taken) of white solid, m.p. 136.5-137°C.

| | | |
|---|---|---|
| Analysis: Calculated for $C_{27}H_{28}N_2O_2S$: | C,72.94; | H,6.35; N,6.30 |
| Found : | C,72.82; | H,6.36; N,6.28 |

## Preparation 62

### α,α-Diphenyl-3-piperidinepropanenitrile fumarate [1:1].

A mixture of 302.41 g (0.68 mole) of 1-[(4-methylphenyl) sulfonyl]-α,α-diphenyl-3-piperidinepropanenitrile, hydrogen bromide (48%, 750 ml), and phenol (260 g, 2.76 mole) was stirred vigorously while heating at reflux for 3-1/2 hr. The reaction mixture was cooled to room temperature and made alkaline with 50% sodium hydroxide-ice. The aqueous phase was extracted several times with chloroform, and the chloroform layer was back extracted with 5% sodium hydroxide. The chloroform layer was dried, filtered, and solvent removed. NMR analysis showed about 80% product was obtained, so the same reaction sequence was repeated. The chloroform layer gave a brown oil which was converted to the oxalate salt. A portion of this oxalate salt was converted to the free base by partitioning in chloroform and dilute aqueous sodium hydroxide and separating and evaporating the chloroform layer and converted to the fumarate salt. The salt was recrystallized from methanol-diethyl ether and dried *in vacuo* at 80°C. overnight to give 6.53 g of white crystalline product, m.p. 181-182°C.

| | | |
|---|---|---|
| Analysis: Calculated for $C_{24}H_{26}N_2O_4$: | C,70.92; | H,6.45; N,6.89 |
| Found : | C,70.46; | H,6.41; N,6.85 |

## Preparation 63

### α,α-Diphenyl-3-piperidinepropanamide maleate [1:1].

A solution of 52.01 g (0.179 mole) of α,α-diphenyl-3-piperidinepropanenitrile was stirred overnight at 85°C. in 280 ml of 90% sulfuric acid. The reaction mixture was allowed to cool to room temperature and then poured into 50% sodium hydroxide/ice mix. The basic layer was extracted with chloroform. The chloroform layer was dried, filtered, and the solvent removed to give a fluffy solid, the free base of

the title compound. A 3 g portion of the free base was converted to the maleate salt and recrystallized from methanol-diethyl ether. The salt obtained was dried *in vacuo* overnight at 80°C. This furnished 2.15 g of white crystalline product, m.p. 177-179°C.

Analysis: Calculated for $C_{24}H_{28}N_2O_5$: C,67.91; H,6.65; N,6.60
Found : C,67.85; H,6.85; N,6.55

Preparation 64

1-[(2-Chloroethyl)sulfonyl]-4-fluorobenzene.

A solution of 30% hydrogen peroxide (153 g, 1.34 mole) in 400 ml of glacial acetic acid was prepared. To this ice cooled solution was added a solution of 2-chloroethyl p-fluorophenylsulfide (70.11 g, 0.369 mole) in 200 ml of glacial acetic acid. The resulting solution was stirred 72 hours at room temperature. The volume of acetic acid was concentrated on a rotary evaporator. The residual material was dissolved in chloroform and extracted with a solution of sodium bicarbonate and sodium sulfite. The chloroform layer was then dried, filtered, and solvent removed to give a white solid. A 5 g portion of this white solid was recrystallized from methylene chloride-isopropyl ether. The white solid was dried *in vacuo* at 80°C. overnight. This furnished 1.84 g (32.6% yield base on aliquot taken) of white crystalline solid, m.p. 72.5-74°C.

Analysis: Calculated for $C_8H_8SO_2FCl$: C,43.15; H,3.62;
Found : C,43.52; H,3.66

Preparation 65

4-Fluoro-α-(4-fluorophenyl)benzeneacetonitrile.

4-Fluorophenylacetonitrile (70.0 g, 62.2 ml, d=1.126, 0.518 mole) was heated to 120°C. Bromine (83.0 g, 26.6 ml d=3.119, 0.525 mole) was added dropwise over 1 hr while maintaining a temperature of 120°C. The solution was stirred for 1/2 hr at 120°C. and then flushed vigorously with nitrogen for 3/4 hr (solution A).

In a separate 2-liter flask was placed aluminum chloride (85.0 g, 0.644 mole). Fluorobenzene (200 g, 2.08 mole d=1.024, 195.3 ml) was added dropwise with stirring over

1/2 hr while flushing with nitrogen (Mixture B).

Solution A was added dropwise to Mixture B starting at room temperature. The temperature rose to 50°C. The reaction was stirred at this temperature for 1/3 hr. The temperature was raised to 70°C. and maintained there for 1/3 hr. The reaction became vigorous and a part of the material was lost. The mixture which could be recovered was added to ice/75 ml of concentrated hydrochloric acid. The aqueous phase was extracted several times with chloroform. The solvent layer was dried, filtered, and solvent removed to give a green solid. The solid was recrystallized from isopropanol; the solid was washed with cold isopropanol twice and dried *in vacuo* at 55°C. overnight. This produced 29.72 g (25.1%) of light yellow solid, m.p. 62-63.5°C.

Analysis: Calculated for $C_{14}H_9NF_2$: C,73.36; H,3.96; N,6.11
Found : C,73.55; H,3.88; N,6.10

Preparation 66

α,α-Bis(4-fluorophenyl)-1-[(4-methylphenyl)sulfonyl]-4-piperidineacetonitrile.

The sodium salt of 4-fluoro-α-(4-fluorophenyl)benzeneacetonitrile was prepared in dimethyl sulfoxide from its free base, 28.0 g (0.1223 mole) and 4.90 g of 60% sodium hydride (0.1227 mole). The salt was stirred for 1 hr at room temperature. To the mixture was added 50.0 g (0.1223 mole) of 1-[(4-methylphenyl)sulfonyl]-4-piperidinol ester with 4-methylbenzenesulfonic acid over five minutes in solid form while stirring under nitrogen. The resulting solution was stirred 15 hours at 65°C. and then allowed to stand at room temperature for 72 hours. The solution was stripped to dryness, and the residue was dissolved in chloroform and extracted several times with 5% sodium hydroxide. The chloroform layer was dried (anhydrous sodium sulfate), filtered, and solvent removed to give 111.36 g of solid. The solid was triturated with isopropyl ether and placed in the freezer. After washing the solid several times with isopropyl ether, 55.41 g of white solid was obtained. A 3 g sample was then triturated with 50-50 (v/v) hot

isopropyl alcohol-methanol and placed in the freezer. The white solid collected was washed with isopropyl ether and dried in vacuo at 80°C. overnight. This produced 2.28 g of white crystalline product, m.p. 190-191°C.

Analysis: Calculated for $C_{26}H_{24}N_2O_2SF_2$: C,66.94; H,5.18; N,6.00

Found : C,66.92; H,5.17; N,5.99

## Preparation 67

α,α-Bis(4-fluorophenyl)-4-piperidineacetonitrile cxalate, diethyl ether [1:1:0.5].

A solution of 52.41 g (0.1125 mole) of α,α-bis-(4-fluorophenyl)-1-[(4-methylphenyl)sulfonyl]-4-piperidine acetonitrile was heated at reflux for 3-1/2 hr in 200 ml of 48% hydrobromic acid with phenol (50.0 g, 0.53 mole). The reaction mixture was cooled to room temperature and then made alkaline with ice/50% sodium hydroxide mixture. The alkaline phase was extracted several times with chloroform. The chloroform layer was back extracted with 5% sodium hydroxide. The chloroform layer was dried (anhydrous sodium sulfate), filtered, and solvent removed to give 34.24 g of dark brown oil. The entire oil was converted to the oxalate salt and recrystallized from methanol-diethyl ether. The salt obtained was dried in vacuo overnight at 80°C. to give 34.24 g (69.3%) of white crystalline solid, m.p. 124-127°C.

Analysis: Calculated for $C_{23}H_{25}N_2F_2O_{4.5}$: C,62.86; H,5.73; N,6.37

Found : C,62.30; H,5.78 N,6.17

## Preparation 68

N-[3-(3-Chloropropoxy)phenyl]urea.

A mixture of 45.6 g (0.3 mole) of 1-(3-hydroxyphenyl) urea, 94.5 g (0.6 mole) of 1-bromo-3-chloropropane, 124.4 g (0.9 mole) of anhydrous potassium carbonate and 1 liter of acetone was heated at reflux with mechanical stirring for 20 hrs. The mixture was concentrated and the residue was slurried with 1.5 liter of water. The mixture was filtered and the filter cake was recrystallized from isopropanol to

yield 57.0 g (83%)of off-white solid, m.p. 141-143°C.

Analysis: Calculated for $C_{10}H_{13}ClN_2O_2$: C,52.52; H,5.73; N,12.25

Found : C,52.37; H,5.79; N,12.17

Preparation 69

N-[4-(3-Chloropropoxy)phenyl]carbamic acid ethyl ester.

A mixture of 6.6 g (0.036 mole) of 4-hydroxyphenyl) carbamic acid ethyl ester, 11.5 g (0.072 mole) of 1-bromo-3-chloropropane, 13.8 (0.10 mole) of anhydrous potassium carbonate and 150 ml of acetone was heated at reflux for 21 hours. The mixture was cooled and filtered. The filtrate was concentrated under reduced pressure to give a solid residue. The solid was triturated with petroleum ether (30-60°C.), collected by filtration and recrystallized from isopropanol to yield 7.7 g (83%) of white solid, m.p. 91-93°C.

Analysis: Calculated for $C_{12}H_{16}ClNO_3$: C,55.93; H,6.26; N,5.43

Found : C,55.93; H,6.28; N,5.46

Preparation 70

α-(4-Fluorophenyl)-2-pyridineacetonitrile.

A sample of sodium hydride (60%, 1.60 g, 0.04 mole) was washed with dry hexanes. After removal of hexanes a 100 ml portion of dimethyl sulfoxide was added. To this mixture was added a solution of 4-fluorophenylacetonitrile (5.41 g, 0.04 mole). The mixture was stirred 3 hours at room temperature under nitrogen. 2-Bromopyridine (6.32 g, 0.04 mole) was added to the mixture, and the reaction mixture was then stirred overnight at 65°C. The reaction mixture was poured into 1.2 liters of water and the aqueous phase was extracted several times with chloroform (the chloroform layer was filtered using Celite). The combined chloroform layer was extracted with water and 5% sodium hydroxide. The chloroform layer was dried over sodium sulfate, filtered, and solvent removed to give a red oil. The oil was subjected to flash chromatography on silica gel using 10% ethyl acetate-90% hexanes and 20% ethyl

acetate-80% hexanes for elution. Fractions of similar purity were combined and solvent removed *in vacuo*. The oil obtained was dried *in vacuo* overnight at 80°C. to give 2.43 g (28.6%) of clear oil.

$^1H$ ($CDCl_3$): $\delta$ 8.5 (m,1, proton adjacent to N in pyridine nucleus), 6.8-7.8 (m, 7, aromatics), 5.3 (s, 1, methine).

Analysis: Calculated for $C_{13}H_9N_2F$: C,73.57; H,4.27; N,13.20
Found : C,73.23; H,4.23; N,13.12

## Preparation 71

### α-(4-Fluorophenyl)-α-[1-[(4-methylphenyl)sulfonyl]-4-piperidinyl]-2-pyridineacetonitrile hemihydrate.

The sodium salt of the free base of α-(4-fluorophenyl)-2-pyridineacetonitrile was formed in dimethyl sulfoxide from sodium hydride (60%, 5.16 g, 0.129 mole) and the free base of α-(4-fluorophenyl)-2-pyridineacetonitrile (27.36 g, 0.129 mole). The salt was stirred in dimethylsulfoxide 4-1/2 hr at room temperature. Next, 4-methylphenylsulfonic acid ester with 1-[(4-methylbenzene)sulfonyl]-4-piperidinol, (52,8 g, 0.129 mole) was added and the reaction mixture was stirred 2 hours at room temperature. The reaction mixture was stirred overnight at 80°C. The solvent was removed *in vacuo* and the residue obtained was dissolved in chloroform. The chloroform was extracted with water and 5% sodium hydroxide. The chloroform layer was dried over sodium sulfate and filtered. Solvent was removed to give a dark brown residue. This material was triturated with acetone to give 36.2 g of white solid. A one gram portion was triturated with acetone and then recrystallized from methylene chloride-acetone. The solid was dried *in vacuo* overnight at 80°C. to give 0.74 g (62.4% based on aliquot taken) of white crystals, m.p. 228-229°C.

Analysis: Calculated for $C_{25}H_{25}N_3O_{2.5}SF$: C,65.90; H,5.49; N,9.16
Found : C,65.86; H,5.27; N,9.16

Preparation 72

α-(4-Fluorophenyl)-α-(4-piperidinyl)-2-pyridine-acetonitrile oxalate [2:3].

A solution of α-(4-fluorophenyl)-α-[1-[(4-methylphenyl) sulfonyl]-4-piperidinyl]-2-pyridineacetonitrile, (30.86 g, 0.0687 mole) and phenol (75 g, 0.8 mole) in 200 ml of 48% hydrobromic acid was heated at reflux for 3 hours. The mixture was cooled in ice and made alkaline with ice - 50% sodium hydroxide. The aqueous layer was extracted with chloroform and the chloroform layer was extracted with 5% sodium hydroxide. The chloroform layer was dried over sodium sulfate, filtered, and solvent removed to give a dark brown oil. The entire oil was converted to the oxalate salt in methanol-diethyl ether. A one gram portion was taken and recrystallized from methanol-diethyl ether and dried *in vacuo* at 80°C. overnight. This furnished 0.90 g (80.2% based on aliquot taken) of white crystalline, m.p. 98°C. (softened 70°C.).

Analysis: Calculated for $C_{21}H_{21}N_3O_8F$: C,58.60; H,4.92; N,9,76

Found : C,58.77; H,5.01 N,10.04

Preparation 73

3-(8-Quinolinyloxy)-1-propanol.

A solution of 8-hydroxyquinoline (36.0 g, 0.25 mole) and potassium tert-butoxide (28.0 g, 0.25 mole) in 80 ml of dimethyl sulfoxide was stirred for 1 hour at room temperature. 3-Chloro-1-propanol (24.0 g, 0.25 mole) was added and the solution was heated overnight at 70°C. The solution was poured into 500 ml of water. A brown solid/mass was obtained. The solid was washed with several portions of water and then triturated with acetone. The solid was filtered and dried in vacuo at 80°C. overnight to give 35.67 g (70.3%) of light brown solid, m.p. 126-127°C.

Analysis: Calculated for $C_{12}H_{13}NO_2$: C,70.92; H,6.45; N,6.89

Found : C,70.94; H,6.49; N,6.87

Preparation 74

8-(3-Chloropropoxy)quinoline.

A solution of 3-(8-quinolinyloxy)-1-propanol (32.0 g, 0.158 mole) and thionyl chloride (24.0 g, 0.203 mole) was heated at reflux for 5 hours in 300 ml of dry benzene (dried over 4A molecular sieves). The reaction mixture was cooled to room temperature and then stripped to dryness. The residue was treated with potassium carbonate solution (30 g in 500 ml of water). The gummy residue was dissolved in chloroform and the solution was extracted with the potassium carbonate solution. The chloroform layer was dried over anhydrous sodium sulfate, filtered, and solvent removed to give a dark mass which crystallized. The mass was treated with 500 ml of boiling hexane. The hexane layer was decanted off from insoluble oil. A white solid crystallized on cooling the hexane layer. The solid was dried in vacuo at room temperature overnight to give 26.69 g (76.2%) of white crystalline solid, m.p. 69-71°C.

Analysis: Calculated for $C_{12}H_{12}NOCl$: C,65.02; H,5.45; N,6.32
Found : C,65.19; H,5.51; N,6.27

Preparation 75

α,α-Bis(4-fluorophenyl)-1-[(4-methylphenyl)sulfonyl]-3-piperidineacetonitrile.

The sodium salt of 4-fluoro-α-(4-fluorophenyl) benzeneacetonitrile was formed in 500 ml of dimethylsulfoxide from (39.42 g, 0.172 mole) of its free base and sodium hydride (60%) (6.88 g, 0.172 mole). The reaction mixture was stirred for one hour at room temperature. 1-[(4-Methylphenyl)sulfonyl]-3-piperidinol-4-methylphenylsulfonate ester (70.4 g, 0.172 mole) was added and the solution was stirred overnight at 65°C. The solution was stripped to dryness on a rotary evaporator. The residue obtained was dissolved in chloroform and the chloroform layer was extracted with 5% sodium hydroxide and also water. Removal of chloroform gave a dark brown oil. An eight gram portion of this oil was subjected to flash chromatography on silica gel using 15% ethyl acetate-85% hexane for elution. Fractions of similar purity were combined

and solvent was removed *in vacuo*. The residue was dried *in vacuo* overnight at 80°C. to give 4.75 g (45.9% based on aliquot taken) of white amorphous material.

$^1$H NMR ($CDCl_3$): 6.9-7.6 δ (m, 12, aromatic), 3.7-4.0 (m, 2, protons adjacent to sulfonamide nitrogen), 2.4 (5, 3, methyl), 1.3-2.9 (m, 7, aliphatics).

Analysis: Calculated for $C_{26}H_{24}N_2O_2SF_2$: C,66.93; H,5.18; N,6.00

Found : C,66.62; H,5.20; N,5.89

## Preparation 76

α,α-Bis(4-fluorophenyl)-3-piperidineacetonitrile maleate [1:1].

A solution of α,α-bis(4-fluorophenyl)-1-[(4-methylphenyl)sulfonyl]-3-piperidineacetonitrile (25.00 g, 0.0536 mole) in 125 ml of 48% hydrobromic acid containing phenol (25.00 g, 0.2657 mole) was heated at reflux for 3-1/2 hours. The solution was cooled to room temperature and diluted to 1 liter with ice while being made alkaline with 50% sodium hydroxide. The purple aqueous phase was extracted with three 300 ml portions of chloroform. The chloroform layer was back extracted with two 150 ml portions of 1N sodium hydroxide. The chloroform layer was dried over anhydrous sodium sulfate, filtered, and solvent removed to give a light brown oil. The oil was converted to the maleate salt which was recrystallized from methanol-diethyl ether. The precipitate was dried *in vacuo* overnight at 80°C. to give 13.05 g (56.8%) of white crystals, m.p. 115-118°C.

Analysis: Calculated for $C_{23}H_{22}N_2O_4F_2$: C,64.48; H,5.18; N,6.54

Found : C,64.04; H,5.15; N,6.50

Preparation 77

4-[Bis(4-fluorophenyl)methyl]-1-(3-chloropropyl) piperidine.

A solution of 4-[bis(4-fluorophenyl)methyl]-1-piperidinepropanol (40.27 g, 0.117 mole free base) and thionyl chloride (17.90 g, 0.150 mole) in 350 ml of chloroform was stirred at room temperature for 1/2 hour. The solution was heated at reflux for 6 hours, cooled to room temperature, and then stripped to dryness. The gum obtained was dissolved in chloroform and extracted with saturated sodium bicarbonate. The chloroform layer was dried (anhydrous sodium sulfate), filtered, and solvent removed to give a reddish-brown oil (42.11 g). An eight gram sample was subjected to flash chromatography on silica gel using 50-50 v/v of ethyl acetate-hexane for elution. After combining fractions, removing solvent and drying the oil *in vacuo*, 6.84 g. (84.6% yield-based on aliquot) of brown oil was obtained.

Analysis: Calculated for $C_{21}H_{24}NOF_2Cl_2$: C,69.32; H,6.65; N,3.85

Found : C,69.09; H,6.60; N,3.84

Preparation 78

7-Hydroxy-4-oxo-4H-1-benzopyran-2-carboxylic acid ethyl ester.

To a warm, stirred solution of 18.4 g (0.8 mole) of sodium metal in 250 ml of absolute ethanol was added dropwise a solution of 30.4 g (0.2 mole) of 2,4-dihydroxyacetophenone and 58.5 g (0.4 mole) of diethyloxalate in 50 ml of absolute ethanol and 50 ml of absolute ethyl ether over a 30 min period. The mixture was heated at reflux for 4 hours and then poured into a solution of 200 ml of concentrated hydrochloric acid and 1.8 liter of water. The mixture was extracted with two 500 ml portions of ethyl ether and the combined extracts were concentrated under reduced pressure to give a solid residue.

The solid was dissolved in a mixture of 250 ml of ethanol and 3 ml of concentrated hydrochloric acid and

heated tt reflux for 2 hours. The mixture was concentrated under reduced pressure and the solid residue was triturated with ethyl ether, collected by filtration, and recrystallized from 95% ethanol to yield 28.1 g (60%) of a tan powder, m.p. 217-221°C.

Analysis: Calculated for $C_{12}H_{10}O_5$: C,61.54; H,4.30
Found : C,61.68; H,4.34

Preparation 79

α,α-Diphenyl-1-(phenylmethyl)-4-piperidineacetonitrile hydrochloride [1:1].

To a prewashed slurry of 8.00 g (.19 mole), 57% sodium hydride in 300 ml of dimethylsulfoxide was added 32.80 g (0.17 mole) diphenylacetonitrile. The solution was heated at 65°C. for 1 hr during which time the solution became deep red. 1-(Phenylmethyl)-4-piperidinol ester with benzenesulfonic acid (0.17 mole) was then added in 50 ml of dimethylsulfoxide and the solution stirred overnight at 60°C. The solution was cooled and poured into 1 liter of water. The aqueous solution was extracted with toluene (3 x 150 ml). The toluene extracts were treated with 500 ml of sulfuric acid which caused a gummy residue to precipitate. The residue was taken up in a mixture of methylene chloride and 10% sodium hydroxide. The layers were separated, the aqueous layer extracted with methylene chloride and the combined extracts dried over magnesium sulfate. Concentration gave 35.0 g (57%) of a tan solid, m.p. 138-142°C.

A small portion was converted to the hydrochloride salt which was recrystallized from methanol/diethyl ether to give white powder, m.p $>$250°C.

Analysis: Calculated for $C_{26}H_{27}ClN_2$: C,77.50; H,6.75; N,6.95
Found : C,77.09; H,6.76; N,7.04

Preparation 80

α,α-Diphenyl-1-(phenylmethyl)-3-piperidinepropanenitrile hydrochloride [1:1].

A mixture of 7.25 g, (0.025 mole) of α,α-diphenyl-3-piperidinepropanenitrile, 4.28 g, (0.025 mole) of benzyl bromide and potassium carbonate (5.53 g, 0.04 mole) was stirred overnight at room temperature in 300 ml of acetonitrile containing potassium iodide (0.3 g). The reaction mixture was stripped to dryness, and the resulting residue was dissolved in chloroform. The chloroform layer was extracted several times with water, dried, filtered, and solvent removed to give an oil. The oil was converted to the hydrochloride salt via ethereal hydrogen chloride. The white solid was recrystallized from methanol-diethyl ether and dried in vacuo at 80°C. overnight. A yield of 7.04 g (67.5%) of white solid, m.p. 243-246°C. with decomposition was obtained.

Analysis: Calculated for $C_{27}H_{29}N_2Cl$: C,77.77; H,7.01; N,6.72

Found : C,77.36; H,6.97; N,6.67

Preparation 81

α,α-Diphenyl-1-(phenylmethyl)-4-piperidineacetamide fumarate [1:1].

A solution of 5.88 g, (0.02 mole) of α,α-diphenyl-4-piperidineacetamide in acetonitrile was prepared by warming with a hair dryer. To this solution was added 3.42 g, (0.02 mole) of benzyl bromide and potassium carbonate, 6.91 g (0.05 mole). This mixture was stirred overnight at room temperature and then heated at reflux for 5 hours. The reaction mixture was stripped to dryness, and the residue obtained was partitioned between chloroform-water and chloroform-5% sodium hydroxide. Removal of chloroform gave an oil which was subjected to column chromatography on silica gel using mixtures of ethyl acetate-dimethoxyethane for elution. Suitable fractions were combined and converted to the fumarate salt. The salt was recrystallized from methanol-diethyl ether and dried overnight at 80°C.

in vacuo. A yield of 1.65 g (10%) of white crystalline material, m.p. 218-220°C. was obtained.

Analysis: Calculated for $C_{30}H_{32}N_2O_5$: C,71.98; H,6.44; N,5.60
Found : C,71.60; H,6.47; N,5.51

Preparation 82

α,α-Diphenyl-1-(phenylmethyl)-3-piperidinepropanamide hydrate [1:0.5].

A mixture of 7.70 g (0.025 mole) of α,α-diphenyl-3-piperidinepropanamide, 4.28 g (0.025 mole) of benzyl bromide and potassium carbonate (5.54 g, 0.04 mole) was heated overnight at gentle reflux in 300 ml of acetonitrile containing potassium iodide (0.3 g). The reaction mixture was stripped to dryness and partitioned between chloroform-water and chloroform 5% sodium hydroxide. Removal of chloroform gave an oil. This oil was subjected to column chromatography on silica gel using dimethoxyethane and ethyl acetate for elution. A yield of 3.34 g (32.8%) of yellow amorphous solid , after combining column fractions and drying at 80°C. in vacuo overnight was obtained.

$^1$H NMR ($CDCl_3$) δ 7.1-7.6 (m, 15, aromatic), 5.5-6.0 (br s, 2, $NH_2$), 3.4 (s, 2, $CH_2$), 3.1 (s, 1, 1/2 $H_2O$) 1.0-2.7 (m, 11, alphatic).

Analysis: Calculated for $C_{27}H_{31}N_2O_{1.5}$: C,79.57; H,7.67; N,6.87
Found : C,79.65; H,7.46; N,6.88

Preparation 83

α,α-Bis(4-fluorophenyl)-1-(phenylmethyl)-4-piperidineacetonitrile . hydrochloride hydrate [1:1:0.5].

A mixture of α,α-bis(4-fluorophenyl)-4-piperidineacetonitrile, 6.05 g (0.019 mole), benzyl bromide, 3.32 g (0.019 mole), and potassium carbonate, 5.53 g (0.04 mole) was heated overnight at gentle reflux in 350 ml of acetonitrile containing potassium iodide. The reaction mixture was stripped to dryness and partitioned between chloroform and water. The chloroform layer was dried over anhydrous sodium sulfate, filtered, and solvent removed to give 7.55 g of light yellow oil. The oil was

converted to the hydrochloride salt using ethereal hydrogen chloride and the salt was recrystallized from methanol-diethyl ether. The white solid obtained by filtration was washed with diethyl ether and dried _in vacuo_ overnight at 80°C. A yield of 3.85 g (45.2%) of white crystals, m.p. 283°C. with decomposition was obtained.

Analysis: Calculated for $C_{26}H_{26}N_2O_{0.5}F_2Cl$: C,69.71; H,5.85; N,6.25

Found : C,70.07; H,5.68; N,6.25

Preparation 84

α,α-Bis(4-fluorophenyl)-1-(phenylmethyl)-3-pyrrolidinepropanenitrile hydrate [1:0.5].

The sodium salt of 4-fluoro-α-(4-fluorophenyl) benzeneacetonitrile was prepared in dimethyl sulfoxide from 41.9 g (0.183 mole) of the free base and 7.32 g (0.183 mole) of 60% sodium hydride. After stirring at room temperature for 3 hrs, a solution of 63.18 g (0.183 mole) of 1-phenylmethyl-3-pyrrolidinemethanol methanesulfonate ester in dimethylsulfoxide was added. The resulting solution was stirred overnight at 60°C. The solvent was removed _in vacuo_ via a rotary evaporator. The oil obtained was dissolved in chloroform and extracted several times with 1N sulfuric acid. The chloroform layer was extracted with 5% sodium hydroxide. dried over anhydrous sodium sulfate, filtered, and solvent removed to give 59.6 g of dark brown oil. A 10 g fraction was subjected to flash chromatography on silica gel using 50-50 v/v ethyl acetate-hexane and 100% ethyl acetate for elution. Fractions of similar purity were combined and solvent was removed _in vacuo_. A dark brown oil was obtained and dried at 80°C. _in vacuo_ overnight. A yield of 4.13 g (33.5% based on aliquot used) of dark brown oil was obtained. $^1H$ NMR ($CDCl_3$): δ 6.8-7.4 (m, 13 aromatic), 3.5 (s, 2, N-$CH_2$- 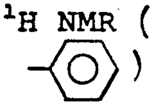) 1.1-2.8 (m, 9, aliphatic).

Analysis: Calculated for $C_{26}H_{25}N_2O_{0.5}F_2$: C,75.89; H,6.12; N,6.81

Found : C,75.93; H,6.00; N,6.55

Example 1

4-(Diphenylmethylene)-1-(3-phenoxypropyl)piperidine oxalate [1:1].

A mixture of 3.3 g (0.013 mole) of 4-diphenylmethylenepiperidine, 3.3 g (0.015 mole) of (3-bromopropoxy)benzene and 5.3 g (0.05 mole) of anhydrous sodium carbonate in 100 ml of 1-butanol was heated at reflux for 20 hr. The mixture was concentrated under reduced pressure and the residue was partitioned between water and benzene. The benzene layer was washed with water and brine, dried over anhydrous sodium sulfate and concentrated under reduced pressure to give an oil as residue, the free base of the title compound. The free base was converted to the oxalic acid salt and the solid was recrystallized from absolute ethanol to yield 4.3 g (70%) of the title product as a white powder, m.p. 175-178°C.

Analysis: Calculated for $C_{29}H_{31}NO_5$: C,73.55; H,6.60; N,2.96
Found : C,73.59; H,6.64; N,2.83

Example 2

α,α-Bis-(4-fluorophenyl)-1-(3-phenoxypropyl)-4-piperidine-methanol oxalate hydrate [1:1:0.5].

A mixture of 3.37 g (0.011 mole) of [α,α-bis(p-fluoro-phenyl)]-4-piperidinemethanol, 2.52 g (0.011 mole) of (3-bromo-propoxy)benzene and sodium bicarbonate (0.92 g, 0.011 mole) in 200 ml of 1-butanol was heated overnight at reflux. The butanol was removed by the rotary evaporator, and the residue partitioned between chloroform and water. Removal of the chloroform *in vacuo* gave a dark brown oil, the free base of the title compound. The free base was converted to the oxalate salt and the salt was recrystallized from methanol-diethyl ether to give 1.41 g (23.9%) of white solid, m.p. 153°C.

Analysis: Calculated for $C_{29}H_{32}NO_{6.5}F_2$: C,64.92; H,6.01; N,2.61
Found : C,65.27; H,5.87; N,2.61

Example 3

4-[Bis(4-fluorophenyl)methylene]-1-(3-phenoxypropyl)-piperidine oxalate [1:1].

A solution of 7.37 g (0.0168 mole) α,α-bis(4-fluorophenyl)-1-(3-phenoxypropyl)-4-piperidinemethanol in 100 ml of methanol containing 100 ml of 6N hydrochloric acid was gently refluxed for 4 hr. The reaction mixture was cooled, made alkaline with ice/50% sodium hydroxide, and diluted to 1 liter with water. The aqueous phase was extracted with chloroform, and removal of chloroform gave an oil. The oil was converted to the oxalate salt and recrystallized from methanol-diethyl ether to give 3.45 g (40.3%) of white solid, m.p. 190-192°C.

Analysis: Calculated for $C_{29}H_{29}NO_5F_2$: C,68.36; H,5.74; N,2.75

Found : C,68.43; H,5.75; N,2.69

Example 4

α,α-Bis(4-fluorophenyl)-1-(3-phenoxypropyl)-4-piperidinemethanol oxalate [1:1].

To a mixture of 5.10 g (0.21 mole) of magnesium turnings and a crystal of iodine in 800 ml of dry tetrahydrofuran (distilled from lithium aluminum hydride) was added a solution of 36.75 g (0.21 mole) of p-bromofluorobenzene in 100 ml of tetrahydrofuran. The reaction flask was cooled in an ice bath during this addition, and the reaction mixture was under an atmosphere of nitrogen. The mixture was stirred at ambient temperature for 1 hr. A solution of 20.17 g (0.0693 mole) of ethyl N-(3-phenoxypropyl)isonipecotate in 100 ml of tetrahydrofuran was added and the solution was stirred at room temperature for 16 hr. The mixture was poured into an icy solution of ammonium chloride and the aqueous mixture was extracted with methylene chloride. The methylene chloride solution was extracted with dilute sodium hydroxide and was dried over magnesium sulfate. The solvent was removed *in vacuo* to give a gummy residue. The residue was treated with a solution of oxalic acid in methanol and the salt was recrystallized from methanol-ether

to give 24.17 g (66.2%) of white crystalline solid, m.p. 153-155°C.

Analysis: Calculated for $C_{28}H_{31}NO_6F_2$: C,66.02; H,5.92; N,2.66
Found : C,65.78; H,5.93; N,2.63

Example 5

4-(Diphenylmethyl)-1-(4-phenoxybutyl)piperidine fumarate [1:1].

A solution of 6.99 g (0.0278 mole) of 4-diphenylmethylpiperidine, 6.64 g (0.029 mole) of (4-bromobutoxy)benzene and 5 g (0.060 mole) of sodium bicarbonate in 400 ml of 1-butanol was refluxed for 11 hr. The solvent was removed *in vacuo*, and the residue was partitioned between methylene chloride and dilute sodium hydroxide. The methylene chloride solution was dried over magnesium sulfate, and the solvent was removed *in vacuo* to give an oil, the free base of the title compound. The free base was dissolved in 500 ml of ether, and a small amount of solid was filtered from the solution. To the filtrate was added a solution of 3.2 g (0.0276 mole) of fumaric acid in 60 ml of methanol. A white precipitate was collected to give 7.97 g (55.7%) of white crystalline solid, m.p. 146-147°C.

Analysis: Calculated for $C_{32}H_{37}NO_5$: C,74.54; H,7.23; N,2.72
Found : C,74.68; H,7.24; N,2.68

Example 6

4-(Diphenylmethyl)-1-(3-phenoxypropyl)piperidine fumarate [1:1].

Following the procedure of Example 5, 4-(diphenylmethyl) piperidine and (3-bromopropoxy)benzene were reacted to give the free base of the title compound which was reacted with fumaric acid in methanol to give the white fumarate salt in 71% yield, m.p. 171-172°C.

Analysis: Calculated for $C_{31}H_{35}NO_5$: C,74.23; H,7.03; N,2.79
Found : C,74.62; H,7.03; N,2.73

Example 7

4-[Bis(4-fluorophenyl)methyl]-1-(3-phenoxypropyl) piperidine oxalate [1:1].

Following the procedure of Example 2, 4-[bis(4-fluorophenyl)methyl]piperidine and (3-bromopropoxy)benzene were reacted to give the free base of the title compound which was reacted with oxalic acid recrystallizing from methanol-diethyl ether to give the white oxalate salt in 60% yield, m.p. 178-181°C.

Analysis: Calculated for $C_{29}H_{31}NO_5F_2$: C,68.09; H,6.11; N,2.74
Found : C,68.37; H,6.13; N,2.76

Example 8

4-(Diphenylmethyl)-1-(2-phenoxyethyl)piperidine fumarate [1:1].

Following the procedure of Example 5, 4-(diphenylmethyl) piperidine and (2-bromoethoxy)benzene were reacted to give the free base of the title compound which was reacted with fumaric acid in ether-methanol mixture to give the white fumarate salt in 85% yield, m.p. 189-190°C.

Analysis: Calculated for $C_{30}H_{33}NO_5$: C,73.90; H,6.82; N,2.87
Found : C,74.07; H,6.91; N,2.85

Example 9

4-[Bis(4-fluorophenyl)methyl]-1-(2-phenoxyethyl) piperidine oxalate [1:1].

A mixture of 5.83 g (0.02 mole) of 4-[bis(4-fluorophenyl) methyl]piperidine, 4.02 g (0.02 mole) of (2-bromoethoxy)benzene and sodium carbonate (3.18 g, 0.03 mole) was heated overnight at gentle reflux in 300 ml of 1-butanol. The reaction was filtered and solvent removed in vacuo. The residue was dissolved in chloroform and extracted with water and 5% sodium hydroxide. Removal of chloroform gave an oil which was converted to the oxalate salt. The salt was recrystallized from methanol-diethyl ether to give 6.0 g (60.3%) of white crystalline product, m.p. 180-182°C.

Analysis: Calculated for $C_{28}H_{29}NO_5F_2$: C,67.60; H,5.88; N,2.82
Found : C,67.68; H,5.87; N,2.81

Example 10

4-[Bis(4-fluorophenyl)methyl]-1-(4-phenoxybutyl) piperidine oxalate [1:1].

Following the procedure of Example 2, 4-[bis(4-fluorophenyl)methyl]piperidine and (4-bromopropoxy)benzene were reacted to give the free base of the title compound which was reacted with oxalic acid to give the white oxalate salt (recrystallizing from methanol-diethyl ether), in 48% yield, m.p. 206°C.

Analysis: Calculated for $C_{30}H_{33}NO_5F_2$: C,68.56; H,6.33; N,2.67
Found : C,68.79; H,6.35; N,2.67

Example 11

4-[(4-Fluorophenyl)-phenylmethyl]-1-(3-phenoxypropyl) piperidine fumarate [1:1].

A mixture of 5.4 g (0.02 mole) of 4[α-(p-fluorophenyl)-α-phenylmethyl]piperidine, 4.5 g (0.021 mole) of (3-bromopropoxy)benzene and 8.0 g (0.075 mole) of anhydrous sodium carbonate in 150 ml of acetonitrile was refluxed for about 20 hr and concentrated under reduced pressure to give a gummy residue. The residue was purified by column chromatography on 160 g of Florisil® and the product was eluted with 2% acetone in benzene to give an oil, the free base of the title compound. The free base was reacted with fumaric acid and the salt was recrystallized from isopropyl alcohol to give 4.0 g (38%) of white solid, m.p. 169-171°C. (with decomposition).

Analysis: Calculated for $C_{31}H_{34}FNO_5$: C,71.66; H,6.60; N,2.70
Found : C,71.37; H,6.55; N,2.66

Example 12

4-[Bis(4-fluorophenyl)methyl]-1-[2-(2,6-dichlorophenoxy) ethyl]piperidine.

A mixture of 6.13 g (0.021 mole) of 4-[bis(4-fluorophenyl)methyl]piperidine, 5.38 g (0.03 mole) of 2-(2-bromoethoxy)-1,3-dichlorobenzene was heated overnight at gentle reflux in 200 ml of 1-butanol. The reaction mixture was filtered and stripped to dryness. The residue was dissolved in chloroform and extracted with water and 5% sodium

hydroxide solution. The oil which was obtained was chromatographed on 300 g of silica gel using hexane-ethyl acetate (50/50 v/v) as eluant. The fractions containing product were combined and solvent removed to furnish an oil. The oil was dried overnight *in vacuo* at 80°C. This furnished 5.99 g (59%) of product oil.

Analysis: Calculated for $C_{26}H_{25}NOF_2Cl_2$: C,65.55; H,5.29; N,2.94

Found : C,65.43 H,5.34 N,2.77

The $^1$H NMR spectrum of the subject compound was obtained in $CDCl_3$, containing tetramethylsilane and is consistent with the structure indicated by the title,

| | | |
|---|---|---|
| δ 1.1-2.3 | aliphatic protons (cyclic) | 7H |
| δ 2.8 or triplet | $CH_2$ next to N | 2H |
| δ 2.8-3.1 | Hydrogen next to N | 2H |
| δ 3.5 doublet | methine proton | 1H |
| δ 4.1 triplet | $CH_2$ next to oxygen | 2H |
| δ 6.8-7.4 | aromatic protons | 11H |

## Example 13

1-[3-(4-Chlorophenoxy)propyl]-α,α-bis(4-fluorophenyl)-4-piperidinemethanol.

A mixture of 3.0 g (0.01 mole) of [α,α-bis(p-fluorophenyl)]-4-piperidinemethanol, 2.0 g (0.01 mole) of 1-chloro-4-(3-chloropropoxy)benzene, 5.3 g (0.05 mole) of anhydrous sodium carbonate and 0.3 g of potassium iodide in 100 ml of 1-butanol was refluxed for 20 hr to give, after working up as in Example 1 (recrystallizing the free base from isopropyl alcohol), 1.7 g (36%) of white solid, m.p. 92-93°C.

Analysis: Calculated for $C_{27}H_{28}ClF_2NO_2$: C,68.71; H,5.98; N,2.97

Found C,68.66; H,5.99; N,2.92

0235463

Example 14

4-[Bis(4-fluorophenyl)methyl]-1-[3-(2-fluorophenoxy)propyl]piperidine oxalate [1:1].

A mixture of 5.85 g (0.02 mole) of 4-[bis(4-fluorophenyl)methyl]piperidine, 3.76 g (0.02 mole) of 2-(3-chloropropoxy)-1-fluorobenzene, and sodium carbonate (4.80 g, 0.045 mole) in 300 ml of 1-butanol containing 0.3 g of potassium iodide was heated overnight at gentle reflux. The reaction mixture was stripped to dryness and the resulting oil partitioned between chloroform-5% sodium hydroxide and then between chloroform-water. Removal of chloroform gave an oil which was converted to the oxalate salt. The salt was recrystallized from methanol-diethyl ether. The salt was subsequently triturated with isopropanol, and was dried overnight at 80°C. to give 5.82 g (55%) of product, m.p. 182-183°C.

Analysis: Calculated for $C_{28}H_{30}NO_5F_3$: C,65.78; H,5.71; N,2.65
Found : C,66.05; H,5.79; N,2.59

Example 15

4-[Bis(4-fluorophenyl)methyl]-1-[3-(3-fluorophenoxy)propyl]piperidine mandelate [1:1].

Following the procedure of Example 14, 4-[bis(4-fluorophenyl)methyl]piperidine and 3-(3-chloropropoxy)-1-fluorobenzene were reacted to give the free base of the title compound which was reacted with mandelic acid to give the white mandelate salt (recrystallizing from isopropyl alcohol) in 62% yield, m.p. 145-147.5°C.

Analysis: Calculated for $C_{35}H_{36}NO_4F_3$: C,71.05; H,6.13; N,2.37
Found : C,71.10; H,6.20; N,2.36

Example 16

4-[Bis(4-fluorophenyl)methyl]-1-[3-(4-chlorophenoxy)propyl]piperidine fumarate [1:1].

Following the procedure of Example 14, 4-[bis(4-fluorophenyl)methyl]piperidine and 1-[4-(3-chloropropoxy)]chlorobenzene were reacted to give the free base of the title compound. The free base was chromatographed on silica gel eluting with hexane-ethyl acetate and reacted with fumaric

acid (recrystallizing from methanol-diethyl ether) in 9% yield, m.p. 169-170°C.

Analysis: Calculated for $C_{31}H_{32}NO_5F_2Cl$: C,65.10; H,5.64; N,2.45

Found : C,64.85; H,5.63; N,2.46

Example 17

4-[Bis(4-fluorophenyl)methyl]-1-[3-(4-fluorophenoxy)propyl]piperidine.

Following the combined procedures of Examples 14 and 16, 4-[bis(4-fluorophenyl)methyl]piperidine and 4-(3-chloropropoxy)-1-fluorobenzene were reacted and worked up by chromatography in Example 16, to give the free base in 53% yield as a yellow oil after drying *in vacuo* at 80°C. overnight.

Analysis: Calculated for $C_{27}H_{28}NOF_3$: C,73.78; H,6.42; N,3.19

Found : C,73.64; H,6.39; N,3.14

Example 18

4-[Bis(4-fluorophenyl)methyl]-1-[3-(4-methoxyphenoxy)propyl]piperidine fumarate [1:1].

Following the procedure of Example 14, 4-[bis(4-fluorophenyl)methyl]piperidine and 1-(3-chloropropoxy)-4-methoxybenzene were reacted to give the free base of the title compound which was reacted with fumaric acid to give the white fumarate salt (recrystallizing from methanol-diethyl ether) in 64% yield, m.p. 172-173°C.

Analysis: Calculated for $C_{32}H_{35}NO_6F_2$: C,67.71; H,6.22; N,2.47

Found : C,67.89; H,6.25; N,2.39

Example 19

4-[Bis(4-fluorophenyl)methyl]-1-[3-(2-methoxyphenoxy)propyl]piperidine.

A mixture of 5.99 g (0.021 mole) of 4-[bis(4-fluorophenyl)methyl]piperidine, 4.35 g (0.022 mole) of 2-(3-chloropropoxy)-1-methoxybenzene ether, and sodium carbonate (3.18 g, 0.03 mole) in 1-butanol was heated overnight at gentle reflux. The reaction mixture was filtered and stripped to dryness. The residue was dissolved in chloroform and the solution was

extracted with water and 5% sodium hydroxide. Removal of chloroform gave a dark brown oil. The oil was chromatographed on silica gel using acetone-ethyl acetate for elution. After combining fractions and removing solvent, an oil was obtained. The oil was dried *in vacuo* at 80°C. overnight. This gave 3.18 g (33.5%) of title product.

Analysis: Calculated for $C_{28}H_{31}NO_2F_2$: C,74.48; H,6.92; N,3.12
Found : C,74.42; H,6.95; N,3.00

The $^1$H NMR spectrum of the subject compound was obtained in $CDCl_3$ containing tetramethyl silane and is consistent with the structure indicated by the title,

| | | |
|---|---|---|
| δ 6.8 | singlet; or protons on ring containing methoxy group. | 4H |
| δ 6.8-7.3 | aromatic protons on fluorophenyl rings. | 8H |
| δ 4.0 | triplet $CH_2$-O. | 2H |
| δ 3.8 | singlet O-$CH_3$. | 3H |
| δ 3.4 | doublet; methine proton. | 1H |
| δ 0.8-3.1 | multiplet. | 13H |

## Example 20

α,α-Bis(4-fluorophenyl)-1-[3-(2-methoxyphenoxy)propyl]-4-piperidinemethanol.

Following the procedure of Example 1, [α,α-bis(p-fluorophenyl)]-4-piperidinemethanol and 1-chloro-3-(2-methoxyphenoxy)propane were reacted using in addition potassium iodide catalyst to give the title compound in 66% yield, (recrystallizing from isopropyl alcohol), m.p. 127-218°C.

Analysis: Calculated for $C_{28}N_{31}F_2NO_3$: C,71.93; H,6.68; N,3.00
Found : C,71.88; H,6.67; N,2.98

## Example 21

4-[Bis(4-fluorophenyl)methylene]-1-[3-(2-methoxyphenoxy)propyl]piperidine oxalate [1:1].

Following the procedure of Example 14, 4-[bis(4-fluorophenyl)methylene]piperidine and 2-(3-chloropropoxy)-1-methoxybenzene were reacted using in addition potassium iodide catalyst to give the free base of the title compound which was reacted with oxalic acid to give the white oxalate salt (recrystallizing from methanol-diethyl ether) in 73% yield, m.p. 184-186°C.

Analysis: Calculated for $C_{30}H_{31}NO_6F_2$: C,66.78; H,5.79; N,2.60
Found : C,66.74; H,5.79; N,2.61

Example 22

4-[Bis(4-fluorophenyl)methyl]-1-[3-(3,4-dimethoxyphenoxy)propyl]piperidine oxalate [1:1].

A mixture of 6.02 g (0.021 mole) of 4-[bis(4-fluorophenyl)methyl]piperidine, 4.83 g (0.021 mole) of 4-(3-chloropropoxy)-1,2-dimethoxybenzene, and potassium carbonate (5.52 g, 0.04 mole) was refluxed overnight in 300 ml of 1-butanol containing potassium iodide (0.3 g). The reaction mixture was stripped to dryness and partitioned between chloroform and water several times. The chloroform layer was dried over anhydrous sodium sulfate and then filtered. The chloroform was removed by rotary evaporator. The oil obtained was converted to the oxalate salt and then recrystallized from methanol-diethyl ether and methanol isopropanol ether. This furnished 7.77 g (64.7%) of white solid, m.p. 188°C.

Analysis: Calculated for $C_{31}H_{35}NO_7F_2$: C,65.14; H,6.17; N,2.43
Found : C,64.78; H,6.14; N,2.44

Example 23

4-[Bis(4-methylphenyl)methyl]-1-[3-(2,6-dimethoxyphenoxy)propyl]piperidine fumarate [1:1].

Following the procedure of Example 22, 4-[bis(4-methylphenyl)methyl]piperidine and 2-(3-chloropropoxy)-1,3-dimethoxybenzene were reacted to give the free base of the title compound which was reacted with fumaric acid to give the white fumarate salt (recrystallizing from methanol-diethyl ether) in 66% yield, m.p. 206-207°C.

Analysis: Calculated for $C_{35}H_{43}NO_7$: C,71.29; H,7.35; N,2.38
Found : C,71.24; H,7.38; N,2.36

Example 24

4-[Bis(4-fluorophenyl)methylene]-1-[3-(3,4-dimethoxyphenoxy)propyl] piperidine oxalate [1:1].

Following the procedure of Example 22, 4-[ bis(4-fluorophenyl)methylene]piperidine and 4-(3-chloropropoxy)-1,2-dimethoxybenzene were reacted to give the free base of the title compound which was reacted with oxalic acid to give. the cream colored oxalate salt (recrystallizing from methanol-diethyl ether) in 51% yield, m.p. 173-176°C.

Analysis: Calculated for $C_{31}H_{33}NO_7F_2$: C,65.37; H,5.84; N,2.46
Found : C,65.02; H,5.83; N,2.50

Example 25

4-[Bis(4-fluorophenyl)methyl]-1-[3-(2,6-dimethoxyphenoxy) propyl]piperidine oxalate hydrate [1:1:1].

Following the procedure of Example 22, but substituting dimethoxy ethane for butanol, 4-[bis(4-fluorophenyl)methyl] piperidine and 2-(3-chloropropoxy)-1,3-dimethoxybenzene were reacted to give the free base of the title compound which was reacted with oxalic acid to give the white oxalate salt (recrystallizing from methanol-diethyl ether) in 9% yield, m.p. 132-134°C.

Analysis: Calculated for $C_{31}H_{37}NO_8F_2$: C,63.15; H,6.32; N,2.38
Found : C,62.89; H,5.98; N,2.41

Example 26

4-[Bis(4-fluorophenyl)methyl]-1-[3-(3,5-dimethoxyphenoxy) propyl]piperidine.

A mixture of 5.51 g (0.019 mole) of 4-[bis(4-fluorophenyl)methyl]piperidine, 4.42 g (0.019 mole) of 1-(3-chloropropoxy)-3,5-dimethoxybenzene and potassium carbonate (5.53 g, 0.04 mole) was heated overnight at reflux in 350 ml of 1-butanol containing potassium iodide (0.3 g). The reaction mixture was stripped to dryness and the residue partitioned between chloroform-5% sodium hydroxide and chloroform-water. Removal of chloroform gave a brown oil. The oil was subjected to chromatography on a silica gel column using a gradient elution series of hexane-ethyl acetate and ethyl acetate-dimethoxyethane. After combining proper fractions eluted from the column and removing solvent,

the residual oil was dried in vacuo overnight at 80°C. This produced 2.61 g (28.5%) of brown oil.

Analysis: Calculated for $C_{29}H_{33}NO_3F_2$: C,72.33; H,6.91; N,2.91
Found : C,71.62; H,6.80; N,2.98

The $^1H$ NMR spectrum of the subject compound was obtained in $CDCl_3$ containing tetramethylsilane and is consistent with the structure indicated by the title: $\delta$ 7.0 (multiplet, aromatic protons on fluorophenyl ring, 6.0 (singlet, aromatic protons on methoxyphenyl ring, 3H), 2.8 (triplet, methylene next to ether oxygen, 2H), 3.75 (singlet, $OCH_3$, 6H), 3.4 (doublet, methine attached to two aromatic rings, 1H), 0.75 - 2.6 (multiplet, remaining aliphatics, 13H).

## Example 27

4-[Bis(4-methoxyphenyl)methyl]-1-[3-(3,4-dimethoxyphenoxy)propyl]piperidine.

A mixture of 5.58 g (0.02 mole) of 4-[bis(4-methoxyphenyl)methyl]piperidine, 4.83 g (0.021 mole) of 4-(3-chloropropoxy)-1,2-dimethoxybenzene, and potassium carbonate, 5.52 g (0.04 mole) was heated overnight at reflux in 350 ml of 1-butanol containing potassium iodide (0.3 g). The reaction mixture was stripped to dryness, and the residue partitioned between chloroform-5% sodium hydroxide and chloroform-water. Removal of chloroform gave a dark brown oil. The oil was subjected to column chromatography on a silica gel column with elution via ethyl acetate-dimethoxy ethane. This produced 4.72 g (46.7%) of dark brown oil.

Analysis: Calculated for $C_{31}H_{39}NO_5$: C,73.64; H,7.77; N,2.77
Found : C,72.38; H,7.70; N,2.72

The $^1H$ NMR spectrum of the subject compound was obtained in $CDCl_3$ containing tetramethylsilane and is consistent with the structure indicated by the title: $\delta$ 7.1 (multiplet, aromatic protons ortho to methine of $-\overset{H}{\overset{|}{C}}-(4-OCH_3C_6H_4)_2$, 4H) 6.75 (multiplet, aromatic protons adjacent to methoxy groups, 5H), 6.4 (multiplet, aromatic protons adjacent to ether linkage, 2H), 3.9 (triplet, methylene protons next to ether linkage, 2H), 3.7 ($OCH_3$, 6H), 3.6 ($OCH_3$, 6H), 3.3 (doublet, methine attached to aromatic rings, 1H), 0.75-3.0 (multiplet, aliphatic protons, 13H).

Example 28

4-[Bis(4-methoxyphenyl)methyl]-1-[3-(4-methoxyphenoxy) propyl]-piperidine fumarate hydrate [1:1:0.5].

Following the procedure of Example 22, 4-[bis(4-methoxyphenyl)methyl]piperidine and 4-(3-chloropropoxy)-1-methoxybenzene were reacted to give the free base of the title compound which was separated by extracting with sodium hydroxide-chloroform and reacted with fumaric acid to give the title salt (recrystallizing from methanol-diethyl ether several times as well as isopropyl alcohol) in 15% yield, m.p. 163-165°C.

Analysis: Calculated for $C_{34}H_{42}NO_{8.5}$: C,67.98; H,7.05; N,2.33
Found : C,68.16; H,6.97; N,2.34

Example 29

1-[4-[3-[4-[Bis(4-fluorophenyl)methylene]-1-piperidinyl] propoxy]phenyl]ethanone oxalate [1:1].

Following the procedure of Example 2, 4-[bis(4-fluorophenyl)methylene]piperidine and 1-[4-(3-chloropropoxy)phenyl] ethanone, substituting sodium carbonate for sodium bicarbonate, were reacted to give the free base of the title compound which was reacted with oxalic acid to give the oxalate salt (recrystallizing from methanol-diethyl ether) in 59% yield, m.p. 196-198°C.

Analysis: Calculated for $C_{31}H_{33}NO_6F_2$: C,67.26; H,6.01; N,2.53
Found : C,66.94; H,6.01; N,2.40

Example 30

1-[4-[3-[4-[Bis(4-fluorophenyl)methyl]-1-piperidinyl] propoxy]phenyl]ethanone, oxalate [1:1].

Following the procedure of Example 2, 4-[bis(4-fluorophenyl)methyl]piperidine and 1-[4-(3-chloropropoxy)phenyl] ethanone and substituting sodium carbonate for sodium bicarbonate were reacted to give the free base of the title compound which was reacted with oxalic acid to give the oxalate salt (recrystallizing from methanol-diethyl ether) in 75% yield, m.p. 141-143°C.

Analysis: Calculated for $C_{31}H_{33}NO_6F_2$: C,67.26; H,6.01; N,2.53
Found : C,66.94; H,6.01; N,2.40

Example 31

1-[4-[3-[4-[Bis(4-fluorophenyl)hydroxymethyl]-1-piperidinyl]propoxy]-phenyl]ethanone compound with 2-propanol [1:1].

Following the procedure of Example 1, [α,α-bis(p-fluorophenyl)]-4-piperidinemethanol and 1-[4-(3-chloropropoxy)phenyl]ethanone were reacted using potassium iodide catalyst to give the free base of the title compound which when recrystallized from isopropyl alcohol gave the white title compound in 71% yield, m.p. 72-84°C.

Analysis: Calculated for $C_{28}H_{31}F_2NO_3 \cdot C_3H_8O$ : C,71.22; H,7.28 N,2.60

Found : C,71.26; H,7.34; N,2.56

NMR indicated that the solid contained one mole of 2-propanol as a solvate.

Example 32

1-[4-[3-[4-[Bis(4-fluorophenyl)hydroxymethyl]-1-piperidinyl]propoxy]-3-methylphenyl]ethanone.

Following the procedure of Example 1, [α,α-bis(p-fluorophenyl)]-4-piperidinemethanol and 1-[4-(3-chloropropoxy)-3-methylphenyl]ethanone were reacted using potassium iodide catalyst to give the white title compound (recrystallizing from isopropyl alcohol) in 76% yield, m.p. 116-117°C.

Analysis: Calculated for $C_{30}H_{33}F_2NO_3$: C,73.00; H,6.74; N,2.84

Found : C,72.90; H,6.80; N,2.78

Example 33

4-[3-[4-[Bis(4-fluorophenyl)hydroxymethyl]-1-piperidinyl]propoxy] benzonitrile.

Following the procedure of Example 1, [α,α-bis(p-fluorophenyl)]-4-piperidinemethanol and 4-(3-chloropropoxy) benzonitrile were reacted using potassium iodide as catalyst to give the white title compound (recrystallizing from isopropyl alcohol-isopropyl ether) in 30% yield, m.p. 107-108°C.

Analysis: Calculated for $C_{28}H_{28}F_2N_2O_2$: C,72.71; H,6.10; N,6.06

Found : C,72.82; H,6.11; N,6.05

Example 34

4-[3-[4-[Bis(4-fluorophenyl)methyl]-1-piperidinyl] propoxy]benzonitrile fumarate [1:1].

Following the procedure of Example 22, 4-[bis(4-fluorophenyl)methyl]piperidine and 4-(3-chloropropoxy)cyanobenzene were reacted using potassium iodide catalyst to give the free base of the title compound which was reacted with fumaric acid to give the fumarate salt which was (recrystallized from methanol-diethyl ether) in 53% yield, m.p. 167°C.

Analysis: Calculated for $C_{32}H_{32}N_2O_5F_2$: C,68.32; H,5.73; N,4.98

Found : C,68.10; H,5.70; N,4.94

Example 35

4-[3-[4-[Bis(4-fluorophenyl)hydroxymethyl]-1-piperidinyl] propoxy]benzoic acid ethyl ester hydrochloride [1:1].

A mixture of 6.0 g (0.02 mole) of [α,α-bis(p-fluorophenyl]-4-piperidinemethanol, 5.0 g (0.02 mole) of 4-(3-chloropropoxy)benzoic acid methyl ester, 7.4 g (0.07 mole) of anhydrous sodium carbonate, 0.3 g of potassium iodide and 150 ml of dimethylformamide was heated on a steam bath for 20 hr and then poured into 1.5 liter of ice-water. A gum precipitated and the aqueous solution was decanted. The gum was dissolved in benzene and the solution was washed with water and dilute sodium hydroxide solution, dried over anhydrous sodium sulfate and concentrated under reduced pressure to give 9.2 g of gum as residue. The gum was purified by column chromatography on 200 g of Florisil® and the desired product was eluted with 20% acetone in benzene. The fractions containing the free base of the title compound were combined and concentrated under reduced pressure to give a gum, the free base, as residue. The free base was converted to the hydrochloric acid salt which was recrystallized from 2-propanol to give 5.3 g (49%) of white powder, m.p. 193.5-194.5°C.

Analysis: Calculated for $C_{30}H_{34}ClF_2NO_4$: C,65.99; H,6.28; N,2.57

Found : C,66.16; H,6.32; N,2.56

Example 36

4-[3-[4-[Bis(4-fluorophenyl)hydroxymethyl]-1-piperidinyl] propoxy]benzoic acid hydrochloride hydrate [1:1:0.5].

A solution of 2.7 g (0.005 mole) of 4-[3-[4-[bis(4-fluorophenyl)hydroxymethyl]-1-piperidinyl]propoxy]benzoic acid ethyl ester and 1.2 g (0.022 mole) of potassium hydroxide in 50 ml of ethanol and 20 ml of water was heated on a steam bath for 2 hr. Acetic acid, 10 ml, was added and the solution was poured into 500 ml of ice water and the mixture was allowed to stand at ambient temperature overnight. Sodium chloride was added to the mixture to give a coagulated solid. The solid was collected by filtration and air dried. The solid was dissolved in 20 ml of isopropyl alcohol and the solution was poured into 30 ml of ethereal hydrogen chloride. The salt which gradually crystallized was collected by filtration, washed with ethyl ether and dried to give 0.2 g (8%) of white powder, m.p. 148-158°C. with decomposition.

Analysis: Calculated for $C_{18}H_{30}ClF_2NO_4.0.5\ H_2O$: C,63.82; H,5.93; N,2.66

Found C,63.97; H,6.25; N,2.51

Example 37

4-[3-[4-[Bis(4-fluorophenyl)methylene]-1-piperidinyl] propoxy]benzoic acid ethyl ester hydrobromide [1:1].

A mixture of 6.09 g (0.021 mole)of 4-[bis(4-fluorophenyl) methylene]piperidine, 5.20 g (0.02 mole) of 1-[4-(3-chloro-propoxy)-phenyl]carbethoxybenzene and sodium carbonate 4.30 g (0.04 mole) in 230 ml of 1-butanol containing potassium iodide (0.3 g) was heated overnight at gentle reflux. The reaction mixture was stripped to dryness and partitioned between chloroform water and chloroform - 5% sodium hydroxide. Removal of chloroform gave an oil. The oil was converted to the hydrobromide salt using hydrogen bromide in glacial acetic acid. The acetic acid and excess hydrogen bromide were removed <u>in vacuo</u>. The salt was recrystallized from methanol-diethyl ether. The salt was washed with water to remove acetamide present as an impurity. The salt was washed with diethyl ether and dried <u>in vacuo</u> overnight at 80°C. A yield of 6.81 g (59.5%) of white solid, m.p. 192-194°C., was

obtained.

Analysis: Calculated for $C_{30}H_{32}NO_3F_2Br$: C,62.94; H,5.63; N,2.45
Found : C,62.83; H,5.58; N,2.45

Example 38

4-[3-[4-[Bis(4-fluorophenyl)methyl]-1-piperidinyl]propoxy]benzoic acid ethyl ester hydrobromide [1:1].

Following the procedure of Example 14, 4-[bis(4-fluorophenyl)methyl]piperidine and 4-(3-chloropropoxy)benzoic acid ethyl ester were reacted using potassium iodide as catalyst to give the free base which was reacted with hydrogen bromide in glacial acetic acid. The oil was stripped to dryness and the solid obtained was recrystallized from isopropyl alcohol-diethyl ether to give the white salt in 20% yield, m.p. 142-144°C.

Analysis: Calculated for $C_{30}H_{34}NO_3F_2Br$: C,62.72; H,5.97; N,2.44
Found : C,62.66; H,5.95; N,2.45

Example 39

4-[3-[4-[Bis(4-methoxyphenyl)methyl]-1-piperidinyl]propoxy]benzoic acid butyl ester.

A mixture of 6.22 g (0.02 mole) of 4-[bis(4-methoxyphenyl)methyl]piperidine, 4.84 g (0.02 mole) of 4-(3-chloropropoxy)benzoic acid ethyl ester, and potassium carbonate, 5.60 g (0.04 mole) in 350 ml of 1-butanol was refluxed overnight with potassium iodide. The reaction mixture was stripped to dryness and the residue partitioned between chloroform-5% sodium hydroxide then chloroform-water. Removal of chloroform gave an oil. This oil was chromatographed on a 200 g silica gel comumn packed in 50/50 v/v hexane-ethyl acetate. The material was eluted with hexane-ethyl acetate mixtures and finally 1% methanol-ethyl acetate.

From the chromatography was obtained 5.09 g (46.6%) of an oil.

Analysis: Calculated for $C_{34}H_{43}NO_5$: C,74.83; H,7.94; N,2.57
Found : C,74.19; H,7.91; N,2.53

The $^1H$ NMR spectrum was obtained in tetramethylsilane and is consistent with the structure indicated by the title, $\delta$ 8.0 (H's ortho to $CO_2$, 2H), 6.8 (m, aromatic, 10H), 4.2 (m, $CH_2$ alpha to O, 4H), 3.7 (S, $OCH_3$, 6H), 0.9-3.5 (m, aliphatics, 21H).

Example 40

4-[3-[4-[Bis(4-methoxyphenyl)methyl]-1-piperidinyl] propoxy]benzoic acid ethyl ester fumarate hydrate [1:1:0.5].

Following the procedure of Example 22, but substituting dimethylformamide at 73°C. for butanol, 4-[bis(4-methoxyphenyl)methyl]piperidine and 4-(3-chloropropoxy)benzoic acid ethyl ester were reacted to give the free base of the title compound which was reacted with fumaric acid, to give the white fumarate salt (recrystallizing from methanol-diethyl ether) in 27% yield, m.p. 147.5-148.5°C.

Analysis: Calculated for $C_{36}H_{44}NO_{9.5}$: C,67.27; H,6.90; N,2.18
Found : C,67.26; H,6.78; N,2.19

Example 41

4-[3-[4-[Bis(4-fluorophenyl)hydroxymethyl]-1-piperidinyl] ethoxy]benzoic acid ethyl ester hydrochloride.

Following the procedure of Example 35, $\alpha,\alpha$-bis(p-fluoro-phenyl)-4-piperidine methanol and 4-(2-chloroethoxy)benzoic acid ethyl ester are reacted and the hydrochloride salt is prepared.

Example 42

4-[3-[4-[Bis(4-fluorophenyl)hydroxymethyl]-1-piperidinyl] propoxy]-3-methoxybenzeneacetic acid ethyl ester hydrochloride.

Following the procedure of Example 35, [$\alpha,\alpha$-bis(p-fluorophenyl)]-4-piperidinemethanol and 4-(3-chloropropoxy)-3-methoxybenzeneacetic acid ethyl ester are reacted and the hydrochloride salt is prepared.

Example 43

4-[Bis(4-fluorophenyl)methylene]-1-[3-[4-(1,1-dimethylethyl)phenoxy]propyl]piperidine fumarate [1:1].

Following the procedure of Example 9, 4-[bis(4-fluorophenyl)methylene]piperidine and 4-(3-chloropropoxy)-(1,1-dimethylethyl)benzene were reacted using potassium iodide catalyst to give an oil which was dissolved in ethyl acetate and filtered through silica gel to give the free base of the title compound. The free base was reacted with fumaric acid to give the white fumarate salt (recrystallizing from isopropyl alcohol-diethyl ether) in 40% yield, m.p. 208.5-209.5°C.

Analysis: Calculated for $C_{35}H_{39}NO_5F_2$: C,71.05; H,6.64; N,2.37
Found : C,70.91; H,6.57; N,2.38

Example 44

4-[Bis(4-fluorophenyl)methyl]-1-[3-[4-(1,1-dimethylethyl)phenoxy]propyl]piperidine fumarate hydrate [1:1:0.5].

Following the procedure of Example 9, 4-[bis(4-fluorophenyl)methyl]piperidine and 4-(3-chloropropoxy)-(1,1-dimethylethyl)benzene were reacted using potassium iodide catalyst to give the free base of the title compound which was reacted with fumaric acid to give the white fumarate salt (recrystallizing from methanol-diethyl ether and isopropyl alcohol-diethyl ether) in 55% yield, m.p. 194-196°C. with decomposition.

Analysis: Calculated for $C_{35}H_{42}NO_{5.5}F_2$: C,69.75; H,7.02; N,2.32
Founc : C,70.01; H,6.89; N,2.44

Example 45

4-[Bis(4-methoxyphenyl)methyl]-1-[3-[4-(1,1-dimethylethyl)phenoxy]propyl]piperidine oxalate [1:1].

Following the procedure of Example 22, 4-[bis(4-methoxyphenyl)methyl]piperidine and 4-(3-chloropropoxy)-(1,1-dimethylethyl)benzene were reacted using potassium iodide catalyst to give the free base which was reacted with oxalic acid to give the white oxalate salt (recrystallizing from methanol-diethyl ether) in 35% yield, m.p. 212°C.

Analysis: Calculated for $C_{35}H_{45}NO_7$: C,71.04; H,7.67; N,2.37
Found : C,70.91; H,7.70; N,2.35

Example 46

1-[3-[4-(1,1-Dimethylethyl)phenoxy]propyl]-α,α-bis(4-fluorophenyl)-4-piperidinemethanol.

Following the procedure of Example 1, [α,α-bis(p-fluorophenyl)]-4-piperidinemethanol and 4-(3-chloropropoxy)-(1,1-dimethylethyl)benzene were reacted using potassium iodide catalyst to give white powder (recrystallizing from isopropyl alcohol) in 41% yield, m.p. 126-127°C.

Analysis: Calculated for $C_{31}H_{37}F_2NO_2$: C,75.43; H,7.56; N,2.84
Found : C,75.21; H,7.58; N,2.82

Example 47

4-[Bis(4-fluorophenyl)methyl]-1-[3-[3-(trifluoromethyl)phenoxy]propyl]piperidine oxalate [1:1].

Following the procedure of Example 9, 4-[bis-(4-fluorophenyl)methyl]piperidine and 1-[3-chloropropoxy]-3-trifluoromethylbenzene were reacted using potassium iodide catalyst to give the free base of the title compound which was reacted with oxalic acid to give the white oxalate salt (recrystallizing from methanol-diethyl ether) in 39% yield, m.p. 185-186°C.

Analysis: Calculated for $C_{30}H_{30}NO_5F_5$: C,62.17; H,5.22; N,2.42
Found : C,62.54; H,5.27; N,2.52

Example 48

N-[4-[3-[4-[Bis(4-methylphenyl)methyl]-1-piperidinyl]propoxy]phenyl] Acetamide fumarate hydrate [1:1:0.5].

Following the procedure of Example 22 but substituting dimethylformamide at 73°C. for refluxing butanol, 4-[bis-(4-methylphenyl)methyl]piperidine and N-[4-(3-chloropropoxy)phenyl]acetamide were reacted using potassium iodide catalyst to give the free base of the title compound which was reacted with fumaric acid to give the white fumarate hydrate (recrystallizing from methanol-diethyl ether), m.p. 149-152°C.

Analysis: Calculated for $C_{35}H_{43}N_2O_{6.5}$: C,70.57; H,7.28; N,4.70
Found : C,70.80; H,7.28; N,4.65

Example 49

N-[4-[3-[4-[Bis(4-fluorophenyl)methyl]-1-piperidinyl]propoxy]phenyl]acetamide hydrobromide [1:1].

A mixture of 25.68 g (0.089 mole) 4-[bis(4-fluorophenyl)methyl]piperidine, 20.3 g (0.089 mole) of N-[4-(3-chloropropoxy)phenyl]acetamide, and potassium carbonate, 21.4 g, (0.155 mole) was stirred overnight at 70-80°C. in 350 ml of dimethylformamide. The reaction mixture was stripped to dryness and the residue was partitioned between chloroform and water; removal of chloroform gave a dark red oil. The oil was dissolved in glacial acetic acid, and the hydrobromide salt was formed with hydrobromic acid in glacial acetic acid. Solvent was removed in vacuo, and the residue was recrystallized from methanol-diethyl ether. A yield of 21.68 g (43.5%) of pale-white solid, m.p. 223-225°C. was obtained.

Analysis: Calculated for $C_{29}H_{33}N_2O_2F_2Br$: C,62.26; H,5.95; N,5.01
Found : C,61.99; H,5.94; N,5.01

Example 50

4-[3-[4-[Bis(4-fluorophenyl)methyl]-1-piperidinyl]propoxy]benzeneamine fumarate hydrate [1:1:0.5].

A solution of 11.8 g (0.02709 mole) of N-[4-[3-[bis(4-fluorophenyl)methyl]-1-piperidinyl]propoxy]acetamide was heated at gentle reflux for four hours in 500 ml of methanol containing 500 ml of 6N hydrochloric acid. The reaction was stopped and allowed to cool overnight. The reaction mixture was evaporated to a small volume on the rotary evaporator, diluted with water and made alkaline with 5% sodium hydroxide. The reaction mixture was then partitioned between the alkaline phase and chloroform. The chloroform layer was dried, filtered, and solvent removed to give an oil. The oil was converted to the fumarate salt and the salt was recrystallized from methanol-diethyl ether. The white solid obtained was dried overnight in vacuo at 80°C. to give 8.49 g (71%) of white crystalline product, m.p. 121.5-124.0°C.

Analysis: Calculated for $C_{31}H_{35}N_2O_{5.5}F_2$: C,66.30; H,6.28; N,4.99
Found : C,66.49; H,6.13; N,4.92

Example 51

N-[4-[3-[4-[Bis(4-fluorophenyl)hydroxymethyl]-1-piperidinyl]propoxy]phenyl]acetamide hydrochloride hydrate [1:1:1].

A mixture of 3.0 g (0.01 mole) of [α,α-bis(p-fluorophenyl)]-4-piperidinemethanol, 2.3 g (0.01 mole) of N-[4-(3-chloropropoxy)phenyl]acetamide, 5.3 g (0.05 mole) of anhydrous sodium carbonate and 0.3 g of potassium iodide in 100 ml of 1-butanol gave a gum as residue. The gum was purified by column chromatography on 80 g of Florisil® and the product was eluted with 20% acetone in benzene. The combined fractions containing product were concentrated under reduced pressure to give a glass as residue. The glass was dissolved in ethyl ether, filtered through cotton, and the filtrate treated with ethereal hydrogen chloride. The resulting solid was collected by filtration, washed with ethyl ether and dried to yield 2.1 g (38%) of white solid, m.p. 135-170°C. (with decomposition).

Analysis: Calculated for $C_{29}H_{33}ClF_2N_2O_3 \cdot H_2O$: C,63.44; H,6.43; N,5.10

Found : C,63.32; H,6.56; N,4.92

Example 52

α,α-Bis(4-fluorophenyl)-1-[3-(4-nitrophenoxy)propyl]-4-piperidinemethanol.

Following the procedure of Example 1 and using potassium iodide catalyst, a mixture of 9.1 g (0.03 mole) of [α,α-bis(p-fluorophenyl)]-4-piperidinemethanol and 6.7 g (0.03 mole) of 1-(3-chloropropoxy)-4-nitrobenzene were reacted to give 10.5 g of the title compound which was recrystallized from isopropyl ether, m.p. 93.5-94.5°C.

Analysis: Calculated for $C_{27}H_{28}F_2N_2O_4$: C,67.21; H,5.85; N,5.81

Found : C,67.05; H,5.83; N,5.74

## Example 53

4-[3-[4-[Bis(4-fluorophenyl)hydroxymethyl]-1-piperidinyl] propoxy]benzamide.

Following the procedure of Example 1 and using potassium iodide catalyst, a mixture of 3.0 g (0.01 mole) of [α,α-bis (p-fluorophenyl)]-4-piperidinemethanol, 1 g (0.01 mole) of 4-(3-chloropropoxy)benzamide and 6.9 g (0.05 mole) of anhydrous potassium carbonate in 100 ml of 1-butanol were reacted to give 3.0 g (63%) of white powder, m.p., 200-204°C. The recrystallizing solvent used was absolute ethanol.

Analysis: Calculated for $C_{28}H_{30}F_2N_2O_3$: C,69.98; H,6.29; N,5.83
Found : C,69.61; H,6.49; N,5.70

## Example 54

4-[Bis(4-fluorophenyl)methyl]-1-[2-(1-naphthalenyloxy) ethyl]piperidine hydrochloride [1:1].

A mixture of 2.84 g (0.0099 mole) of 4-[α,α-bis(p-fluorophenyl)methyl]piperidine, 3.01 g (0.012 mole) of 1-(2-bromoethoxy)naphthalene and 5.0 g (0.060 mole) of sodium bicarbonate in 400 ml of 1-butanol was refluxed for 16 hr. The solvent was removed *in vacuo* and the residue was partitioned between methylene chloride and dilute sodium hydroxide. The methylene chloride solution was dried over magnesium sulfate, and the solvent was removed *in vacuo* to give an oil. This was dissolved in a mixture of ether and methanol, an excess of ethereal hydrochloride was added, and a white precipitate was collected to give 3.13 g (54%) of white crystalline solid, m.p. 155-158°C.

Analysis: Calculated for $C_{30}P_{30}MOF_2Cl$: C,72.94; H,6.12; N,2.84
Found : C,73.20; H,6.10; N,2.78

Example 55

4-[Bis(4-fluorophenyl)methyl]-1-[2-(2-naphthalenyloxy) ethyl]piperidine oxalate [1:1].

Following the procedure of Example 54 and substituting 2-(2-bromoethoxy)naphthalene and oxalic acid for hydrogen chloride, the title compound was obtained in 61.9% yield as white crystalline solid, m.p. 168-171°C.

Analysis: Calculated for $C_{32}H_{31}NO_5F_2$: C,70.19; H,5.71; N,2.56

Found : C,70.26; H,5.75; N,2.63

Example 56

1-[4-[3-[4-[Bis(4-fluorophenyl)methyl]-1-piperidinyl] propoxy]-3-methoxyphenyl]ethanone oxalate [1:1].

The title compound was prepared by the method described in U. S. Patent 3,956,296 (See Example 13 of that patent) as follows: A mixture of 4.75 g (0.0165 mole) of 4-[α,α-bis(p-fluorophenyl)methyl]piperidine, 4.0 g (0.0165 mole) of 3-(p-acetyl-o-methoxyphenoxy)propyl chloride and 1.4 g (0.0165 mole) of sodium bicarbonate in 60 ml of dimethylformamide was heated at 80°C. for about 2 hours. TLC showed no product at this point. The temperature was raised to 100°C. for 1 hr, at which time TLC showed the reaction to be complete. After cooling, the reaction mixture was filtered and the dimethylformamide was removed under reduced pressure. The crude product was dissolved in chloroform and filtered and the filtrate was concentrated under reduced pressure to give 7.7 g (94%) of crude product. The solid was dissolved in benzene and placed on a Florisil® column. Upon eluting with an acetone-benzene gradient, 5.5 g of product was obtained. The oxalate salt was prepared and upon recrystallization from isopropanol-methanol gave 3.8 g of salt, m.p. 164.5-166°C.

Analysis: Calculated for $C_{32}H_{35}F_2NO_7$: C,65.86; H,6.05; N,2.40

Found : C,66.11; H,6.13; N,2.39

Example 57

1-[4-[3-[4-[Bis(4-fluorophenyl)methyl]-1-piperidinyl] propoxy]-3-methoxyphenyl]ethanone fumarate [5:6].

A mixture of 58.26 g (0.203 mole) of 4-[bis(4-fluorophenyl)methyl)piperidine, 54.5 g (0.225 mole) of 1-chloro-3-(4-acetyl-2-methoxyphenoxy)propane, 18.7 g (0.223 mole) of sodium bicarbonate and 1.2 g (0.0072 mole) of potassium iodide in 800 ml of 1-butanol was refluxed for 16 hr. The hot reaction mixture was filtered, and the solvent was removed *in vacuo* from the filtrate. The residue was partitioned between methylene chloride and dilute sodium hydroxide. The methylene chloride solution was dried over magnesium sulfate, and the solvent was removed *in vacuo* to give an oil. The oil was dissolved in 600 ml of anhydrous ether, and 4.91 g of a solid was collected at room temperature. The ether solution was then treated with a solution of 30.2 g (0.26 mole) of fumaric acid in methanol. Anhydrous ether was added and 99.88 g (77.7%), m.p. 160-163°C. of title compound was isolated. This was recrystallized from isopropanol-diethyl ether, (2.5 g, 0.0216 mole of additional fumaric acid was added) to give 2 crops of title compound. [Crop I - 44.15 g, m.p. 163-164.5°C.; Crop II - 38.75 g, m.p. 161-163°C.]. An additional 10.00 g (8.786%), m.p. 159-162°C. of title compound collected from the original ether-methanol filtrate.

Analysis: Calculated for $C_{34.8}H_{37.8}NO_{7.8}F_2$*: C,66.05; H,6.02; N,2.21

Found : C,65.96; H,6.18; N,2.16

*NMR showed that the salt contained 1.2 equivalents of fumaric acid.

Example 58

1-[4-[3-[4-[Bis(4-fluorophenyl)methylene]-1-piperidinyl] propoxy]-3-methoxyphenyl]ethanone oxalate [1:1].

The title compound was prepared by the method described in U. S. Patent 3,922,276 (See Ex. 12 of that patent) as follows: A mixture of 4.7 g (0.0165 mole) of 4-[α,α-bis(p-fluorophenyl)methylene]piperidine, 4.0 g (0.0165 mole) of 3-(p-acetyl-o-methoxyphenoxy)propyl chloride and 1.4 g of sodium bicarbonate in 60 ml of dimethylformamide was heated

at 100°C. overnight. After cooling, the reaction mixture was filtered and the dimethylformamide was removed at reduced pressure. The residuel oil was dissolved in benzene and placed on a Florisil® column. Elution with a gradient of acetone-benzene gave 5.7 g (70%) of a viscous brown oil. The free base was reacted with oxalic acid to give the oxalate salt, m.p. 169-170°C. after recrystallization from isopropyl alcohol and drying under nitrogen.

Analysis: Calculated for $C_{32}H_{33}F_2NO_7$: C,66.08; H,5.72; N,2.41

Found : C,66.01; H,5.67; N,2.40

Example 59

1-[4-[3-[4-[(4-Fluorophenyl)phenylmethylene]-1-piperidinyl]propoxy]-3-methoxyphenyl]ethanone oxalate [1:1].

The title compound was prepared by the method described in U. S. 3,922,276 (See Ex. 12 of that patent) as follows: A mixture of 7.1 g (0.027 mole) of 4-[α-(p-fluorophenyl)-α-phenylmethylene]piperidine, 6.5 g (0.027 mole) of 3-(p-acetyl-o-methoxyphenoxy)propyl chloride and 2.3 g. (0.027 mole) of sodium bicarbonate in 100 ml of dimethylformamide was stirred and heated at 100°C. for approximately 8 hours. The mixture was filtered and the dimethylformamide was removed under reduced pressure. The residual oil was dissolved in chloroform and the mixture was filtered. The filtrate was concentrated under vacuum to give 11.5 g. of crude free base (92%). The free base was reacted with oxalic acid to give the oxalate salt, m.p. 143-145°C. after recrystallization from methylisobutyl ketone.

Analysis: Calculated for $C_{32}H_{34}FNO_7$: C,68.19; H,6.08; N,2.49

Found : C,68.14; H,6.12; N,2.54

Example 60

1-[3-Methoxy-4-[3-[4-[phenyl[3-(trifluoromethyl)phenyl] methylene]-1-piperidinyl]propoxy]phenyl]ethanone oxalate [1:1].

The title compound was prepared by the method described in U. S. Patent 3,922,276 (See Ex. 10 of that patent) as follows: A mixture of 5.0 g (0.0157 mole) of 4-[α-phenyl-α-(m-trifluoromethylphenyl)methylene]piperidine, 3.82 g (0.0157 mole) of 3-(p-acetyl-o-methoxyphenoxy)propyl chloride and 2.52 g (0.03 mole) of sodium bicarbonate in 75 ml of 1-butanol was stirred and heated at reflux for 17-1/2 hrs. . The mixture was cooled and filtered, and the filtrate was concentrated under reduced pressure. The glassy residue obtained weighed 4.25 g (52%) and was dissolved in benzene and placed on a Florisil® column. Using an acetone-benzene gradient elution, product was obtained as a glassy residue. This residue was dissolved in ether and the oxalate salt was obtained. The salt has a glassy appearance, m.p. 120-125°C.

Analysis: Calculated for $C_{33}H_{34}F_{3}NO_{7}$: C,64.59; H,5.58; N,2.28
Found : C,64.34; H,5.72; N,2.04

Example 61

1-[4-[3-[4{Cyclohexylphenylmethylene)-1-piperidinyl] propoxy]-3-methoxyphenyl]ethanone oxalate [1:1].

The free base of the title compound was obtained as in Example 1 of U. S. Patent 3,922,276 by reacting 4[(α-cyclohexyl-α-phenyl)methylene]piperidine with 3-(p-acetyl-o-methoxyphenoxy) propyl chloride in a mixture with sodium bicarbonate in dimethylformamide and converted to the oxalate salt, m.p. 184-185°C.

Analysis: Calculated for $C_{32}H_{41}NO_{7}$: C,69.67; H,7.49; N,2.54
Found : C,69.83; H,7.58; N,2.56

## Example 62

**1-[4-[3-[4-(Cyclohexylphenylmethyl)-1-piperidinyl] propoxy]-3-methoxyphenyl]ethanone oxalate hydrate [1:1:0.5].**

A mixture of 5.2 g (0.02 mole) of 4-[(α-cyclohexyl-α-phenyl)methyl]piperidine, 4.9 g (0.02 mole) of 3-(p-acetyl-o-methoxyphenoxy)propyl chloride and 1.7 g (0.02 mole) of sodium bicarbonate in 100 ml of dimethylformamide was stirred and heated at 100°C. for 4 hrs. The reaction mixture was cooled, filtered, and the dimethylformamide was removed under reduced pressure. The residual material was dissolved in benzene and placed on a Florisil® column. Elution using an acetone-benzene gradient gave 7.0 g (74.5%) of free base of the title compound. The oxalate salt was prepared and recrystallized from isopropanol, m.p. 155-160°C.

Analysis: Calculated for $C_{64}H_{88}N_2O_{15}$: C,68.31; H,7.88; N,2.49
Found : C,68.60; H,7.78; N,2.42

The free base of the title compound was obtained by reacting 4-[(α-cyclohexyl-α-phenyl)methyl]piperidine and 3-(p-acetyl-o-methoxyphenoxy)propyl chloride in a mixture with sodium bicarbonate, isolated and reacted with oxalic acid. The oxalate salt was recrystallized from isopropanol, m.p. 155-160°C.

## Example 63

**4-[3-[4-[Bis(4-fluorophenyl)methylene]-1-piperidinyl] propoxy]-alpha-methylbenzenemethanol oxalate [1:1].**

A solution of 1-[4-[3-[4-[bis(4-fluorophenyl)methylene]-1-piperidinyl]propoxy]phenyl]ethanone, 3.56 g (0.0077 mole) and sodium borohydride, 1.51 g (0.04 mole) was stirred 6 hrs at room temperature. The reaction mixture was stripped to dryness and partitioned between chloroform-water and chloroform-5% sodium hydroxide. Removal of chloroform gave an oil which was converted to the oxalate salt. Recrystallization from methanol-diethyl ether gave 2.67 g (62.1%) of white crystalline product, m.p. 142-145°C.

Analysis: Calculated for $C_{31}H_{33}NO_6F_2$: C,67.26; H,6.01; N,2.53
Found : C,67.17; H,5.92; N,2.47

Example 64

4-[3-[4-[Bis(4-fluorophenyl)methyl]-1-piperidinyl]propoxy]-3-methoxy-$\alpha$-methylbenzenemethanol.

Sodium borohydride (3.0 g, 0.079 mole) was added to 250 ml of 95% ethanol. To the mixture was added 4.40 g (0.00885 mole) of 1-[3-(p-acetyl-o-methoxyphenoxy)propyl] 4-[$\alpha$,$\alpha$-bis(p-fluorophenyl)methyl]piperidine in 100 ml of 95% ethanol over 15 minutes. The resulting solution was stirred 2-1/2 hr at room temperature. The reaction mixture was stripped to dryness and partitioned between chloroform and 5% sodium hydroxide. The organic layer was back extracted with 5% sodium hydroxide and water; removal of chloroform gave an oil. The oil formed a white solid in diethyl ether. The white solid was filtered off and recrystallized from methylene chloride-diethyl ether. This furnished 2.16 g (49.2%) of white solid, m.p. 132-135°C.

Analysis: Calculated for $C_{30}H_{35}NO_3F_2$: C,72.72; H,7.12; N,2.83

Found : C,72.28; H,7.21; N,2.52

Example 65

1-[4-[3-[4-(Diphenylmethyl)-1-piperidinyl]propoxy]-3-methoxyphenyl]ethanone oxalate [1:1].

A mixture of 5.0 g (0.02 mole) of 4-($\alpha$-phenylbenzyl) piperidine, 4.85 g (0.02 mole) of 3-(p-acetyl-o-methoxyphenoxy)propyl chloride, and 3.4 g (0.04 mole) of sodium bicarbonate in 100 ml of dimethylformamide was heated at 100°C. for about 3 hrs. The reaction mixture was cooled, filtered and the filtrate was concentrated under reduced pressure. The residual oil was dissolved in chloroform and the chloroform was filtered to remove insolubles. The filtrate was concentrated under reduced pressure to give 8.6 g of a red oil (94.5%). The oil was dissolved in a mixture of 4:1 ether-isopropanol and treated with 2.3 g of oxalic acid dihydrate. The oxalate salt crystallized upon standing and trituration in ether gave 8.4 g of salt melting at 149-155°C. Recrystallization from isobutyl methyl ketone gave 7.0 g of the salt, m.p. 153-155°C. (See Ex. 11, U. S.

3,956,296).

Analysis: Calculated for $C_{32}H_{37}NO_7$: C,70.18; H,6.81; N,2.56
Found : C,70.00; H,6.76; N,2.56

Example 66

1-[4-[3-[4-[Bis(4-fluorophenyl)hydroxymethyl]-1-piperidinyl]propoxy]-3-methoxyphenyl]ethanone.

A mixture of 5.0 g (0.0165 mole) of α,α-bis(p-fluorophenyl)-4-piperidinemethanol, 4.0 g (0.0165 mole) of 3-(p-acetyl-o-methoxyphenoxy)propyl chloride and 1.4 g (0.0165 mole) of sodium bicarbonate in 60 ml of dimethylformamide was stirred and heated at 80°C. for two hours. The temperature was raised to 100°C. for one hour. After cooling, the reaction mixture was filtered and the dimethylformamide was removed at reduced pressure. The residual oil which crystallized on standing in ether was dissolved in benzene and placed on a Florisil® column. Using a gradient elution of acetone-benzene, 1.8 g (21.4%) of product was obtained from the column, m.p. 141.5-143°C. (See Ex. 12, U.S. 3,956,296).

Analysis: Calculated for $C_{30}H_{33}F_2NO_4$: C,70.71; H,6.53; N,2.75
Found : C,70.49; H,6.58; N,2.59

Example 67

1-[4-[3-[4-[(4-Fluorophenyl)hydroxyphenylmethyl]-1-piperidinyl]propoxy]-3-methoxyphenyl]ethanone.

A mixture of 6.5 g (0.023 mole) of α-(p-fluorophenyl)-α-phenyl-4-piperidinemethanol, 5.5 g (0.023 mole) of 3-(p-acetyl-o-methoxyphenoxy)propyl chloride and 1.92 g (0.023 mole) of sodium bicarbonate in 80 ml of dimethylformamide was heated at 100-110°C. for 2 hrs. The reaction mixture was cooled and filtered and the dimethylformamide was removed at reduced pressure. The residual oil was dissolved in chloroform and filtered. The chloroform was removed at reduced pressure. The solid residue which remained weighed 8.6 g (77%) and was recrystallized from ethanol to give 3.1 g of material melting at 147-148°C. A sample was dried over refluxing toluene and submitted for analysis. (See

Ex. 14, U.S. 3,956,296).

Analysis: Calculated for $C_{30}H_{34}NO_4F$: C,73.30; H,6.97; N,2.85
Found : C,73.15; H,7.05; N,2.77

Example 68

1-[4-[3-[4-(Diphenylhydroxymethyl)-1-piperidinyl]propoxy]-3-methoxyphenyl]ethanone oxalate [1:1].

A mixture of 5.2 g (0.0194 mole) of α,α-diphenyl-4-piperidinemethanol, 4.7 g (0.0194 mole) of 3-(p-acetyl-o-methoxyphenoxy)propyl chloride and 1.6 g (0.0194 mole) of sodium bicarbonate in 60 ml of dimethylformamide was stirred at 100°C. for 3 hrs. After cooling, the reaction mixture was filtered and the dimethylformamide was removed under reduced pressure. The residual oil weighed 8.3 g (90%). Some of the product crystallized upon trituration in anhydrous ether and was collected by filtration. The filtrate was evaporated to dryness and the residue was dissolved in hot benzene-isooctane. Upon cooling, the crystalline product was obtained. A total yield of 6.3 g of solid product was obtained. The solid free base was converted to the oxalate salt. Recrystallization from isobutyl methyl ketone gave the off-white solid melting at 174-176°C. (See Ex. 15, U.S. 3,956,296).

Analysis: Calculated for $C_{32}H_{37}NO_8$: C,68.19; H,6.62; N,2.49
Found : C,68.34; H,6.75; N,2.42

Example 69

1-[4-[3-[4-[Hydroxyphenyl [3-(trifluoromethyl)phenyl]methyl]-1-piperidinyl]propoxy]-3-methoxyphenyl]ethanone hydrochloride hydrate [1:1:0.5].

A mixture of 7.0 g (0.021 mole) of α-phenyl-α-(m-trifluoromethylphenyl)-4-piperidinemethanol, 5.1 g (0.021 mole) of 3-(p-acetyl-o-methoxyphenoxy)propyl chloride and 3.0 g (0.036 mole) of sodium bicarbonate in 125 ml of dry dimethylformamide was stirred and heated at 90-95°C. for 5 hours. The mixture was cooled and filtered. An excess of water was added to the reaction mixture. The mixture was extracted several times with benzene and the collected extracts were dried over anhydrous sodium sulfate. The mixture was filtered and the filtrate was concentrated under reduced pressure. The

crude solid which was obtained was dissolved in benzene and placed on a Florisil® column. Elution using an acetone-benzene gradient gave a gummy solid. The gum was dissolved in ether and the hydrochloride salt was prepared. The hydrochloride salt weighed 3.1 g (25%) and became a clear melt at 95°C. (See Ex. 16, U.S. 3,956,296).

Analysis: Calculated for $C_{62}H_{72}Cl_2F_6N_2O_8$: C,63.42; H,6.18; N,2.39

Found : C,63.68; H,6.03; N,2.33

Example 70

1-[4-[3-[4-(Cyclohexylhydroxyphenylmethyl)-1-piperidinyl]propoxy]-3-methoxyphenyl]ethanone hydrochloride [1:1].

A mixture of 3.9 g (0.143 mole) of α-cyclohexyl-α-phenyl-4-piperidinemethanol, 3.5 g (0.143 mole) of 3-(p-acetyl-o-methoxyphenoxy)propyl chloride and 2.35 g (0.28 mole) of sodium bicarbonate in 100 ml of dimethylformamide was heated at 100°C. for 4 hrs. After cooling, the reaction mixture was diluted with about 600 ml of water and extracted with benzene. The collected benzene extracts were washed with water and dried over anhydrous magnesium sulfate. The mixture was filtered, and the filtrate was concentrated under reduced pressure. A crude solid weighing 5.1 g (74.5%) was obtained. The solid was dissolved in ether, and the ether solution was treated with an excess of ethereal hydrogen chloride. The hydrochloride salt obtained was recrystallized from isobutyl methyl ketone to give 4.0 g of the salt, m.p. 152-155°C. (See Ex. 17, U.S. 3,956,296).

Analysis: Calculated for $C_{30}H_{42}ClNO_4$: C,69.82; H,8.20; N,2.71

Found : C,69.50; H,8.31; N,2.62

Example 71

1-[4-[2-[4-[Bis(4-fluorophenyl)hydroxymethyl]-1-piperidinyl]ethoxy]-3-methoxyphenyl]ethanone.

Following the procedure of Example 1 and utilizing potassium iodide catalyst, a mixture of 3.0 g (0.01 mole) of $\alpha,\alpha$-bis(p-fluorophenyl)-4-piperidinemethanol, 2.3 g (0.01 mole) of 1-[4-(2-chloroethoxy)-3-methoxyphenyl] ethanone and sodium carbonate in butanol, the title compound was prepared in 22% yield, m.p. 131-135°C. after recrystallization from isopropyl alcohol.

Analysis: Calculated for $C_{28}H_{31}F_2NO_4$: C,70.29; H,6.31; N,2.83

Found : C,70.00; H,6.39 N,2.60

Example 72

1-[4-[4-[4-[Bis(4-fluorophenyl)hydroxymethyl]-1-piperidinyl]butoxy]-3-methoxyphenyl]ethanone.

This compound was prepared according to the procedure used to synthesize the compound of Example 35. A mixture of 3.0 g (0.01 mole) of $\alpha,\alpha$-bis(p-fluorophenyl)-4-piperidinemethanol, 3.0 g (0.01 mole) of 1-[4-(4-bromobutoxy)-3-methoxyphenyl]ethanone, 5.3 g (0.05 mole of anhydrous sodium carbonate and 0.3 g of potassium iodide in 100 ml of dimethylformamide gave, after purification by column chromatography on Florisil® (acetone-benzene). 0.8 g (15%) of off-white powder, m.p. 104-105°C. after recrystallization from 2-propanol-isopropyl ether.

Analysis: Calculated for $C_{31}H_{35}F_2NO$: C,71.11; H,6.74; N,2.68

Found : C,70.84; H,6.71; N,2.65

Example 73

1-[4-[5-[4-[Bis(4-fluorophenyl)hydroxymethyl]-1-piperidinyl] pentoxy]-3-methoxyphenyl]ethanone.

Following the procedure of Example 1 and utilizing potassium iodide catalyst, a mixture of 3.0 g (0.01 mole) of $\alpha,\alpha$-bis(p-fluorophenyl)-4-piperidinemethanol, 2.7 g (0.01 mole) of 1-[4-(5-chloropentoxy)-3-methoxyphenyl]ethanone and sodium carbonate in butanol, the title compound was prepared in 65% yield as white solid after recrystallization

from isopropyl alcohol, m.p. 117.5-118.5°C.

Analysis: Calculated for $C_{32}H_{37}F_2NO_4$: C,71.49; H,6.94; N,2.61

Found : C,71.51; H,7.06; N,2.50

Example 74

1-[4-[2-[4-[Bis(4-fluorophenyl)methyl]-1-piperidinyl]ethoxy]-3-methoxyphenyl]ethanone.

A mixture of 4-[bis(4-fluorophenyl)methyl]piperidine, 4.88 g (0.017 mole), 1-[4-(2-chloroethoxy)-3-methoxyphenyl]ethanone, 3.86 g (0.017 mole), and potassium carbonate, 5.53 g (0.04 mole) was heated overnight at gentle reflux in 350 ml of 1-butanol containing potassium iodide (0.3 g). The reaction mixture was filtered and stripped to dryness. The dark brown oil obtained was dissolved in chloroform and extracted with 1N sulfuric acid and 5% sodium hydroxide. The chloroform layer was dried, filtered, and solvent removed. This furnished a brown oil which was subjected to flash chromatography on silica gel using hexane-ethyl acetate for elution. A white solid was obtained by evaporating the fractions containing the product. The solid was extracted with diethyl ether and the mixture was placed in the freezer overnight. A white solid was obtained which was dried at 80°C. in vacuo overnight. A yield of 2.2 g (27%) of white crystalline solid, m.p. 129-131°C. was obtained.

Analysis: Calculated for $C_{29}H_{31}NO_3F_2$: C,72.63; H,6.52; N,2.92

Found : C,72.52; H,6.45; N,2.87

Example 75

1-[4-[3-[4-[Bis(4-chlorophenyl)methylene]-1-piperidinyl]propoxy]-3-methoxyphenyl]ethanone.

A mixture of 3.96 g (0.01305 mole) of 4-[bis(4-chlorophenyl)methylene]piperidine, 3.16 g (0.013 mole) of 1-[4-(3-chloropropoxy)-3-methoxyphenyl]ethanone in 300 ml of 1-butanol containing 0.3 g of potassium iodide was heated overnight at gentle reflux. The reaction mixture was stripped to dryness and partitioned between chloroform-water and chloroform-5% sodium hydroxide. Removal of

chloroform gave an oil which crystallized from isopropyl alcohol. The solid was again crystallized from isopropyl alcohol to give 4.16 g (61%) of light yellow solid, m.p. 143-144°C.

Analysis: Calculated for $C_{30}H_{31}NO_3Cl_2$: C,68.70; H,5.96; N,2.67
Found : C,69.11; H,6.02; N,2.55

Example 76

1-[4-[3-[4-[(4-Fluorophenyl)phenylmethyl]-1-piperidinyl]propoxy]-3-methoxyphenyl]ethanone oxalate [1:1].

A solution of 4.42 g (0.0164 mole) of 4-[(4-fluorophenyl)phenylmethyl]piperidine, 4.11 g (0.0170 mole) of 1-[4-(3-chloropropoxy)-3-methoxyphenyl]ethanone, and 0.01 g of potassium iodide in 1-butanol was refluxed for 18 hr. The solvent was removed in vacuo, and the residue was partitioned between methylene chloride and dilute sodium hydroxide. The solvent was removed in vacuo to give an oil. A solution of the oil in methanol was treated with an equivalent of oxalic acid, ethyl ether was added, and 6.39 g (68.9%) of white crystalline solid, m.p. 161-163°C. was obtained.

Analysis: Calculated for $C_{32}H_{36}NO_7F$: C,67.95; H,6.42; N,2.48
Found : C,67.92; H,6.42; N,2.44

Example 77

1-[4-[3-[4-[Bis(4-methoxyphenyl)methyl]-1-piperidinyl]propoxy]-3-methoxyphenyl]ethanone oxalate [1:1].

A mixture of 7.78 g (0.025 mole) of 4-[bis(4-methoxyphenyl)methyl]piperidine, 6.05 g (0.025 mole) of 1-[4-(3-chloropropoxy)-3-methoxyphenyl]ethanone, and potassium carbonate (5.53 g, 0.04 mole) in 300 ml of 1-butanol containing potassium iodide (0.3 g) was refluxed overnight. The reaction mixture was stripped to dryness and the residue was partitioned between chloroform and water; removal of chloroform in vacuo gave a dark brown oil. The oil was subjected to column chromatography on silica gel using a gradient elution composed of methanol and ethyl acetate. The corresponding fractions from the column were combined

and reacted with oxalic acid. Recrystallization of the salt from methanol-diethyl ether gave 4.16 g (27.4%) of white solid, m.p. 163.5-165°C.

Analysis: Calculated for $C_{34}H_{41}NO_8$: C,67.20; H,6.80; N,2.31
Found : C,66.76; H,6.84; N,2.26

Example 78

1-[4-[3-[4-[Bis(4-methylphenyl)methyl]-1-piperidinyl]propoxy]-3-methoxyphenyl]ethanone.

A mixture of 5.10 g (0.018 mole) of 4-[bis(4-methylphenyl)methyl]piperidine and 4.42 g (0.018 mole) of 1-[4-(3-chloropropoxy)-3-methylphenyl]ethanone in 350 ml of 1-butanol was heated overnight at gentle reflux with potassium carbonate (5.53 g, 0.04 mole) and potassium iodide (0.3 g). The reaction mixture was stripped to dryness and the resulting residue was partitioned between chloroform-5% sodium hydroxide and chloroform-water. Removal of chloroform gave a dark brown oil. The oil was subjected to column chromatography on a silica gel column with a gradient elution series of hexane-ethyl acetate and ethyl acetate-dimethoxy-ethane. The proper fractions from the column were combined. This resulted in 2.60 g (29.7%) of oil (after drying in vacuo at 80°C.overnight).

Analysis: Calculated for $C_{32}H_{39}NO_3$: C,79.14; H,8.09; N,2.88
Found : C,78.70; H,8.08; N,2.80

$^1H$ NMR ($CDCl_3$):

$\delta$7.5 (multiplet, aromatic protons next to ketone, 2H), 6.7-7.6 (multiplet, aromatic proton, 9H), 4.0 (triplet, methylene adjacent to ether oxygen,2H), 3.8 (singlet, $OCH_3$, 3H), 3.3 (doublet, methine next to rings, 1H), 2.5 (singlet, methyl of ketone, 3H), 2.2 (singlet, methyl groups attached to aromatic rings, 6H), 1.0-3.0 (multiplet, remaining aliphatic protons, 13H).

Example 79

1-[4-[4-[4-[Bis(4-fluorophenyl)methyl]-1-piperidinyl]butoxy]-3-methoxyphenyl]ethanone.

A mixture of 6.15 g (0.021 mole) of 4-[bis(4-fluorophenyl)methyl]piperidine and 6.45 g (0.02 mole) of 1-[4-(4-bromobutoxy)-3-methoxyphenyl]ethanone in 350 ml of acetonitrile was stirred overnight at room temperature with potassium carbonate, 5.53 g (0.04 mole) and potassium iodide (0.3 g). The mixture was then heated five hours at reflux. The reaction mixture was stripped to dryness on a rotary evaporator, and the residue was partitioned between chloroform-5% sodium hydroxide and chloroform-water. Removal of chloroform gave a dark brown oil. The oil was subjected to chromatography on a silica gel column and eluted with a hexane-ethyl acetate-dimethoxyethane series. Fractions from the column were combined and solvent removed by pumping in vacuo overnight at 80°C. This provided 3.34 g (31.3%) of brown oil.

| | | |
|---|---|---|
| Analysis: Calculated for $C_{31}H_{35}NO_3F_2$ | : C,73.35; | H,6.95; N,2.76 |
| Found | : C,72.34; | H,6.92; N,2.70 |

NMR analysis was obtained as follows:

$^1H$ NMR ($CDCl_3$):

$\delta$ 6.8-7.6 (multiplet, aromatics, 11H), 4.1 (triplet methylene next to ether linkage, 2H), 3.4-3.6 (doublet, methine attached to two fluorophenyl rings, 1H), 3.8 (singlet, $OCH_3$, 3H), 2.5 (singlet, $COCH_3$, 3H), 1.1-3.0 (multiplet, remaining aliphatics, 15H).

Example 80

4-[3-[4-[Bis(4-fluorophenyl)hydroxymethyl]-1-piperidinyl]propoxy]-3-methoxybenzoic acid methyl ester.

Following the procedure of Example 1 and utilizing potassium iodide catalyst and substituting dimethylformamide for butanol, a mixture of 5.4 g (0.021 mole) of 4-(3-chloropropoxy)-3-methoxybenzoic acid methyl ester, 6.0 g (0.02 mole) of [α,α-bis(p-fluorophenyl)]-4-piperidinemethanol, 7.4 g (0.07 mole) of anhydrous sodium carbonate and 0.3 g of potassium iodide in 150 ml of dimethylformamide was

reacted to give 5.7 g (53%) of white solid, m.p. 131-132.5°C. after recrystallization from isopropyl alcohol.

Analysis: Calculated for $C_{30}H_{33}F_2NO_5$: C,68.56; H,6.33; N,2.67
Found : C,68.23; H,6.35; N,2.60

Example 81

α,α-[Bis(4-fluorophenyl)]-1-[3-[4-(methylthio)phenoxy]propyl]-4-piperidinemethanol.

Following the procedure of Example 1, a mixture of 3.0 g (0.01 mole) of [α,α-bis(p-fluorophenyl)]-4-piperidinemethanol, 2.2 g (0.01 mole) of 1-chloro-3-(4-methylthiophenoxy)propane, 5.3 g (0.05 mole) of anhydrous sodium carbonate and 0.3 g of potassium iodide in 100 ml of 1-butanol was reacted to give 2.3 g (48%) of white powder, m.p. 113-115°C. after recrystallization from isopropyl ether.

Analysis: Calculated for $C_{28}H_{31}F_2NO_2S$: C,69.54; H,6.46; N,2.90
Found : C,69.57; H,6.51; N,2.85

Example 82

α,α-[Bis(4-fluorophenyl)]-1-[3-[4-(methylsulfonyl)phenoxy]propyl]-4-piperidinemethanol fumarate [1:1].

Following the procedure of Example 1, a mixture of 3.0 g (0.01 mole) of [α,α-bis(p-fluorophenyl)]-4-piperidinemethanol, 2.5 g (0.01 mole) of 1-(3-chloropropoxy)-4-(methylsulfonyl)benzene, 5.3 g (0.05 mole) of anhydrous sodium carbonate and 0.3 g of potassium iodide in 100 ml of 1-butanol was reacted to give a brown gum as residue. The gummy residue was reacted with fumaric acid and the fumarate salt obtained was recrystallized from acetonitrile to give 3.0 g (48%) of white solid, m.p. 176-178°C.

Analysis: Calculated for $C_{32}H_{35}F_2NO_8S$: C,60.85; H,5.59; N,2.22
Found : C,60.72; H,5.54; N,2.20

Example 83

4-[3-[4-[Bis(4-fluorophenyl)hydroxymethyl]-1-piperidinyl] propoxy]-3-methoxybenzeneacetic acid ethyl ester hydrochloride.

Following the procedure of Example 45, [α,α-bis(p-fluorophenyl)]-4-piperidinemethanol and 4-(3-chloropropoxy)-3-methoxybenzeneacetic acid, ethyl ester are reacted and the hydrochloride salt is prepared.

Example 84

4-[3-[4-[Bis(4-fluorophenyl)hydroxymethyl]-1-piperidinyl] ethoxy]benzoic acid ethyl ester hydrochloride.

Following the procedure of Example 45, α,α-bis(p-fluorophenyl)-4-piperidinemethanol and 4-(2-chloroethoxy)benzoic acid ethyl ester are reacted and the hydrochloride salt is prepared.

Example 85

4-[3-[4-[Bis(4-fluorophenyl)hydroxymethyl]-1-piperidinyl] propoxy]-3-methoxybenzeneacetic acid sodium salt hemihydrate.

This compound was prepared according to the procedure of Example 1. A mixture of 3.0 g (0.01 mole) of [α,α-bis(p-fluorophenyl)]-4-piperidinemethanol, 2.9 g (0.01 mole) of 4-(3-chloropropoxy)-3-methoxybenzeneacetic acid ethyl ester, 5.3 g (0.05 mole) of anhydrous sodium carbonate and 0.3 g of potassium iodide in 150 ml of acetonitrile gave the ester as a gum. The gum was converted to the hydrochloride with ethereal hydrogen chloride to give a white solid. The solid could not be recrystallized so it was partitioned between methylene chloride and a 5% sodium hydroxide solution. An emulsion resulted which was let stand until the layers separated. During this time a solid precipitated. The mixture was filtered. The filter cake was recrystallized from ethyl acetate to yield 0.7 g (13%) of fluffy, white solid, m.p. 102-112°C.

Analysis: Calculated for $C_{30}H_{32}F_2NNaO_5 \cdot 0.5\ H_2O$:
C,64.74; H,5.98; N,2.52

Found C,64.50; H,5.97; N,2.39

Example 86

7-[3-[4-[Bis(4-fluorophenyl)hydroxymethyl]-1-piperidinyl]propoxy]-2H-1-benzopyran-2-one.

This compound was prepared according to the procedure of Example 1. A mixture of 3.0 g (0.01 mole) of [α,α-bis (p-fluorophenyl)]-4-piperidinemethanol, 2.4 g (0.01 mole of 7-(3-chloropropoxy)-2H-1-benzopyran-2-one, 5.3 g (0.05 mole) of anhydrous sodium carbonate and 0.3 g of potassium iodide in 100 ml of 1-butanol gave 3.6 g (71%) of pale yellow crystals, m.p. 99-120°C. with decomposition. Recrystallizing solvent used was 2-propanol.

Analysis: Calculated for $C_{30}H_{29}F_2NO_4$: C,71.27; H,5.78; N,2.77

Found : C,71.02; H,5.89; N,2.63

Example 87

2-[3-[4-[Bis(4-fluorophenyl)hydroxymethyl]-1-piperidinyl]propoxy]benzoic acid ethyl ester fumarate [4:3].

This compound is prepared according to the procedure of Example 1. A mixture of 3.0 g (0.01 mole) of [α,α-bis (p-fluorophenyl)]-4-piperidinemethanol, 2.4 g (0.01 mole) of 2-(3-chloropropoxy)benzoic acid ethyl ester, 5.3 g (0.05 mole) of anhydrous sodium carbonate and 0.3 g of potassium iodide in 100 ml of dimethylformamide gave 5.7 g of gum as residue. The gum was purified by column chromatography on 100 g of silica gel. Fractions eluted with 35% acetone in benzene were combined and concentrated to give 3.0 g of pale yellow gum as residue. The gum was converted to the fumaric acid salt and the solid was recrystallized twice from 2-propanol to yield 2.0 g (32%) of white solid, m.p. 138-141°C.

Analysis: Calculated for $C_{33}H_{36}F_2NO_7$: C,66.43; H,6.08; N,2.35

Found : C,66.25; H,6.08; N,2.27

Example 88

2-[3-[4-[Bis(4-fluorophenyl)methyl]-1-piperidinyl]propoxy]benzoic acid ethyl ester.

A mixture of 32.79 g (0.116 mole) of 4-[bis(4-fluorophenyl)methyl]piperidine, 27.04 g (0.114 mole) of 2-(3-chloropropoxy)benzoic acid ethyl ester, and potassium

carbonate, 19.40 g (0.140 mole) was heated overnight at reflux in 500 ml of diethoxyethane containing potassium iodide (0.4 g). The reaction was filtered and stripped to dryness. The residue obtained was dissolved in chloroform and extracted with 5% sodium hydroxide, sodium sulfite, and water. The chloroform layer was dried (anhydrous sodium sulfate), filtered, and solvent removed to furnish a dark brown oil (56.20 g). The oil was subjected to flash chromatography on an 83.5 g silica gel column (eluted with ethyl acetate). Fractions were combined with similar purity. One sample of 6.49 g (56.5%) was dried in vacuo at 80°C. overnight and analyzed.

$^1H$ NMR ($CDCl_3$): $\delta$ 7.8 (m, 1, aromatic proton ortho to ester) 7.0 (m, 11, aromatic), 4.3 (q, 2, C-O-$CH_2$), 4.1 (t, 2, -$OCH_2$), 3.5 (d, 1, methine), 1,3 (t, 3, $CH_3$), 1.7-3.0 (m, 13, aliphatic).

Analysis: Calculated for $C_{30}H_{33}NO_3F_2$: C,73.00; H,6.74; N,2.84

Found : C,72.98; H,6.70; N,2.93

## Example 89

1-[4-[5-[4-[Bis(4-fluorophenyl)methyl]-1-piperidinyl]pentoxy]-3-methoxyphenyl]ethanone hemihydrate.

A mixture of 6.03 g (0.021 mole) of 4-[bis(4-fluorophenyl)methyl]piperidine, 5.69 g (0.021 mole) of 1-[4-(5-chloropentoxy)-3-methoxyphenyl]ethanone, and potassium carbonate (5.53 g, 0.04 mole) was heated overnight at gentle reflux in 350 ml of 1-butanol containing potassium iodide (0.2 g). The reaction mixture was cooled at room temperature, filtered, and stripped to dryness. The residue obtained was dissolved in chloroform and extracted several times with water. The chloroform layer was dried (sodium sulfate), filtered, and solvent removed to give a brown oil. This oil was subjected to flash chromatography on silica gel using ethyl acetate and 2% methanol-ethyl acetate for elution. Fractions of similar purity were combined and solvent removed.

The sample was dried in vacuo at 70°C. overnight after being exposed to the atmosphere for 24 hours. A yield of 2.7 g (24.6%) of brown oil was obtained.

$^{1}H$ NMR ($CDCl_3$): $\delta$6.8-7.6 (m, 11, aromatic), $\delta$4.1 (t, 2, $-OCH_3$), $\delta$3.9 (s, 3, $OCH_3$) $\delta$3.4-3.6 (d, 1, methine of difluorophenyl group), $\delta$2.5 (s, 3, $-\underset{O}{\overset{\|}{C}}-CH_3$), $\delta$1-3.0 (m, 18, aliphatics and 0.5 $H_2O$)

Analysis: Calculated for $C_{32}H_{38}NO_{3.5}F_2$: C,72.43; H,7.22; N,2.64
Found : C,72.75; H,7.23; N,2.57

Example 90

4-[3-[4-[Bis(4-fluorophenyl)methyl]-1-piperidinyl]propoxy]benzamide fumarate [2:3].

A mixture of 6.10 g (0.02125 mole) of 4-[bis(4-fluorophenyl)methyl]piperidine and 4.53 g (0.02125 mole) of 4-(3-chloropropoxy)benzamide in 350 ml of 1-butanol containing potassium carbonate (5.53 g, 0.02125 mole) and potassium iodide (0.2 g) was heated overnight at gentle reflux. The reaction was filtered and stripped to dryness. The residue obtained was dissolved in chloroform and extracted with water. The chloroform layer was dried, filtered, and solvent removed to give an oil. This material was converted to the fumarate salt and recrystallized from methanol-diethyl ether. The white crystalline solid obtained was dried in vacuo overnight at 65°C. A yield of 5.47 g (40.3%) of white crystalline product was obtained, m.p. 193-194°C.

Analysis: Calculated for $C_{34}H_{36}N_2O_8F_2$: C,63.94; H,5.68; N,4.39
Found : C,64.03; H,5.73; N,4.37

Example 91

4-[Bis(4-fluorophenyl)methyl]-1-[3-[4-(methylsulfonyl)phenoxy]propyl]piperidine oxalate [1:1].

A mixture of 6.02 g (0.021 mole) of 4-[bis(4-fluorophenyl)methyl]piperidine and 5.22 g (0.021 mole) of 1-(3-chloropropoxy)-4-(methylsulfonyl)benzene in 350 ml of 1-butanol containing potassium carbonate (5.53 g, 0.04 mole) and potassium iodide (0.2 g) was heated overnight at gentle reflux. The reaction was filtered and stripped to dryness. The residue obtained was dissolved in chloroform and extracted with water. The chloroform layer was dried, filtered, and solvent removed to give an oil. The dark brown oil was converted to the oxalate salt and recrystallized from methanol-diethyl ether to give a white solid. This material was dried in vacuo overnight at 65°C. A yield of 6.21 g (50.1%) of white crystalline solid, m.p. 202-204°C. was obtained.

Analysis: Calculated for $C_{30}H_{33}NSO_7F_2$: C,61.11; H,5.64; N,2.38
Found : C,60.99; H,5.64; N,2.36

Example 92

1-[4-[6-[4-[Bis(4-fluorophenyl)hydroxymethyl]-1-piperidinyl]hexyloxy]-3-methoxyphenyl]ethanone.

Following the procedure of Example 1 and utilizing potassium iodide catalyst, a mixture of [α,α-bis(p-fluorophenyl)]-4-piperidinemethanol and 1-[4-(6-chlorohexoxy)-3-methoxyphenyl]ethanone and sodium carbonate in butanol, the title compound is prepared.

Example 93

1-[4-[3-[4-[Bis(4-fluorophenyl)hydroxymethyl]-1-piperidinyl]propoxy]-2-methoxyphenyl]ethanone.

Following the procedure of Examples 1 and 66, [α,α-bis(p-fluorophenyl)]-4-piperidinemethanol and 3-(p-acetyl-m-methoxyphenoxy)propyl chloride are reacted to give the title compound.

Example 94

α,α-Bis(4-fluorophenyl)-1-[3-(2-hydroxyphenoxy)propyl]-4-piperidinemethanol.

Following the procedure of Example 2 and using potassium iodide catalyst, [α,α-bis(p-fluorophenyl)]-4-piperidinemethanol and 2-(3-chloropropoxy)-1-benzyloxybenzene are reacted to give 1-[3-(2-benzyloxyphenoxy)propyl]-α,α-bis(4-fluorophenyl)-4-piperidinemethanol which is reacted with hydrogen over palladium on carbon catalyst to give the title compound.

Example 95

α,α-[Bis(4-fluorophenyl)]-1-[3-[4-(methylsulfinyl)phenoxy]propyl]-4-piperidine methanol fumarate.

Following the procedure of Examples 1 and 82, [α,α-bis(p-fluorophenyl)]-4-piperidenemethanol and 1-(3-chloropropoxy)-4-(methylsulfinyl)benzene are reacted to give the free base of the title compound which is then reacted with fumaric acid to give the title compound.

Example 96

4-[3-[4-[Bis(4-fluorophenyl)hydroxymethyl]-1-piperidinyl]propoxy]benzenesulfonamide hydrochloride [1:1].

This compound was prepared according to the procedure of Example 1. A mixture of 3.0 g (0.01 mole) of [α,α-bis(p-fluorophenyl)]-4-piperidinemethanol, 2.5 g (0.01 mole) of 4-(3-chloropropoxy)benzenesulfonamide, 5.3 g (0.05 mole) of anhydrous sodium carbonate and 0.3 g of potassium iodide in 100 ml of 1-butanol gave a gum as residue. The gum was converted to the hydrochloride with ethereal hydrogen chloride and the solid was recrystallized from absolute ethanol to yield 3.5 g (64%) of white solid, m.p. 152-175°C.

Analysis: Calculated for $C_{27}H_{31}ClF_2N_2O_4S$: C,58.64; H,5.65; N,5.06

Found : C,58.43; H,5.68; N,5.06

Example 97

N-[4-[3-[4-[Bis(4-fluorophenyl)hydroxymethyl]-1-piperidinyl]propoxy]phenyl]methanesulfonamide.

Following the procedure of Example 1, [α,α-bis(p-fluorophenyl)]-4-piperidinemethanol and N-[4-(3-bromopropoxy)phenyl]methanesulfonamide are reacted to give the title compound.

Example 98

N-[4-[3-[4-[Bis(4-fluorophenyl)hydroxymethyl]-1-piperidinyl]propoxy]phenyl]-N'-methylurea.

Following the procedure of Example 1, [α,α-bis(p-fluorophenyl)]-4-piperidinemethanol and N-[4-(3-bromopropoxy)phenyl]-N'-methylurea are reacted to give the title compound.

Example 99

[4-[3-[4-[Bis(4-fluorophenyl)hydroxymethyl]-1-piperidinyl]propoxy]phenyl]carbamic acid ethyl ester.

Following the procedure of Example 1, [α,α-bis(p-fluorophenyl)]-4-piperidinemethanol and [4-(3-bromopropoxy)phenyl]carbamic acid ethyl ester are reacted to give the title compound.

Example 100

N-[3-[3-[4-[Bis(4-fluorophenyl)hydroxymethyl]-1-piperidinyl]propoxy]phenyl]urea.

Following the procedure of Example 1, [α,α-bis(fluorophenyl)]-4-piperidinemethanol and N-[3-(3-bromopropoxy)phenyl]urea are reacted to give the title compound.

Example 101

4-[3-[4-[Bis(4-fluorophenyl)hydroxymethyl]-1-piperidinyl]propoxy]-3-methoxybenzoic acid sodium salt.

Following the procedures of Examples 1 and 85 but substituting 4-(3-chloropropoxy)-2-methoxybenzoic acid for the corresponding 3-methoxy compound, the title compound is prepared.

Example 102

1-[4-[3-[4-[Bis(4-fluorophenyl)hydroxymethyl]piperidinyl]propoxy]-2-hydroxyphenyl]ethanone.

Following the procedure of Example 1, [α,α-bis(p-fluorophenyl)-4-piperidinemethanol and 1-[4-(3-bromopropoxy)-2-hydroxyphenyl]ethanone are reacted to give the title compound.

Example 103

7-[3-[4-[Bis(4-fluorophenyl)hydroxymethyl]-1-piperidinyl]propoxy]-4-oxo-4H-1-benzopyran-2-carboxylic acid ethyl ester hydrochloride.

A mixture of 3.0 g (0.01 mole) of [α,α-bis(p-fluorophenyl)]-4-piperidinemethanol, 3.1 g (0.01 mole) of 7-(3-chloropropoxy)-4-oxo-4H-1-benzopyran-2-carboxylic acid ethyl ester, 5.3 g (0.05 mole) of anhydrous sodium carbonate and 0.3 g of potassium iodide in 150 ml of acetonitrile heated at reflux for 48 hr gave a gum as residue. The gum was purified by column chromatography on 120 g of Florisil®. The desired fractions eluted with 10% acetone in benzene were combined and concentrated under reduced pressure to give a glass as residue. The glass was dissolved in ether-isopropanol and treated with ethereal hydrogen chloride. The solid which precipitated was collected by filtration and recrystallized from absolute ethanol to give 1.9 g (31%) of white solid, m.p. 191°C. with decomposition.

Analysis: Calculated for $C_{33}H_{34}ClF_2NO_6$: C,64.55; H,5.58; N,2.28

Found : C,64.41; H,5.51; N,2.26

Example 104

7-[3-[4-[Bis(4-fluorophenyl)hydroxymethyl]-1-piperidinyl]propoxy]-2,3-dihydro-4H-1-benzopyran-4-one hydrochloride.

Following the procedure of Example 103, [α,α-bis(p-fluorophenyl)]-4-piperidinemethanol and 7-(3-bromopropoxy)-2,3-dihydro-4H-1-benzopyran-4-one are reacted to give the title compound.

Example 105

1-[4-[3-[4-(Diphenylmethylene)-1-piperidinyl]propoxy]-3-methoxyphenyl]ethanone oxalate hydrate [1:1:0.5].

A mixture of 7.5 g (0.030 mole) of 4-diphenylmethylenepiperidine, 6.3 g (0.032 mole) of 3-(p-acetyl-o-methoxy-phenoxy)propyl bromide, 25 g of potassium carbonate and 150 ml of toluene was heated at reflux for 16 hrs, cooled, filtered and the solvent evaporated at reduced pressure. The residual oil was taken up in benzene, washed with water, dried over magnesium sulfate and then the solvent was evaporated. The free base was dissolved in isopropanol and treated with 3.8 g (0.03 mole) of oxalic acid dihydrate in dry ether. The white salt which separated was recrystallized from an isopropanol-methanol mixture. The product weighed 8.5 g (54%), m.p. 186-188°C.

Analysis: Calculated for $C_{32}H_{38}NO_{7.5}$: C,69.29; H,6.54; N,2.53
Found : C,69.20; H,6.49; N,2.71

Example 106

1-[4-[3-[4-(Cyclohexylphenylmethyl)-1,2,3,6-tetrahydro-pyridin-1-yl]propoxy]-3-methoxyphenyl]ethanone oxalate hydrate [1:1:0.5].

The free base of the title compound was obtained by reacting 4-(α-cyclohexylphenylmethyl)-1,2,3,6-tetrahydropyridine with 3-(p-acetyl-o-methoxyphenoxy)propyl chloride in a mixture with sodium bicarbonate in dimethylformamide and isolated on a Florisil® column eluting with benzene. The title salt was prepared, m.p. 110°C.

Analysis: Calculated for $C_{64}H_{84}N_2O_{15}$: C,68.55; H,7.55; N,2.50
Found : C,68.79; H,7.64; N,2.47

Example 107

1-[4-[3-[4-[Bis(4-fluorophenyl)hydroxymethyl]-1-piperidinyl]propoxy] -2-methoxyphenyl]ethanone hydrochloride [1:1].

This compound was prepared according to the procedure used to synthesize the compound of Example 1. A mixture of 3.0 g (0.01 mole) of [α,α-bis(p-fluorophenyl)]-4-piperidinemethanol, 2.4 g (0.01 mole) of 1-[4-(3-chloropropoxy)-2-methoxyphenyl]ethanone, 5.3 g (0.05 mole) of anhydrous sodium carbonate and 0.3 g of potassium iodide in 100 ml of 1-butanol gave a gum as residue. The gum was purified by column chromatography on 80 g of Florisil® and the fractions eluted with 20% acetone in benzene were combined and concentrated under reduced pressure to give a solid as residue. The solid was converted to the hydrochloride and this solid was recrystallized from 2-propanol-isopropyl ether to yield 2.2 g (40%) of white powder, m.p. 196-197°C.

Analysis: Calculated for $C_{30}H_{34}ClF_2NO_4$: C,65.99; H,6.28; N,2.57

Found : C,65.87; H,6.31 N,2.54

Example 108

4-[Bis(4-fluorophenyl)methyl]-1-[3-(2,6-dichlorophenoxy)propyl]piperidine.

A mixture of 4-[bis(4-fluorophenyl)methyl]piperidine (free base 6.90 g, 0.024 mole), 1,3-dichloro-2-(3-chloropropoxy)benzene (5.72 g, 0.024 mole), and potassium carbonate (5.54 g, 0.04 mole) was heated overnight at gentle reflux in 350 ml of 1-butanol containing potassium iodide (0.2 g). The reaction was stripped to dryness. The residue was partitioned several times between chloroform and water. The chloroform layer was dried, filtered, and solvent removed to give an oil. The oil was placed in the refrigerator overnight in 50 ml of methanol. A white solid was obtained and dried *in vacuo* overnight at 80°C. A yield of 3.26 g (27.7%) of white crystalline solid, m.p. 101.5-103°C. was obtained.

Analysis: Calculated for $C_{27}H_{27}NOCl_2F_2$: C,66.13; H,5.55; N,2.85

Found : C,66.12; H,5.56; N,2.88

Example 109

4-[Bis(4-fluorophenyl)methyl]-1-[3-(2,6-dichloro-phenoxy)propyl]piperidine oxalate [1:1].

Free base of the compound of Example 108 was converted to the oxalate salt and recrystallized from methanol-diethyl ether and dried *in vacuo* at 80°C. overnight, m.p. 158-161°C.

Analysis: Calculated for $C_{28}H_{29}NO_5Cl_2F_2$: C,60.01; H,5.04; N,2.44

Found : C,60.02; H,5.07; N,2.46

Example 110

2-[3-[4-[Bis(4-fluorophenyl)methyl]-1-piperidinyl] propoxy]benzonitrile.

A mixture of 7.41 g (0.025 mole) of 4-[bis(4-fluoro-phenyl)methyl]piperidine, 4.90 g (0.025 mole) of 2-(3-chloropropoxy)benzonitrile, and potassium carbonate, 5.54 g (0.04 mole) was heated overnight at reflux in 350 ml of 1-butanol containing potassium iodide (0.2 g). The mixture was stripped to dryness and the resulting residue was partitioned several times between water and chloroform. The chloroform layer was dried (anhydrous sodium sulfate), filtered, and solvent removed to give a brown oil. The oil was triturated with diethyl ether and placed in a freezer overnight. White crystals were obtained and dried *in vacuo* overnight at room temperature. A yield of 5.15 g (46.1%) of analytically pure material, m.p. 88.5-90°C. was obtained.

Analysis: Calculated for $C_{28}H_{28}N_2OF_2$: C,75.31; H,6.32; N,6.27

Found : C,75.16; H,6.34; N,6.26

Example 111

α,α- Bis(4-fluorophenyl)-1-[2-(phenylthio)ethyl]-4-piperidinemethanol maleate [1:1].

A Grignard solution was prepared in tetrahydrofuran (ice bath) from magnesium, 5.81 g (0.242 mole) and p-fluorobromobenzene, 42.4 g (0.242 mole). This Grignard reagent in about 350 ml of tetrahydrofuran was stirred about 3 hr at room temperature. The reaction mixture was then transferred to a 500 ml addition funnel (under nitrogen). This solution was added dropwise to a tetrahydrofuran solution of 1-[2-(phenylthio)ethyl]-4-piperidinecarboxylic acid ethyl ester, 29.10 g (0.1 mole) in about 200 ml of tetrahydrofuran. The solution was stirred overnight at room temperature, then poured onto ice containing 35 g of ammonium chloride. The solution was extracted with chloroform and the chloroform back extracted with 5% sodium hydroxide. Removal of chloroform gave a dark brown oil. The oil was converted to the maleate salt and recrystallized from methanol-diethyl ether to give 5.0 g (56.5% yield based on aliquot taken) of white crystalline product, m.p. 171-173°C.

Analysis: Calculated for $C_{30}H_{31}NO_5SF_2$: C,64.85; H,5.62; N,2.52

Found : C,64.80; H,5.62; N,2.45

Example 112

4-[Bis(4-fluorophenyl)methylene]-1-[2-(phenylthio)ethyl]piperidine.

α,α-Bis(4-fluorophenyl)-1-[2-(phenylthio)ethyl-4-piperidinemethanol maleate, 26.13 g (0.047 mole) was converted to the free base by partitioning with methylene chloride and weak alkaline solution and evaporating the organic layer to give an oil. The oil was dissolved in 150 ml of methanol containing 100 ml of 6 N hydrochloric acid and the solution was heated 4-1/2 hr at gentle reflux. The reaction mixture was cooled to room temperature, made alkaline with an ice-50% sodium hydroxide mixture and extracted with chloroform. The chloroform layer was evaporated to leave an oil which crystallized. Trituration of the solid in methanol followed by refrigeration and filtering gave the title compound,

m.p. 101.5-103.5°C. in 60% yield

Analysis: Calculated for $C_{26}H_{25}NSF_2$: C,74.08; H,5.98; N,3.32
Found : C,74.12; H,5.96; N,3.25

Example 113

α,α-Bis(4-fluorophenyl)-1-[2-[(4-chlorophenyl)sulfonyl]ethyl]-4-piperidinemethanol.

A mixture of 5.85 g (0.0193 mole) of α,α-bis(4-fluorophenyl)-4-piperidinemethanol, 4.76 g (0.020 mole) of 2-chloroethyl-p-chlorophenylsulfone and 4.90 g (0.0462 mole) of sodium carbonate in 600 ml of acetonitrile was heated at 65°C. for 18 hr. The solvent was removed in vacuo, and the residue was partitioned between methylene chloride and dilute sodium hydroxide. The methylene chloride solution was dried over sodium sulfate and the solvent was removed in vacuo to give a solid. This was recrystallized from a mixture of methylene chloride (400 ml), methanol (50 ml) and hexane (200 ml) to give 6.99 g (71.7%) of white crystalline solid, m.p. 211-212°C.

Analysis: Calculated for $C_{26}H_{26}NO_3SF_2Cl$: C,61.72; H,5.18; N,2.77
Found : C,61.51; H,5.16; N,2.81

Example 114

1-[2-[(4-Chlorophenyl)sulfonyl]ethyl]-4-[bis(4-fluorophenyl)methylene]piperidine.

A mixture of 2.74 g (0.0054 mole) of α,α-bis(4-fluorophenyl)-1-[2-[(4-chlorophenyl)sulfonyl]ethyl]-4-piperidinemethanol in 100 ml of glacial acetic acid and 40 ml of 2 M sulfuric acid was refluxed for 6 hr. The solvent was removed in vacuo and the resulting solid was recrystallized from methylene chloride-hexane to give 1.95 g (74%) of white crystalline solid, m.p. 152-153°C.

Analysis: Calculated for $C_{26}H_{24}NO_2SClF_2$: C,63.99; H,4.96; N,2.87
Found : C,64.24; H,4.95; N,2.84

Example 115

4-[Bis(4-fluorophenyl)methyl]-1-[3-(phenylsulfonyl)propyl]piperidine fumarate [1:1.5].

A mixture of 5.74 g (0.02 mole) of 4-[bis(4-fluorophenyl)methyl]piperidine, 4.36 g (0.02 mole) of 3-chloropropyl phenyl sulfone, 3.18 g (0.03 mole) of sodium carbonate, and potassium iodide (0.3 g) in 300 ml of n-butanol was refluxed overnight. The reaction mixture was stripped to dryness and the residue partitioned between chloroform-5% sodium hydroxide and then between chloroform-water. Removal of chloroform gave an oil which was converted to the fumarate salt. Recrystallization from isopropyl alcohol-diethyl ether gave 3.31 g (25.7%) of white solid, m.p. 172-173°C.

Analysis: Calculated for $C_{22}H_{35}NO_8F_2S$: C,61.58; H,5.48; N,2.18

Found : C,61.69; H,5.54; N,2.14

Example 116

4-[Bis(4-fluorophenyl)methyl]-1-[2-[(4-chlorophenyl)sulfonyl]ethyl]piperidine maleate [1:1].

A mixture of 4.22 g (0.0147 mole) of 4-[bis(4-fluorophenyl)methyl]piperidine, 3.63 g (0.0152 mole) of 2-chloroethyl-p-chlorophenyl sulfone, and 4.1 g (0.039 mole) of sodium carbonate in 400 ml of acetonitrile was refluxed for 22 hr. The solvent was removed in vacuo, and the residue was partitioned between methylene chloride and dilute sodium hydroxide. The methylene chloride solution was dried (magnesium sulfate) and the solvent was removed in vacuo to give an oil. This was converted to the maleate salt, and the salt was recrystallized from methanol-diethyl ether to give 6.84 g (76.9%) of white crystalline solid, m.p. 185-186°C.

Analysis: Calculated for $C_{30}H_{30}NO_6SF_2Cl$: C,59.45; H,4.99; N,2.31

Found : C,59.57; H,4.99; N,2.32

Example 117

4-[Bis(4-fluorophenyl)methyl]-1-[2-(phenylsulfonyl)ethyl]piperidine maleate [1:1].

A mixture of 5.20 g (0.016 mole) of 4-[bis(4-fluorophenyl)methyl]piperidine, 3.32 g (0.0162 mole) of 2-chloroethyl phenyl sulfone, and 500 g (0.0362 mole) of potassium carbonate in 500 ml of acetonitrile was refluxed for 19 hr. The solvent was removed in vacuo, and the residue was partitioned between methylene chloride and dilute sodium hydroxide. The organic solution was dried, (magnesium sulfate), and the solvent was removed in vacuo to give an oil. This was converted to the maleate salt, and the salt was recrystallized from methanol-ether to give 4.82 g (52.4%) of white crystalline solid, m.p. 183.5-184.5°C.

Analysis: Calculated for $C_{30}H_{31}NO_6F_2S$: C,63.04; H,5.47; N,2.45

Found : C,62.88; H,5.40; N,2.45

Example 118

1-(2,3-Dihydro-1,4-benzodioxan-2-ylmethyl)-α,α-diphenyl-4-piperidineacetonitrile.

A mixture of 6.24 g (0.027 mole) of 2-(bromomethyl)-1,4-benzodioxan, 0.027 mole of α,α-diphenyl-4-piperidineacetonitrile and potassium carbonate (6.91 g, 0.05 mole) was stirred overnight at room temperature. The reaction was refluxed for 5 hours and then stirred overnight at room temperature. The reaction mixture was stripped to dryness. The residue was dissolved in chloroform and the solution was extracted with water and 5% sodium hydroxide. Removal of chloroform gave an oil. NMR showed a 2/1 ratio of product to starting material. The reaction was then refluxed overnight in 350 ml of 1-butanol containing potassium iodide (0.3 g) and potassium carbonate (6.91 g, 0.05 mole). The reaction mixture was stripped to dryness, and the resulting residue was partitioned between chloroform-water and chloroform-5% sodium hydroxide. Removal of chloroform gave an oil which was crystallized from methanol and dried in vacuo at 80°C. overnight. A yield

of 7.18 g (62.6%) of white crystalline product, m.p. 130-131.5°C. was obtained.

Analysis: Calculated for $C_{28}H_{28}N_2O_2$: C,79.22; H,6.65; N,6.60
Found : C,78.88; H,6.62; N,6.56

Example 119

1-[3-(4-Acetyl-2-methoxyphenoxy)propyl]-α,α-diphenyl-4-piperidineacetonitrile oxalate [1:1].

A mixture of 6.78 g (0.05 mole) of 1-[4-(3-chloropropoxy)-3-methoxyphenyl]ethanone, 7.72 g (0.028 mole) of α,α-diphenyl-4-piperidineacetonitrile was heated overnight at reflux in 350 ml of 1-butanol containing potassium iodide (0.4 g). The reaction mixture was stripped to dryness, and the residue was dissolved in chloroform. The chloroform layer was extracted with 1N sulfuric acid and then with 5% sodium hydroxide. The chloroform was removed in vacuo to give a brown oil. The oil was converted to the oxalate salt, and the salt crystallized from methanol. The salt was dried in vacuo at 80°C. overnight. A yield of 8.36 g (52.2%) of white solid, m.p. 226-227°C. with decomposition was obtained.

Analysis: Calculated for $C_{33}H_{36}N_2O_7$: C,69.21; H,6.34; N,4.89
Found : C,68.78; H,6.32; N,4.84

Example 120

1-[3-(4-Acetyl-2-methoxyphenoxy)propyl]-α,α-diphenyl-3-piperidinepropanenitrile.

A mixture of 5.80 g (0.02 mole) of α,α-diphenyl-3-piperidinepropanenitrile, 4.84 g (0.02 mole) of 1-[4-(3-chloropropoxy)-3-methoxyphenyl]ethanone, and potassium carbonate, 5.60 g (0.04 mole) was heated overnight at 68°C. in 400 ml of dimethylformamide containing potassium iodide (0.3 g). The reaction mixture was stripped to dryness and partitioned between chloroform-water and chloroform-5% sodium hydroxide. The chloroform layer was dried, filtered, and solvent removed to give an oil. The oil was subjected to column chromatography on a silica gel column with elution

via ethyl acetate. Fractions containing the product were combined and transferred to brown glass bottles with acetone. The oil was triturated with diethyl ether and pumped to dryness *in vacuo* at 80°C. overnight. A yield of 2.37 g (23.9%) of oil was obtained.

Analysis: Calculated for $C_{32}H_{36}N_2O_3$: C,77.39; H,7.31; N,5.64
Found : C,77.00; H,7.35; N,5.63

Example 121

1-[4-(4-Acetyl-2-methoxyphenoxy)butyl]-α,α-diphenyl-3-piperidinepropanenitrile.

A mixture of 5.80 g (0.02 mole) of α,α-diphenyl-3-piperidinepropanenitrile, 6.02 g (0.02 mole) of 1-[4-(4-bromobutoxy)-3-methoxyphenyl]ethanone, and 5.60 g, (0.04 mole) of potassium carbonate was stirred overnight at room temperature in 400 ml of acetonitrile containing potassium iodide (0.3 g). The mixture was then stirred overnight at reflux, then stripped to dryness and the resulting residue was partitioned between chloroform-water and chloroform-5% sodium hydroxide. The chloroform layer was dried, filtered, and solvent removed to give a dark yellow oil. The oil was subjected to column chromatography on silica gel with elution via ethyl acetate. Pure fractions were combined, and the oil was triturated with diethyl ether. The oil was dried *in vacuo* at 80°C. overnight. A yield of 5.96 g (58.4%) of light yellow oil was obtained. $^1H$ NMR ($CDCl_3$) δ 7.2 - 7.8 (m, 12, aromatic), 6.9 (d, 1, aromatic), 4.1 (t, 2, $CH_2O$), 3.9 (s, 3, $-OCH_3$), 2.5 (s, 3, $C-CH_3$), 1.1 - 2.4 (m, 17, aliphatic).

Analysis: Calculated for $C_{33}H_{38}N_2O_3$: C,77.61; H,7.50; N,5.48
Found : C,76.96; H,7.55; N,5.35

Example 122

1-[3-(4-Acetyl-2-methoxyphenoxy)propyl]-α,α-diphenyl-3-pyrrolidineacetamide.

A mixture of 5.00 g (.018 mole) of α,α-diphenyl-3-pyrrolidineacetamide, 4.14 g (.018 mole) of 3-chloro-1-[(4-acetyl-2-methoxy)phenoxy]propane and 1.91 g (.018 mole) of sodium carbonate in 100 ml of 1-butanol was refluxed 18 hrs. The solution was cooled and concentrated _in vacuo_. The residue was taken up in 300 ml of methylene chloride, washed with 100 ml dilute sodium hydroxide, 100 ml dilute hydrochloric acid, and 100 ml dilute sodium hydroxide, dried over magnesium sulfate and concentrated to yield 7.70 g (88%) of yellow glass. The glass was crystallized from methylene chloride-diethyl ether to yield 5.70 g (67%) of a fine tan powder which contained ether. The powder was recrystallized from ethyl acetate and acetonitrile to give an off-white powder, m.p., 143.5-148.5C.

Analysis: Calculated for $C_{30}H_{34}N_2O_4$: C,74.05; H,7.04; N,5.76

Found : C,73.80; H,7.01; N,5.80

Example 123

1-[3-(4-Acetyl-2-methoxyphenoxy)propyl]-α,α-diphenyl-4-piperidineacetamide fumarate hydrate [1:0.5:1].

A mixture of 5.88 g (0.02 mole) of α,α-diphenyl-4-piperidineacetamide, 4.84 g (0.02 mole) of 1-[4-(3-chloropropoxy)-3-methoxyphenyl]ethanone, and 6.91 g (0.05 mole) potassium carbonate was heated at reflux overnight in 350 ml of 1-butanol containing potassium iodide (0.3 g). The reaction mixture was stripped to dryness. The residue obtained was partitioned between chloroform-water and chloroform-5% sodium hydroxide layer. The chloroform layer was dried, filtered, and solvent removed by rotary evaporator. The residue obtained was converted to the fumarate salt. The salt was recrystallized from methanol-diethyl ether. The white solid obtained was dried _in vacuo_ overnight at 80°C. A yield of 3.71 g (32.2%) of white solid,

m.p. 211-213°C. was obtained.

Analysis: Calculated for $C_{33}H_{40}N_2O_7$: C,68.73; H,6.99; N,4.86

Found : C,68.56; H,6.72; N,4.60

Example 124

1-[4-(4-Acetyl-2-methoxyphenoxy)butyl]-α,α-diphenyl-4-piperidineacetamide fumarate hydrate [1:0.5:1].

A mixture of 6.0 g (0.02 mole) of α,α-diphenyl-4-piperidineacetamide, 6.02 g (0.02 mole) of 1-[4-(4-bromobutoxy)-3-methoxyphenyl]ethanone and potassium carbonate (5.60 g, 0.04 mole) was stirred overnight at room temperature in 300 ml of acetonitrile containing potassium iodide (0.2 g). The mixture was then heated overnight at gentle reflux. The mixture was stripped to dryness and the residue obtained was partitioned between chloroform and water. The chloroform layer was dried, filtered, and solvent removed to give a gummy residue. This material was converted to the fumarate salt. The salt was recrystallized from methanol-diethyl ether and was dried in vacuo overnight at 80°C. to give 5.91 g (50.0%) of white solid, m.p. 169-171°C.

Analysis: Calculated for $C_{34}H_{42}N_2O_7$: C,69.13; H,7.17; N,4.74

Found : C,69.22; H,6.89; N,4.44

Example 125

1-[3-(4-Acetyl-2-methoxyphenoxy)propyl]-α,α-diphenyl-3-piperidinepropanamide hydrate [1:0.5].

A mixture of 7.00 g (0.0227 mole) of α,α-diphenyl-3-piperidinepropanamide, 5.50 g (0.0227 mole) of 1-[4-(3-chloropropoxy)-3-methoxyphenyl]ethanone and potassium carbonate (11.2 g, 0.08 mole) was heated overnight at reflux in 350 ml of 1-butanol containing potassium iodide (0.3 g). The reaction mixture was stripped to dryness and the resulting residue was partitioned between chloroform-water and chloroform-5% sodium hydroxide. The chloroform layer was dried, filtered, and solvent removed to give an oil. The oil was subjected to column chromatography on silica gel and eluted with dimethoxyethane-

ethyl acetate. Fractions of pure material were combined and dried *in vacuo* at 80°C. overnight. A yield of 3.75 g (31.5%) of a light yellow amorphous solid was obtained, $^1$H NMR ($CDCl_3$): $\delta$ 7.7 (m, 12, aromatic), 6.9 (d, 1, aromatic), 5.8 - 6.4 (br s, 2, $NH_2$), 5.1 (br s, 1-1/2 $H_2O$), 3.9 (t, 2, $CH_2O$), 3.9 (s, 3, $OCH_3$) 2.5 (s, 3, $C-CH_3$), 1.1 - 2.4 (m, 15, aliphatic).

Analysis: Calculated for $C_{32}H_{39}N_2O_{4.5}$: C,73.40; H,7.51; N,5.35

Found : C,73.42; H,7.47; N,5.22

Example 126

4-[Bis(4-fluorophenyl)methyl]-1-[(2,3-dihydro-1,4-benzodioxan-2-yl)methyl]piperidine oxalate [1:1].

A mixture of 5.53 g (0.019 mole) of 4-[bis(4-fluorophenyl)methyl]piperidine, 4.39 g (0.019 mole) of 2-(bromomethyl)-2,3-dihydro-1,4-benzodioxan, and potassium carbonate 7.80 g (0.056 mole) was stirred overnight at room temperature in 250 ml of acetonitrile. The reaction mixture was stripped to dryness and partitioned between chloroform and water. Removal of chloroform gave a yellowish brown oil which was converted to the oxalate salt. The salt was recrystallized from methanol-diethyl ether. A yield of 3.63 g (36.3%) of white solid, m.p. 142-145°C. was obtained.

Analysis: Calculated for $C_{29}H_{29}NO_6F_2$: C,66.28; H,5.56; N,2.61

Found : C,66.06; H,5.50; N,2.61

Example 127

1-[2-(2,6-Dichlorophenoxy)ethyl]-$\alpha,\alpha$-diphenyl-3-piperidinepropanenitrile.

A mixture of 6.09 g (0.021 mole) of $\alpha,\alpha$-diphenyl-3-piperidinepropanenitrile and 5.63 g (0.021 mole) of 2-(2-bromoethoxy)-1,3-dichlorobenzene in 350 ml of acetonitrile was stirred overnight at room temperature with potassium carbonate, 5.53 g (0.04 mole) and 0.2 g of potassium iodide. The mixture was then heated overnight at gentle reflux. The mixture was then stripped to dryness and the resulting residue was dissolved in chloroform. The chloroform was extracted with 5% sodium hydroxide and water. The chloroform

layer was then dried, filtered, and solvent removed to give 12.2 g of oil. The oil was subjected to flash chromatography on silica gel using 20% ethyl acetate-hexane and 50% ethyl acetate-hexane for elution. Fractions of pure material were combined and solvent removed *in vacuo*. The brown oil was dried overnight *in vacuo* at 80°C. A yield of 5.49 g (54.5%) of brown oil was obtained.

$^1$H NMR ($CDCL_3$) $\delta$ 6.8-7.4 (m, 13, aromatic), 4.1 (t, 2, $-OCH_2$), 0.7-2.9 (m, 13, remaining aliphatic)

Analysis: Calculated for $C_{28}H_{28}N_2OCl_2$: C,70.14; H,5.89; N,5.84
Found : C,70.08; H,5.88; N,5.76

Example 128

1-[5-(4-Acetyl-2-methoxyphenoxy)pentyl]-α,α-diphenyl-3-piperidinepropanenitrile hydrate [1:0.5].

A mixture of 5.80 g (0.02 mole) of α,α-diphenyl-3-piperidinepropanenitrile, 5.42 g (0.02 mole) of 1-[4-(5-chloropentoxy)-3-methoxyphenyl]ethanone, and potassium carbonate, 5.53 g (0.04 mole) was heated overnight at gentle reflux in 350 ml of 1-butanol containing potassium iodide (0.3 g). The reaction mixture was stripped to dryness and the residue dissolved in chloroform. The chloroform layer was extracted with water and then dried with anhydrous sodium sulfate. The chloroform was removed by rotary evaporation following filtration. The oil obtained upon removal of chloroform was subjected to column chromatography (flash chromatography) on silica gel with methanol-ethyl acetate (4:96 v/v) for elution. Similar fractions were combined and removal of solvent gave a clear brown oil. The oil was dried *in vacuo* overnight at 80°C. The oil was triturated with diethyl ether and dried *in vacuo* again, at 80°C. overnight. A yield of 6.37 g (59.6%) of clear brown oil was obtained,

$^1$H NMR ($CDCl_3$) $\delta$ 7.2-7.8 (m, 12, aromatic), 6.9 (d, 1, aromatic), 4.1 (t, 2, $CH_2O$), 3.9 (s, 3, $OCH_3$), 2.5 (s, 3, $\overset{O}{\ddot{C}}-CH_3$), 1.1-2.4 (m, 19, aliphatic).

Analysis: Calculated for $C_{34}H_{41}N_2O_{3.5}$: C,76.52; H,7.74; N,5.25
Found : C,76.37; H,7.65; N,5.19

Example 129

1-[3-(4-Acetyl-2-methoxyphenoxy)propyl]-α,α-bis(4-fluorophenyl)-4-piperidineacetonitrile maleate [1:1].

A mixture of α,α-bis(4-fluorophenyl)-4-piperidineacetonitrile, 6.45 g (0.021 mole), 1[4-(3-chloropropoxy)-3-methoxyphenyl]ethanone (5.00 g, 0.021 mole), and potassium carbonate, 5.53 g (0.04 mole), was heated overnight at gentle reflux in 350 ml of 1-butanol containing potassium iodide (0.2 g). The reaction mixture was stripped to dryness and then partitioned several times between chloroform and water. The chloroform layer was dried, filtered, and solvent removed to give a dark brown oil. The oil was converted to the maleate salt. The salt was recrystallized from methanol-diethyl ether and dried in vacuo at 80°C. overnight. A yield of 6.80 g (51.8%) of white crystals, m.p. 158-160°C. was obtained.

| | | |
|---|---|---|
| Analysis: Calculated for $C_{35}H_{36}N_2O_7F_2$: | C,66.24; | H,5.72; N,4.14 |
| Found : | C,66.13; | H,5.69; N,4.41 |

Example 130

1-[3-(2,6-Dichlorophenoxy)propyl]-α,α-bis(4-fluorophenyl)-4-acetonitrile maleate [1:1].

A mixture of 5.99 g (0.0192 mole) of α,α-bis(4-fluorophenyl)-4-piperidineacetonitrile oxalate and 1,3-dichloro-2-(3-chloropropoxy)benzene, 4.76 g (0.02 mole) was heated at reflux overnight in 350 ml of 1-butanol containing potassium carbonate, 5.53 g (0.04 mole), and potassium iodide, 0.2 g. The reaction mixture was stripped to dryness and the resulting residue was partitioned several times between water and chloroform. The chloroform layer was dried (anhydrous sodium sulfate), filtered, and solvent removed to give a light brown oil. The entire oil was converted to the maleate salt. The salt was recrystallized from methanol-diethyl ether, and dried in vacuo overnight at 80°C. A yield of 4.55 g (37.5%) of white crystals, m.p. 162-163°C. was obtained.

Analysis: Calculated for $C_{32}H_{30}N_2O_5F_2Cl_2$: C,60.83; H,4.79; N,4.14

Found : C,60.89; H,4.82; N,4.44

Example 131

1-[3-(2,6-Dichlorophenoxy)propyl]-α,α-diphenyl-3-piperidinepropanenitrile oxalate [1:1].

A mixture of 5.80 g (0.02 mole) of α,α-diphenyl-3-piperidinepropanenitrile and 1,3-dichloro-2-(3-chloropropoxy)benzene, 4.76 g (0.02 mole) in 350 ml of 1-butanol was heated overnight at reflux with potassium carbonate (5.54 g, 0.04 mole) and potassium iodide (0.2 g). The reaction mixture was stripped to dryness and the residue partitioned several times between water and chloroform. The chloroform layer was dried (anhydrous sodium sulfate), filtered, and solvent removed to give an oil. The entire oil was converted to the oxalate salt. The salt was recrystallized from methanol-diethyl ether, and was dried *in vacuo* overnight at 80°C. A yield of 7.23 g (62%) of white crystals, m.p. 159-161°C. was obtained.

Analysis: Calculated for $C_{31}H_{32}N_2O_5Cl_2$: C,63.81; H,5.53; N,4.80

Found : C,64.02; H,5.61; N,4.86

Example 132

1-[4-(4-Acetyl-2-methoxyphenoxy)butyl]-α,α-bis(4-fluorophenyl)-4-piperidineacetonitrile fumarate [1:1].

A mixture of 5.38 g (0.0172 mole) of α,α-bis(4-fluorophenyl)-4-piperidineacetonitrile and 5.20 g (0.0172 mole) of 1-[4-(4-bromobutoxy)-3-methoxyphenyl]ethanone was heated overnight at reflux in 350 ml of acetonitrile containing potassium carbonate, 5.54 g (0.04 mole) and potassium iodide, 0.2 g. The reaction mixture was stripped to dryness and the residue obtained was partitioned several times between chloroform and water. The chloroform layer was dried (anhydrous sodium sulfate), filtered, and solvent removed to give an oil. The oil was converted to the fumarate salt. The salt was recrystallized from methanol-diethyl ether, and was dried *in vacuo* overnight at

80°C. A yield of 5.78 g (51.7%) of white crystals, m.p. 181.5-182°C. was obtained.

Analysis: Calculated for $C_{36}H_{38}N_2O_7F_2$: C,66.66; H,5.90; N,4.32

Found : C,66.56; H,5.92; N,4.28

Example 133

1-[2-(2,6-Dichlorophenoxy)ethyl]-α,α-diphenyl-3-piperidinepropanamide.

A mixture of 7.65 g (0.025 mole) of α,α-diphenyl-3-piperidinepropanenitrile, 2-(2-bromoethoxy)-1,3-dichlorobenzene (6.70 g, 0.025 mole), and potassium carbonate, 5.54 g (0.04 mole) was stirred overnight at reflux in 350 ml of acetonitrile containing potassium iodide, (0.2 g). The reaction mixture was stripped to dryness and the residue was partitioned between chloroform and water several times. The chloroform layer was dried (anhydrous sodium sulfate), filtered, and solvent removed to give 12.91 g of brown oil. The entire oil was subjected to flash chromatography on silica gel using 75-25 v/v ethyl acetate-hexane and 100% ethyl acetate for elution. Similar fractions were combined and solvent was removed. The resulting oil was dried in vacuo overnight at 65°C. A yield of 6.98 g (56.1%) of clear oil was obtained.
$^1H$ NMR ($CDCl_3$): δ 6.9-7.5 (m, 13, aromatic), 6.0 (br 2, $NH_2$), 4.0 (t, 2, $O-CH_2$), 2.7 (t, 2, $N-CH_2$), 1.0-2.3 (m, 11, aliphatics).

Analysis: Calculated for $C_{28}H_{30}N_2O_2Cl_2$: C,67.61; H,6.08; N,5.63

Found : C,67.52; H,6.08; N,5.63

Example 134

α-[1-[3-(4-Acetyl-2-methoxyphenoxy)propyl]-4-piperidinyl]-α-(4-fluorophenyl)-2-pyridineacetonitrile fumarate [1:1].

A mixture of α-(4-fluorophenyl)-α-(4-piperidinyl)-2-pyridineacetonitrile (7.18 g, 0.024 mole), 1-[4-(3-chloropropoxy)-3-methoxyphenyl]ethanone (5.89 g, 0.024 mole), and potassium carbonate (5.54 g, 0.04 mole) was

heated overnight in 350 ml of 1-butanol containing potassium iodide (0.15 g). The reaction mixture was stripped to dryness and the residue obtained was partitioned between chloroform and water. The chloroform layer was extracted with 1N sulfuric acid, 5% sodium hydroxide and water. The chloroform layer was dried over sodium sulfate, filtered, and the solvent removed to give an oil. The oil was converted to the fumarate salt and recrystallized from methanol-diethyl ether. A white solid was obtained and dried <u>in vacuo</u> overnight at 80°C. to give 9.43 g (62.7%) of white crystals, m.p. 166-167°C.

Analysis: Calculated for $C_{24}H_{36}N_3O_7F$: C,66.11; H,5.87; N,6.80

Found : C,65.57; H,5.89; N,6.70

*(1/4 $H_2O$ found by NMR).

<u>Example 135</u>

<u>α-[1-[3-(4-Acetyl-2-methoxyphenoxy)propyl[-4-piperidinyl]-α-(4-fluorophenyl)-2-pyridineacetonitrile fumarate hydrate [1:1:1].</u>

A portion of the compound prepared in Example 134 was exposed to the air for 3 days, m.p. 166-167°C.

Analysis: Calculated for $C_{34}H_{38}N_3O_8F$: C,64.24; H,6.02; N,6.61

Found : C,64.07; H,5.82; N,6.58

<u>Example 136</u>

<u>α,α-Diphenyl-1-[3-(8-quinolinyloxy)propyl]-3-piperidinepropanenitrile hydrate [1:0.5].</u>

A mixture of α,α-diphenyl-3-piperidinepropanenitrile (8.12 g, 0.028 mole), 8-(3-chloropropoxy)quinoline (6.18 g, 0.028 mole) and potassium carbonate (5.53 g, 0.04 mole) was heated at reflux overnight in 350 ml of 1-butanol containing potassium iodide (0.3 g). The reaction mixture was filtered and stripped to dryness on a rotary evaporator. The residue obtained was dissolved in chloroform and extracted with 5% sodium hydroxide and water. The chloroform layer was dried over anhydrous sodium sulfate, filtered, and solvent removed to give a dark red mass. This material was subjected to flash chromatography on a

silica gel column using 10% methanol-ethyl acetate, 20% methanol-ethyl acetate, and 50% methanol-ethyl acetate for elution. Fractions of similar purity were combined and solvent was removed by rotary evaporator. The residue obtained was dried *in vacuo* at 80°C. overnight to give 5.29 g (39%) of a dark black residue.

$^1H$ NMR ($CDCl_3$): $\delta$ 8.9 (m, 1, proton ortho to N in ring), 7.9-8.1 (m, 1, proton para to N in ring), 6.9-7.6 (m, 14, aromatics), 4.2 (t, 2, methylenes adjacent to oxygen atom), 1.1-2.7 (m, 16, aliphatic protons and 1H from 0.5 $H_2O$).

| | | | |
|---|---|---|---|
| Analysis: Calculated for $C_{32}H_{33}N_3O_{1.5}$: | C,79.31; | H,7.07; | N,8.67 |
| Found : | C,79.47; | H,7.19; | N,8.69 |

Example 137

8-[3-[4-[Bis(4-fluorophenyl)methyl]-1-piperidinyl]propoxy]quinoline hydrate [1:0.5].

A mixture of 4-($\alpha$-p-fluorophenyl)-p-fluorobenzylpiperidine (8.03 g, 0.028 mole), 8-(3-chloropropoxy) quinoline (6.18 g, 0.028 mole), and potassium carbonate (5.53 g, 0.04 mole) was heated overnight at gentle reflux in 350 ml of 1-butanol containing potassium iodide (0.3 g). The reaction mixture was filtered through activated charcoal and solvent was removed by rotary evaporator. The residue was dissolved in chloroform and then extracted with 5% sodium hydroxide and water. The chloroform layer was dried over anhydrous sodium, filtered, and solvent removed to provide a dark red mass. This material was subjected to flash chromatography on silica gel using 10, 20 and 50% methanol in ethyl acetate for elution. Fractions with similar purity were combined and solvent removed. The residue was dried at 80°C. *in vacuo* overnight to give 3.74 g (28%) of a dark red product.

$^1H$ NMR ($CDCl_3$): $\delta$ 8.9 (m, 1, proton ortho to N in ring), 7.9-8.1 (m, 1, proton para to N in ring), 6.8-7.4 (m, 12, aromatic), 4.2 (t, 2, $CH_2$ attached to -O), 3.5 (d, 1, methine attached to two aromatic rings), 1.0-3.2 (m, 14, aliphatic protons and 1H for 0.5 $H_2O$).

Analysis: Calculated for $C_{30}H_{30.50}N_2O_{7.5}F_2$: C,75.53; H,6.44; N,5.87

Found :C,75.66; H,6.56; N,5.86

Example 138

2-[3-[4-[Bis(4-fluorophenyl)methyl]-1-piperidinyl]propoxy]benzoic acid hydrate [1:0.5].

A solution of 2-[3-[4-[bis(4-fluorophenyl)methyl]-1-piperidinyl]propoxy]benzoic acid ethyl ester (25.3 g, 0.051 mole) in 400 ml of 200 ethanol containing potassium hydroxide (16.8 g, 0.30 mole) was heated at reflux for 4 hours. The reaction mixture was concentrated to a volume of approximately 200 ml. The reaction mixture was made acidic with 1N sulfuric acid. The acidic layer was extracted with chloroform. The chloroform layer was dried over anhydrous sodium sulfate, filtered, and solvent removed to give a gummy residue. The material was subjected to flash chromatography using 20% methanol-80% ethyl acetate and 50% methanol-50% ethyl acetate for elution. Fractions of similar purity were combined. Solvent was removed *in vacuo* and the residue was dried *in vacuo* overnight at 80°C. to give 5.72 g (23.6%) of material.

$^1H$ NMR ($CDCl_3$) $\delta$ 9.2 (br 2, COOH and 0.5 $H_2O$), 7.8 (m, 1, H next to COOH), 6.7-7.3 (m, 11, aromatic), 4.1 (m, 2, $CH_2$ next to O), 1.5-3.66 (m, 14, aliphatics).

Analysis: Calculated for: $C_{28}H_{30}NO_{3.5}F_2$: C,70.87; H,6.37 N,2.95

Found : C,71.42; H,6.31; N,3.01

Example 139

4-[Bis(4-fluorophenyl)methyl]-N-phenyl-1-piperidinepropanamine maleate [1:2].

A solution of 4-[bis(4-fluorophenyl)methyl]-1-(3-chloropropyl)piperidine (35.27 g, 0.097 mole) in 200 ml of aniline (204.4, 2.2 mole) was stirred overnight at 100°C. The solution was cooled to room temperature and then extracted several times with 1N sulfuric acid. The chloroform layer was extracted with 5% sodium hydroxide

and then dried over anhydrous sodium sulfate. The solution was filtered and then solvent removed *in vacuo* to give a dark brown oil. The oil was converted to the maleate salt which was recrystallized from methanol-diethyl ether. The solid obtained was dried *in vacuo* overnight at 80°C. to give 29.84 g (47.1%) of light brown solid, m.p. 153-154°C.

Analysis: Calculated for $C_{27}H_{30}N_2OF_2$: C,64.41; H,5.87; N,4.29

Found : C,64.36; H,5.87; N,4.39

## Example 140

**N-[3-[4-[Bis(4-fluorophenyl)methyl]-1-piperidinyl] propyl]-N-methylbenzeneamine.**

A solution of 4-[bis(4-fluorophenyl)methyl]-1-(3-chloropropyl)piperidine (8.43 g, 0.023 mole) in 100 ml of N-methylaniline was stirred 30 hours at 100°C. The reaction mixture was cooled to room temperature and diluted with 300 ml of chloroform. The chloroform layer was extracted with three 100 ml portions of 1N sulfuric acid (each neutral to litmus paper). The fourth 100 ml portion of 1N sulfuric acid used for extraction was strongly acidic to litmus paper. This fourth fraction was made alkaline with ice and 50% sodium hydroxide and then extracted with chloroform. The chloroform layer was back extracted with 5% sodium hydroxide and dried over anhydrous sodium sulfate. Chloroform was removed by rotary evaporation to give a reddish brown oil. This oil was subjected to flash chromatography on silica gel using ethyl acetate for elution. Fractions of similar purity were combined and solvent removed. The residue was obtained and dried *in vacuo* overnight at 80°C. to give 4.41 g (44.1%) of brown oil.

$^1H$ NMR ($CDCl_3$): $\delta$ 6.6-7.2 (m, 13, aromatic), 3.5 (d, 1, methine attached to aromatic nuclei), 3.3 (t, 2, methylene attached to aromatic N), 2.9 (s, 3, N-$CH_3$), 1.1-2.7 (m, 13, aliphatics).

Analysis: Calculated for $C_{28}H_{32}N_2F_2$: C,77.39; H,7.42; N,6.45

Found : C,77.44; H,7.44; N,6.42

Example 141

1-[3-(4-Acetyl-2-methoxyphenoxy)propyl]-α,α-bis(4-fluorophenyl)-3-piperidineacetonitrile oxalate hydrate [1:1:1].

A mixture of α,α-bis(4-fluorophenyl)piperidineacetonitrile (6.55 g, 0.02 mole), 1-[4-(3-chloropropoxy)-3-methoxyphenyl]ethanone (5.08 g, 0.02 mole), and potassium carbonate (5.53 g, 0.04 mole) was heated overnight at gentle reflux in 350 ml of 1-butanol containing potassium iodide (0.2 g). The reaction mixture was stripped to dryness and the residue obtained was dissolved in chloroform. The chloroform was extracted with water and 5% sodium hydroxide. The chloroform layer was dried over anhydrous sodium sulfate, filtered, and solvent removed to give a dark brown oil. This oil was converted to the oxalate salt which was recrystallized twice from methanol-diethyl ether. The salt was dried in vacuo overnight at 80°C. to give 2.28 g (17.4%) of white crystalline solid, m.p. 108-109°C.

Analysis: Calculated for $C_{33}H_{36}N_2O_8F_2$: C,63.25; H,5.79 N,4.47

Found : C,63.08; H,5.48; ,4.39

Example 142

1-[3-(4-Cyanophenoxy)propyl]-α,α-bis(4-fluorophenyl)-3-piperidineacetonitrile.

A mixture of 4-(3-chloropropoxybenzonitrile (6.39 g, 0.0327 mole), α,α-bis(4-fluorophenyl)piperidineacetonitrile (10.22 g, 0.0327 mole), and potassium carbonate (5.54 g, 0.04 mole) was heated overnight at reflux in 350 ml of 1-butanol containing potassium iodide (0.2 g). The reaction mixture was stripped to dryness and the residue obtained was partitioned between chloroform and water. The chloroform layer was back extracted with water. The chloroform layer was dried over anhydrous sodium sulfate, filtered, and solvent removed to give an oil. The oil was flash chromatographed on 300 g of silica gel using 50% hexane-50% ethyl acetate and 75% ethyl acetate-25% hexane for elution. Fractions of similar purity were combined and

the resulting oil was dried in vacuo overnight at 80°C. The material crystallized while drying overnight. The solid obtained was recrystallized from equal volumes of isopropanol and low boiling petroleum ether. The solid obtained was dried in vacuo overnight at 80°C. to give 5.84 g (37.9%) of white crystalline product, m.p. 132-133°C.

Analysis: Calculated for $C_{29}H_{27}N_3OF_2$: C,73.87; H,5.77 N,8.91

Found : C,73.56; H,5.75; N,8.85

Example 143

4-[3-[4-[Bis(4-fluorophenyl)methyl]-1-piperidinyl]propoxy]-3-methoxybenzonitrile.

The sodium salt of 4-hydroxy-3-methoxybenzonitrile was prepared by reacting sodium hydride (60%, 0.8 g, 0.02 mole), 4-hydroxy-3-methoxybenzonitrile (3.00 g, 0.02 mole), and 250 ml of dry dimethylsulfoxide. Initially, the solution had a cloudy white color, but shortly (about 10 minutes) after stirring, the solution had a clear brown color. The reaction mixture was stirred for 30 minutes at room temperature. A dimethylsulfoxide solution of 4-[bis(4-fluorophenyl)methyl]-1-(3-chloropropyl)piperidine (7.26 g, 0.02 mole) was added and the resulting solution was stirred overnight at 50°C. The dimethylsulfoxide was removed in vacuo and the residue obtained was dissolved in chloroform. The organic layer was extracted with 5% sodium hydroxide and also water. The chloroform layer was dried over sodium sulfate, filtered, and solvent removed to give a dark brown oil. The oil was flash chromatographed on silica gel using ethyl acetate and 2% methanol-ethyl acetate for elution. Fractions of similar purity were combined and solvent removed in vacuo. The residue was dried in vacuo over night at 80°C. to give 4.40 g (46.2%) of brown oil. $^1H$ NMR ($CDCl_3$): δ 6.8-7.3 (m, 11, aromatics), 4.1 (t, 2, $-CH_2O-$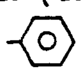 methylenes), 3.8 (s, 3, $-OCH_3$), 3.4-3.6 (d, 1, methine attached to carbon containing two fluorophenyl groups), 1.2-3.0 (m, 13, aliphatics).

Analysis: Calculated for $C_{29}H_{30}N_2O_2F_2$: C,73.09; H,6.34; N,5.88

Found : C,72.38; H,6.34; N,5.78

*NMR shows 1/4 $H_2O$ and a trace of ethyl acetate.

Example 144

4-[Bis(4-fluorophenyl)methyl]-1-[3-(1-naphthalenyloxy)propyl]piperidine hydrobromide hydrate [1:1:0.5].

The sodium salt of α-naphthol was formed in 300 ml of dimethyl sulfoxide from α-naphthol (2.59 g, 0.018 mole) and sodium hydride (60%, 0.72 g, 0.018 mole). The sodium salt was stirred 2-1/2 hours at room temperature. 4-[bis(4-fluorophenyl)methyl]-1-(3-chloropropyl)piperidine (6.52 g, 0.018 mole of free base) in 100 ml of dimethyl sulfoxide was added and the resulting solution was stirred overnight at 65°C. The solvent was removed in vacuo and the residue obtained was partitioned between chloroform-water, chloroform-1N sulfuric acid, and chlofoform-5% sodium hydroxide. The chloroform layer was dried over sodium sulfate, filtered and solvent removed to give a green oil. The oil was converted to the hydrobromide salt which was recrystallized from methanol-diethyl ether. The white salt obtained was dried in vacuo at 80°C. overnight. The crystalline solid was left exposed to the air overnight to give 1.88 g (18.6%) of white crystalline solid, m.p. 220-223°C.

Analysis: Calculated for $C_{31}H_{33}NO_{1.5}F_2Br$: C,66.31; H,5.92; N,2.49

Found : C,66.46; H,5.84; N,2.49

Example 145

2-[3-[4-[Bis(4-fluorophenyl)methyl]-1-piperidinyl]-propoxy]quinoline hydrate [1:0.5].

The sodium salt of 4-[bis(4-fluorophenyl)methyl]-1-piperidinepropanol was formed in 300 ml of dimethyl sulfoxide from its free base (6.90 g, 0.02 mole) and sodium hydride (60%, 0.8 g, 0.02 mole). 2-Chloroquinoline (3.26 g, 0.02 mole) was added and the reaction mixture was heated at 60°C. over the weekend. The reaction mixture was

stripped to dryness and the residue obtained was dissolved in chloroform. The chloroform layer was extracted with water and 5% sodium hydroxide. The chloroform layer was dried over sodium sulfate, filtered, and solvent removed to give an oil. The oil was subjected to flash chromatography on silica gel using ethyl acetate for elution. Fractions of similar purtiy were combined and solvent removed. The residue was dried _in vacuo_ overnight at 80°C. to give 5.16 g (53.6%) of clear brown oil.

$^1$H NMR ($CDCl_3$):$\delta$ 6.8-7.9 (m, 14, aromatics), 4.5 (t, 2, $-OCH_2$), 3.4 and 3.6 (d, 1, methine attached to two aromatic rings), 1.2-3.1 (m, 13, aliphatics remaining).

Analysis: Calculated for $C_{30}H_{31}N_2O_{1.5}F_2$: C,74.82; H,6.49; N,5.82

Found : C,74.56; H,6.36; N,5.69

Example 146

4-[Bis(4-fluorophenyl)methyl]-1-[3-(2-naphthalenyloxy propyl]piperidine hydrate [1:0.5].

The sodium salt of 2-naphthol was prepared in 300 ml of dimethyl sulfoxide from 2-naphthol (3.00 g, 0.0208 mole) and sodium hydride (60%, 0.83 g, 0.0208 mole). The solution was stirred 1 hour at room temperature and had a clear brown color. 4-[Bis(4-fluorophenyl)methyl]-1-(3-chloropropyl)piperidine (7.55 g, 0.0208 mole) in 100 ml of dimethylsulfoxide was added. The resulting solution was stirred overnight at 60°C. The solvent was removed _in vacuo_ and the residue obtained was partitioned between chloroform-water and chloroform-5% sodium hydroxide. The chloroform layer was dried over anhydrous sodium sulfate, filtered, and solvent removed to give a brown oil. The oil (free base) was subjected to flash chromatography on silica gel using 50-50 ethyl acetate-hexanes and 75-25 ethyl acetate-hexanes for elution. Fractions of similar purity were combined and solvent removed to give an oil. The oil was dried _in vacuo_ at 80°C. overnight to give 2.97 g (32.3%) of a dark brown glass after being exposed overnight to laboratory air.

$^1H$ NMR ($CDCl_3$): $\delta$ 6.8-7.8 (m, 15, aromatics), 4-4.3 (t, 2, $-OCH_2$), 3.4-3.6 (d, 1, methine attached to two fluorophenyl groups), 1.1-3.0 (m, 13, aliphatics).

Analysis: Calculated for $C_{30}H_{31}NOF_2$: C,77.48; H,6.71; N,2.91
Found : C,77.86; H,6.65; N,2.94

Example 147

3-[3-[4-[Bis(4-fluorophenyl)methyl]-1-piperidinyl]propoxy]benzonitrile hydrate [1:0.5].

A mixture of 4-[bis(4-fluorophenyl)methyl]piperidine (7.85 g, 0.027 mole), 3-(3-chloropropoxy)-1-benzonitrile (5.33 g, 0.027 mole), and potassium carbonate (5.84 g, 0.027 mole) was heated overnight at reflux in 350 ml of 1-butanol containing potassium iodide (0.2 g). The reaction mixture was filtered and stripped to dryness. The residue obtained was dissolved in chloroform and extracted with water and 5% sodium hydroxide. The chloroform layer was dried over sodium sulfate, filtered, and solvent removed to give a brown oil. The oil was subjected to flash chromatography on silica gel using ethyl acetate-hexanes for elution. Fractions of similar purity were combined and solvent removed. The residue was dried *in vacuo* overnight at 80°C. to give 5.65 g (45.9%) of dark brown oil.

$^1H$ NMR ($CDCl_3$): $\delta$ 6.7-7.3 (m, 12, aromatics), 3.8-4.1 (t, 2, methylenes adjacent to oxygen atom), 3.4-3.6 (d, 1, methine attached to two phenyl rings), 1.1-3.0 (m, 13, remaining aliphatic protons).

Analysis: Calculated for $C_{28}H_{29}N_2O_{1.5}F_2$: C,73.83; H,6.42; N,6.15
Found : C,74.05; H,6.27; N,6.09

Table 1 (Page 1)

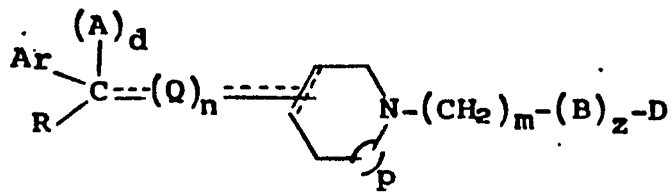

| Ex. No. | p | Ar | R | $(A)_d$ | $(Q)_n$ | Ring Position | $(B)_z$ | m | D | Salt |
|---|---|---|---|---|---|---|---|---|---|---|
| 1 | 1 | $C_6H_5$- | $C_6H_5$- | - | - | 4 | 0 | 3 | $C_6H_5$- | oxalate |
| 2 | 1 | 4-F-$C_6H_4$- | 4-F-$C_6H_4$- | OH | - | 4 | 0 | 3 | $C_6H_5$- | oxalate 0.5 $H_2O$ |
| 3 | 1 | 4-F-$C_6H_4$- | 4-F-$C_6H_4$- | - | - | 4 | 0 | 3 | $C_6H_5$- | oxalate |
| 4 | 1 | 4-F-$C_6H_4$- | 4-F-$C_6H_4$- | OH | - | 4 | 0 | 3 | $C_6H_5$- | oxalate |
| 5 | 1 | $C_6H_5$- | $C_6H_5$- | H | - | 4 | 0 | 3 | $C_6H_5$- | fumarate |
| 6 | 1 | $C_6H_5$- | $C_6H_5$- | H | - | 4 | 0 | 3 | $C_6H_5$- | fumarate |
| 7 | 1 | 4-F-$C_6H_4$- | 4-F-$C_6H_4$- | H | - | 4 | 0 | 3 | $C_6H_5$- | oxalate |
| 8 | 1 | $C_6H_5$- | $C_6H_5$- | H | - | 4 | 0 | 2 | $C_6H_5$- | fumarate |
| 9 | 1 | 4-F-$C_6H_4$- | 4-F-$C_6H_4$- | H | - | 4 | 0 | 2 | $C_6H_5$- | oxalate |
| 10 | 1 | 4-F-$C_6H_4$- | 4-F-$C_6H_4$- | H | - | 4 | 0 | 4 | $C_6H_5$- | oxalate |
| 11 | 1 | 4-F-$C_6H_4$- | $C_6H_5$- | H | - | 4 | 0 | 3 | $C_6H_5$- | fumarate |
| 12 | 1 | 4-F-$C_6H_4$- | 4-F-$C_6H_4$- | H | - | 4 | 0 | 2 | 2,6-$Cl_2$-$C_6H_3$- | - |
| 13 | 1 | 4-F-$C_6H_4$- | 4-F-$C_6H_4$- | OH | - | 4 | 0 | 3 | 4-Cl-$C_6H_4$- | - |
| 14 | 1 | 4-F-$C_6H_4$- | 4-F-$C_6H_4$- | H | - | 4 | 0 | 3 | 2-F-$C_6H_4$- | oxalate |
| 15 | 1 | 4-F-$C_6H_4$- | 4-F-$C_6H_4$- | H | - | 4 | 0 | 3 | 3-F-$C_6H_4$- | mandelate |
| 16 | 1 | 4-F-$C_6H_4$- | 4-F-$C_6H_4$- | H | - | 4 | 0 | 3 | 4-Cl-$C_6H_4$- | fumarate |
| 17 | 1 | 4-F-$C_6H_4$- | 4-F-$C_6H_4$- | H | - | 4 | 0 | 3 | 4-F-$C_6H_4$- | - |
| 18 | 1 | 4-F-$C_6H_4$- | 4-F-$C_6H_4$- | H | - | 4 | 0 | 3 | 4-$OCH_3$-$C_6H_4$- | fumarate |
| 19 | 1 | 4-F-$C_6H_4$- | 4-F-$C_6H_4$- | H | - | 4 | 0 | 3 | 2-$OCH_3$-$C_6H_4$- | - |

Table 1 (Page 2)

| Ex. No. | p | Ar | R | $(A)_d$ | $(Q)_n$ | Ring Position | $(B)_z$ | m | D | Salt |
|---|---|---|---|---|---|---|---|---|---|---|
| 20 | 1 | $4-F-C_6H_4-$ | $4-F-C_6H_4-$ | OH | - | 4 | 0 | 3 | $2-OCH_3-C_6H_4-$ | - |
| 21 | 1 | $4-F-C_6H_4-$ | $4-F-C_6H_4-$ | - | - | 4 | 0 | 3 | $2-OCH_3-C_6H_4-$ | oxalate |
| 22 | 1 | $4-F-C_6H_4-$ | $4-F-C_6H_4-$ | H | - | 4 | 0 | 3 | $3,4-(OCH_3)_2-C_6H_3-$ | oxalate |
| 23 | 1 | $4-CH_3-C_6H_4-$ | $4-CH_3-C_6H_4-$ | H | - | 4 | 0 | 3 | $2,6-(OCH_3)_2-C_6H_3-$ | fumarate |
| 24 | 1 | $4-F-C_6H_4-$ | $4-F-C_6H_4-$ | - | - | 4 | 0 | 3 | $3,4-(OCH_3)_2-C_6H_3-$ | |
| 25 | 1 | $4-F-C_6H_4-$ | $4-F-C_6H_4-$ | H | - | 4 | 0 | 3 | $2,6-(OCH_3)_2-C_6H_3-$ | oxalate, $H_2O$ |
| 26 | 1 | $4-F-C_6H_4-$ | $4-F-C_6H_4-$ | H | - | 4 | 0 | 3 | $3,5-(OCH_3)_2-C_6H_3-$ | - |
| 27 | 1 | $4-OCH_3-C_6H_4-$ | $4-OCH_3-C_6H_4-$ | H | - | 4 | 0 | 3 | $3,4-(OCH_3)_2-C_6H_3-$ | - |
| 28 | 1 | $4-OCH_3-C_6H_4-$ | $4-OCH_3-C_6H_4-$ | H | - | 4 | 0 | 3 | $4-OCH_3-C_6H_4-$ | fumarate, 0.5 $H_2O$ |
| 29 | 1 | $4-F-C_6H_4-$ | $4-F-C_6H_4-$ | - | - | 4 | 0 | 3 | $4-C(O)CH_3-C_6H_4-$ | oxalate |
| 30 | 1 | $4-F-C_6H_4-$ | $4-F-C_6H_4-$ | H | - | 4 | 0 | 3 | $4-C(O)CH_3-C_6H_4-$ | oxalate |
| 31 | 1 | $4-F-C_6H_4-$ | $4-F-C_6H_4-$ | OH | - | 4 | 0 | 3 | $4-C(O)CH_3-C_6H_4-$ | 2-propanol |
| 32 | 1 | $4-F-C_6H_4-$ | $4-F-C_6H_4-$ | OH | - | 4 | 0 | 3 | $2-CH_3-4-C(O)CH_3-C_6H_3$ | - |
| 33 | 1 | $4-F-C_6H_4-$ | $4-F-C_6H_4-$ | OH | - | 4 | 0 | 3 | $4-CN-C_6H_4-$ | - |
| 34 | 1 | $4-F-C_6H_4-$ | $4-F-C_6H_4-$ | H | - | 4 | 0 | 3 | $4-CN-C_6H_4-$ | fumarate |
| 35 | 1 | $4-F-C_6H_4-$ | $4-F-C_6H_4-$ | OH | - | 4 | 0 | 3 | $4-C(O)OC_2H_5-C_6H_4-$ | HCl |
| 36 | 1 | $4-F-C_6H_4-$ | $4-F-C_6H_4-$ | OH | - | 4 | 0 | 3 | $4-C(O)OH-C_6H_4-$ | HCl, 0.5 $H_2O$ |
| 37 | 1 | $4-F-C_6H_4-$ | $4-F-C_6H_4-$ | - | - | 4 | 0 | 3 | $4-C(O)OC_2H_5-C_6H_4-$ | HBr |
| 38 | 1 | $4-F-C_6H_4-$ | $4-F-C_6H_4-$ | H | - | 4 | 0 | 3 | $4-C(O)OC_2H_5-C_6H_4-$ | HBr |
| 39 | 1 | $4-OCH_3-C_6H_4-$ | $4-OCH_3-C_6H_4-$ | H | - | 4 | 0 | 3 | $4-C(O)OC_4H_9-C_6H_4-$ | - |
| 40 | 1 | $4-OCH_3-C_6H_4-$ | $4-OCH_3-C_6H_4-$ | H | - | 4 | 0 | 3 | $4-C(O)OC_2H_5-C_6H_4-$ | fumarate, 0.5 $H_2O$ |

Table 1 (Page 3)

| Ex. No. | p | Ar | R | $(A)_d$ | $(Q)_n$ | Ring Position | $(B)_z$ | m | D | Salt |
|---|---|---|---|---|---|---|---|---|---|---|
| 41 | 1 | 4-F-$C_6H_4$- | 4-F-$C_6H_4$- | OH | - | 4 | 0 | 2 | 4-C(O)O$C_2H_5$-$C_6H_4$- | HCl |
| 42 | 1 | 4-F-$C_6H_4$- | 4-F-$C_6H_4$- | OH | - | 4 | 0 | 3 | 2-$OCH_3$-4-$CH_2$-C(O)O$C_2H_5$-$C_6H_4$- | HCl |
| 43 | 1 | 4-F-$C_6H_4$- | 4-F-$C_6H_4$- | - | - | 4 | 0 | 3 | 4-t-butyl-$C_6H_4$- | fumarate |
| 44 | 1 | 4-F-$C_6H_4$- | 4-F-$C_6H_4$- | H | - | 4 | 0 | 3 | 4-t-butyl-$C_6H_4$- | fumarate, 0.5 $H_2O$ |
| 45 | 1 | 4-$OCH_3$-$C_6H_4$- | 4-$OCH_3$-$C_6H_4$- | H | - | 4 | 0 | 3 | 4-t-butyl-$C_6H_4$- | oxalate |
| 46 | 1 | 4-F-$C_6H_4$- | 4-F-$C_6H_4$- | OH | - | 4 | 0 | 3 | 4-t-butyl-$C_6H_4$- | - |
| 47 | 1 | 4-F-$C_6H_4$- | 4-F-$C_6H_4$- | H | - | 4 | 0 | 3 | 3-$CF_3$-$C_6H_4$- | oxalate |
| 48 | 1 | 4-$CH_3$-$C_6H_4$- | 4-$CH_3$-$C_6H_4$- | H | - | 4 | 0 | 3 | 4-NHC(O)$CH_3$-$C_6H_4$- | fumarate, 0.5 $H_2O$ |
| 49 | 1 | 4-F-$C_6H_4$- | 4-F-$C_6H_4$- | H | - | 4 | 0 | 3 | 4-NHC(O)$CH_3$-$C_6H_4$- | HBr |
| 50 | 1 | 4-F-$C_6H_4$- | 4-F-$C_6H_4$- | H | - | 4 | 0 | 3 | 4-$NH_2$-$C_6H_4$- | fumarate, 0.5 $H_2O$ |
| 51 | 1 | 4-F-$C_6H_4$- | 4-F-$C_6H_4$- | H | - | 4 | 0 | 3 | 4-NHC(O)$CH_3$-$C_6H_4$- | HCl, $H_2O$ |
| 52 | 1 | 4-F-$C_6H_4$- | 4-F-$C_6H_4$- | OH | - | 4 | 0 | 3 | 4-$NO_2$-$C_6H_4$- | - |
| 53 | 1 | 4-F-$C_6H_4$- | 4-F-$C_6H_4$- | OH | - | 4 | 0 | 3 | 4-C(O)$NH_2$-$C_6H_4$- | - |
| 54 | 1 | 4-F-$C_6H_4$- | 4-F-$C_6H_4$- | H | - | 4 | 0 | 2 | 1-$C_{10}H_7$- | HCl |
| 55 | 1 | 4-F-$C_6H_4$- | 4-F-$C_6H_4$- | H | - | 4 | 0 | 2 | 2-$C_{10}H_7$- | oxalate |
| 56 | 1 | 4-F-$C_6H_4$- | 4-F-$C_6H_4$- | H | - | 4 | 0 | 3 | 2-$OCH_3$-4-C(O)$CH_3$-$C_6H_3$- | oxalate |
| 57 | 1 | 4-F-$C_6H_4$- | 4-F-$C_6H_4$- | H | - | 4 | 0 | 3 | 2-$OCH_3$-4-C(O)$CH_3$-$C_6H_3$- | 1.2 fumarate |
| 58 | 1 | 4-F-$C_6H_4$- | 4-F-$C_6H_4$- | - | - | 4 | 0 | 3 | 2-$OCH_3$-4-C(O)$CH_3$-$C_6H_3$- | oxalate |

Table 1 (Page 4)

| Ex. No. | p | Ar | R | $(A)_d$ | $(Q)_n$ | Ring Position | $(B)_z$ | m | D | Salt |
|---|---|---|---|---|---|---|---|---|---|---|
| 59 | 1 | $4-F-C_6H_4-$ | $C_6H_5$ | - | - | 4 | 0 | 3 | $2-OCH_3-4-C(O)CH_3-C_6H_3-$ | oxalate |
| 60 | 1 | $3-CF_3-C_6H_4-$ | $C_6H_5-$ | - | - | 4 | 0 | 3 | $2-OCH_3-4-C(O)CH_3-C_6H_3$ | oxalate |
| 61 | 1 | $C_6H_5-$ | $C_6H_{11}$ | - | - | 4 | 0 | 3 | $2-OCH_3-4-C(O)CH_3-C_6H_3-$ | oxalate |
| 62 | 1 | $C_6H_5-$ | $C_6H_{11}-$ | H | - | 4 | 0 | 3 | $2-OCH_3-4-C(O)CH_3-C_6H_3-$ | oxalate, 0.5 $H_2O$ |
| 63 | 1 | $4-F-C_6H_4-$ | $4-F-C_6H_4-$ | - | - | 4 | 0 | 3 | $4-COHCH_3-C_6H_4-$ | oxalate |
| 64 | 1 | $4-F-C_6H_4-$ | $4-F-C_6H_4-$ | H | - | 4 | 0 | 3 | $2-OCH_3-4-COHCH_3-C_6H_3-$ | - |
| 65 | 1 | $C_6H_5-$ | $C_6H_5-$ | H | - | 4 | 0 | 3 | $2-OCH_3-4-C(O)CH_3-C_6H_3-$ | oxalate |
| 66 | 1 | $4-F-C_6H_4-$ | $4-F-C_6H_4-$ | OH | - | 4 | 0 | 3 | $2-OCH_3-4-C(O)CH_3-C_6H_3-$ | - |
| 67 | 1 | $4-F-C_6H_4-$ | $C_6H_5-$ | OH | - | 4 | 0 | 3 | $2-OCH_3-4-C(O)CH_3-C_6H_3-$ | - |
| 68 | 1 | $C_6H_5-$ | $C_6H_5-$ | OH | - | 4 | 0 | 3 | $2-OCH_3-4-C(O)CH_3-C_6H_3-$ | oxalate |
| 69 | 1 | $3-CF_3-C_6H_4-$ | $C_6H_5-$ | OH | - | 4 | 0 | 3 | $2-OCH_3-4-C(O)CH_3-C_6H_3-$ | HCl, 0.5 $H_2O$ |
| 70 | 1 | $C_6H_5-$ | $C_6H_{11}-$ | OH | - | 4 | 0 | 3 | $2-OCH_3-4-C(O)CH_3-C_6H_3-$ | HCl |
| 71 | 1 | $4-F-C_6H_4-$ | $4-F-C_6H_4-$ | OH | - | 4 | 0 | 2 | $2-OCH_3-4-C(O)CH_3-C_6H_3-$ | - |
| 72 | 1 | $4-F-C_6H_4-$ | $4-F-C_6H_4-$ | OH | - | 4 | 0 | 4 | $2-OCH_3-4-C(O)CH_3-C_6H_3-$ | - |
| 73 | 1 | $4-F-C_6H_4-$ | $4-F-C_6H_4-$ | OH | - | 4 | 0 | 5 | $2-OCH_3-4-C(O)CH_3-C_6H_3-$ | - |

Table 1 (Page 5)

| Ex. No. | p | Ar | R | $(A)_d$ | $(Q)_n$ | Ring Position | $(B)_z$ | m | D | Salt |
|---|---|---|---|---|---|---|---|---|---|---|
| 74 | 1 | $4-F-C_6H_4-$ | $4-F-C_6H_4-$ | H | - | 4 | 0 | 2 | $2-OCH_3-4-C(O)CH_3-C_6H_3-$ | - |
| 75 | 1 | $4-Cl-C_6H_4-$ | $4-Cl-C_6H_4-$ | - | - | 4 | 0 | 3 | $2-OCH_3-4-C(O)CH_3-C_6H_3-$ | - |
| 76 | 1 | $4-F-C_6H_4-$ | $C_6H_5-$ | H | - | 4 | 0 | 3 | $2-OCH_3-4-C(O)CH_3-C_6H_3-$ | oxalate |
| 77 | 1 | $4-OCH_3-C_6H_4-$ | $4-OCH_3-C_6H_4-$ | H | - | 4 | 0 | 3 | $2-OCH_3-4-C(O)CH_3-C_6H_3-$ | oxalate |
| 78 | 1 | $4-CH_3-C_6H_4-$ | $4-CH_3-C_6H_4-$ | H | - | 4 | 0 | 3 | $2-OCH_3-4-C(O)CH_3-C_6H_3-$ | - |
| 79 | 1 | $4-F-C_6H_4-$ | $4-F-C_6H_4-$ | H | - | 4 | 0 | 4 | $2-OCH_3-4-C(O)CH_3-C_6H_3-$ | - |
| 80 | 1 | $4-F-C_6H_4-$ | $4-F-C_6H_4-$ | OH | - | 4 | 0 | 3 | $2-OCH_3-4-C(O)OCH_3-C_6H_3-$ | - |
| 81 | 1 | $4-F-C_6H_4-$ | $4-F-C_6H_4-$ | OH | - | 4 | 0 | 3 | $4-SCH_3-C_6H_4-$ | - |
| 82 | 1 | $4-F-C_6H_4-$ | $4-F-C_6H_4-$ | OH | - | 4 | 0 | 3 | $4-S(O)_2CH_3-C_6H_4-$ | fumarate |
| 83 | 1 | $4-F-C_6H_4-$ | $4-F-C_6H_4-$ | OH | - | 4 | 0 | 3 | $2-OCH_3-4-CH_2-C(O)OC_2H_5-C_6H_3-$ | HCl |
| 84 | 1 | $4-F-C_6H_4-$ | $4-F-C_6H_4-$ | OH | - | 4 | 0 | 2 | $4-C(O)OC_2H_5-C_6H_4-$ | HCl |
| 85 | 1 | $4-F-C_6H_4-$ | $4-F-C_6H_4-$ | OH | - | 4 | 0 | 3 | $2-OCH_3-4-CH_2C(O)-ON_a-C_6H_3$ | 0.5 $H_2O$ |
| 86 | 1 | $4-F-C_6H_4-$ | $4-F-C_6H_4-$ | OH | - | 4 | 0 | 3 | 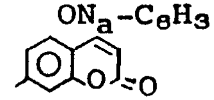 | - |
| 87 | 1 | $4-F-C_6H_4-$ | $4-F-C_6H_4-$ | OH | - | 4 | 0 | 3 | $2-C(O)OC_2H_5-C_6H_4-$ | 0.75 fumarate |
| 88 | 1 | $4-F-C_6H_4-$ | $4-F-C_6H_4-$ | H | - | 4 | 0 | 3 | $2-(C)OC_2H_5-C_6H_4-$ | - |
| 89 | 1 | $4-F-C_6H_4-$ | $4-F-C_6H_4-$ | H | - | 4 | 0 | 5 | $2-OCH_3-4-C(O)CH_3-C_6H_3-$ | 0.5 $H_2O$ |

Table 1 (Page 6)

| Ex. No. | p | Ar | R | $(A)_d$ | $(Q)_n$ | Ring Position | $(B)_z$ | m | D | Salt |
|---|---|---|---|---|---|---|---|---|---|---|
| 90 | 1 | 4-F-$C_6H_4$- | 4-F-$C_6H_4$- | H | - | 4 | 0 | 3 | 4-C(O)$NH_2$-$C_6H_4$- | 1.5 fumarate |
| 91 | 1 | 4-F-$C_6H_4$- | 4-F-$C_6H_4$- | H | - | 4 | 0 | 3 | 4-S$(O)_2$$CH_3$-$C_6H_4$- | oxalate |
| 92 | 1 | 4-F-$C_6H_4$- | 4-F-$C_6H_4$- | OH | - | 4 | 0 | 6 | 2-$OCH_3$-4-C(O)$CH_3$-$C_6H_3$- | - |
| 93 | 1 | 4-F-$C_6H_4$- | 4-F-$C_6H_4$- | OH | - | 4 | 0 | 3 | 3-$OCH_3$-4-C(O)$CH_3$-$C_6H_3$- | - |
| 94 | 1 | 4-F-$C_6H_4$- | 4-F-$C_6H_4$ | OH | - | 4 | 0 | 3 | 2-OH-$C_6H_4$ | - |
| 95 | 1 | 4-F-$C_6H_4$- | 4-F-$C_6H_4$- | OH | - | 4 | 0 | 3 | 4-S(O)$CH_3$-$C_6H_4$- | fumarate |
| 96 | 1 | 4-F-$C_6H_4$- | 4-F-$C_6H_4$- | OH | - | 4 | 0 | 3 | 4-S$(O)_2$$NH_2$-$C_6H_4$- | HCl |
| 97 | 1 | 4-F-$C_6H_4$- | 4-F-$C_6H_4$- | OH | - | 4 | 0 | 3 | 4-NHS$(O)_2$$CH_3$-$C_6H_4$- | - |
| 98 | 1 | 4-F-$C_6H_4$- | 4-F-$C_6H_4$ | OH | - | 4 | 0 | 3 | 4-NHC(O)$NHCH_3$-$C_6H_4$- | - |
| 99 | 1 | 4-F-$C_6H_4$- | 4-F-$C_6H_4$- | OH | - | 4 | 0 | 3 | 4-NHC(O)$OC_2H_5$-$C_6H_4$- | - |
| 100 | 1 | 4-F-$C_6H_4$- | 4-F-$C_6H_4$- | OH | - | 4 | 0 | 3 | 3-NHC(O)$NH_2$-$C_6H_4$- | - |
| 101 | 1 | 4-F-$C_6H_4$- | 4-F-$C_6H_4$- | OH | - | 4 | 0 | 3 | 2-$OCH_3$-4-COOH-$C_6H_3$- | sodium |
| 102 | 1 | 4-F-$C_6H_4$- | 4-F-$C_6H_4$- | OH | - | 4 | 0 | 3 | 3-OH-4-C(O)$CH_3$-$C_6H_4$- | - |
| 103 | 1 | 4-F-$C_6H_4$- | 4-F-$C_6H_4$- | OH | - | 4 | 0 | 3 | 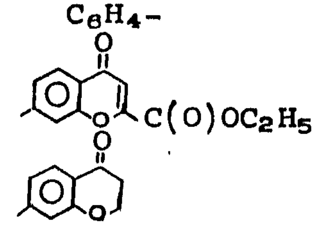 | HCl |
| 104 | 1 | 4-F-$C_6H_4$ | 4-F-$C_6H_4$- | OH | - | 4 | 0 | 3 | | - |

Table 1 (Page 7)

| Ex. No. | p | Ar | R | $(A)_d$ | $(Q)_n$ | Ring Position | $(B)_z$ | m | D | Salt |
|---|---|---|---|---|---|---|---|---|---|---|
| 105 | 1 | $C_6H_5$- | $C_6H_5$- | - | - | 4 | O | 3 | 2-$OCH_3$-4-C(O)$CH_3$-$C_6H_3$- | oxalate 0.5 $H_2O$ |
| 106 | 1 | $C_6H_5$- | $C_6H_{11}$- | H | - | 4* | O | 3 | 2-$OCH_3$-4-C(O)$CH_3$-$C_6H_3$- | oxalate 0.5 $H_2O$ |
| 107 | 1 | 4-F-$C_6H_4$- | 4-F-$C_6H_4$- | OH | - | 4 | O | 3 | 3-$OCH_3$-4-C(O)$CH_3$-$C_6H_3$- | HCl |
| 108 | 1 | 4-F-$C_6H_4$- | 4-F-$C_6H_4$- | H | - | 4 | O | 3 | 2,6-$Cl_2$-$C_6H_3$- | - |
| 109 | 1 | 4-F-$C_6H_4$- | 4-F-$C_6H_4$- | H | - | 4 | O | 3 | 2,6-$Cl_2$-$C_6H_3$- | oxalate |
| 110 | 1 | 4-F-$C_6H_4$- | 4-F-$C_6H_4$- | H | - | 4 | O | 3 | 2-CN-$C_6H_4$- | - |
| 111 | 1 | 4-F-$C_6H_4$- | 4-F-$C_6H_4$- | -OH | - | 4 | S | 2 | $C_6H_5$- | maleate |
| 112 | 1 | 4-F-$C_6H_4$- | 4-F-$C_6H_4$- | - | - | 4 | S | 2 | $C_6H_5$- | - |
| 113 | 1 | 4-F-$C_6H_4$- | 4-F-$C_6H_4$- | -OH | - | 4 | -S(O)$_2$ | 2 | 4-Cl-$C_6H_4$- | - |
| 114 | 1 | 4-F-$C_6H_4$- | 4-F-$C_6H_4$- | - | - | 4 | -S(O)$_2$- | 2 | 4-Cl-$C_6H_4$- | - |
| 115 | 1 | 4-F-$C_6H_4$- | 4-F-$C_6H_4$- | H | - | 4 | -S(O)$_2$- | 3 | $C_6H_5$- | 1.5 fumarate |
| 116 | 1 | 4-F-$C_6H_4$- | 4-F-$C_6H_4$- | H | - | 4 | -S(O)$_2$- | 2 | 4-Cl-$C_6H_4$- | maleate |
| 117 | 1 | 4-F-$C_6H_4$- | 4-F-$C_6H_4$- | H | - | 4 | -S(O)$_2$- | 2 | $C_6H_5$- | maleate |
| 118 | 1 | $C_6H_5$- | $C_6H_5$- | -CN | - | 4 | - | 1 | 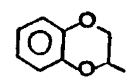 | - |
| 119 | 1 | $C_6H_5$- | $C_6H_5$- | -CN | - | 4 | O | 3 | 2-$OCH_3$-4-C(O)$CH_2$-$C_6H_3$- | oxalate |
| 120 | 1 | $C_6H_5$- | $C_6H_5$- | -CN | -$CH_2$ | 3 | - | 3 | 2-$OCH_3$-4-C(O)$CH_3$-$C_6H_3$- | - |
| 121 | 1 | $C_6H_5$- | $C_6H_5$- | -CN | -$CH_2$- | 3 | O | 4 | 2-$OCH_3$-4-C(O)$CH_3$-$C_6H_3$- | - |
| 122 | 0 | $C_6H_5$- | $C_6H_5$- | -C(O)$NH_2$ | - | 3 | O | 3 | 2-$OCH_3$-4-C(O)$CH_3$-$C_6H_3$- | - |

Table 1 (Page 8)

| Ex. No. | p | Ar | R | $(A)_d$ | $(Q)_n$ | Ring Position | $(B)_z$ | m | D | Salt |
|---|---|---|---|---|---|---|---|---|---|---|
| 123 | 1 | $C_6H_5-$ | $C_6H_5-$ | $-C(O)NH_2-$ | - | 4 | O | 3 | $2-OCH_3-4-C(O)CH_3-C_6H_3-$ | 0.5 fumarate, $H_2O$ |
| 124 | 1 | $C_6H_5-$ | $C_6H_5-$ | $-C(O)NH_2-$ | - | 4 | O | 4 | $2-OCH_3-4-C(O)CH_3-C_6H_3-$ | fumarate 0.5 $H_2O$ |
| 125 | 1 | $C_6H_5-$ | $C_6H_5-$ | $-C(O)NH_2-$ | $-CH_2-$ | 3 | O | 3 | $2-OCH_3-4-C(O)CH_3-C_6H_3-$ | 0.5 $H_2O$ |
| 126 | 1 | $4-F-C_6H_4-$ | $4-F-C_6H_4-$ | H | - | 4 | - | 1 | 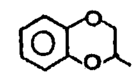 | oxalate |
| 127 | 1 | $C_6H_5-$ | $C_6H_5-$ | -CN | $-CH_2-$ | 3 | O | 2 | $2,6-Cl_2-C_6H_3-$ | - |
| 128 | 1 | $C_6H_5-$ | $C_6H_5-$ | -CN | $-CH_2-$ | 3 | O | 5 | $2-OCH_3-4-(O)CH_3-C_6H_3-$ | 0.5 $H_2O$ |
| 129 | 1 | $4-F-C_6H_4-$ | $4-F-C_6H_4-$ | -CN | - | 4 | O | 3 | $2-OCH_3-4-C(O)CH_3-C_6H_3-$ | maleate |
| 130 | 1 | $4-F-C_6H_4-$ | $4-F-C_6H_4-$ | -CN | - | 4 | O | 3 | $2,6-Cl_2-C_6H_3-$ | maleate |
| 131 | 1 | $C_6H_5-$ | $C_6H_5-$ | -CN | $-CH_2-$ | 3 | O | 3 | $2,6-Cl_2-C_6H_3-$ | oxalate |
| 132 | 1 | $4-F-C_6H_4-$ | $4-F-C_6H_4-$ | -CN | - | 4 | O | 4 | $2-OCH_3-4-C(O)CH_3-C_6H_3-$ | fumarate |
| 133 | 1 | $C_6H_5-$ | $C_6H_5-$ | $-C(O)NH_2$ | $-CH_2-$ | 3 | O | 2 | $2,6-Cl_2-C_6H_3-$ | - |
| 134 | 1 | $4-F-C_6H_4-$ | 2-pyridinyl | -CN | - | 4 | O | 3 | $2-OCH_3-4-C(O)CH_3-C_6H_3-$ | fumarate |
| 135 | 1 | $4-F-C_6H_4-$ | 2-pyridinyl | -CN | - | 4 | O | 3 | $2-OCH_3-4-C(O)CH_3-C_6H_3-$ | fumarate $H_2O$ |
| 136 | 1 | $C_6H_5-$ | $C_6H_5-$ | -CN | $-CH_2-$ | 3 | O | 3 | 8-quinolinyl | 0.5 $H_2O$ |
| 137 | 1 | $4-F-C_6H_4-$ | $4-F-C_6H_4-$ | H | - | 4 | O | 3 | 8-quinolinyl | 0.5 $H_2O$ |
| 138 | 1 | $4-F-C_6H_4-$ | $4-F-C_6H_4-$ | H | - | 4 | O | 3 | $2-C(O)OH-C_6H_4-$ | 0.5 $H_2O$ |
| 139 | 1 | $4-F-C_6H_4-$ | $4-F-C_6H_4-$ | H | - | 4 | -NH- | 3 | $C_6H_5-$ | 2 maleate |

Table 1 (Page 9)

| Ex. No. | p | Ar | R | $(A)_d$ | $(Q)_n$ | Ring Position | $(B)_z$ | m | D | Salt |
|---|---|---|---|---|---|---|---|---|---|---|
| 140 | 1 | $4-F-C_6H_4-$ | $4-F-C_6H_4-$ | H | - | 4 | $-NCH_3-$ | 3 | $C_6H_5-$ | - |
| 141 | 1 | $4-F-C_6H_4-$ | $4-F-C_6H_4-$ | -CN | - | 3 | 0 | 3 | $2-OCH_3-4-C(O)CH_3-C_6H_3$ | oxalate hydrate |
| 142 | 1 | $4-F-C_6H_4-$ | $4-F-C_6H_4-$ | -CN | - | 3 | 0 | 3 | $4-CN-C_6H_4-$ | - |
| 143 | 1 | $4-F-C_6H_4-$ | $4-F-C_6H_4-$ | H | - | 4 | 0 | 3 | $2-OCH_3-4-CN-C_6H_3-$ | - |
| 144 | 1 | $4-F-C_6H_4-$ | $4-F-C_6H_4-$ | H | - | 4 | 0 | 3 | $1-C_{10}H_7-$ | HBr, 0.5 $H_2O$ |
| 145 | 1 | $4-F-C_6H_4-$ | $4-F-C_6H_4-$ | H | - | 4 | 0 | 3 | 2-quinolinyl | 0.5 $H_2O$ |
| 146 | 1 | $4-F-C_6H_5-$ | $4-F-C_6H_4-$ | H | - | 4 | 0 | 3 | $2-C_{10}H_7-$ | 0.5 $H_2O$ |
| 147 | 1 | $4-F-C_6H_4-$ | $4-F-C_6H_4-$ | H | - | 4 | 0 | 3 | $3-CN-C_6H_4-$ | 0.5 $H_2O$ |

Footnote:
*1,2,3,6-tetrahydropyridine.

Screening Method for Calcium Channel Blocking Activity In Isolated Rabbit Aorta.

A non-fasted rabbit is killed by cervical dislocation. Spiral arterial strips are prepared from the thoracic aorta by the method of Furchgott, R. F., and Bhadrakom, S. (1953), J. PHARMACOL. EXP. THER. 108: 129-43. The strips are suspended in water-jacketed, 10 ml, organ baths that are kept at 37°C. and aerated with a mixture of 95% oxygen and 5% carbon dioxide. An isometric recording of tissue response is made with a Grass force-displacement transducer (Model FT03C) and a Grass polygraph.

The loading tension on the strips is about 1 g. About 90 min is allowed for maximum relaxation to occur, and during this time the bath is changed at 15 to 20 min intervals. The bath contains a physiological solution, hereafter referred to as normal bath solution, prepared in glass-distilled water and adjusted to pH 7.4. The composition of the solution in millimoles per liter will be

| | |
|---|---|
| sodium chloride | 120 |
| potassium chloride | 5.6 |
| calcium chloride | 2.6 |
| magnesium chloride 6-hydrate | 1.2 |
| sodium dihydrogen phosphate hydrate | 1.5 |
| sodium bicarbonate | 25.0 |
| glucose | 9.1 |

Strips are first checked for viability based on their response to norepinephrine at a final bath concentration of $10^{-5}$ M; then they are washed with the normal bath solution until resting tension has returned to baseline. Following this, the normal bath solution is replaced with a physiological solution, hereafter referred to as a calcium-free bath solution, having the same pH and composition as the normal bath solution except that the calcium is omitted. Strips are then incubated in this calcium-free solution for 10 min. During this time the

solution is exchanged by fresh physiological (calcium-free) solution three times.

Strips are then tested for completeness of calcium depletion by incubation for 15 min in potassium depolarizing solution. The composition of the depolarizing solution in millimoles per liter is

| | |
|---|---|
| sodium chloride | 32.2 |
| potassium chloride | 100.0 |
| magnesium chloride 6-hydrate | 1.2 |
| trihydroxymethyl hydrochloride | 12 |
| glucose | 9.1 |

The solution is prepared in glass-distilled water and adjusted to pH 7.4. If the strips contract, they will be washed with physiological solution and then reincubated in the depolarizing solution. The process is repeated if necessary until the strips are unresponsive and thus calcium depleated. Alternatively, the strips may be rewashed with depolarizing solution until they are calcium depleted.

Cumulative concentration response curves (controls) are made with calcium chloride as the agonist by the method of Van Rossum, J. M. (1963), ARCH. INT. PHARMACODYN. 143: 299-330. Final bath concentrations of calcium chloride will be 0.1 millimolar, 0.3 millimolar, and 1.0 millimolar (See Godfraind, T. & Kaba, A. (1969) BRIT. J. PHARMACOL. 36: 549-60). Responses are allowed to reach a plateau before adding the next increment of calcium chloride.

After control responses are obtained, the strips are washed with normal bath solution containing test drug (Formula I) at $10^{-7}$ molar concentration for about one hour at 15-20 min intervals.(See Broekaert, A. and Godfraind, T. (1979)). During this time the tissues have returned to resting tension.

The strips are then incubated in the physiological solution (calcium-free) for 10 min and finally in the depolarizing solution for 15 min, both of which solutions at this point contain Formula I test drug. If the strips contract in the depolarizing solution, they will be washed

as mentioned above until unresponsive. The cumulative concentration response to calcium chloride is then made over the range of concentrations used for the control determination.

As a final test to determine selectivity and whether α-blocking activity is present, the strips are washed with the normal bath solution containing the research compound until the resting tension is again at baseline. The strips are then retested for their response to norepinephrine at a final bath concentration of $10^{-5}$ molar.

In the foregoing primary screen, a minimum of 2 strips are used to test each drug at $10^{-7}$ molar. Those compounds which consistently produce at least 20% inhibition of the calcium induced contractions will be tested further. For those test drugs giving interesting positive results, a $PA_2$ value may be obtained, see Van Rossum (1963) ibid. Reference articles which may be used for comparison are lidoflazine, diltiazem, verapamil, nifedipine or other appropriate drugs. In this test, the more active compounds such as those of Examples 56, 57 and 60 showed 100% change (reduction) in contraction at $10^{-7}$ molar concentration of these agents caused by 1 millimolar concentration of calcium. Compared to the reference calcium channel blocking drugs, these compounds were found to be superior.

<u>Test Method for Antihypertensive Effect of Orally Administered Drugs to Unanesthetized Spontaneously Hypertensive Rats.</u>

<u>Surgical Preparation of Rats</u>

Charles River, spontaneously hypertensive rats are anesthetized with sodium pentobarbital (50 mg/kg, IP). The abdomen and the top of head are shaved and cleaned. A midline incision, approximately 5 mm long, is made in the skin of the dorsal surface of the animal's neck. Brass tubing, 22 cm long with a slight bend in the end, is passed through the incision, under the skin diagonally down the animal's back and around to the right side of the lower abdomen of the rat.

The animal is then taped to the table in a supine position. A midline incision approximately 4 cm long is made with scissors in the skin and another through the abdominal muscle wall. With small blunt hemostats, the skin is separated from the abdominal muscle at the midline to expose the tip of the brass tube. A small opening is made through the abdominal muscle at the appropriate angle with the blunt tips of the hemostats.

The distal end of a modified Week's cannula is inserted in the abdominal cavity and the other end is threaded through the brass tube until it exits at the base of the animal's neck. The brass tubing is removed and the 7 mm cured polyethylene tip of the cannula is aligned and positioned for insertion into the abdominal aorta. The positioned cannula is filled with isotonic saline.

The abdominal viscera is gently moved to the side, exposing the aorta in the region of bifurcation. The aorta is isolated and 2 silk ligatures, 1 to 1.5 cm apart, are placed around it. The ligatures are used to briefly and gently occlude blood flow. The abdominal aorta is punctured craniad to the bifurcation with the tip of a 23-gauge hypodermic needle. The needle is removed and the tip of the cannula inserted through this opening toward the heart. Caution is taken to keep the tip vertically aligned in the aorta. Blood is allowed to flow back through the cannula to check correct insertion. The cannula is cleared of blood with a 0.4 cc flush of isotonic saline. The stability of the cannula in the artery is ensured by suturing the ligature tied around the cannula to the dorsal muscle layers lying directly beside the aorta. The cannula is also satured to the abdominal wall at the point of exit. The abdominal viscera is repositioned and the abdominal wall and skin sutured in separate layers with blanket stitch. The animal is given 0.2 ml Combiotic® (procaine penicillin G and dihydrostreptomycin sulfate).

The end of the cannula exteriorized at the base of the neck is tied off and passed through an L-shaped piece of aluminum tubing fastened to the skull by screws and dental

cement (Purdy and Ashbrook, 1978), J. PHARM. PHARMACOLOGY 30: 436-41.

For protection and attachment of the cannula to the cage, the cannula is inserted through a length of flexible metal spring, which is attached to the aluminum tubing and to a part of a swivel device that permits the animal to move with relative freedom around the cage. During recovery, each rat is given a bottle of 5% dextrose containing terramycin (1 tsp. Pfizer Terramycin soluble powder/L 5% dextrose) to drink.

Blood Pressure Recordings

On the day following surgery, the tied-off cannula is reopened and attached to the swivel device. One end of a saline filled length of polyethylene 50 tubing is attached to the swivel and the other to a Statham pressure transducer (Model P23ID) creating a continuous saline-arterial connection. Continuous tracings from the direct aortic blood pressure are recorded on a Grass polygraph (Model 7). Heart rate is determined from the blood pressure pulse.

The electrical output of the blood pressure signal from the polygraph is fed into a Buxco Channel Cardiovascular analyzer (Model 12). The blood pressure signals are averaged for a 1-min period and measurements of blood pressure and heart rate is printed on a Texas Instruments data terminal (Model 700 ASR).

Maintenance of Rats

To maintain patency of cannula and to permit the animal to be used for maximum time, animals are continuously infused with heparin in sterile saline (2 mg/ml) at a rate of .05 to .06 ml/hr. Purina Mouse Chow and water are available ad libitum. A solution containing 5% dextrose and terramycin is given once weekly. Surgically prepared rats may be used more than once during a study. A minimum of 3 days must lapse before rats are used again. A rat is used only once in a dosage group.

Experimental Procedure

Each surgically prepared rat is individually housed in a separate cage. Each cage is labeled with the lot number and sequential rat number. Single doses of 10, 20, and 30 mg/kg of the test drug calculated on free base content is administered orally by using a syringe and size 16 gavage tube. Control article is PEG-300: saline at ratio of 1:1. Reference articles are verapamil and nifedipine. The carrier for compounds of Formula I and verapamil is PEG-300: saline, 1:1, and for nifedipine, it is ethanol. The dosage volume for test and control articles is 1 ml/kg body weight. Arterial blood pressure and heart rate are measured in each rat prior to and at 30, 60, 90, 120, 180, 240, 300, 360 minutes and 24 hours after drug administration.

The more active compounds such as compounds of Examples 56 and 57 are slightly less active in lowering blood pressure in hypertensive rats than nifedipine, but duration of action is longer.

Procedure for Determining Effect of Compounds on Coronary Blood Flow.

The procedure used to determine the effect of the aforementioned compounds on coronary arterial blood flow is described as follows.

Mongrel dogs of either sex were anesthetized with phenobarbital sodium (100 mg/kg) and pentobarbital sodium (100 mg total dose). The trachea was surgically exposed, a tracheal tube was inserted and the dog was artificially respired with room air using a Harvard Model 613 Respirator. The heart was exposed by a left thoracotomy at the fourth intercostal space. An approximately 1.5 cm segment of the left anterior descending coronary artery was exposed and a Statham electromagnetic blood flow probe was implanted around the vessel. The flow probe cable was connected to a Statham Model 2201 Blood Flow Meter. Continuous recordings of carotid arterial blood pressure, and of coronary arterial blood flows, were obtained using a Grass Model 5 Polygraph.

The compounds were administered via a femoral vein. Changes in both magnitude and duration of change in coronary blood flow from pre-drug levels were determined. Generally, multiple doses of the compounds tested were administered to a single dog. Appropriate intervals between doses were allowed to permit the blood flow to return to control levels.

Illustratively, the compounds of Examples 56, 57, 61, 62, 69, 74, 76, 89, 105, 106, and 128 showed an increase in coronary arterial blood flow at 0.5 mg/kg of the compounds of about 75-120 ml/min.

Screening Procedure for Antihistamine Activity

The compounds of the present invention exhibit antihistaminic activity in guinea pigs. The method of testing is a modification of the procedure of Tozzi et al (Agents and Actions, Vol. 4/4, 264-270, 1974) as follows: Guinea pigs are fasted 18-24 hrs in individual cages. Water is available *ad libitum*. On the test day, animals in groups of 3 are injected intraperitoneally with 30 mg/kg of the test compound prepared in an appropriate vehicle. Thirty minutes later histamine at a dosage level of 1.2 mg/kg (= 2 x the $LD_{99}$) is injected into a marginal ear vein. Survival of the guinea pigs for 24 hrs is positive evidence of antihistaminic activity. If the vehicle used for the test compound is other than water, its effect is established by testing an equal amount as a control. The dose protecting 50% of the animals ($PD_{50}$) from death may be established from dose-response curves. Compounds such as in Examples 58 and 105 were found to be active at dosages at least as low as 3 mg/kg.

Screening Procedure for Gastric Antisecretory Activity In Pyloric-Ligated Rats.

Female Sprague-Dawley rats weighing 130-180 g were starved 24 hours in individual screen-bottom cages with water *ad libitum*. Animals were arranged into groups of 9 rats each for treated animals and 8 rats for controls. Each group was injected intraduodenally at the time of

pyloric-ligation with test drug in doses of 25.0 mg/kg (0.2 ml/100 g body weight). Rats dosed with deionized water (2 ml $kg^{-1}$) served as controls. Four hours following ligation, rats were killed, the stomachs removed, gastric juice collected and the volume determined. Total hydrochloric acid output was determined by potentiometic titration to pH 7.0 endpoint using a Radiometer TTA-61 autopipetting titration system. Statistical analysis was performed by using the "Student's t-test" significance. Illustratively, at a dose of 25 mg/kg, significant reduction of secretion occurred of about 85% in volume and 98% in acid was obtained for the compound of Example 58. Similarly, reduction in volume obtained for the compound of Example 105 was about 65% for both volume and acid.

## Pharmaceutical Compositions and Administration

Compositions for administration to living animals are comprised of at least one of the compounds of Formula I according to the methods of treatment of the invention in association with a pharmaceutical carrier or excipient. Effective quantities of the compounds may be administered in any one of various ways, for example, orally as in elixirs, capsules, tablets or coated tablets, parenterally in the form of sterile solutions, suspensions and in some cases intravenously in the form of sterile solutions, intranasally and to the throat or bronchial region in the form of drops, gargles, syrups, powders, etc. or subcutaneously. Suitable tableting excipients include lactose, potato and maize starches, talc, gelatin, stearic and silicic acids, magnesium stearate and polyvinyl pyrrolidone.

For the parenteral administration, the carrier or excipient can be comprised of a sterile parenterally acceptable liquid, e.g., water or arachis oil contained in ampoules.

Advantageously, the compositions are formulated as dosage units, each unit being adapted to supply a fixed dose of active ingredients. Tablets, coated tablets, capsules, ampoules, sprays and suppositories are examples

of preferred dosage forms. It is only necessary that the active ingredient constitute an effective amount such that a suitable effective dosage will be consistent with the dosage form employed, in multiples if necessary. The exact individual dosages, as well as daily dosages, will of course be determined according to standard medical principles under the direction of a physician or veterinarian. Generally, the following guide to projected human oral dosages is derived by knowledge of the activity obtained in animal screening tests for the various indications in the methods of the invention compared to activity of known agents in the field in the same animal screening tests. However, the amount of the active compounds administered need not be limited by these comparisons due to uncertainty in transposing comparative animal data to human treatments.

Oral dosages projected for hypertension for an adult human are of the order of 40-300 mg/day divided into 2 or 3 doses. Thus, for example, two capsules each containing 10-50 mg active agent of Formula I could be administered 2-3 times daily for blood pressure lowering.

Oral dosages projected for use in the treatment of angina for an adult human are of the order of 60-400 mg/day divided into 2 or 3 doses. Thus, for example, two capsules each containing 10-30 mg active agent of Formula I could be administered 2-5 daily to increase coronary blood flow.

Oral dosages projected for use as antihistamines for an adult human are of the order 10-120 mg/day divided into 2 or 3 doses. Thus, for example, one or two capsules each containing 10-40 mg active agent of Formula I could be administered 2-3 times daily for temporary relief of cough due to minor throat and bronchial irritation which may occur with the common cold or with inhaled irritants.

Oral dosages projected for use as antisecretory agents for an adult human are of the order of 4 to 150 mg/day divided into 2 or 3 doses. Thus, for example, one or two doses each containing 0.5 to 50 mg active agent of Formula I could be administered 2-3 times daily for temporary relief

due to excessive acid release in the stomach.

Other routes of administration such as subcutaneous, intraperitoneal, intravenous, etc. are possible with dosage forms being adapted to the situation as will be obvious to one skilled in the art of medicine.

Various modifications and equivalents will be apparent to one skilled in the art and may be made in the compounds, methods of treatment and compositions of the present invention without departing from the spirit or scope thereof, and it is therefore to be understood that the invention is to be limited only by the scope of the appended claims.

CLAIMS FOR THE CONTRACTING STATES: BE, FR, DE, IT, LU, NL, SE, CH and GB.

1. A compound having the formula:

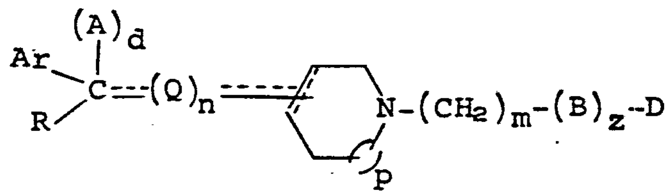

wherein;

p is zero, one or two;

A is hydrogen, $-O-R^1$, $-C{\equiv}N$, $-\underset{\underset{O}{\Vert}}{C}NR^1R^2$, $-\overset{\overset{O}{\Vert}}{C}-R^1$, $-\overset{\overset{O}{\Vert}}{C}-O-R^1$, $-O-\overset{\overset{O}{\Vert}}{C}-R^1$, $-CH_2OR^1$, $-CH_2NR^1R^2$;

m is zero to six inclusive;

Q is $-CH-$, $-CH_2-$ or $-\overset{OH}{\underset{H}{C}}-$;

d and n are selected from zero or one and the dotted lines represent double bonds which may form consistent with the valence of carbon;

Ar, D and R are selected independently from:

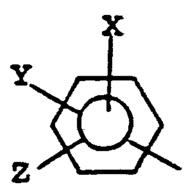 , 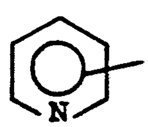 , 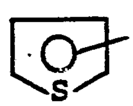 , 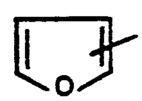 or 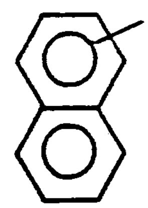

and in addition, R may have the values:

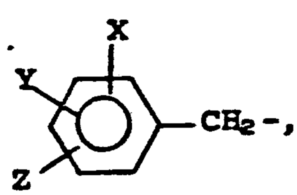, cycloalkyl or loweralkyl; and

D may have additionally the values:

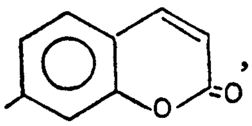, 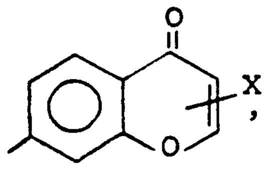, 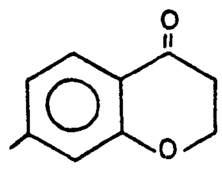, 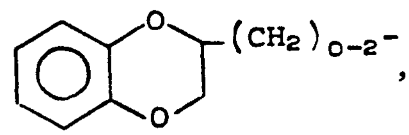,

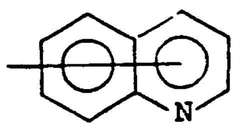,

or $Ar(CH_2)_{1-4}$-; X, Y, and Z are selected from the group consisting of hydrogen, loweralkyl, halogen, $-NO_2$, $-O-R^1$, $-C(O)-R^1$, $-CF_3$, $-C{\equiv}N$, $-C(O)-N(R^1)_2$, $-N(R^1)_2$, $-C(O)OR^1$, $-SO_2R^2$, $-SR^2$, $-S(O)R^2$, $-N(R^1)-C(O)-R^1$, $-CH_2COOM$, $-SO_2N(R^1)(R^2)$, $-N(R^1)SO_2CH_3$, $-N(R^1)C(O)-N(R^1)(R^2)$, or $-N(R^1)C(O)-OR^2$;

B is selected from O, S, $-S(O)-$, $-S(O)_2-$, $-N(R^1)-$, and $-N-C(O)-O-R^1$;

z is one or zero with the proviso that z cannot be zero at the same time n is zero when one of the following occurs at the same time that D is phenyl or substituted phenyl: $(A)_d$ is hydrogen, $(A)_d$ is cyano, $(A)_d$ is aminocarbonyl, or a double bond forms between the $\alpha$ carbon and a carbon of the central heterocyclic amine ring;

$R^1$ is selected from hydrogen, loweralkyl, phenyl and phenylloweralkyl;

$R^2$ is selected from loweralkyl, phenyl and phenylloweralkyl;

M is a pharmaceutically acceptable metal ion, or a pharmaceutically acceptable salts thereof, (including acid addition salts, quaternary salts, and hydrates and alcoholates thereof) with the further proviso that $(B)_z$ cannot represent oxygen at the same time D is phenyl or substituted phenyl when n is zero and $(A)_d$ is hydrogen or hydroxyl or when d is zero and a double bond forms between the $\alpha$ carbon and a carbon of a saturated central heterocyclic amine ring.

2. A compound as claimed in claim 1, wherein Ar is unsaturated phenyl or 4-saturated phenyl.

3. A compound as claimed in claim 1 or 2, wherein Ar is halo-substituted phenyl, trifluoromethyl-substituted phenyl, loweralkyl-substituted phenyl or loweralkoxy-substituted phenyl.

4. A compound as claimed in claim 1, 2 or 3, wherein R is phenyl, 4-substituted phenyl or loweralkyl.

5. A compound as claimed in any one of claims 1 to 4, wherein R is halo-substituted phenyl, loweralkyl-substituted phenyl or loweralkoxy-substituted phenyl.

6. A compound as claimed in any one of claims 1 to 5, wherein M is two to five inclusive.

7. A compound as claimed in any one of claims 1 to 6, wherein p is one.

8. A compound as claimed in any one of claims 1 to 7, wherein the left hand substituent in the formula, as drawn, is in the 4-position relative to the ring nitrogen atom.

9. 7-[3-[4-[Bis(4-fluorophenyl)hydroxymethyl]-1-piperidinyl]propoxy]-2H-1-benzopyran-2-one or a pharmaceutically acceptable salt thereof,

7-[3-[4-[bis(4-fluorophenyl)hydroxymethyl]-1-piperidinyl]propoxy]-4-oxo-4H-1-benzopyran-2-carboxylic acid ethyl ester or a pharmaceutically acceptable salt thereof,

7-[3-[4-[bis(4-fluorophenyl)hydroxymethyl]-1-piperidinyl]propoxy]-2,3-dihydro-4H-1-benzopyran-4-one or a pharmaceutically acceptable salt thereof,

α,α-bis(4-fluorophenyl)-1-[2-(phenylthio)ethyl]-4-piperidinemethanol or a pharmaceutically acceptable salt thereof,

4-[bis(4-fluorophenyl)methylene]-1-[2-(phenylthio)ethyl]piperidine or a pharmaceutically acceptable salt thereof,

α,α-bis(4-fluorophenyl)-1-[2-[(4-chlorophenyl)sulfonyl]ethyl]-4-piperidinemethanol or a pharmaceutically acceptable salt thereof,

1-[2-[(4-chlorophenyl)sulfonyl]ethyl]-4-[bis(4-fluorophenyl)methylene] piperidine or a pharmaceutically acceptable salt thereof,

4-[bis(4-fluorophenyl)methyl]-1-[3-(phenylsulfonyl)propyl]piperidine or a pharmaceutically acceptable salt thereof,

4-[bis(4-fluorophenyl)methyl]-1-[2-[(4-chlorophenyl)sulfonyl]ethyl] piperidine or a pharmaceutically acceptable salt thereof, or

4-[bis(4-fluorophenyl)methyl]-1-[2-(phenylsulfonyl)ethyl]piperidine or a pharmaceutically acceptable salt thereof.

10. 1-(2,3-Dihydro-1,4-benzodioxan-2-ylmethyl)-α,α-diphenyl-4-piperidineacetonitrile or a pharmaceutically acceptable salt thereof,

1-[3-(4-acetyl-2-methoxyphenoxy)propyl]-α,α-diphenyl-4-piperidineacetonitrile or a pharmaceutically acceptable salt thereof,

1-[3-(4-acetyl-2-methoxyphenoxy)propyl]-α,α-diphenyl-3-piperidinepropane-nitrile or a pharmaceutically acceptable salt thereof,

1-[4-(4-acetyl-2-methoxyphenoxy)butyl]-α,α-diphenyl-3-piperidinepropane-nitrile or a pharmaceutically acceptable salt thereof,

1-[3-(4-acetyl-2-methoxyphenoxy)propyl]-α,α-diphenyl-3-pyrrolidineacetamide or a pharmaceutically acceptable salt thereof,

1-[3-(4-acetyl-2-methoxyphenoxy)propyl]-α,α-diphenyl-4-piperidineacetamide or a pharmaceutically acceptable salt thereof,

1-[4-(4-acetyl-2-methoxyphenoxy)butyl]-α,α-diphenyl-4-piperidineacetamide or a pharmaceutically acceptable salt thereof,

1-[3-(4-acetyl-2-methoxyphenoxy)propyl]-α,α-diphenyl-3-piperidine-propanamide or a pharmaceutically acceptable salt thereof,

4-[bis(4-fluorophenyl)methyl]-1-[(2,3-dihydro-1,4-benzodioxan-2-yl)methyl] piperidine or a pharmaceutically acceptable salt thereof,

1-[2-(2,6-dichlorophenoxy)ethyl]-α,α-diphenyl-3-piperidinepropane-nitrile or a pharmaceutically acceptable salt thereof, or

1-[5-(4-acetyl-2-methoxyphenoxy)pentyl]-α,α-diphenyl-3-piperidinepropane-nitrile or a pharmaceutically acceptable salt thereof.

11. 1-[3-(4-Acetyl-2-methoxyphenoxy)propyl]-α,α-bis(4-fluorophenyl)-4-piperidineacetonitrile or a pharmaceutically acceptable salt thereof,

1-[3-(2,6-dichlorophenoxy)propyl]-α,α-bis(4-fluorophenyl)-4-acetonitrile or a pharmaceutically acceptable salt thereof,

1-[3-(2,6-dichlorophenoxy)propyl]-α,α-diphenyl-3-piperidinepropanenitrile or a pharmaceutically acceptable salt thereof,

1-[4-(4-acetyl-2-methoxyphenoxy)butyl]-α,α-bis(4-fluorophenyl)-4-piperidineacetonitrile or a pharmaceutically acceptable salt thereof,

1-[2-(2,6-dichlorophenoxy)ethyl]-α,α-diphenyl-3-piperidinepropanamide or a pharmaceutically acceptable salt thereof,

α-[1-[3-(4-acetyl-2-methoxyphenoxy)propyl]-4-piperidinyl]-α-(4-fluorophenyl)-2-pyridineacetonitrile or a pharmaceutically acceptable salt thereof,

α-[1-[3-(4-acetyl-2-methoxyphenoxy)propyl]-4-piperidinyl]-α-(4-fluorophenyl)-2-pyridineacetonitrile or a pharmaceutically acceptable salt thereof,

α,α-diphenyl-1-[3-(8-quinolinyloxy)propyl]-3-piperidinepropanenitrile or a pharmaceutically acceptable salt thereof,

8-[3-[4-[bis(4-fluorophenyl)methyl]-1-piperidinyl]propoxy]quinoline or a pharmaceutically acceptable salt thereof, or

4-[bis(4-fluorophenyl)methyl]-N-phenyl-1-piperidinepropanamine or a pharmaceutically acceptable salt thereof.

12. N-[3-[4-[Bis(4-fluorophenyl)methyl]-1-piperidinyl]propyl]-N-methylbenzene-amine or a pharmaceutically acceptable salt thereof,

1-[3-(4-acetyl-2-methoxyphenoxy)propyl]-α,α-bis(4-fluorophenyl)-3-piperidineacetonitrile or a pharmaceutically acceptable salt thereof,

1-[3-(4-cyano-phenoxy)propyl]-α,α-bis(4-fluorophenyl)-3-piperidineacetonitrile or a pharmaceutically acceptable salt thereof,

4-[bis(4-fluoro-phenyl)methyl]-1-[3-(1-naphthylenyloxy)propyl]piperidine or a pharmaceutically acceptable salt thereof,

2-[3-[4-[bis(4-fluorophenyl)methyl]-1-piperidinyl]propoxy]quinoline or a pharmaceutically acceptable salt thereof, or

4-[bis(4-fluoro-phenyl)methyl]-1-[3-(2-naphthalenyloxy)propyl]piperidine or a pharmaceutically acceptable salt thereof.

13. The use of a compound having calcium antagonist property selected from the group of compounds having the formula:

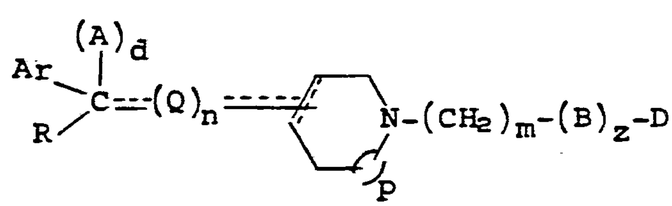

wherein;

p is zero, one or two;

A is hydrogen, $-O-R^1$, $-C{\equiv}N$, $-\underset{O}{\underset{\|}{C}}NR^1R^2$, $-\overset{O}{\overset{\|}{C}}-R^1$, $-\overset{O}{\overset{\|}{C}}-O-R^1$, $-O-\overset{O}{\overset{\|}{C}}-R^1$, $-CH_2OR_1$, $-CH_2NR^1R^2$;

m is zero to six inclusive;

Q is $-CH-$, $-CH_2-$ or $-\overset{OH}{\underset{H}{C}}-$;

d and n are selected from zero or one and the dotted lines represent double bonds which may form consistent with the valence of carbon;

Ar, D and R are selected independently from: of

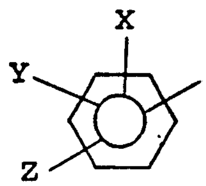, 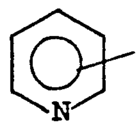, 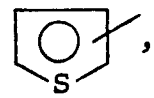, 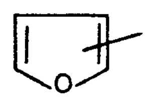 or 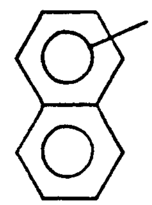

and in addition, R may have the values:

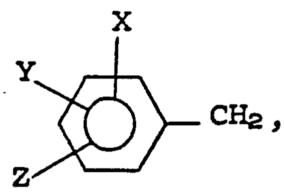 cycloalkyl or loweralkyl and

D may have additionally the values:

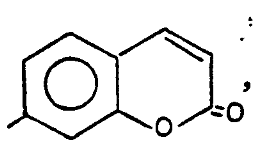, 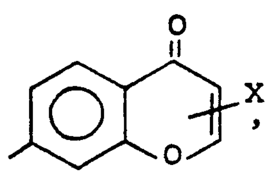, 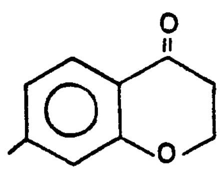, 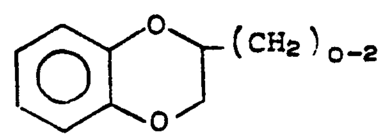,

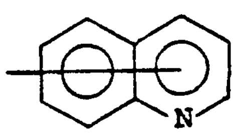, or $Ar(CH_2)_{1-4}-$; X, Y and Z are selected from the group consisting of hydrogen, loweralkyl, halogen, $-NO_2$, $-O-R^1$, $-C(O)-R^1$, $-CF_3$, $-C{\equiv}N$, $-C(O)-N(R^1)_2$, $-N(R^1)_2$, $-C(O)OR^1$, $-SO_2R^2$, $-SR^2$, $-S(O)R^2$, $-N(R^1)-C(O)-R^1$, $-CH_2COOM$, $-SO_2N(R^1)(R^2)$, $-N(R^1)SO_2CH_3$, $-N(R^1)C(O)-N(R^1)(R^2)$, or $-N(R^1)C(O)-OR^2$;

B is selected from O, S, $-S(O)-$, $-S(O)_2-$, $-N(R^1)-$, and $-N-C(O)-O-R^1$;

z is one or zero with the proviso that z cannot be zero at the same time n is zero when one of the following occurs at the same time that D is phenyl or substituted

phenyl: $(A)_d$ is hydrogen, $(A)_d$ is cyano, $(A)_d$ is aminocarbonyl, or a double bond forms between the $\alpha$-carbon and a carbon of the central heterocyclic amine ring;

$R^1$ is selected from hydrogen, loweralkyl, phenyl, and phenylloweralkyl;

$R^2$ is selected from loweralkyl, phenyl and phenylloweralkyl;

M is a pharmaceutically acceptable metal ion, and the pharmaceutically acceptable salts thereof, including acid addition salts, quaternary salts, and hydrates and alcoholates thereof, in the preparation of an antihypertensive agent.

14. The use as claimed in claim 13, wherein the compound is also defined by any one of claims 2 to 8.

15. The use of
4-(diphenylmethylene)-1-(3-phenoxypropyl)piperidine or a pharmaceutically acceptable salt thereof,

α,α-bis(4-fluorophenyl)-1-(3-phenoxypropyl)-4-piperidinemethanol or a pharmaceutically acceptable salt thereof,
4-[bis(4-fluorophenyl)methylene]-1-(3-phenoxypropyl)-piperidine or a pharmaceutically acceptable salt thereof,

4-(diphenylmethyl)-1-(4-phenoxybutyl)piperidine or a pharmaceutically acceptable salt thereof,

4-(diphenylmethyl)-1-(3-phenoxypropyl)piperidine or a pharmaceutically acceptable salt thereof,

4-[bis(4-fluorophenyl)methyl]-1-(3-phenoxypropyl)piperidine or a pharmaceutically acceptable salt thereof,

4-(diphenylmethyl)-1-(2-phenoxyethyl)piperidine or a pharmaceutically acceptable salt thereof,

4-[bis(4-fluorophenyl)methyl]-1-(2-phenoxyethyl)piperidine or a pharmaceutically acceptable salt thereof,

4-[bis(4-fluorophenyl)methyl]-1-(4-phenoxybutyl)piperidine or a pharmaceutically acceptable salt thereof,

4-[(4-fluorophenyl)phenylmethyl]-1-(3-phenoxypropyl) piperidine or a pharmaceutically acceptable salt thereof,

4-[bis(4-fluorophenyl)methyl]-1-[2-(2,6-dichlorophenoxy) ethyl]piperidine or a pharmaceutically acceptable salt thereof,

1-[3-(4-chlorophenoxy)propyl]-α,α-bis(4-fluorophenyl)-4-piperidinemethanol or a pharmaceutically acceptable salt thereof,

4-[bis(4-fluorophenyl)methyl]-1-[3-(2-fluorophenoxy)propyl] piperidine or a pharmaceutically acceptable salt thereof,

4-[bis(4-fluorophenyl)methyl]-1-[3-(3-fluorophenoxy) propyl]piperidine or a pharmaceutically acceptable salt thereof,

4-[bis(4-fluorophenyl)methyl]-1-[3-(4-chlorophenoxy)propyl] piperidine or a pharmaceutically acceptable salt thereof,

4-[bis(4-fluorophenyl)methyl]-1-[3-(4-fluorophenoxy)propyl] piperidine or a pharmaceutically acceptable salt thereof, or

4-[bis(4-fluorophenyl)methyl]-1-[3-(4-methoxyphenoxy)propyl] piperidine or a pharmaceutically acceptable salt thereof in the preparation of an antihypertensive agent.

16. The use of

4-[bis(4-fluorophenyl)methyl]-1-[3-(2-methoxyphenoxy)propyl] piperidine or a pharmaceutically acceptable salt thereof,

α,α-bis(4-fluorophenyl)-1-[3-(2-methoxyphenoxy)propyl]-4-piperidinemethanol or a pharmaceutically acceptable salt thereof,

4-[bis(4-fluorophenyl)methylene]-1-[3-(2-methoxyphenoxy) propyl]piperidine or a pharmaceutically acceptable salt thereof,

4-[bis(4-fluorophenyl)methyl]-1-[3-(3,4-dimethoxyphenoxy) propyl]piperidine or a pharmaceutically acceptable salt thereof,

4-[bis(4-methylphenyl)methyl]-1-[3-(2,6-dimethoxyphenoxy) propyl]piperidine or a pharmaceutically acceptable salt thereof,

4-[bis(4-fluorophenyl)methylene]-1-[3-(3,4-dimethoxyphenoxy) propyl]piperidine or a pharmaceutically acceptable salt thereof,

4-[bis(4-fluorophenyl)methyl]-1-[3-(2,6-dimethoxyphenoxy) propyl]piperidine or a pharmaceutically acceptable salt thereof,

4-[bis(4-fluorophenyl)methyl]-1-[3-(3,5-dimethoxyphenoxy) propyl]piperidine or a pharmaceutically acceptable salt thereof,

4-[bis(4-methoxyphenyl)methyl]-1-[3-(3,4-dimethoxyphenoxy) propyl]piperidine or a pharmaceutically acceptable salt thereof,

4-[bis(4-methoxyphenyl)methyl]-1-[3-(4-methoxyphenoxy)propyl] piperidine or a pharmaceutically acceptable salt thereof,

1-[4-[3-[4-[bis(4-fluorophenyl)methylene]-1-piperidinyl] propoxy]phenyl]ethanone or a pharmaceutically acceptable salt thereof,

1-[4-[3-[4-[bis(4-fluorophenyl)methyl]-1-piperidinyl]propoxy] phenyl]ethanone or a pharmaceutically acceptable salt thereof,

1-[4-[3-[4-[bis(4-fluorophenyl)hydroxymethyl]-1-piperidinyl] propoxy]phenyl]ethanone or a pharmaceutically acceptable salt thereof,

1-[4-[3-[4-[bis(4-fluorophenyl)hydroxymethyl]-1-piperidinyl] propoxy]-3-methylphenyl]ethanone or a pharmaceutically acceptable salt thereof,

4-[3-[4-[bis(4-fluorophenyl)hydroxymethyl]-1-piperidinyl] propoxy]benzonitrile or a pharmaceutically acceptable salt thereof,

4-[3-[4-[bis(4-fluorophenyl)methyl]-1-piperidinyl]propoxy] benzonitrile or a pharmaceutically acceptable salt thereof,

4-[3-[4-[bis(4-fluorophenyl)hydroxymethyl]-1-piperidinyl] propoxy]benzoic acid ethyl ester or a pharmaceutically acceptable salt thereof, or

4-[3-[4-[bis(4-fluorophenyl)hydroxymethyl]-1-piperidinyl] propoxy]benzoic acid or a pharmaceutically acceptable salt thereof, in the preparation of an antihypertensive agent.

17. The use of

4-[3-[4-[bis(4-fluorophenyl)methylene]-1-piperidinyl] propoxy]benzoic acid ethyl ester or a pharmaceutically acceptable salt thereof,

4-[3-[4-[bis(4-fluorophenyl)methyl]-1-piperidinyl]propoxy] benzoic acid ethyl ester or a pharmaceutically acceptable salt thereof,

4-[3-[4-[bis(4-methoxyphenyl)methyl]-1-piperidinyl]propoxy] benzoic acid butyl ester or a pharmaceutically acceptable salt thereof,

4-[3-[4-[bis(4-methoxyphenyl)methyl]-1-piperidinyl]propoxy] benzoic acid ethyl ester or a pharmaceutically acceptable salt thereof,

4-[bis(4-fluorophenyl)methylene]-1-[3-[4-(1,1-dimethylethyl)phenoxy]propyl]piperidine or a pharmaceutically acceptable salt thereof,

4-[bis(4-fluorophenyl)methyl]-1-[3-[4-(1,1-dimethylethyl) phenoxy]propyl]piperidine or a pharmaceutically acceptable salt thereof,

4-[bis(4-methoxyphenyl)methyl]-1-[3-[4-(1,1-dimethylethyl) phenoxy]propyl]piperidine or a pharmaceutically acceptable salt thereof,

1-[3-[4-(1,1-dimethyl)phenoxy]propyl]-α,α-bis(4-fluorophenyl)-4-piperidinemethanol or a pharmaceutically acceptable salt thereof,

4-[bis(4-fluorophenyl)methyl]-1-[3-[3-(trifluoromethyl) phenoxy]propyl]piperidine or a pharmaceutically acceptable salt thereof,

N-[4-[3-[4-[bis(4-methylphenyl)methyl]-1-piperidinyl]propoxy]phenyl]acetamide or a pharmaceutically acceptable salt thereof,

N-[4-[3-[4-[bis(4-fluorophenyl)methyl]-1-piperidinyl]propoxy]phenyl]acetamide or a pharmaceutically acceptable salt thereof,

4-[3-[4-[bis(4-fluorophenyl)methyl]-1-piperidinyl]propoxy]benzeneamine or a pharmaceutically acceptable salt thereof,

N-[4-[3-[4-[bis(4-fluorophenyl)hydroxymethyl]-1-piperidinyl]propoxy]phenyl]acetamide or a pharmaceutically acceptable salt thereof,

α,α-bis(4-fluorophenyl)-1-[3-(4-nitrophenoxy)propyl]-4-piperidinemethanol or a pharmaceutically acceptable salt thereof,

4-[3-[4-[bis(4-fluorophenyl)hydroxymethyl]-1-piperidinyl]propoxy]benzamide or a pharmaceutically acceptable salt thereof,

4-[bis(4-fluorophenyl)methyl]-1-[2-(1-naphthalenyloxy)ethyl]piperidine or a pharmaceutically acceptable salt thereof,

4-[bis(4-fluorophenyl)methyl]-1-[2-(2-naphthalenyloxy)ethyl]piperidine or a pharmaceutically acceptable salt thereof, or

1-[4-[3-[4-[bis(4-fluorophenyl)methyl]-1-piperidinyl]propoxy]-3-methoxyphenyl]ethanone or a pharmaceutically acceptable salt thereof, in the preparation of an antihypertensive agent.

18. The use of

1-[4-[3-[4-[bis(4-fluorophenyl)methyl]-1-piperidinyl] propoxy]-3-methoxyphenyl]ethanone or a pharmaceutically acceptable salt thereof,

1-[4-[3-[4-[bis(4-fluorophenyl)methylene]-1-piperidinyl] propoxy]-3-methoxyphenyl]ethanone or a pharmaceutically acceptable salt thereof,

1-[4-[3-[4-[(4-fluorophenyl)phenylmethylene]-1-piperidinyl] propoxy]-3-methoxyphenyl]ethanone or a pharmaceutically acceptable salt thereof,

1-[3-methoxy-4-[3-[4-[phenyl[3-(trifluoromethyl)phenyl] methylene]-1-piperidinyl]propoxy]phenyl]ethanone or a pharmaceutically acceptable salt thereof,

1-[4-[3-[4-(cyclohexylphenylmethylene)-1-piperidinyl] propoxy]-3-methoxyphenyl]ethanone or a pharmaceutically acceptable salt thereof,

1-[4-[3-[4-(cyclohexylphenylmethyl)-1-piperidinyl]propoxy]-3-methoxyphenyl]ethanone or a pharmaceutically acceptable salt thereof,

4-[3-[4-[bis(4-fluorophenyl)methylene]-1-piperidinyl] propoxy]-α-methylbenzenemethanol or a pharmaceutically acceptable salt thereof,

4-[3-[4-[bis(4-fluorophenyl)methyl]-1-piperidinyl]propoxy] 3-methoxy-α-methylbenzenemethanol or a pharmaceutically acceptable salt thereof,

1-[4-[3-[4-(diphenylmethyl)-1-piperidinyl]propoxy]-3-methoxyphenyl]ethanone or a pharmaceutically acceptable salt thereof,

1-[4-[3-[4-[bis(4-fluorophenyl)hydroxymethyl]-1-piperidinyl]propoxy]-3-methoxyphenyl]ethanone or a pharmaceutically acceptable salt thereof,

1-[4-[3-[4-[(4-fluorophenyl)hydroxyphenylmethyl]-1-piperidinyl]propoxy]-3-methoxyphenyl]ethanone or a pharmaceutically acceptable salt thereof,

1-[4-[3-[4-(diphenylhydroxymethyl)-1-piperidinyl]propoxy]-3-methoxyphenyl]ethanone or a pharmaceutically acceptable salt thereof,

1-[4-[3-[4-[hydroxyphenyl[-3-(trifluoromethyl)phenyl]methyl]-1-piperidinyl]propoxy]-3-methoxyphenyl]ethanone or a pharmaceutically acceptable salt thereof,

1-[4-[3-[4-(cyclohexylhydroxyphenylmethyl)-1-piperidinyl]propoxy]-3-methoxyphenyl]ethanone or a pharmaceutically acceptable salt thereof,

1-[4-[2-[4-[bis(4-fluorophenyl)hydroxymethyl]-1-piperidinyl]ethoxy]-3-methoxyphenyl]ethanone or a pharmaceutically acceptable salt thereof, or

1-[4-[4-[4-[bis(4-fluorophenyl)hydroxymethyl]-1-piperidinyl]butoxy]-3-methoxyphenyl]ethanone or a pharmaceutically acceptable salt thereof, in the preparation of an antihypertensive agent.

19. The use of

1-[4-[5-[4-[bis(4-fluorophenyl)hydroxymethyl]-1-piperidinyl]-pentoxy]-3-methoxyphenyl]ethanone or a pharmaceutically acceptable salt thereof,

1-[4-[2-[4-[bis(4-fluorophenyl)methyl]-1-piperidinyl] ethoxy]-3-methoxyphenyl]ethanone or a pharmaceutically acceptable salt thereof,

1-[4-[3-[4-[bis(4-chlorophenyl)methylene]-1-piperidinyl] propoxy]-3-methoxyphenyl]ethanone or a pharmaceutically acceptable salt thereof,

1-[4-[3-[4-[(4-fluorophenyl)phenylmethyl]-1-piperidinyl] propoxy]-3-methoxyphenyl]ethanone or a pharmaceutically acceptable salt thereof,

1-[4-[3-[4-[bis(4-methoxyphenyl)methyl]-1-piperidinyl] propoxy]-3-methoxyphenyl]ethanone or a pharmaceutically acceptable salt thereof,

1-[4-[3-[4-[bis(4-methylphenyl)methyl]-1-piperidinyl] propoxy]-3-methoxyphenyl]ethanone or a pharmaceutically acceptable salt thereof,

1-[4-[4-[4-[bis(4-fluorophenyl)methyl]-1-piperidinyl] butoxy]-3-methoxyphenyl]ethanone or a pharmaceutically acceptable salt thereof,

4-[3-[4-[bis(4-fluorophenyl)hydroxymethyl]-1-piperidinyl] propoxy]-3-methoxybenzoic acid methyl ester or a pharmaceutically acceptable salt thereof,

α,α-[bis(4-fluorophenyl)]-1-[3-[4-(methylthio)phenoxy] propyl]-4-piperidinemethanol or a pharmaceutically acceptable salt thereof,

α,α-[bis(4-fluorophenyl)]-1-[3-[4-(methylsulfonyl)phenoxy] propyl]-4-piperidinemethanol or a pharmaceutically acceptable salt thereof,

4-[3-[4-[bis(4-fluorophenyl)hydroxymethyl]-1-piperidinyl] propoxy]-3-methoxybenzeneacetic acid or a pharmaceutically acceptable salt thereof,

7-[3-[4-[bis(4-fluorophenyl)hydroxymethyl]-1-piperidinyl] propoxy]-2H-1-benzopyran-2-one or a pharmaceutically acceptable salt thereof,

2-[3-[4-[bis(4-fluorophenyl)hydroxymethyl]-1-piperidinyl] propoxy]benzoic acid ethyl ester or a pharmaceutically acceptable salt thereof,

2-[3-[4-[bis(4-fluorophenyl)methyl]-1-piperidinyl]propoxy] benzoic acid ethyl ester or a pharmaceutically acceptable salt thereof,

1-[4-[5-[4-[bis(4-fluorophenyl)methyl]-1-piperidinyl] pentoxy]-3-methoxyphenyl]ethanone or a pharmaceutically acceptable salt thereof,

4-[3-[4-[bis(4-fluorophenyl)methyl]-1-piperidinyl]propoxy] benzamide or a pharmaceutically acceptable salt thereof, or

4-[bis(4-fluorophenyl)methyl]-1-[3-[4-(methylsulfonyl) pentoxy]propyl]piperidine or a pharmaceutically acceptable salt thereof, in the preparation of an antihypertensive agent.

20. The use of
4-[3-[4-[bis(4-fluorophenyl)hydroxymethyl]-1-piperidinyl]propoxy]benzenesulfonamide or a pharmaceutically acceptable salt thereof,

7-[3-[4-[bis(4-fluorophenyl)hydroxymethyl]-1-piperidinyl]propoxy]-4-oxo-4H-1-benzopyran-2-carboxylic acid ethyl ester or a pharmaceutically acceptable salt thereof,

1-[4-[3-[4-(diphenylmethylene)-1-piperidinyl]propoxy]-3-methoxyphenyl]ethanone or a pharmaceutically acceptable salt thereof,

1-[4-[3-[4-(cyclohexylphenylmethyl)-1,2,3,6-tetrahydro-pyridin-1-yl]propoxy]-3-methoxyphenyl]ethanone or a pharmaceutically acceptable salt thereof,

1-[4-[3-[4-[bis(4-fluorophenyl)hydroxymethyl]-1-piperidinyl]propoxy]-2-methoxyphenyl]ethanone or a pharmaceutically acceptable salt thereof,

1-[3-(2,6-dichlorophenoxy)propyl]-4-[bis(4-fluorophenyl)methyl]piperidine or a pharmaceutically acceptable salt thereof,

2-[3-[4-[bis(4-fluorophenyl)methyl]-1-piperidinyl]propoxy]benzonitrile or a pharmaceutically acceptable salt thereof,

α,α-bis(4-fluorophenyl)-1-[2-(phenylthio)ethyl]-4-piperidinemethanol or a pharmaceutically acceptable salt thereof,

4-[bis(4-fluorophenyl)methylene]-1-[2-(phenylthio)ethyl]piperidine or a pharmaceutically acceptable salt thereof,

α,α-bis(4-fluorophenyl)-1-[2-[(4-chlorophenyl)sulfonyl]ethyl]-4-piperidinemethanol or a pharmaceutically acceptable salt thereof,

1-[2-[(4-chlorophenyl)sulfonyl]ethyl]-4-[bis(4-fluorophenyl)methylene]piperidine or a pharmaceutically acceptable salt thereof,

4-[bis(4-fluorophenyl)methyl]-1-[3-(phenylsulfonyl)propyl]piperidine or a pharmaceutically acceptable salt thereof,

4-[bis(4-fluorophenyl)methyl]-1-[2-[(4-chlorophenyl)sulfonyl]ethyl]piperidine or a pharmaceutically acceptable salt thereof,

4-[bis(4-fluorophenyl)methyl]-1-[2-(phenylsulfonyl)ethyl]piperidine or a pharmaceutically acceptable salt thereof,

1-(2,3-dihydro-1,4-benzodioxan-2-ylmethyl)-α,α-diphenyl-4-piperidineacetonitrile or a pharmaceutically acceptable salt thereof,

1-[3-(4-acetyl-2-methoxyphenoxy)propyl]-α,α-diphenyl-4-piperidineacetonitrile or a pharmaceutically acceptable salt thereof,

1-[3-(4-acetyl-2-methoxyphenoxy)propyl]-α,α-diphenyl-3-piperidinepropanenitrile or a pharmaceutically acceptable salt thereof, or

1-[4-(4-acetyl-2-methoxyphenoxy)butyl]-α,α-diphenyl-3-piperidinepropanenitrile or a pharmaceutically acceptable salt thereof, in the preparation of an antihypertensive agent.

21. The use of

1-[3-(4-acetyl-2-methoxyphenoxy)propyl]-α,α-diphenyl-3-pyrrolidineacetamide or a pharmaceutically acceptable salt thereof,

1-[3-(4-acetyl-2-methoxyphenoxy)propyl]-α,α-diphenyl-4-piperidineacetamide or a pharmaceutically acceptable salt thereof,

1-[4-(4-acetyl-2-methoxyphenoxy)butyl]-α,α-diphenyl-4-piperidineacetamide or a pharmaceutically acceptable salt thereof,

1-[3-(4-acetyl-2-methoxyphenoxy)propyl]-α,α-diphenyl-3-piperidinepropanamide or a pharmaceutically acceptable salt thereof,

4-[bis(4-fluorophenyl)methyl]-1-[(2,3-dihydro-1,4-benzodioxan-2-yl)methyl]piperidine or a pharmaceutically acceptable salt thereof,

1-[2-(2,6-dichlorophenoxy)ethyl]-α,α-diphenyl-3-piperidinepropanenitrile or a pharmaceutically acceptable salt thereof,

1-[5-(4-acetyl-2-methoxyphenoxy)pentyl]-α,α-diphenyl-3-piperidinepropanenitrile or a pharmaceutically acceptable salt thereof,

1-[3-(4-acetyl-2-methoxyphenoxy)propyl]-α,α-bis(4-fluorophenyl)-4-piperidineacetonitrile or a pharmaceutically acceptable salt thereof,

1-[3-(2,6-dichlorophenoxy)propyl]-α,α-bis(4-fluorophenyl)-4-acetonitrile or a pharmaceutically acceptable salt thereof,

1-[3-(2,6-dichlorophenoxy)propyl]-α,α-diphenyl-3-piperidinepropanenitrile or a pharmaceutically acceptable salt thereof,

1-[4-(4-acetyl-2-methoxyphenoxy)butyl]-α,α-bis(4-fluorophenyl)-4-piperidineacetonitrile or a pharmaceutically acceptable salt thereof,

1-[2-(2,6-dichlorophenoxy)ethyl]-α,α-diphenyl-3-piperidinepropanamide or a pharmaceutically acceptable salt thereof,

α-[1-[3-(4-acetyl-2-methoxyphenoxy)propyl]-4-piperidinyl]-α-(4-fluorophenyl)-2-pyridineacetonitrile or a pharmaceutically acceptable salt thereof,

α,α-diphenyl-1-[3-(8-quinolinyloxy)propyl]-3-piperidinepropanenitrile or a pharmaceutically acceptable salt thereof,

8-[3-[4-[bis(4-fluorophenyl)methyl]-1-piperidinyl]propoxy] quinoline or a pharmaceutically acceptable salt thereof,

2-[3-[4-[bis(4-fluorophenyl)methyl]-1-piperidinyl]propoxy] benzoic acid or a pharmaceutically acceptable salt thereof,

4-[bis(4-fluorophenyl)methyl]-N-phenyl-1-piperidinepropanamine or a pharmaceutically acceptable salt thereof,

N-[3-[4-[bis(4-fluorophenyl)methyl]-1-piperidinyl]propyl]-N-methylbenzeneamine or a pharmaceutically acceptable salt thereof,

1-[3-(4-acetyl-2-methoxyphenoxy)propyl]-α,α-bis(4-fluorophenyl)-3-piperidineacetonitrile or a pharmaceutically acceptable salt thereof.

1-[3-(4-cyanophenoxy)propyl]-α,α-bis(4-fluorophenyl)-3-piperidineacetonitrile or a pharmaceutically acceptable salt thereof.

4-[3-[4-[bis(4-fluorophenyl)methyl]-1-piperidinyl]propoxy]-3-methoxybenzonitrile or a pharmaceutically acceptable salt thereof.

4-[bis(4-fluorophenyl)methyl]-1-[3-(1-naphthalenyloxy)propyl]piperidine or a pharmaceutically acceptable salt thereof.

2-[3-[4-[bis(4-fluorophenyl)methyl]-1-piperidinyl]propoxy]quinoline or a pharmaceutically acceptable salt thereof.

4-[bis(4-fluorophenyl)methyl]-1-[3-(2-naphthalenyloxy)propyl]piperidine or a pharmaceutically acceptable salt thereof. or

3-[3-[4-[bis(4-fluorophenyl)methyl]-1-piperidinyl]propoxy]benzonitrile or a pharmaceutically acceptable salt thereof. in the preparation of an antihypertensive agent.

22. The use of a compound selected from the group having the formula:

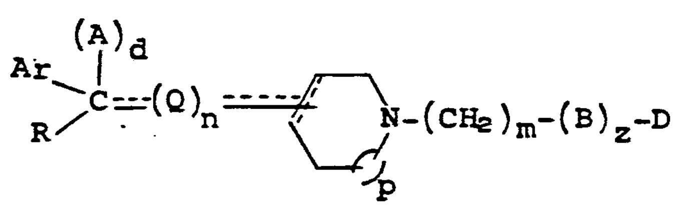

wherein;

p is zero, one or two;

A is hydrogen, $-O-R^1$, $-C{\equiv}N$, $-\underset{\underset{O}{\parallel}}{C}NR^1R^2$, $-\overset{\overset{O}{\parallel}}{C}-R^1$, $-\overset{\overset{O}{\parallel}}{C}-O-R^1$, $-O-\overset{\overset{O}{\parallel}}{C}-R^1$, $-CH_2OR^1$, $-CH_2NR^1R^2$;

m is zero to six inclusive;

Q is $-CH-$, $-CH_2-$ or $-\overset{\overset{OH}{|}}{\underset{\underset{H}{|}}{C}}-$;

d and n are selected from zero or one and the dotted lines represent double bonds which may form consistent with the valence of carbon;

Ar, D and R are selected independently from:

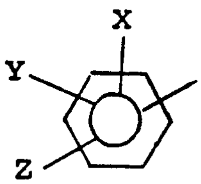, 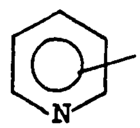, 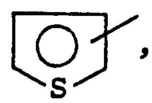, 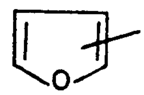 or 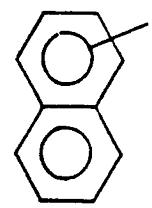

and in addition, R may have the values:

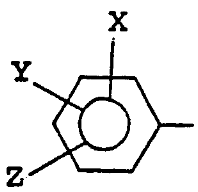 $CH_2$, cycloalkyl or loweralkyl and

D may have additionally the values:

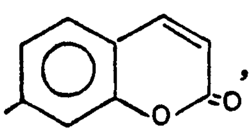, 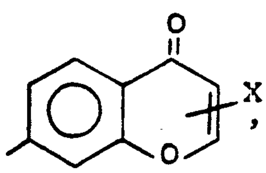, 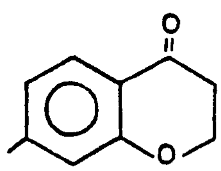, 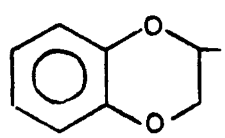 $(CH_2)_{0-2}-$,

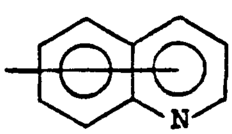,

or $Ar(CH_2)_{1-4}-$; X, Y and Z are selected from the group consisting of hydrogen, loweralkyl, halogen, $-NO_2$, $-O-R^1$, $-\underset{\underset{O}{\parallel}}{C}-R^1$, $-CF_3$, $-C{\equiv}N$, $-\overset{\overset{O}{\parallel}}{C}-N(R^1)_2$, $-N(R^1)_2$, $-C(O)OR^1$, $-SO_2R^2$, $-SR^2$, $-S(O)R^2$, $-\underset{\underset{R^1}{|}}{N}-\underset{\underset{O}{\parallel}}{C}-R^1$, $-CH_2COOM$, $-SO_2N{<}^{R^1}_{R^2}$, $-\underset{\underset{R^1}{|}}{N}SO_2CH_3$, $-\underset{\underset{R^1}{|}}{N}\overset{\overset{O}{\parallel}}{C}-N{<}^{R^1}_{R^2}$, or $-\underset{\underset{R^1}{|}}{N}\overset{\overset{O}{\parallel}}{C}-OR^2$;

B is selected from O, S, $-\overset{O}{S}-$, $-\underset{O}{\overset{O}{S}}-$, $-\underset{R^1}{N}-$, and $-N-\overset{O}{C}-O-R^1$;

z is one or zero with the proviso that z cannot be zero at the same time n is zero when one of the following occurs at the same time that D is phenyl or substituted phenyl: $(A)_d$ is hydrogen, $(A)_d$ is cyano, $(A)_d$ is aminocarbonyl or a double bond forms between the $\alpha$-carbon and a carbon of the central heterocyclic amine ring;

$R^1$ is selected from hydrogen, loweralkyl, phenyl and phenylloweralkyl;

$R^2$ is selected from loweralkyl, phenyl and phenylloweralkyl;

M is a pharmaceutically acceptable metal ion, and the pharmaceutically acceptable salts thereof, (including acid addition salts, quaternary salts, and hydrates and alcoholates thereof) in the preparation of an agent for use in the treatment of angina.

23. The use as claimed in claim 22, wherein the compound is also defined by any one of claims 2 to 8.

24. The use of 1-[4-[3-[4-[bis(4-fluorophenyl)methyl]-1-piperidinyl]propoxy]-3-methoxyphenyl]ethanone or a pharmaceutically acceptable salt thereof,

1-[4-[3-[4-[bis(4-fluorophenyl)methylene]-1-piperidinyl] propoxy]-3-methoxyphenyl]ethanone or a pharmaceutically acceptable salt thereof.

1-[3-methoxy-4-[3-[4-[phenyl[3-(trifluoromethyl)phenyl] methylene]-1-piperidinyl]propoxy]phenyl]ethanone or a pharmaceutically acceptable salt thereof.

1-[4-[3-[4-(cyclohexylphenylmethylene)-1-piperidinyl] propoxy]-3-methoxyphenyl]ethanone or a pharmaceutically acceptable salt thereof.

1-[4-[3-[4-(cyclohexylphenylmethyl)-1-piperidinyl]propoxy]-3-methoxyphenyl]ethanone or a pharmaceutically acceptable salt thereof.

1-[4-[3-[4-[hydroxyphenyl[-3-(trifluoromethyl)phenyl] methyl]-1-piperidinyl]propoxy]-3-methoxyphenyl]ethanone or a pharmaceutically acceptable salt thereof,

1-[4-[3-[4-(cyclohexylhydroxyphenylmethyl)-1-piperidinyl] propoxy]-3-methoxyphenyl]ethanone or a pharmaceutically acceptable salt thereof,

1-[4-[2-[4-[bis(4-fluorophenyl)methyl]-1-piperidinyl] ethoxy]-3-methoxyphenyl]ethanone or a pharmaceutically acceptable salt thereof,

1-[4-[3-[4-[(4-fluorophenyl)phenylmethyl]-1-piperidinyl] propoxy]-3-methoxyphenyl]ethanone or a pharmaceutically acceptable salt thereof,

1-[4-[5-[4-[bis(4-fluorophenyl)methyl]-1-piperidinyl] pentoxy]-3-methoxyphenyl]ethanone or a pharmaceutically acceptable salt thereof,

1-[4-[3-[4-(diphenylmethylene)-1-piperidinyl]propoxy]-3-methoxyphenyl]ethanone or a pharmaceutically acceptable salt thereof,

1-[4-[3-[4-(cyclohexylphenylmethyl)-1,2,3,6-tetrahydro-pyridin-1-yl]propoxy]-3-methoxyphenyl]ethanone or a pharmaceutically acceptable salt thereof,

1-(2,3-dihydro-1,4-benzodioxan-2-ylmethyl)-α,α-diphenyl-4-piperidineacetonitrile or a pharmaceutically acceptable salt thereof,

1-[3-(4-acetyl-2-methoxyphenoxy)propyl]-α,α-diphenyl-4-piperidineacetonitrile or a pharmaceutically acceptable salt thereof.

1-[3-(4-acetyl-2-methoxyphenoxy)propyl]-α,α-diphenyl-3-piperidinepropanenitrile or a pharmaceutically acceptable salt thereof.

1-[4-(4-acetyl-2-methoxyphenoxy)butyl]-α,α-diphenyl-3-piperidinepropanenitrile or a pharmaceutically acceptable salt thereof.

1-[2-(2,6-dichlorophenoxy)ethyl]-α,α-diphenyl-3-piperidinepropanenitrile or a pharmaceutically acceptable salt thereof. or

1-[5-(4-acetyl-2-methoxyphenoxy)pentyl]-α,α-diphenyl-3-piperidinepropanenitrile or a pharmaceutically acceptable salt thereof. in the preparation of an agent for use in the treatment of angina.

25. The use of a compound selected from the group having the formula:

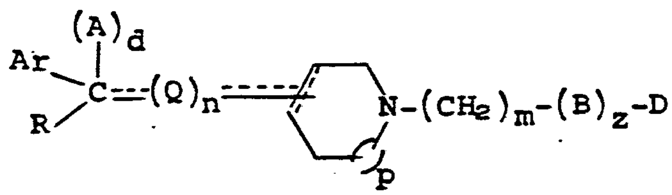

wherein;

p is zero, one or two;

A is hydrogen, $-O-R^1$, $-C\equiv N$, $-C(=O)NR^1R^2$, $-C(=O)-R^1$, $-C(=O)-O-R^1$, $-O-C(=O)-R^1$, $-CH_2-OR^1$, $-CH_2NR^1R^2$;

m is zero to six inclusive;

Q is $-CH-$, $-CH_2-$ or $-C(OH)(H)-$;

d and n are selected from zero or one and the dotted lines represent double bonds which may form consistent with the valence of carbon;

Ar, D and R are selected independently from:

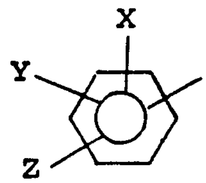 , 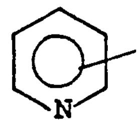 , 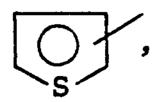 , 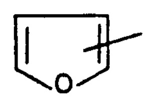 or 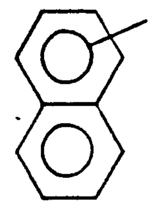 .

and in addition, R may have the values:

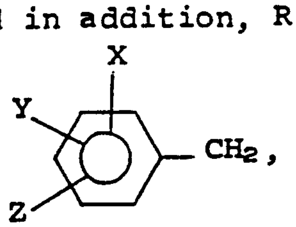 , cycloalkyl or loweralkyl and

D may have additionally the values:

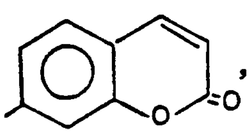 , 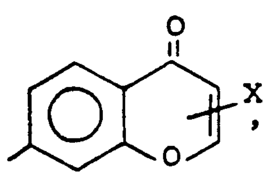 , 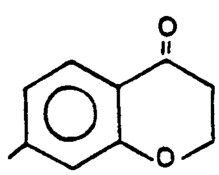 , 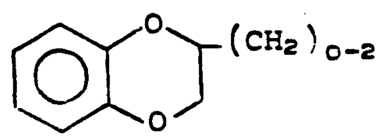 ,

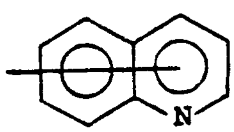 ,

or $Ar(CH_2)_{1-4}-$; X, Y and Z are selected from the group consisting of hydrogen, loweralkyl, halogen, $-NO_2$, $-O-R^1$, $-\underset{\underset{O}{\|}}{C}-R^1$, $-CF_3$, $-C{\equiv}N$, $-\overset{\overset{O}{\|}}{C}-N(R^1)_2$, $-N(R^1)_2$, $-C(O)OR^1$, $-SO_2R^2$, $-SR^2$, $-S(O)R^2$, $-\underset{R^1}{\underset{|}{N}}-\underset{\underset{O}{\|}}{C}-R^1$, $-CH_2COOM$, $-SO_2N\begin{smallmatrix}R^1\\R^2\end{smallmatrix}$, $-\underset{R^1}{\underset{|}{N}}SO_2CH_3$, $-\underset{R^1}{\underset{|}{N}}\overset{\overset{O}{\|}}{C}-N\begin{smallmatrix}R^1\\R^2\end{smallmatrix}$, or $-\underset{R^1}{\underset{|}{N}}\overset{\overset{O}{\|}}{C}-OR^2$;

B is selected from O, S, $-\overset{\overset{O}{\|}}{S}-$, $-\overset{\overset{O}{\|}}{\underset{\underset{O}{\|}}{S}}-$, $-\underset{R^1}{\underset{|}{N}}-$, and $-\overset{|}{N}-\overset{\overset{O}{\|}}{C}-O-R^1$;

z is one or zero with the proviso that z cannot be zero at the same time n is zero when one of the following occurs at the same time that D is phenyl or substituted

phenyl: $(A)_d$ is hydrogen, $(A)_d$ is cyano, $(A)_d$ is aminocarbonyl, or a double bond forms between the α-carbon and a carbon of the central heterocyclic amine ring;

$R^1$ is selected from hydrogen, loweralkyl, phenyl, and phenylloweralkyl;

$R^2$ is selected from loweralkyl, phenyl, and phenylloweralkyl;

M is a pharmaceutically acceptable metal ion, and the pharmaceutically acceptable salts thereof, (including acid addition salts, quaternary salts, and hydrates and alcoholates thereof) in the preparation of an antihistamine agent.

26. The use as claimed in claim 25, wherein the compound is also defined by any one of claims 2 to 8.

27. The use of 1-[4-[3-[4-[bis(4-fluorophenyl)methylene]-1-piperidinyl]propoxy]-3-methoxyphenyl]ethanone or a pharmaceutically acceptable salt thereof, or 1-[4-[3-[4-(diphenylmethylene)-1-piperidinyl]propoxy]-3-methoxyphenyl]-ethanone or a pharmaceutically acceptable salt thereof in the preparation of an antihistamine agent.

28. The use of a compound selected from the group having the formula :

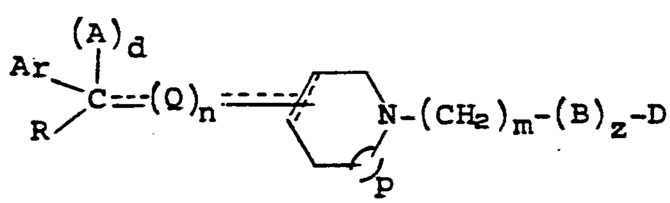

wherein;

p is zero, one or two;

A is hydrogen, $-O-R^1$, $-C{\equiv}N$, $-\overset{}{C}(=O)NR^1R^2$, $-C(=O)-R^1$, $-C(=O)-O-R^1$, $-O-C(=O)-R^1$, $-CH_2OR^1$, $-CH_2NR^1R^2$;

m is zero to six inclusive;

Q is $-CH-$, $-CH_2-$ or $-C(OH)(H)-$;

d and n are selected from zero or one and the dotted lines represent double bonds which may form consistent with the valence of carbon;

Ar, D and R are selected independently from:

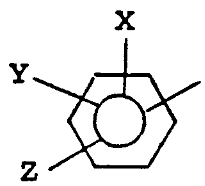 , 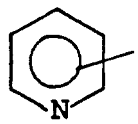 , 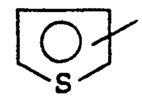 , 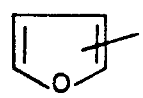 or 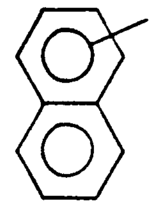

and in addition, R may have the values:

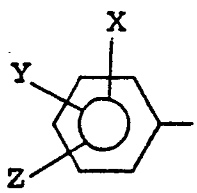 $CH_2$, cycloalkyl or loweralkyl and

D may have additionally the values:

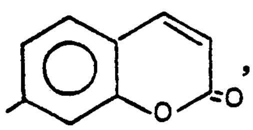 , 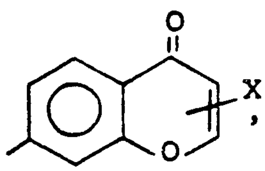 , 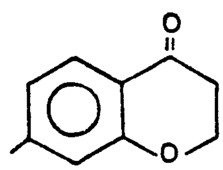 , 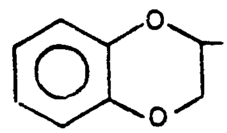 $(CH_2)_{0-2}$- ,

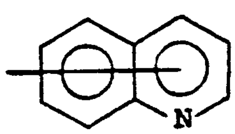 ,

or $Ar(CH_2)_{1-4}$-; X, Y and Z are selected from the group consisting of hydrogen, loweralkyl, halogen, $-NO_2$, $-O-R^1$, $-C(O)-R^1$, $-CF_3$, $-C\equiv N$, $-C(O)-N(R^1)_2$, $-N(R^1)_2$, $-C(O)OR^1$, $-SO_2R^2$, $-SR^2$, $-S(O)R^2$, $-N(R^1)-C(O)-R^1$, $-CH_2COOM$, $-SO_2N(R^1)(R^2)$, $-N(R^1)SO_2CH_3$, $-N(R^1)C(O)-N(R^1)(R^2)$, or $-N(R^1)C(O)-OR^2$;

B is selected from O, S, $-S(O)-$, $-S(O)_2-$, $-N(R^1)-$, and $-N(C(O)-O-R^1)-$;

z is one or zero with the proviso that z cannot be zero at the same time n is zero when one of the following occurs at the same time that D is phenyl or substituted

phenyl: $(A)_d$ is hydrogen, $(A)_d$ is cyano, $(A)_d$ is aminocarbonyl, or a double bond forms between the $\alpha$-carbon and a carbon of the central heterocyclic amine ring;

$R^1$ is selected from hydrogen, loweralkyl, phenyl, and phenylloweralkyl;

$R^2$ is selected from loweralkyl, phenyl, and phenylloweralkyl;

M is a pharmaceutically acceptable metal ion, and the pharmaceutically acceptable salts thereof, (including acid addition salts, quaternary salts, and hydrates and alcoholates thereof) in the preparation of an agent for decreasing gastric secretion and acid release.

29. The use as claimed in claim 28, wherein the compound is also defined by any one of claims 2 to 8.

30. The use of 1-[4-[3-[4-[bis(4-fluorophenyl)methylene]-1-piperidinyl]propoxy]-3-methoxyphenyl]ethanone or a pharmaceutically acceptable salt thereof, or 1-[4-[3-[4-(diphenylmethylene)-1-piperidinyl]propoxy]-3-methoxyphenyl]ethanone or a pharmaceutically acceptable salt thereof, in the preparation of an agent for decreasing gastric secretion and acid release.

31. A compound as defined in any one of claims 1 to 12 for use in human or veterinary medicine.

32. A pharmaceutical or veterinary composition comprising a compound as defined in any one of claims 1 to 12 and a pharmaceutically acceptable carrier therefor.

33. A process for the preparation of a compound having the formula:

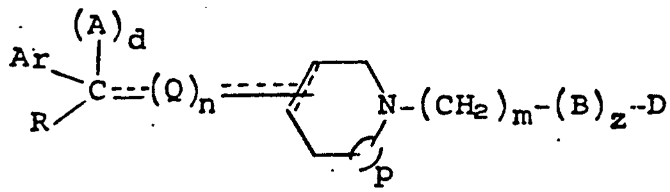

wherein;

p is zero, one or two;

A is hydrogen, $-O-R^1$, $-C\equiv N$, $-\underset{O}{\underset{\|}{C}}NR^1R^2$, $-\overset{O}{\overset{\|}{C}}-R^1$, $-\overset{O}{\overset{\|}{C}}-O-R^1$, $-O-\overset{O}{\overset{\|}{C}}-R^1$, $-CH_2OR^1$, $-CH_2NR^1R^2$;

m is zero to six inclusive;

Q is $-CH-$, $-CH_2-$ or $-\overset{OH}{\underset{H}{C}}-$;

d and n are selected from zero or one and the dotted lines represent double bonds which may form consistent with the valence of carbon;

Ar, D and R are selected independently from:

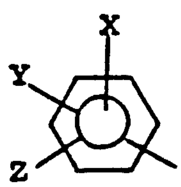 , 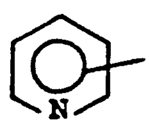 , 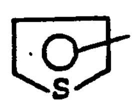 , 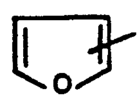 or 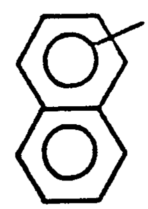

and in addition, R may have the values:

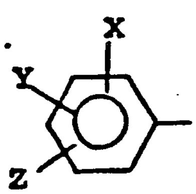 $CH_2$-, cycloalkyl or loweralkyl; and

D may have additionally the values:

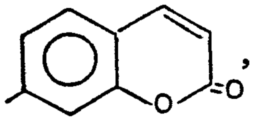, 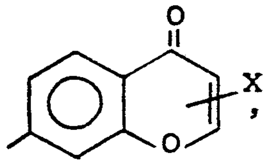, 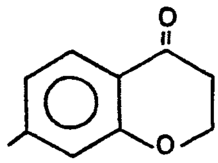, 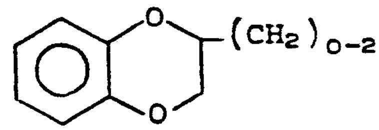,

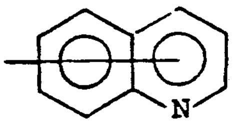,

or $Ar(CH_2)_{1-4}-$; X, Y, and Z are selected from the group consisting of hydrogen, loweralkyl, halogen, $-NO_2$, $-O-R^1$, $-C(O)-R^1$, $-CF_3$, $-C\equiv N$, $-C(O)-N(R^1)_2$, $-N(R^1)_2$, $-C(O)OR^1$, $-SO_2R^2$, $-SR^2$, $-S(O)R^2$, $-N(R^1)-C(O)-R^1$, $-CH_2COOM$, $-SO_2N(R^1)(R^2)$, $-N(R^1)SO_2CH_3$, $-N(R^1)C(O)-N(R^1)(R^2)$, or $-N(R^1)C(O)-OR^2$;

B is selected from O, S, $-S(O)-$, $-S(O)_2-$, $-N(R^1)-$, and $-N(C(O)-O-R^1)-$;

z is one or zero with the proviso that z cannot be zero at the same time n is zero when one of the following occurs at the same time that D is phenyl or substituted phenyl: $(A)_d$ is hydrogen, $(A)_d$ is cyano, $(A)_d$ is aminocarbonyl, or a double bond forms between the $\alpha$ carbon and a carbon of the central heterocyclic amine ring;

$R^1$ is selected from hydrogen, loweralkyl, phenyl and phenylloweralkyl;

$R^2$ is selected from loweralkyl, phenyl and phenylloweralkyl;

M is a pharmaceutically acceptable metal ion, or

a pharmaceutically acceptable salts thereof, (including acid addition salts, quaternary salts, and hydrates and alcoholates thereof) with the further proviso that $(B)_z$ cannot represent oxygen at the same time D is phenyl or substituted phenyl when n is zero and $(A)_d$ is hydrogen or hydroxyl or when d is zero and a double bond forms between the $\alpha$ carbon and a carbon of a saturated central heterocyclic amine ring,

the process comprising either

(A) reacting a compound of the formula

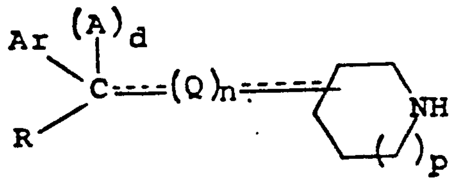

wherein p, A, Q, d, n, Ar and R are as defined for Formula I,

with a compound of the formula

$$X-(CH_2)_m-(B)_z-D$$

wherein m, B, z and D are as defined in Formula I and X represents a halogen atom; or

(B) (when R=Ar and n of (Q)n is zero) reacting a compound of the formula:

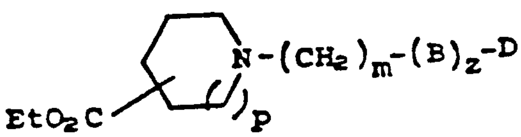

wherein p, m, B, z and D are as defined for Formula I, with a compound of the formula ArMgX, wherein Ar is as defined for Formula I and X is a halogen atom; or

(C) (when (B)z represents -O-) reacting a compound of the formula

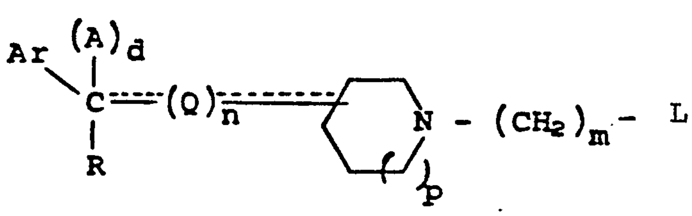

wherein p, A, m, Q, n, d, Ar and R are as defined in Formula I, and L is a leaving group, with a compound M-O-D wherein D is as defined for Formula I and M is an alkali metal; or

(D) (when B)z represents $-N(R_1)-$) reacting a compound of the formula

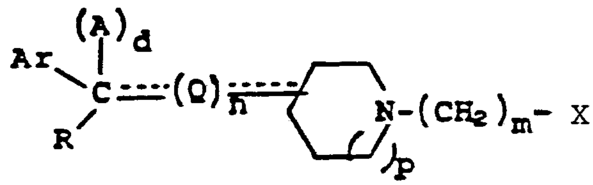

wherein p, A, m, Q, n, d, Ar and R are as defined in Formula I and X is a halogen atom with a compound $HN(R^1)D$, wherein D and $R^1$ are as defined for Formula I; or

(E) (when D is pyridin-2-yl or quinolin-2-yl and (B)z is oxygen) reacting a compound of the formula

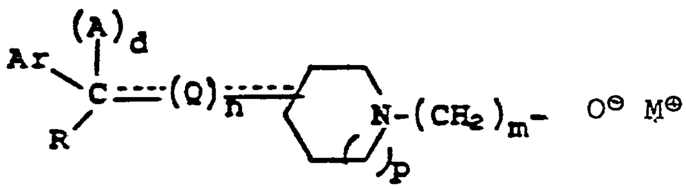

wherein p, A, m, Q, n, d, Ar and R are as defined for Formula I, with 2-halo pyridine or 2-haloquinoline; and

(F) optionally converting a compound (or salt) of Formula I so formed into another compound (or salt) of Formula I.

34. A process as claimed in claim 33 for the preparation of a compound as defined in any one of claims 2 to 12.

CLAIMS FOR THE CONTRACTING STATES: AT, ES and GR.

1. A process for the preparation of a compound having the formula:

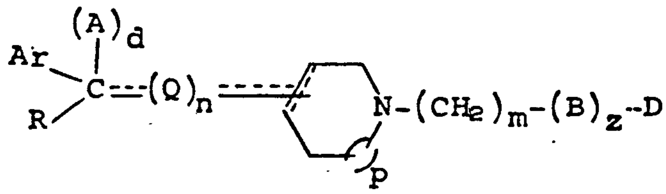

(Formula I)

wherein;

p is zero, one or two;

A is hydrogen, $-O-R^1$, $-C\equiv N$, $-\underset{\underset{O}{\|}}{C}NR^1R^2$, $-\overset{\overset{O}{\|}}{C}-R^1$, $-\overset{\overset{O}{\|}}{C}-O-R^1$, $-O-\overset{\overset{O}{\|}}{C}-R^1$, $-CH_2OR^1$, $-CH_2NR^1R^2$;

m is zero to six inclusive;

Q is $-CH-$, $-CH_2-$ or $-\overset{\overset{OH}{|}}{\underset{\underset{H}{|}}{C}}-$;

d and n are selected from zero or one and the dotted lines represent double bonds which may form consistent with the valence of carbon;

Ar, D and R are selected independently from:

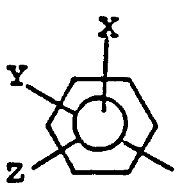 , 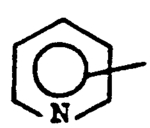 , 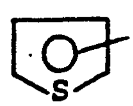 , 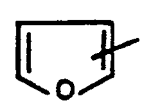 or 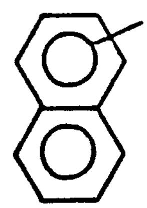

and in addition, R may have the values:

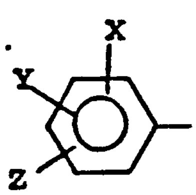$CH_2-$, cycloalkyl or loweralkyl; and

D may have additionally the values:

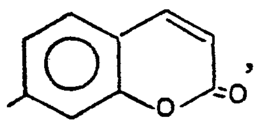, 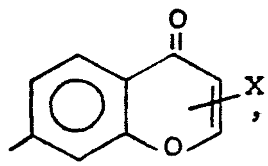, 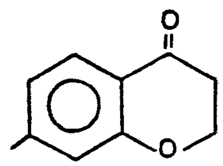, 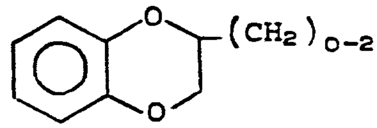,

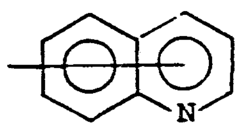,

or $Ar(CH_2)_{1-4}$-; X, Y, and Z are selected from the group consisting of hydrogen, loweralkyl, halogen, $-NO_2$, $-O-R^1$, $-C(=O)-R^1$, $-CF_3$, $-C{\equiv}N$, $-C(=O)-N(R^1)_2$, $-N(R^1)_2$, $-C(O)OR^1$, $-SO_2R^2$, $-SR^2$, $-S(O)R^2$, $-N(R^1)-C(=O)-R^1$, $-CH_2COOM$, $-SO_2N(R^1)(R^2)$, $-N(R^1)SO_2CH_3$, $-N(R^1)C(=O)-N(R^1)(R^2)$, or $-N(R^1)C(=O)-OR^2$;

B is selected from O, S, $-S(=O)-$, $-S(=O)_2-$, $-N(R^1)-$, and $-N-C(=O)-O-R^1$;

z is one or zero with the proviso that z cannot be zero at the same time n is zero when one of the following occurs at the same time that D is phenyl or substituted phenyl: $(A)_d$ is hydrogen, $(A)_d$ is cyano, $(A)_d$ is aminocarbonyl, or a double bond forms between the α carbon and a carbon of the central heterocyclic amine ring;

$R^1$ is selected from hydrogen, loweralkyl, phenyl and phenylloweralkyl;

$R^2$ is selected from loweralkyl, phenyl and phenylloweralkyl;

M is a pharmaceutically acceptable metal ion, or a pharmaceutically acceptable salts thereof, (including acid addition salts, quaternary salts, and hydrates and alcoholates thereof) with the further proviso that $(B)_z$ cannot represent oxygen at the same time D is phenyl or substituted phenyl when n is zero and $(A)_d$ is hydrogen or hydroxyl or when d is zero and a double bond forms between the α carbon and a carbon of a saturated central heterocyclic amine ring,

the process comprising either

(A) reacting a compound of the formula

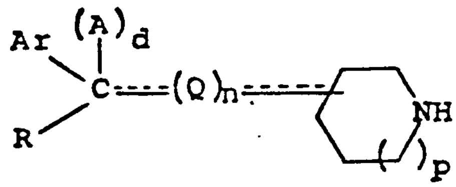

wherein p, A, Q, d, n, Ar and R are as defined for Formula I,
with a compound of the formula

$$X-(CH_2)_m-(B)_z-D$$

wherein m, B, z and D are as defined in Formula I and X represents a halogen atom; or

(B) (when R=Ar and n of (Q)n is zero) reacting a compound of the formula:

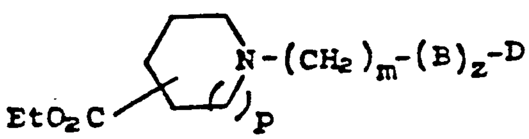

wherein p, m, B, z and D are as defined for Formula I, with a compound of the formula ArMgX, wherein Ar is as defined for Formula I and X is a halogen atom; or

(C) (when (B)z represents -O-) reacting a compound of the formula

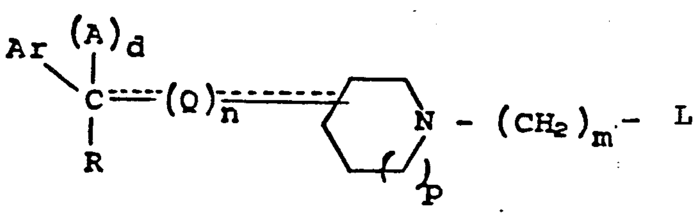

wherein p, A, m, Q, n, d, Ar and R are as defined in Formula I, and L is a leaving group, with a compound M-O-D wherein D is as defined for Formula I and M is an alkali metal; or

(D) (when B)z represents $-N(R_1)-$) reacting a compound of the formula

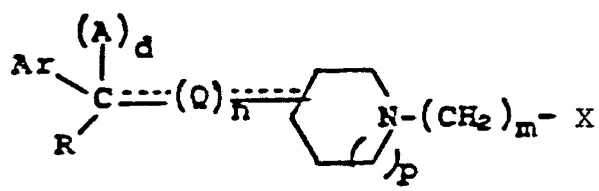

wherein p, A, m, Q, n, d, Ar and R are as defined in Formula I and X is a halogen atom
with a compound $HN(R^1)D$, wherein D and $R^1$ are as defined for Formula I; or

(E) (when D is pyridin-2-yl or quinolin-2-yl and (B)z is oxygen) reacting a compound of the formula

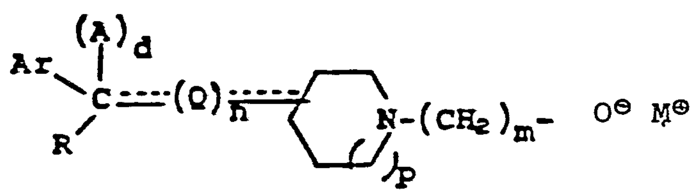

wherein p, A, m, Q, n, d, Ar and R are as defined for Formula I,
with 2-halo pyridine or 2-haloquinoline; and

(F) optionally converting a compound (or salt) of Formula I so formed into another compound (or salt) of Formula I.

2. A process as claimed in claim 1, wherein Ar is unsaturated phenyl or 4-saturated phenyl.

3. A process as claimed in claim 1 or 2, wherein Ar is halo-substituted phenyl, trifluoromethyl-substituted phenyl, loweralkyl-substituted phenyl or loweralkoxy-substituted phenyl.

4. A process as claimed in claim 1, 2 or 3, wherein R is phenyl, 4-substituted phenyl or loweralkyl.

5. A process as claimed in any one of claims 1 to 4, wherein R is halo-substituted phenyl, loweralkyl-substituted phenyl or loweralkoxy-substituted phenyl.

6. A process as claimed in any one of claims 1 to 5, wherein M is two to five inclusive.

7. A process as claimed in any one of claims 1 to 6, wherein p is one.

8. A process as claimed in any one of claims 1 to 7, wherein the left hand substituent in the formula, as drawn, is in the 4-position relative to the ring nitrogen atom.

9. A process as claimed in claim 1 for the preparation of 7-[3-[4-[bis(4-fluorophenyl)hydroxymethyl]-1-piperidinyl]propoxy]-2H-1-benzopyran-2-one or a pharmaceutically acceptable salt thereof,

7-[3-[4-[bis(4-fluorophenyl)hydroxymethyl]-1-piperidinyl]propoxy]-4-oxo-4H-1-benzopyran-2-carboxylic acid ethyl ester or a pharmaceutically acceptable salt thereof,

7-[3-[4-[bis(4-fluorophenyl)hydroxymethyl]-1-piperidinyl]propoxy]-2,3-dihydro-4H-1-benzopyran-4-one or a pharmaceutically acceptable salt thereof,

α,α-bis(4-fluorophenyl)-1-[2-(phenylthio)ethyl]-4-piperidinemethanol or a pharmaceutically acceptable salt thereof,

4-[bis(4-fluorophenyl)methylene]-1-[2-(phenylthio)ethyl]piperidine or a pharmaceutically acceptable salt thereof,

α,α-bis(4-fluorophenyl)-1-[2-[(4-chlorophenyl)sulfonyl]ethyl]-4-piperidinemethanol or a pharmaceutically acceptable salt thereof,

1-[2-[(4-chlorophenyl)sulfonyl]ethyl]-4-[bis(4-fluorophenyl)methylene] piperidine or a pharmaceutically acceptable salt thereof,

4-[bis(4-fluorophenyl)methyl]-1-[3-(phenylsulfonyl)propyl]piperidine or a pharmaceutically acceptable salt thereof,

4-[bis(4-fluorophenyl)methyl]-1-[2-[(4-chlorophenyl)sulfonyl]ethyl] piperidine or a pharmaceutically acceptable salt thereof, or

4-[bis(4-fluorophenyl)methyl]-1-[2-(phenylsulfonyl)ethyl]piperidine or a pharmaceutically acceptable salt thereof.

10. A process as claimed in claim 1 for the preparation of 1-(2,3-dihydro-1,4-benzodioxan-2-ylmethyl)-α,α-diphenyl-4-piperidineacetonitrile or a pharmaceutically acceptable salt thereof,

1-[3-(4-acetyl-2-methoxyphenoxy)propyl]-α,α-diphenyl-4-piperidineacetonitrile or a pharmaceutically acceptable salt thereof,

1-[3-(4-acetyl-2-methoxyphenoxy)propyl]-α,α-diphenyl-3-piperidinepropane-nitrile or a pharmaceutically acceptable salt thereof,

1-[4-(4-acetyl-2-methoxyphenoxy)butyl]-α,α-diphenyl-3-piperidinepropane-nitrile or a pharmaceutically acceptable salt thereof,

1-[3-(4-acetyl-2-methoxyphenoxy)propyl]-α,α-diphenyl-3-pyrrolidineacetamide or a pharmaceutically acceptable salt thereof,

1-[3-(4-acetyl-2-methoxyphenoxy)propyl]-α,α-diphenyl-4-piperidineacetamide or a pharmaceutically acceptable salt thereof,

1-[4-(4-acetyl-2-methoxyphenoxy)butyl]-α,α-diphenyl-4-piperidineacetamide or a pharmaceutically acceptable salt thereof,

1-[3-(4-acetyl-2-methoxyphenoxy)propyl]-α,α-diphenyl-3-piperidine-propanamide or a pharmaceutically acceptable salt thereof,

4-[bis(4-fluoro-phenyl)methyl]-1-[(2,3-dihydro-1,4-benzodioxan-2-yl)methyl] piperidine or a pharmaceutically acceptable salt thereof,

1-[2-(2,6-dichlorophenoxy)ethyl]-α,α-diphenyl-3-piperidinepropane-nitrile or a pharmaceutically acceptable salt thereof, or

1-[5-(4-acetyl-2-methoxyphenoxy)pentyl]-α,α-diphenyl-3-piperidinepropane-nitrile or a pharmaceutically acceptable salt thereof.

11. A process as claimed in claim 1 for the preparation of 1-[3-(4-acetyl-2-methoxyphenoxy)propyl]-α,α-bis(4-fluorophenyl)-4-piperidineacetonitrile or a pharmaceutically acceptable salt thereof,

1-[3-(2,6-dichlorophenoxy)propyl]-α,α-bis(4-fluorophenyl)-4-acetonitrile or a pharmaceutically acceptable salt thereof,

1-[3-(2,6-dichlorophenoxy)propyl]-α,α-diphenyl-3-piperidinepropanenitrile or a pharmaceutically acceptable salt thereof,

1-[4-(4-acetyl-2-methoxyphenoxy)butyl]-α,α-bis(4-fluorophenyl)-4-piperidineacetonitrile or a pharmaceutically acceptable salt thereof,

1-[2-(2,6-dichlorophenoxy)ethyl]-α,α-diphenyl-3-piperidinepropanamide or a pharmaceutically acceptable salt thereof,

α-[1-[3-(4-acetyl-2-methoxyphenoxy)propyl]-4-piperidinyl]-α-(4-fluorophenyl)-2-pyridineacetonitrile or a pharmaceutically acceptable salt thereof,

α-[1-[3-(4-acetyl-2-methoxyphenoxy)propyl]-4-piperidinyl]-α-(4-fluorophenyl)-2-pyridineacetonitrile or a pharmaceutically acceptable salt thereof,

α,α-diphenyl-1-[3-(8-quinolinyloxy)propyl]-3-piperidinepropanenitrile or a pharmaceutically acceptable salt thereof,

8-[3-[4-[bis(4-fluorophenyl)methyl]-1-piperidinyl]propoxy]quinoline or a pharmaceutically acceptable salt thereof, or

4-[bis(4-fluorophenyl)methyl]-N-phenyl-1-piperidinepropanamine or a pharmaceutically acceptable salt thereof.

12. A process as claimed in claim 1 for the preparation of N-[3-[4-[bis(4-fluorophenyl)methyl]-1-piperidinyl]propyl]-N-methylbenzene-amine or a pharmaceutically acceptable salt thereof,

1-[3-(4-acetyl-2-methoxyphenoxy)propyl]-α,α-bis(4-fluorophenyl)-3-piperidineacetonitrile or a pharmaceutically acceptable salt thereof,

1-[3-(4-cyano-phenoxy)propyl]-α,α-bis(4-fluorophenyl)-3-piperidineacetonitrile or a pharmaceutically acceptable salt thereof,

4-[bis(4-fluoro-phenyl)methyl]-1-[3-(1-naphthylenyloxy)propyl]piperidine or a pharmaceutically acceptable salt thereof,

2-[3-[4-[bis(4-fluorophenyl)methyl]-1-piperidinyl]propoxy]quinoline or a pharmaceutically acceptable salt thereof, or

4-[bis(4-fluoro-phenyl)methyl]-1-[3-(2-naphthalenyloxy)propyl]piperidine or a pharmaceutically acceptable salt thereof.

13. The use of a compound having calcium antagonist property selected from the group of compounds having the formula:

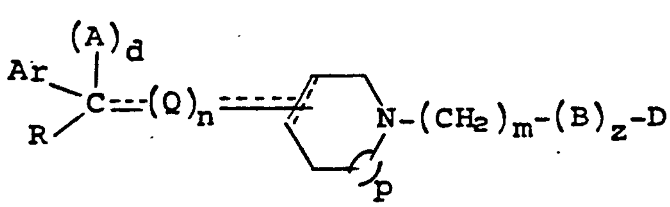

wherein;

p is zero, one or two;

A is hydrogen, $-O-R^1$, $-C \equiv N$, $-\underset{O}{\underset{\|}{C}}NR^1R^2$, $-\overset{O}{\overset{\|}{C}}-R^1$, $-\overset{O}{\overset{\|}{C}}-O-R^1$, $-O-\overset{O}{\overset{\|}{C}}-R^1$, $-CH_2OR_1$, $-CH_2NR^1R^2$;

m is zero to six inclusive;

Q is $-CH-$, $-CH_2-$ or $-\overset{OH}{\underset{H}{C}}-$;

d and n are selected from zero or one and the dotted lines represent double bonds which may form consistent with the valence of carbon;

Ar, D and R are selected independently from: of

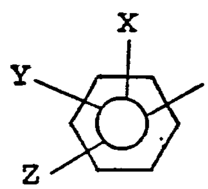, 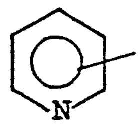, 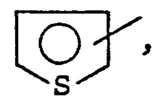, 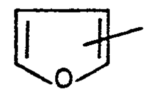 or 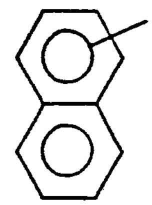

and in addition, R may have the values:

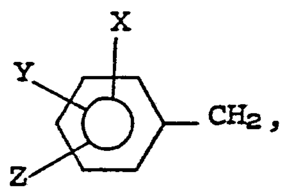, cycloalkyl or loweralkyl and

D may have additionally the values:

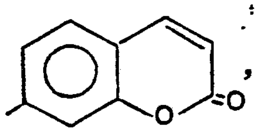, 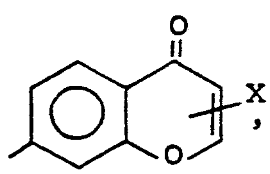, 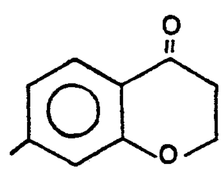, 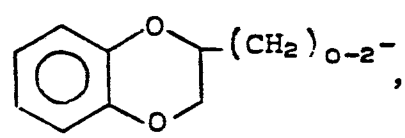,

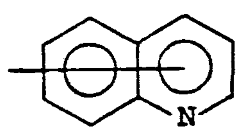,

or $Ar(CH_2)_{1-4}$-; X, Y and Z are selected from the group consisting of hydrogen, loweralkyl, halogen, $-NO_2$, $-O-R^1$, $-\overset{\|}{\underset{O}{C}}-R^1$, $-CF_3$, $-C{\equiv}N$, $-\overset{O}{\overset{\|}{C}}-N(R^1)_2$, $-N(R^1)_2$, $-C(O)OR^1$, $-SO_2R^2$, $-SR^2$, $-S(O)R^2$, $-\underset{R^1}{N}-\underset{O}{C}-R^1$, $-CH_2COOM$, $-SO_2N(R^1)(R^2)$, $-\underset{R^1}{N}SO_2CH_3$, $-\underset{R^1}{N}\overset{O}{\overset{\|}{C}}-N(R^1)(R^2)$, or $-\underset{R^1}{N}\overset{O}{\overset{\|}{C}}-OR^2$;

B is selected from O, S, $-\overset{O}{\overset{\|}{S}}-$, $-\overset{O}{\underset{O}{S}}-$, $-\underset{R^1}{N}-$, and $-\overset{|}{N}-\overset{O}{\overset{\|}{C}}-O-R^1$;

z is one or zero with the proviso that z cannot be zero at the same time n is zero when one of the following occurs at the same time that D is phenyl or substituted

phenyl: $(A)_d$ is hydrogen, $(A)_d$ is cyano, $(A)_d$ is aminocarbonyl, or a double bond forms between the α-carbon and a carbon of the central heterocyclic amine ring;

$R^1$ is selected from hydrogen, loweralkyl, phenyl, and phenylloweralkyl;

$R^2$ is selected from loweralkyl, phenyl and phenylloweralkyl;

M is a pharmaceutically acceptable metal ion, and the pharmaceutically acceptable salts thereof, including acid addition salts, quaternary salts, and hydrates and alcoholates thereof, in the preparation of an antihypertensive agent.

14. The use as claimed in claim 13, wherein the compound is also defined by any one of claims 2 to 8.

15. The use of
4-(diphenylmethylene)-1-(3-phenoxypropyl)piperidine or a pharmaceutically acceptable salt thereof,

α,α-bis(4-fluorophenyl)-1-(3-phenoxypropyl)-4-piperidinemethanol or a pharmaceutically acceptable salt thereof,

4-[bis(4-fluorophenyl)methylene]-1-(3-phenoxypropyl)-piperidine or a pharmaceutically acceptable salt thereof,

4-(diphenylmethyl)-1-(4-phenoxybutyl)piperidine or a pharmaceutically acceptable salt thereof,

4-(diphenylmethyl)-1-(3-phenoxypropyl)piperidine or a pharmaceutically acceptable salt thereof,

4-[bis(4-fluorophenyl)methyl]-1-(3-phenoxypropyl)piperidine or a pharmaceutically acceptable salt thereof,

4-(diphenylmethyl)-1-(2-phenoxyethyl)piperidine or a pharmaceutically acceptable salt thereof,

4-[bis(4-fluorophenyl)methyl]-1-(2-phenoxyethyl)piperidine or a pharmaceutically acceptable salt thereof,

4-[bis(4-fluorophenyl)methyl]-1-(4-phenoxybutyl)piperidine or a pharmaceutically acceptable salt thereof,

4-[(4-fluorophenyl)phenylmethyl]-1-(3-phenoxypropyl) piperidine or a pharmaceutically acceptable salt thereof,

4-[bis(4-fluorophenyl)methyl]-1-[2-(2,6-dichlorophenoxy) ethyl]piperidine or a pharmaceutically acceptable salt thereof,

1-[3-(4-chlorophenoxy)propyl]-α,α-bis(4-fluorophenyl)-4-piperidinemethanol or a pharmaceutically acceptable salt thereof,

4-[bis(4-fluorophenyl)methyl]-1-[3-(2-fluorophenoxy)propyl] piperidine or a pharmaceutically acceptable salt thereof,

4-[bis(4-fluorophenyl)methyl]-1-[3-(3-fluorophenoxy) propyl]piperidine or a pharmaceutically acceptable salt thereof,

4-[bis(4-fluorophenyl)methyl]-1-[3-(4-chlorophenoxy)propyl] piperidine or a pharmaceutically acceptable salt thereof,

4-[bis(4-fluorophenyl)methyl]-1-[3-(4-fluorophenoxy)propyl] piperidine or a pharmaceutically acceptable salt thereof, or

4-[bis(4-fluorophenyl)methyl]-1-[3-(4-methoxyphenoxy)propyl] piperidine or a pharmaceutically acceptable salt thereof in the preparation of an antihypertensive agent.

16. The use of

4-[bis(4-fluorophenyl)methyl]-1-[3-(2-methoxyphenoxy)propyl] piperidine or a pharmaceutically acceptable salt thereof,

α,α-bis(4-fluorophenyl)-1-[3-(2-methoxyphenoxy)propyl]-4-piperidinemethanol or a pharmaceutically acceptable salt thereof,

4-[bis(4-fluorophenyl)methylene]-1-[3-(2-methoxyphenoxy) propyl]piperidine or a pharmaceutically acceptable salt thereof,

4-[bis(4-fluorophenyl)methyl]-1-[3-(3,4-dimethoxyphenoxy) propyl]piperidine or a pharmaceutically acceptable salt thereof,

4-[bis(4-methylphenyl)methyl]-1-[3-(2,6-dimethoxyphenoxy) propyl]piperidine or a pharmaceutically acceptable salt thereof,

4-[bis(4-fluorophenyl)methylene]-1-[3-(3,4-dimethoxyphenoxy) propyl]piperidine or a pharmaceutically acceptable salt thereof,

4-[bis(4-fluorophenyl)methyl]-1-[3-(2,6-dimethoxyphenoxy) propyl]piperidine or a pharmaceutically acceptable salt thereof,

4-[bis(4-fluorophenyl)methyl]-1-[3-(3,5-dimethoxyphenoxy) propyl]piperidine or a pharmaceutically acceptable salt thereof,

4-[bis(4-methoxyphenyl)methyl]-1-[3-(3,4-dimethoxyphenoxy) propyl]piperidine or a pharmaceutically acceptable salt thereof,

4-[bis(4-methoxyphenyl)methyl]-1-[3-(4-methoxyphenoxy)propyl] piperidine or a pharmaceutically acceptable salt thereof,

1-[4-[3-[4-[bis(4-fluorophenyl)methylene]-1-piperidinyl] propoxy]phenyl]ethanone or a pharmaceutically acceptable salt thereof,

1-[4-[3-[4-[bis(4-fluorophenyl)methyl]-1-piperidinyl]propoxy] phenyl]ethanone or a pharmaceutically acceptable salt thereof,

1-[4-[3-[4-[bis(4-fluorophenyl)hydroxymethyl]-1-piperidinyl] propoxy]phenyl]ethanone or a pharmaceutically acceptable salt thereof,

1-[4-[3-[4-[bis(4-fluorophenyl)hydroxymethyl]-1-piperidinyl] propoxy]-3-methylphenyl]ethanone or a pharmaceutically acceptable salt thereof,

4-[3-[4-[bis(4-fluorophenyl)hydroxymethyl]-1-piperidinyl] propoxy]benzonitrile or a pharmaceutically acceptable salt thereof,

4-[3-[4-[bis(4-fluorophenyl)methyl]-1-piperidinyl]propoxy] benzonitrile or a pharmaceutically acceptable salt thereof,

4-[3-[4-[bis(4-fluorophenyl)hydroxymethyl]-1-piperidinyl] propoxy]benzoic acid ethyl ester or a pharmaceutically acceptable salt thereof, or

4-[3-[4-[bis(4-fluorophenyl)hydroxymethyl]-1-piperidinyl] propoxy]benzoic acid or a pharmaceutically acceptable salt thereof, in the preparation of an antihypertensive agent.

17. The use of
4-[3-[4-[bis(4-fluorophenyl)methylene]-1-piperidinyl]propoxy]benzoic acid ethyl ester or a pharmaceutically acceptable salt thereof,

4-[3-[4-[bis(4-fluorophenyl)methyl]-1-piperidinyl]propoxy]benzoic acid ethyl ester or a pharmaceutically acceptable salt thereof,

4-[3-[4-[bis(4-methoxyphenyl)methyl]-1-piperidinyl]propoxy]benzoic acid butyl ester or a pharmaceutically acceptable salt thereof,

4-[3-[4-[bis(4-methoxyphenyl)methyl]-1-piperidinyl]propoxy]benzoic acid ethyl ester or a pharmaceutically acceptable salt thereof,

4-[bis(4-fluorophenyl)methylene]-1-[3-[4-(1,1-dimethylethyl)phenoxy]propyl]piperidine or a pharmaceutically acceptable salt thereof,

4-[bis(4-fluorophenyl)methyl]-1-[3-[4-(1,1-dimethylethyl)phenoxy]propyl]piperidine or a pharmaceutically acceptable salt thereof,

4-[bis(4-methoxyphenyl)methyl]-1-[3-[4-(1,1-dimethylethyl)phenoxy]propyl]piperidine or a pharmaceutically acceptable salt thereof,

1-[3-[4-(1,1-dimethyl)phenoxy]propyl]-α,α-bis(4-fluorophenyl)-4-piperidinemethanol or a pharmaceutically acceptable salt thereof,

4-[bis(4-fluorophenyl)methyl]-1-[3-[3-(trifluoromethyl)phenoxy]propyl]piperidine or a pharmaceutically acceptable salt thereof,

N-[4-[3-[4-[bis(4-methylphenyl)methyl]-1-piperidinyl]propoxy]phenyl]acetamide or a pharmaceutically acceptable salt thereof,

N-[4-[3-[4-[bis(4-fluorophenyl)methyl]-1-piperidinyl]propoxy]phenyl]acetamide or a pharmaceutically acceptable salt thereof,

4-[3-[4-[bis(4-fluorophenyl)methyl]-1-piperidinyl]propoxy]benzeneamine or a pharmaceutically acceptable salt thereof,

N-[4-[3-[4-[bis(4-fluorophenyl)hydroxymethyl]-1-piperidinyl]propoxy]phenyl]acetamide or a pharmaceutically acceptable salt thereof,

α,α-bis(4-fluorophenyl)-1-[3-(4-nitrophenoxy)propyl]-4-piperidinemethanol or a pharmaceutically acceptable salt thereof,

4-[3-[4-[bis(4-fluorophenyl)hydroxymethyl]-1-piperidinyl]propoxy]benzamide or a pharmaceutically acceptable salt thereof,

4-[bis(4-fluorophenyl)methyl]-1-[2-(1-naphthalenyloxy)ethyl]piperidine or a pharmaceutically acceptable salt thereof,

4-[bis(4-fluorophenyl)methyl]-1-[2-(2-naphthalenyloxy)ethyl]piperidine or a pharmaceutically acceptable salt thereof, or

1-[4-[3-[4-[bis(4-fluorophenyl)methyl]-1-piperidinyl]propoxy]-3-methoxyphenyl]ethanone or a pharmaceutically acceptable salt thereof, in the preparation of an antihypertensive agent.

18. The use of 1-[4-[3-[4-[bis(4-fluorophenyl)methyl]-1-piperidinyl] propoxy]-3-methoxyphenyl]ethanone or a pharmaceutically acceptable salt thereof,

1-[4-[3-[4-[bis(4-fluorophenyl)methylene]-1-piperidinyl] propoxy]-3-methoxyphenyl]ethanone or a pharmaceutically acceptable salt thereof,

1-[4-[3-[4-[(4-fluorophenyl)phenylmethylene]-1-piperidinyl] propoxy]-3-methoxyphenyl]ethanone or a pharmaceutically acceptable salt thereof,

1-[3-methoxy-4-[3-[4-[phenyl[3-(trifluoromethyl)phenyl] methylene]-1-piperidinyl]propoxy]phenyl]ethanone or a pharmaceutically acceptable salt thereof,

1-[4-[3-[4-(cyclohexylphenylmethylene)-1-piperidinyl] propoxy]-3-methoxyphenyl]ethanone or a pharmaceutically acceptable salt thereof,

1-[4-[3-[4-(cyclohexylphenylmethyl)-1-piperidinyl]propoxy]-3-methoxyphenyl]ethanone or a pharmaceutically acceptable salt thereof,

4-[3-[4-[bis(4-fluorophenyl)methylene]-1-piperidinyl] propoxy]-α-methylbenzenemethanol or a pharmaceutically acceptable salt thereof,

4-[3-[4-[bis(4-fluorophenyl)methyl]-1-piperidinyl]propoxy] 3-methoxy-α-methylbenzenemethanol or a pharmaceutically acceptable salt thereof,

1-[4-[3-[4-(diphenylmethyl)-1-piperidinyl]propoxy]-3-methoxyphenyl]ethanone or a pharmaceutically acceptable salt thereof,

1-[4-[3-[4-[bis(4-fluorophenyl)hydroxymethyl]-1-piperidinyl]propoxy]-3-methoxyphenyl]ethanone or a pharmaceutically acceptable salt thereof,

1-[4-[3-[4-[(4-fluorophenyl)hydroxyphenylmethyl]-1-piperidinyl]propoxy]-3-methoxyphenyl]ethanone or a pharmaceutically acceptable salt thereof,

1-[4-[3-[4-(diphenylhydroxymethyl)-1-piperidinyl]propoxy]-3-methoxyphenyl]ethanone or a pharmaceutically acceptable salt thereof,

1-[4-[3-[4-[hydroxyphenyl[-3-(trifluoromethyl)phenyl]methyl]-1-piperidinyl]propoxy]-3-methoxyphenyl]ethanone or a pharmaceutically acceptable salt thereof,

1-[4-[3-[4-(cyclohexylhydroxyphenylmethyl)-1-piperidinyl]propoxy]-3-methoxyphenyl]ethanone or a pharmaceutically acceptable salt thereof,

1-[4-[2-[4-[bis(4-fluorophenyl)hydroxymethyl]-1-piperidinyl]ethoxy]-3-methoxyphenyl]ethanone or a pharmaceutically acceptable salt thereof, or

1-[4-[4-[4-[bis(4-fluorophenyl)hydroxymethyl]-1-piperidinyl]butoxy]-3-methoxyphenyl]ethanone or a pharmaceutically acceptable salt thereof, in the preparation of an antihypertensive agent.

19. The use of

1-[4-[5-[4-[bis(4-fluorophenyl)hydroxymethyl]-1-piperidinyl]-pentoxy]-3-methoxyphenyl]ethanone or a pharmaceutically acceptable salt thereof,

1-[4-[2-[4-[bis(4-fluorophenyl)methyl]-1-piperidinyl] ethoxy]-3-methoxyphenyl]ethanone or a pharmaceutically acceptable salt thereof,

1-[4-[3-[4-[bis(4-chlorophenyl)methylene]-1-piperidinyl] propoxy]-3-methoxyphenyl]ethanone or a pharmaceutically acceptable salt thereof,

1-[4-[3-[4-[(4-fluorophenyl)phenylmethyl]-1-piperidinyl] propoxy]-3-methoxyphenyl]ethanone or a pharmaceutically acceptable salt thereof,

1-[4-[3-[4-[bis(4-methoxyphenyl)methyl]-1-piperidinyl] propoxy]-3-methoxyphenyl]ethanone or a pharmaceutically acceptable salt thereof,

1-[4-[3-[4-[bis(4-methylphenyl)methyl]-1-piperidinyl] propoxy]-3-methoxyphenyl]ethanone or a pharmaceutically acceptable salt thereof,

1-[4-[4-[4-[bis(4-fluorophenyl)methyl]-1-piperidinyl] butoxy]-3-methoxyphenyl]ethanone or a pharmaceutically acceptable salt thereof,

4-[3-[4-[bis(4-fluorophenyl)hydroxymethyl]-1-piperidinyl] propoxy]-3-methoxybenzoic acid methyl ester or a pharmaceutically acceptable salt thereof,

α,α-[bis(4-fluorophenyl)]-1-[3-[4-(methylthio)phenoxy] propyl]-4-piperidinemethanol or a pharmaceutically acceptable salt thereof,

α,α-[bis(4-fluorophenyl)]-1-[3-[4-(methylsulfonyl)phenoxy]propyl]-4-piperidinemethanol or a pharmaceutically acceptable salt thereof,

4-[3-[4-[bis(4-fluorophenyl)hydroxymethyl]-1-piperidinyl]propoxy]-3-methoxybenzeneacetic acid or a pharmaceutically acceptable salt thereof,

7-[3-[4-[bis(4-fluorophenyl)hydroxymethyl]-1-piperidinyl]propoxy]-2H-1-benzopyran-2-one or a pharmaceutically acceptable salt thereof,

2-[3-[4-[bis(4-fluorophenyl)hydroxymethyl]-1-piperidinyl]propoxy]benzoic acid ethyl ester or a pharmaceutically acceptable salt thereof,

2-[3-[4-[bis(4-fluorophenyl)methyl]-1-piperidinyl]propoxy]benzoic acid ethyl ester or a pharmaceutically acceptable salt thereof,

1-[4-[5-[4-[bis(4-fluorophenyl)methyl]-1-piperidinyl]pentoxy]-3-methoxyphenyl]ethanone or a pharmaceutically acceptable salt thereof,

4-[3-[4-[bis(4-fluorophenyl)methyl]-1-piperidinyl]propoxy]benzamide or a pharmaceutically acceptable salt thereof, or

4-[bis(4-fluorophenyl)methyl]-1-[3-[4-(methylsulfonyl)pentoxy]propyl]piperidine or a pharmaceutically acceptable salt thereof, in the preparation of an antihypertensive agent.

20. The use of 4-[3-[4-[bis(4-fluorophenyl)hydroxymethyl]-1-piperidinyl] propoxy]benzenesulfonamide or a pharmaceutically acceptable salt thereof,

7-[3-[4-[bis(4-fluorophenyl)hydroxymethyl]-1-piperidinyl] propoxy]-4-oxo-4H-1-benzopyran-2-carboxylic acid ethyl ester or a pharmaceutically acceptable salt thereof,

1-[4-[3-[4-(diphenylmethylene)-1-piperidinyl]propoxy]-3-methoxyphenyl]ethanone or a pharmaceutically acceptable salt thereof,

1-[4-[3-[4-(cyclohexylphenylmethyl)-1,2,3,6-tetrahydro-pyridin-1-yl]propoxy]-3-methoxyphenyl]ethanone or a pharmaceutically acceptable salt thereof,

1-[4-[3-[4-[bis(4-fluorophenyl)hydroxymethyl]-1-piperidinyl]propoxy]-2-methoxyphenyl]ethanone or a pharmaceutically acceptable salt thereof,

1-[3-(2,6-dichlorophenoxy)propyl]-4-[bis(4-fluorophenyl) methyl]piperidine or a pharmaceutically acceptable salt thereof,

2-[3-[4-[bis(4-fluorophenyl)methyl]-1-piperidinyl]propoxy] benzonitrile or a pharmaceutically acceptable salt thereof,

α,α-bis(4-fluorophenyl)-1-[2-(phenylthio)ethyl]-4-piperidinemethanol or a pharmaceutically acceptable salt thereof,

4-[bis(4-fluorophenyl)methylene]-1-[2-(phenylthio)ethyl] piperidine or a pharmaceutically acceptable salt thereof,

α,α-bis(4-fluorophenyl)-1-[2-[(4-chlorophenyl)sulfonyl]ethyl]-4-piperidinemethanol or a pharmaceutically acceptable salt thereof,

1-[2-[(4-chlorophenyl)sulfonyl]ethyl]-4-[bis(4-fluorophenyl)methylene]piperidine or a pharmaceutically acceptable salt thereof,

4-[bis(4-fluorophenyl)methyl]-1-[3-(phenylsulfonyl)propyl]piperidine or a pharmaceutically acceptable salt thereof,

4-[bis(4-fluorophenyl)methyl]-1-[2-[(4-chlorophenyl)sulfonyl]ethyl]piperidine or a pharmaceutically acceptable salt thereof,

4-[bis(4-fluorophenyl)methyl]-1-[2-(phenylsulfonyl)ethyl]piperidine or a pharmaceutically acceptable salt thereof,

1-(2,3-dihydro-1,4-benzodioxan-2-ylmethyl)-α,α-diphenyl-4-piperidineacetonitrile or a pharmaceutically acceptable salt thereof,

1-[3-(4-acetyl-2-methoxyphenoxy)propyl]-α,α-diphenyl-4-piperidineacetonitrile or a pharmaceutically acceptable salt thereof,

1-[3-(4-acetyl-2-methoxyphenoxy)propyl]-α,α-diphenyl-3-piperidinepropanenitrile or a pharmaceutically acceptable salt thereof, or

1-[4-(4-acetyl-2-methoxyphenoxy)butyl]-α,α-diphenyl-3-piperidinepropanenitrile or a pharmaceutically acceptable salt thereof, in the preparation of an antihypertensive agent.

21. The use of

1-[3-(4-acetyl-2-methoxyphenoxy)propyl]-α,α-diphenyl-3-pyrrolidineacetamide or a pharmaceutically acceptable salt thereof,

1-[3-(4-acetyl-2-methoxyphenoxy)propyl]-α,α-diphenyl-4-piperidineacetamide or a pharmaceutically acceptable salt thereof,

1-[4-(4-acetyl-2-methoxyphenoxy)butyl]-α,α-diphenyl-4-piperidineacetamide or a pharmaceutically acceptable salt thereof,

1-[3-(4-acetyl-2-methoxyphenoxy)propyl]-α,α-diphenyl-3-piperidinepropanamide or a pharmaceutically acceptable salt thereof,

4-[bis(4-fluorophenyl)methyl]-1-[(2,3-dihydro-1,4-benzodioxan-2-yl)methyl]piperidine or a pharmaceutically acceptable salt thereof,

1-[2-(2,6-dichlorophenoxy)ethyl]-α,α-diphenyl-3-piperidinepropanenitrile or a pharmaceutically acceptable salt thereof,

1-[5-(4-acetyl-2-methoxyphenoxy)pentyl]-α,α-diphenyl-3-piperidinepropanenitrile or a pharmaceutically acceptable salt thereof,

1-[3-(4-acetyl-2-methoxyphenoxy)propyl]-α,α-bis(4-fluorophenyl)-4-piperidineacetonitrile or a pharmaceutically acceptable salt thereof,

1-[3-(2,6-dichlorophenoxy)propyl]-α,α-bis(4-fluorophenyl)-4-acetonitrile or a pharmaceutically acceptable salt thereof,

1-[3-(2,6-dichlorophenoxy)propyl]-α,α-diphenyl-3-piperidinepropanenitrile or a pharmaceutically acceptable salt thereof,

1-[4-(4-acetyl-2-methoxyphenoxy)butyl]-α,α-bis(4-fluorophenyl)-4-piperidineacetonitrile or a pharmaceutically acceptable salt thereof,

1-[2-(2,6-dichlorophenoxy)ethyl]-α,α-diphenyl-3-piperidinepropanamide or a pharmaceutically acceptable salt thereof,

α-[1-[3-(4-acetyl-2-methoxyphenoxy)propyl]-4-piperidinyl]-α-(4-fluorophenyl)-2-pyridineacetonitrile or a pharmaceutically acceptable salt thereof,

α,α-diphenyl-1-[3-(8-quinolinyloxy)propyl]-3-piperidinepropanenitrile or a pharmaceutically acceptable salt thereof,

8-[3-[4-[bis(4-fluorophenyl)methyl]-1-piperidinyl]propoxy] quinoline or a pharmaceutically acceptable salt thereof,

2-[3-[4-[bis(4-fluorophenyl)methyl]-1-piperidinyl]propoxy] benzoic acid or a pharmaceutically acceptable salt thereof,

4-[bis(4-fluorophenyl)methyl]-N-phenyl-1-piperidinepropanamine or a pharmaceutically acceptable salt thereof,

N-[3-[4-[bis(4-fluorophenyl)methyl]-1-piperidinyl]propyl]-N-methylbenzeneamine or a pharmaceutically acceptable salt thereof,

1-[3-(4-acetyl-2-methoxyphenoxy)propyl]-α,α-bis(4-fluorophenyl)-3-piperidineacetonitrile or a pharmaceutically acceptable salt thereof.

1-[3-(4-cyanophenoxy)propyl]-α,α-bis(4-fluorophenyl)-3-piperidineacetonitrile or a pharmaceutically acceptable salt thereof.

4-[3-[4-[bis(4-fluorophenyl)methyl]-1-piperidinyl]propoxy]-3-methoxybenzonitrile or a pharmaceutically acceptable salt thereof.

4-[bis(4-fluorophenyl)methyl]-1-[3-(1-naphthalenyloxy)propyl]piperidine or a pharmaceutically acceptable salt thereof.

2-[3-[4-[bis(4-fluorophenyl)methyl]-1-piperidinyl]propoxy]quinoline or a pharmaceutically acceptable salt thereof.

4-[bis(4-fluorophenyl)methyl]-1-[3-(2-naphthalenyloxy)propyl]piperidine or a pharmaceutically acceptable salt thereof. or

3-[3-[4-[bis(4-fluorophenyl)methyl]-1-piperidinyl]propoxy]benzonitrile or a pharmaceutically acceptable salt thereof. in the preparation of an antihypertensive agent.

22. The use of a compound selected from the group having the formula:

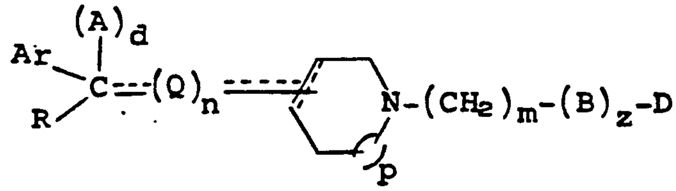

wherein;

p is zero, one or two;

A is hydrogen, $-O-R^1$, $-C{\equiv}N$, $-\underset{\overset{\|}{O}}{C}NR^1R^2$, $-\overset{O}{\overset{\|}{C}}-R^1$, $-\overset{O}{\overset{\|}{C}}-O-R^1$, $-O-\overset{O}{\overset{\|}{C}}-R^1$, $-CH_2OR^1$, $-CH_2NR^1R^2$;

m is zero to six inclusive;

Q is $-CH-$, $-CH_2-$ or $-\overset{OH}{\underset{H}{C}}-$;

d and n are selected from zero or one and the dotted lines represent double bonds which may form consistent with the valence of carbon;

Ar, D and R are selected independently from:

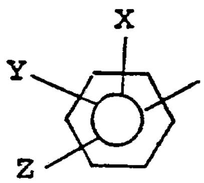,
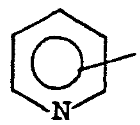,
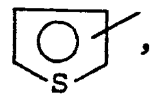,
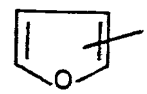 or
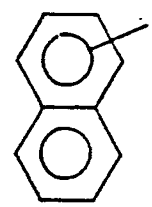

and in addition, R may have the values:

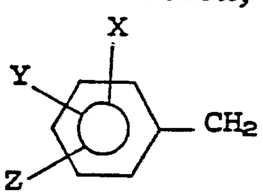 cycloalkyl or loweralkyl and

D may have additionally the values:

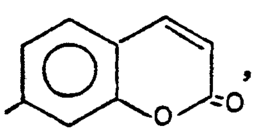,
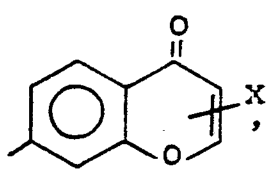,
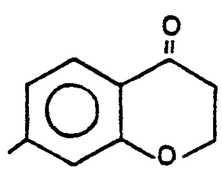,
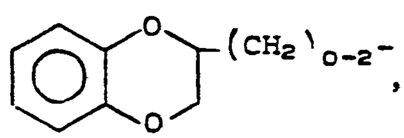,

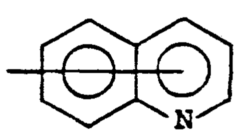,

or $Ar(CH_2)_{1-4}-$; X, Y and Z are selected from the group consisting of hydrogen, loweralkyl, halogen, $-NO_2$, $-O-R^1$, $-\underset{\overset{\|}{O}}{C}-R^1$, $-CF_3$, $-C{\equiv}N$, $-\overset{O}{\overset{\|}{C}}-N(R^1)_2$, $-N(R^1)_2$, $-C(O)OR^1$, $-SO_2R^2$, $-SR^2$, $-S(O)R^2$, $-\underset{R^1}{N}-\underset{O}{C}-R^1$, $-CH_2COOM$, $-SO_2N(R^1)(R^2)$, $-\underset{R^1}{N}SO_2CH_3$, $-\underset{R^1}{N}\overset{O}{\overset{\|}{C}}-N(R^1)(R^2)$, or $-\underset{R^1}{N}\overset{O}{\overset{\|}{C}}-OR^2$;

B is selected from O, S, $-\overset{O}{\overset{\|}{S}}-$, $-\overset{O}{\overset{\|}{\underset{O}{\underset{\|}{S}}}}-$, $-\underset{R^1}{\underset{|}{N}}-$, and $-\overset{|}{N}-\overset{O}{\overset{\|}{C}}-O-R^1$;

z is one or zero with the proviso that z cannot be zero at the same time n is zero when one of the following occurs at the same time that D is phenyl or substituted phenyl: $(A)_d$ is hydrogen, $(A)_d$ is cyano, $(A)_d$ is aminocarbonyl or a double bond forms between the $\alpha$-carbon and a carbon of the central heterocyclic amine ring;

$R^1$ is selected from hydrogen, loweralkyl, phenyl and phenylloweralkyl;

$R^2$ is selected from loweralkyl, phenyl and phenylloweralkyl;

M is a pharmaceutically acceptable metal ion, and the pharmaceutically acceptable salts thereof, (including acid addition salts, quaternary salts, and hydrates and alcoholates thereof) in the preparation of an agent for use in the treatment of angina.

23. The use as claimed in claim 22, wherein the compound is also defined by any one of claims 2 to 8.

24. The use of 1-[4-[3-[4-[bis(4-fluorophenyl)methyl]-1-piperidinyl]propoxy]-3-methoxyphenyl]ethanone or a pharmaceutically acceptable salt thereof,

1-[4-[3-[4-[bis(4-fluorophenyl)methylene]-1-piperidinyl]propoxy]-3-methoxyphenyl]ethanone or a pharmaceutically acceptable salt thereof.

1-[3-methoxy-4-[3-[4-[phenyl[3-(trifluoromethyl)phenyl]methylene]-1-piperidinyl]propoxy]phenyl]ethanone or a pharmaceutically acceptable salt thereof.

1-[4-[3-[4-(cyclohexylphenylmethylene)-1-piperidinyl]propoxy]-3-methoxyphenyl]ethanone or a pharmaceutically acceptable salt thereof.

1-[4-[3-[4-(cyclohexylphenylmethyl)-1-piperidinyl]propoxy]-3-methoxyphenyl]ethanone or a pharmaceutically acceptable salt thereof.

1-[4-[3-[4-[hydroxyphenyl[-3-(trifluoromethyl)phenyl] methyl]-1-piperidinyl]propoxy]-3-methoxyphenyl]ethanone or a pharmaceutically acceptable salt thereof,

1-[4-[3-[4-(cyclohexylhydroxyphenylmethyl)-1-piperidinyl] propoxy]-3-methoxyphenyl]ethanone or a pharmaceutically acceptable salt thereof,

1-[4-[2-[4-[bis(4-fluorophenyl)methyl]-1-piperidinyl] ethoxy]-3-methoxyphenyl]ethanone or a pharmaceutically acceptable salt thereof,

1-[4-[3-[4-[(4-fluorophenyl)phenylmethyl]-1-piperidinyl] propoxy]-3-methoxyphenyl]ethanone or a pharmaceutically acceptable salt thereof,

1-[4-[5-[4-[bis(4-fluorophenyl)methyl]-1-piperidinyl] pentoxy]-3-methoxyphenyl]ethanone or a pharmaceutically acceptable salt thereof,

1-[4-[3-[4-(diphenylmethylene)-1-piperidinyl]propoxy]-3-methoxyphenyl]ethanone or a pharmaceutically acceptable salt thereof,

1-[4-[3-[4-(cyclohexylphenylmethyl)-1,2,3,6-tetrahydro-pyridin-1-yl]propoxy]-3-methoxyphenyl]ethanone or a pharmaceutically acceptable salt thereof,

1-(2,3-dihydro-1,4-benzodioxan-2-ylmethyl)-$\alpha$,$\alpha$-diphenyl-4-piperidineacetonitrile or a pharmaceutically acceptable salt thereof,

1-[3-(4-acetyl-2-methoxyphenoxy)propyl]-α,α-diphenyl-4-piperidineacetonitrile or a pharmaceutically acceptable salt thereof.

1-[3-(4-acetyl-2-methoxyphenoxy)propyl]-α,α-diphenyl-3-piperidinepropanenitrile or a pharmaceutically acceptable salt thereof.

1-[4-(4-acetyl-2-methoxyphenoxy)butyl]-α,α-diphenyl-3-piperidinepropanenitrile or a pharmaceutically acceptable salt thereof.

1-[2-(2,6-dichlorophenoxy)ethyl]-α,α-diphenyl-3-piperidinepropanenitrile or a pharmaceutically acceptable salt thereof. or

1-[5-(4-acetyl-2-methoxyphenoxy)pentyl]-α,α-diphenyl-3-piperidinepropanenitrile or a pharmaceutically acceptable salt thereof. in the preparation of an agent for use in the treatment of angina.

25. The use of a compound selected from the group having the formula:

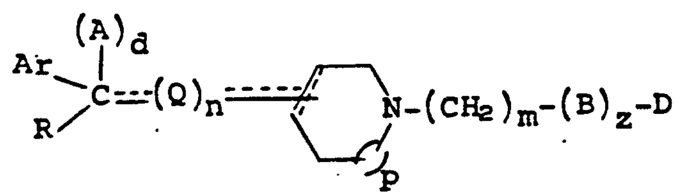

wherein;

p is zero, one or two;

A is hydrogen, $-O-R^1$, $-C{\equiv}N$, $-\underset{\|}{\overset{}{C}}NR^1R^2$ (O), $-\overset{O}{\overset{\|}{C}}-R^1$, $-\overset{O}{\overset{\|}{C}}-O-R^1$, $-O-\overset{O}{\overset{\|}{C}}-R^1$, $-CH_2-OR^1$, $-CH_2NR^1R^2$;

m is zero to six inclusive;

Q is $-CH-$, $-CH_2-$ or $-\overset{OH}{\underset{H}{C}}-$;

d and n are selected from zero or one and the dotted lines represent double bonds which may form consistent with the valence of carbon;

Ar, D and R are selected independently from:

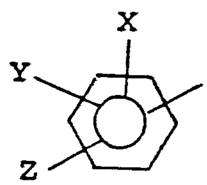 , 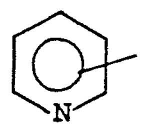 , 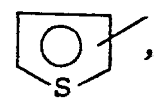 , 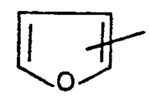 or 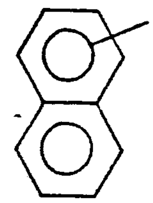 .

and in addition, R may have the values:

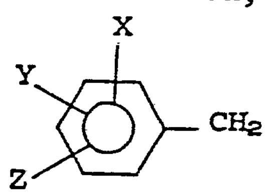 , cycloalkyl or loweralkyl and

D may have additionally the values:

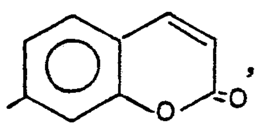 , 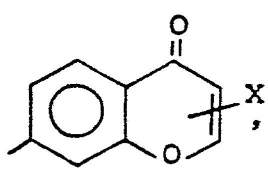 , 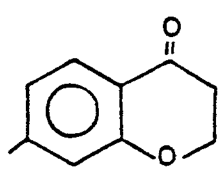 , 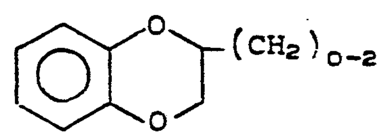 ,

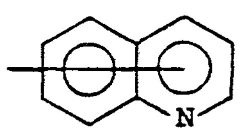 ,

or $Ar(CH_2)_{1-4}$-; X, Y and Z are selected from the group consisting of hydrogen, loweralkyl, halogen, $-NO_2$, $-O-R^1$, $-C(=O)-R^1$, $-CF_3$, $-C\equiv N$, $-C(=O)-N(R^1)_2$, $-N(R^1)_2$, $-C(O)OR^1$, $-SO_2R^2$, $-SR^2$, $-S(O)R^2$, $-N(R^1)-C(=O)-R^1$, $-CH_2COOM$, $-SO_2N(R^1)(R^2)$, $-N(R^1)SO_2CH_3$, $-N(R^1)C(=O)-N(R^1)(R^2)$, or $-N(R^1)C(=O)-OR^2$;

B is selected from O, S, $-S(=O)-$, $-S(=O)_2-$, $-N(R^1)-$, and $-N-C(=O)-O-R^1$;

z is one or zero with the proviso that z cannot be zero at the same time n is zero when one of the following occurs at the same time that D is phenyl or substituted

phenyl: $(A)_d$ is hydrogen, $(A)_d$ is cyano, $(A)_d$ is aminocarbonyl, or a double bond forms between the α-carbon and a carbon of the central heterocyclic amine ring;

$R^1$ is selected from hydrogen, loweralkyl, phenyl, and phenylloweralkyl;

$R^2$ is selected from loweralkyl, phenyl, and phenylloweralkyl;

M is a pharmaceutically acceptable metal ion, and the pharmaceutically acceptable salts thereof, (including acid addition salts, quaternary salts, and hydrates and alcoholates thereof) in the preparation of an antihistamine agent.

26. The use as claimed in claim 25, wherein the compound is also defined by any one of claims 2 to 8.

27. The use of 1-[4-[3-[4-[bis(4-fluorophenyl)methylene]-1-piperidinyl]propoxy]-3-methoxyphenyl]ethanone or a pharmaceutically acceptable salt thereof, or
1-[4-[3-[4-(diphenylmethylene)-1-piperidinyl]propoxy]-3-methoxyphenyl]-ethanone or a pharmaceutically acceptable salt thereof in the preparation of an antihistamine agent.

28. The use of a compound selected from the group having the formula :

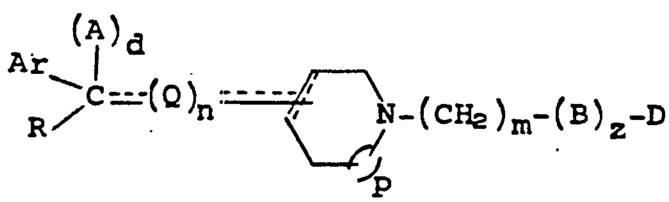

wherein;

p is zero, one or two;

A is hydrogen, $-O-R^1$, $-C{\equiv}N$, $-\underset{\underset{O}{\|}}{C}NR^1R^2$, $-\overset{\overset{O}{\|}}{C}-R^1$, $-\overset{\overset{O}{\|}}{C}-O-R^1$, $-O-\overset{\overset{O}{\|}}{C}-R^1$, $-CH_2OR^1$, $-CH_2NR^1R^2$;

m is zero to six inclusive;

Q is $-CH-$, $-CH_2-$ or $-\overset{\overset{OH}{|}}{\underset{\underset{H}{|}}{C}}-$;

d and n are selected from zero or one and the dotted lines represent double bonds which may form consistent with the valence of carbon;

Ar, D and R are selected independently from:

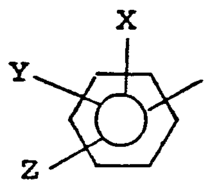 , 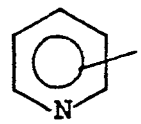 , 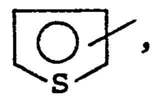 , 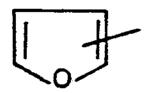 or 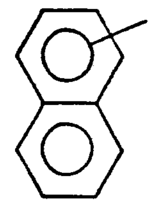

and in addition, R may have the values:

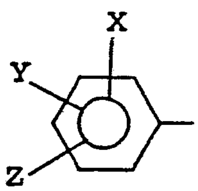 cycloalkyl or loweralkyl and

D may have additionally the values:

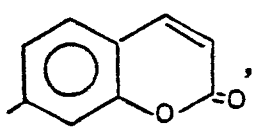 , 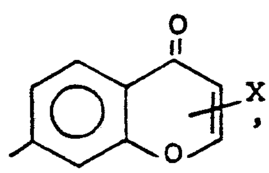 , 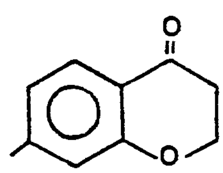 , 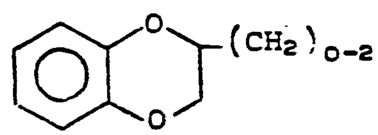 ,

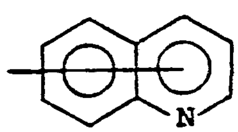 ,

or $Ar(CH_2)_{1-4}-$; X, Y and Z are selected from the group consisting of hydrogen, loweralkyl, halogen, $-NO_2$, $-O-R^1$, $-\underset{O}{\overset{\|}{C}}-R^1$, $-CF_3$, $-C{\equiv}N$, $-\overset{O}{\overset{\|}{C}}-N(R^1)_2$, $-N(R^1)_2$, $-C(O)OR^1$, $-SO_2R^2$, $-SR^2$, $-S(O)R^2$, $-\underset{R^1}{N}-\underset{O}{\overset{\|}{C}}-R^1$, $-CH_2COOM$, $-SO_2N\begin{smallmatrix}R^1\\R^2\end{smallmatrix}$, $-\underset{R^1}{N}SO_2CH_3$, $-\underset{R^1}{N}\overset{O}{\overset{\|}{C}}-N\begin{smallmatrix}R^1\\R^2\end{smallmatrix}$, or $-\underset{R^1}{N}\overset{O}{\overset{\|}{C}}-OR^2$;

B is selected from O, S, $-\overset{O}{\overset{\|}{S}}-$, $-\underset{O}{\overset{O}{S}}-$, $-\underset{R^1}{N}-$, and $-\overset{|}{N}-\overset{O}{\overset{\|}{C}}-O-R^1$;

z is one or zero with the proviso that z cannot be zero at the same time n is zero when one of the following occurs at the same time that D is phenyl or substituted

phenyl: $(A)_d$ is hydrogen, $(A)_d$ is cyano, $(A)_d$ is aminocarbonyl, or a double bond forms between the α-carbon and a carbon of the central heterocyclic amine ring;

$R^1$ is selected from hydrogen, loweralkyl, phenyl, and phenylloweralkyl;

$R^2$ is selected from loweralkyl, phenyl, and phenylloweralkyl;

M is a pharmaceutically acceptable metal ion, and the pharmaceutically acceptable salts thereof, (including acid addition salts, quaternary salts, and hydrates and alcoholates thereof) in the preparation of an agent for decreasing gastric secretion and acid release.

29. The use as claimed in claim 28, wherein the compound is also defined by any one of claims 2 to 8.

30. The use of 1-[4-[3-[4-[bis(4-fluorophenyl)methylene]-1-piperidinyl]propoxy]-3-methoxyphenyl]ethanone or a pharmaceutically acceptable salt thereof, or 1-[4-[3-[4-(diphenylmethylene)-1-piperidinyl]propoxy]-3-methoxyphenyl]ethanone or a pharmaceutically acceptable salt thereof, in the preparation of an agent for decreasing gastric secretion and acid release.